(19) Europäisches Patentamt
European Patent Office
Office européen des brevets

(11) EP 4 371 977 A1

(12) EUROPEAN PATENT APPLICATION
published in accordance with Art. 153(4) EPC

(43) Date of publication:
**22.05.2024 Bulletin 2024/21**

(21) Application number: **22841470.2**

(22) Date of filing: **14.07.2022**

(51) International Patent Classification (IPC):
***C07D 217/14*** (2006.01) ***C07D 401/12*** (2006.01)
***C07D 401/14*** (2006.01) ***C07D 471/04*** (2006.01)
***A61K 31/435*** (2006.01) ***A61K 31/397*** (2006.01)
***A61K 31/437*** (2006.01) ***A61K 31/4545*** (2006.01)
***A61P 35/00*** (2006.01)

(52) Cooperative Patent Classification (CPC):
**A61K 31/397; A61K 31/435; A61K 31/437; A61K 31/4545; A61P 35/00; C07D 217/14; C07D 401/12; C07D 401/14; C07D 471/04**

(86) International application number:
**PCT/CN2022/105788**

(87) International publication number:
**WO 2023/284837 (19.01.2023 Gazette 2023/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority:
**15.07.2021 CN 202110801689**
**10.11.2021 CN 202111324586**
**30.12.2021 CN 202111647530**
**21.02.2022 CN 202210155354**
**17.03.2022 CN 202210267058**
**25.05.2022 CN 202210579865**

(71) Applicant: **Xizang Haisco Pharmaceutical Co., Ltd.**
**Lhoka, Tibet 856099 (CN)**

(72) Inventors:
- **LI, Yao**
  **Chengdu City, Sichuan 611130 (CN)**
- **WANG, Wenjing**
  **Chengdu City, Sichuan 611130 (CN)**
- **SHI, Zongjun**
  **Chengdu City, Sichuan 611130 (CN)**
- **SONG, Changwei**
  **Chengdu City, Sichuan 611130 (CN)**
- **REN, Lei**
  **Chengdu City, Sichuan 611130 (CN)**
- **WANG, Jie**
  **Chengdu City, Sichuan 611130 (CN)**
- **TANG, Pingming**
  **Chengdu City, Sichuan 611130 (CN)**
- **YU, Yan**
  **Chengdu City, Sichuan 611130 (CN)**
- **ZHANG, Chen**
  **Chengdu City, Sichuan 611130 (CN)**
- **YAN, Pangke**
  **Chengdu City, Sichuan 611130 (CN)**

(74) Representative: **Grünecker Patent- und Rechtsanwälte**
**PartG mbB**
**Leopoldstraße 4**
**80802 München (DE)**

# (54) ARYLAMINO DERIVATIVE ESTROGEN RECEPTOR MODULATOR AND USE THEREOF

(57) The present invention relates to a compound as represented by formula (I) and a stereoisomer, solvate, deuterated compound or pharmaceutically acceptable salt or pharmaceutical composition thereof, and the use thereof in the preparation of a drug for treating/preventing ER-mediated diseases, wherein each group in formula (I) is as defined in the description.

EP 4 371 977 A1

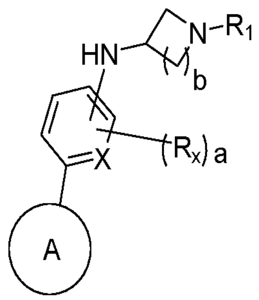

(I)

## Description

### Technical Field

The present invention relates to an arylamino derivative estrogen receptor modulator and the use thereof in the preparation of a drug for treating/preventing an ER-related disease.

### Background Art

Estrogens include estrone, estradiol, etc., which are mainly secreted by the ovaries, with a small amount secreted by the liver, adrenal cortex, and breasts. As the main sex hormone in female animals, estrogen promotes the maturation of female accessory sexual organs and the emergence of secondary sexual characteristics, and maintains normal sexual desire and reproductive functions. Its main function is to diffuse into the nucleus and specifically bind to the estrogen receptor (ER) to form a complex. The activated estrogen receptor quickly forms homo- or heterodimers and binds to DNA enhancers, including estrogen response elements (EREs), and recruits other transcription factors to form transcription initiation complexes to induce transcription. In addition, the activated estrogen receptor can also recruit transcriptional co-factors and bind to activating protein 1 (AP-1) located in the promoter region of target genes, thereby regulating gene transcription activity.

The estrogen receptor (ER) is a transcriptional regulatory protein that mediates ligand activation induced by a variety of biological effects through its interaction with endogenous estrogens. ER contains two subtypes: $ER_\alpha$ and $ER_\beta$ encoded by different genes, respectively. $ER_\alpha$ and $ER_\beta$ show a high degree of similarity at the amino acid level, with a similarity of 97% in the DNA-binding domain and a similarity of 56% in the ligand-binding domain. However, there is only a low homology of 24% at the N-terminus. ER contains 6 domains (A-F), constituting 4 main functional regions. The functional region composed of the N-terminal A/B domains has a ligand-independent transcriptional activation functional region AF-1. AF-1 has constitutive activating activity and activates the transcription of target genes through interaction with basal transcription factors, resurrection factors and other transcription factors. There are multiple phosphorylation sites in this region. There are reports in the literature that the effect of AF-1 depends on protein phosphorylation. The DNA-binding domain (DBD) composed of the C domain is highly conserved and contains two zinc finger domains, which can specifically bind to target DNA. At the same time, the DBD plays an important role in the dimerization of the receptor. The D domain constitutes the hinge region, which links the DBD and the ligand domain (LBD), and is less conserved (the homology between the two subtypes is only 30%). The C-terminal E domain constitutes the ligand binding domain (LBD), which determines the specific binding of ER to ligands such as estrogen, SERM (selective estrogen receptor modulator), and SERD (selective estrogen receptor degrader). LBD possesses ligand-dependent transcriptional activation functional region AF-2, which works synergistically with AF-1 to exert the function of ER receptors to activate target gene transcription. Moreover, LBD has a strong dimerization interface and can still function in the absence of ligands. Therefore, LBD is a key site for receptor dimerization.

$ER_\alpha$ is mainly distributed in the uterus, ovaries, testicles, pituitary gland, kidneys, epididymis and adrenal glands, while $ER_\beta$ is mainly distributed in the prostate, ovaries, lungs, bladder, brain and blood vessels. Since full agonists or full antagonists have serious side effects, researches on selective estrogen receptor modulators (SERMs) emerge as the times require. The "selective" means that SERM acts as an agonist in ERβ-rich areas in some tissues such as bone, liver, and cardiovascular system, and acts as an antagonist in other tissues such as breast. In the uterus (an area where $ER_\alpha$ is dominant), SERM can be an agonist or an antagonist. SERMs currently on the market include Tamoxifen, Raloxifene, Bazedoxifene, Toremifene, etc. However, studies have found that SERMs currently on the market still have serious side effects, for example, long-term use of Tamoxifen and Toremifene can cause endometrial hyperplasia, polyps and endometrial cancer, and common side effects of Raloxifene include hot flashes, leg pain, breast tenderness, vein embolism, etc. Therefore, research and development of new compounds is still an urgent problem that needs to be solved.

Selective estrogen receptor degraders (SERDs) recruit E3 ubiquitin ligases by binding to the estrogen receptor and inducing its conformational changes, resulting in the exposure of hydrophobic residues to the molecular surface. Ubiquitin ligases modify the estrogen receptor by ubiquitination and ultimately direct it to the proteasome for degradation. By degrading the estrogen receptor, we can more completely block the estrogen signaling pathway and prevent it from recruiting transcriptional activators to become "agonists". In addition, SERDs can also degrade mutated estrogen receptors, thereby avoiding the emergence of drug resistance.

Fulvestrant is the only SERD drug currently approved for clinical use for the treatment of ER+ breast cancer, but it has poor druggability properties, is rapidly metabolized and must be administered via monthly intramuscular injections. Compared with complete ER degradation observed in *in vitro* studies, it limits the effective degradation of ER (approximately 50% ER degradation in clinical samples).

Selective estrogen receptor degraders have shown certain therapeutic advantages, but more orally available SERDs still need to be developed so that candidate drugs have better properties, such as better efficacy, lower side

effects, better pharmacokinetic properties, and longer dosing intervals, so as to prevent or treat estrogen receptor-related diseases in a better way. However, there has been no effective oral ER degraders on the market, so oral ER degraders with high inhibitory activity and low toxicity still represent an unmet clinical need.

## Summary of the Invention

The objective of the present invention is to provide a selective estrogen receptor degrader compound, or a stereoisomer, solvate, deuterated compound or pharmaceutically acceptable salt thereof, and the medical application thereof. The compound has the advantages of good efficacy, low toxic and side effects, high safety, high selectivity, good pharmacokinetics, high bioavailability, and no inhibition to CYP enzymes.

Provided is a compound as represented by formula (I), (II), (III), (I'), (II'), (III'), (II'-1), (III'-1), (II'-a), (II'-a-1), (II'-1a) or (II'-1a-1), or a stereoisomer, solvate, deuterated compound, or pharmaceutically acceptable salt thereof,

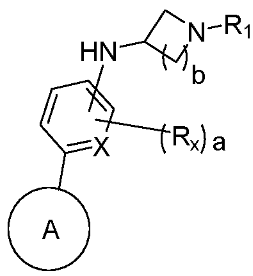
(I)

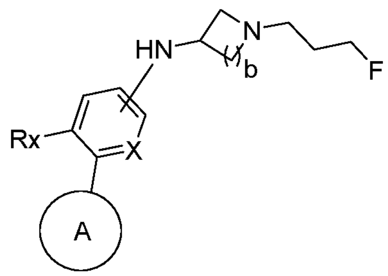
(II)

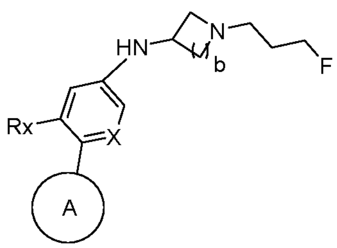
(III)

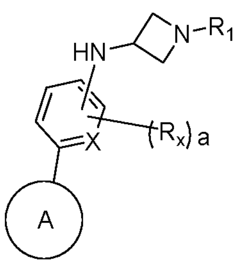
(I')

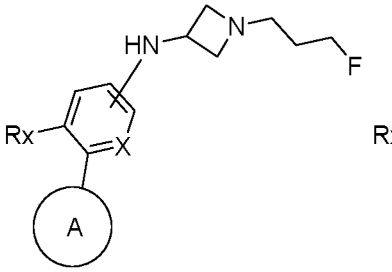
(II')

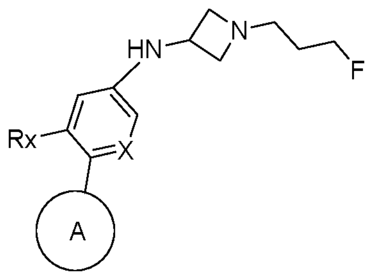
(III')

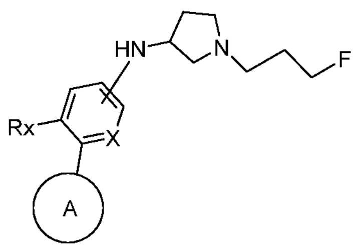
(II'-1)

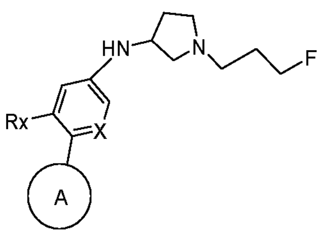
(III'-1)

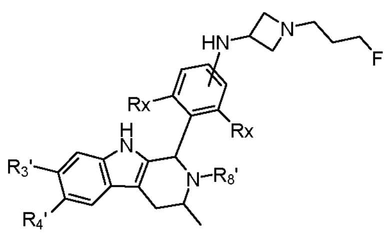
(II'-a)

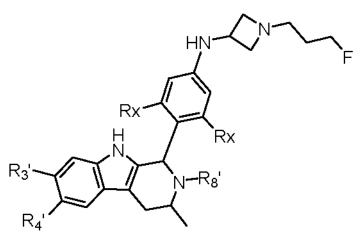
(II'-a-1)

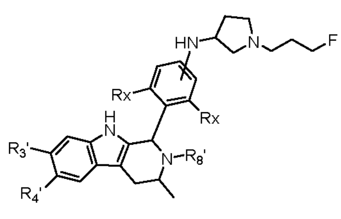
(II'-1a)

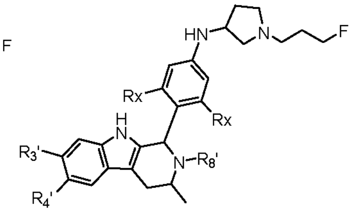
(II'-1a-1)

wherein, ring A is selected from I-a or I-b:

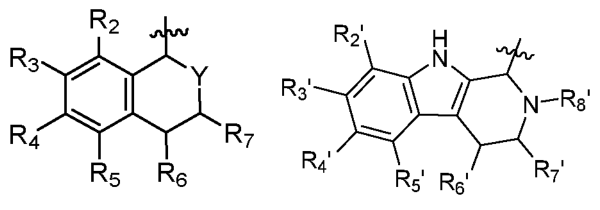

I-a , I-b .

In some specific embodiments, ring A is selected from II-a or II-b:

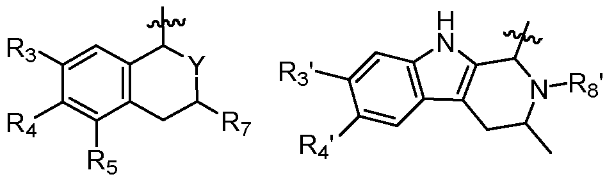

II-a , II-b

X is N or $CR_x$. In some specific embodiments, X is N, CH or CF;

a is 0, 1, 2 or 3. In some specific embodiments, a is 0, 1 or 2. In some specific embodiments, a is 1 or 2;

b is 1 or 2. In some specific embodiments, a is 1. In some specific embodiments, a is 2;

Y is $NR_8$ or $CHR_8$;

$R_1$ is $C_{1-6}$alkyl, $C_{2-6}$alkenyl or $C_{2-6}$alkynyl, the $R_1$ is optionally substituted with 1-3 groups selected from halogen, OH, $NH_2$, $-OC_{1-4}$alkyl, $-NHC_{1-4}$alkyl, $C_{3-6}$cycloalkyl, and 4- to 7-membered heterocycloalkyl containing 1-3 heteroatoms selected from N, S or O, and the cycloalkyl and heterocycloalkyl are optionally further substituted with 1-3 groups selected from halogen, OH, $C_{1-4}$alkyl and $-OC_{1-4}$alkyl. In some specific embodiments, $R_1$ is $C_{1-4}$alkyl, the $R_1$ is optionally substituted with 1-3 groups selected from halogen, OH, $NH_2$, $-OC_{1-4}$alkyl, $-NHC_{1-4}$alkyl, $C_{3-6}$cycloalkyl, and 4- to 7-membered heterocycloalkyl containing 1-3 heteroatoms selected from N, S, or O, wherein, the cycloalkyl and heterocycloalkyl are optionally further substituted with 1-3 groups selected from halogen, OH, $C_{1-4}$alkyl and $-OC_{1-4}$alkyl. In some specific embodiments, $R_1$ is methyl, ethyl, propyl, isopropyl, butyl, isobutyl, or tert-butyl, the $R_1$ is optionally substituted with 1-3 groups selected from F, Cl, Br, I, OH, $NH_2$, $-OC_{1-4}$alkyl, methylamino, dimethylamino, cyclopropyl, cyclobutyl, cyclopentyl, cyclohexyl, tetrahydrofuryl, piperidyl and piperazinyl;

each $R_x$ is independently H, $NH_2$, OH, halogen, $C_{1-4}$alkyl, halo $C_{1-4}$alkyl, $-N(C_{1-4}alkyl)_2$, $-NHC_{1-4}$alkyl or $-OC_{1-4}$alkyl. In some specific embodiments, each $R_x$ is independently H or halogen. In some specific embodiments, each $R_x$ is independently H, F, Cl, Br or I;

$R_2$, $R_5$, $R_6$, $R_2$', $R_5$' and $R_6$' are independently H, CN, OH, $NH_2$, SH or halogen. In some specific embodiments, $R_2$, $R_5$, $R_6$, $R_2$', $R_5$' and $R_6$' are independently H, CN, OH, $NH_2$, SH, F, Cl, Br or I;

$R_3$ and $R_4$ are independently H, halogen, $SF_5$, CN, $-NR_aSO_2R_b$, $-SO_2NHR_a$, - $P(=O)(R_b)_2$, or $-NR_aP(=O)(R_b)_2$;

$R_a$ is H or $R_b$;

$R_b$ is $C_{1-4}$alkyl, $C_{3-6}$cycloalkyl, phenyl, 5- to 6-membered heteroaryl containing 1-3 heteroatoms selected from N, S, or O, or 4- to 7-membered heterocycloalkyl containing 1-3 heteroatoms selected from N, S, or O, and the alkyl, cycloalkyl, phenyl, heteroaryl and heterocycloalkyl are optionally substituted with 1-3 groups selected from halogen, CN, $NH_2$, OH, $C_{1-4}$alkyl, $C_{3-6}$cycloalkyl, 5- to 6-membered heteroaryl containing 1-3 heteroatoms selected from N, S, or O, and 4- to 7-membered heterocycloalkyl containing 1-3 heteroatoms selected from N, S, or O. In some specific embodiments, $R_b$ is methyl, ethyl, propyl, isopropyl, butyl, isobutyl, tert-butyl, cyclopropyl, cyclobutyl, cyclopentyl, cyclohexyl, phenyl, pyrazolyl, furyl, imidazolyl, thiazolyl, isothiazolyl, oxazolyl, isoxazolyl, pyrimidyl, pyridyl, tetrahydrofuryl, piperidyl or piperazinyl, wherein the $R_b$ is optionally substituted with 1-3 groups selected from F, Cl, Br, I, CN, $NH_2$, OH, methyl, ethyl, propyl, isopropyl, butyl, cyclopropyl, cyclobutyl, cyclopentyl, pyrazolyl, furyl, imidazolyl, thiazolyl, isothiazolyl, oxazolyl, isoxazolyl, pyrimidyl, pyridyl, tetrahydrofuryl, piperidyl or piperazinyl;

$R_3$' and $R_4$' are independently H, $-NR_cR_c$', $OR_d$, $C_{2-6}$alkenyl, $C_{2-6}$alkynyl, halogen, $SF_5$, CN, $C_{1-4}$alkyl, $C_{3-6}$cycloalkyl, phenyl, 5- to 6-membered heteroaryl containing 1-3 heteroatoms selected from N, S, or O, or 4- to 7-membered heterocycloalkyl containing 1-3 heteroatoms selected from N, S, or O, the alkyl, alkenyl, alkynyl, cycloalkyl, phenyl, heteroaryl and heterocycloalkyl are optionally substituted with 1-3 groups selected from halogen, CN, $NH_2$, OH, $C_{1-4}$alkyl, $C_{3-6}$cycloalkyl, 5- to 6-membered heteroaryl containing 1-3 heteroatoms selected from N, S, or O, and 4-

to 7-membered heterocycloalkyl containing 1-3 heteroatoms selected from N, S, or O. In some specific embodiments, $R_3$' and $R_4$' are independently H, $-NR_cR_c$', $OR_d$, $C_{2-6}$alkenyl, $C_{2-6}$alkynyl, halogen, $SF_5$, CN, $C_{1-4}$alkyl, $C_{3-6}$cycloalkyl, 5-membered heteroaryl containing 1-3 heteroatoms selected from N, S, or O, or 5- to 6-membered heterocycloalkyl containing 1-3 heteroatoms selected from N, S, or O, wherein the alkyl, alkenyl, alkynyl, cycloalkyl, heteroaryl and heterocycloalkyl are optionally substituted with 1-3 groups selected from halogen, CN, $NH_2$, OH, $C_{1-4}$alkyl, $C_{3-6}$cycloalkyl, 5- to 6-membered heteroaryl containing 1-3 heteroatoms selected from N, S, or O, and 5- to 6-membered heterocycloalkyl containing 1-3 heteroatoms selected from N, S, or O. In some specific embodiments, $R_3$' and $R_4$' are independently H, $-NHR_c$', $OR_d$, ethenyl, propenyl, allyl, ethynyl, propynyl, F, Cl, Br, I, $SF_5$, CN, $NH_2$, OH, methyl, ethyl, propyl, isopropyl, cyclopropyl, cyclobutyl, pyrazolyl, furyl, imidazolyl, thiazolyl, isothiazolyl, oxazolyl, isoxazolyl, pyrimidyl, pyridyl, tetrahydrofuryl, piperidyl or piperazinyl, wherein, as the bond valence permits, the $R_3$' and $R_4$' are optionally substituted with 1-3 groups selected from F, Cl, Br, I, CN, $NH_2$, OH, methyl, ethyl, propyl, cyclopropyl, cyclobutyl, pyrazolyl, furyl, imidazolyl, thiazolyl, isothiazolyl, oxazolyl, isoxazolyl, pyrimidyl, pyridyl, tetrahydrofuryl, piperidyl or piperazinyl;

optionally, one $R_3$' and one $R_4$' together with the atoms to which they are attached form $C_{3-8}$cycloalkyl, or 4- to 12-membered heterocycloalkyl containing 1-3 heteroatoms selected from N, S, or O, the cycloalkyl or heterocycloalkyl is optionally substituted with 1-3 groups selected from halogen, OH, $NH_2$, $C_{1-4}$alkyl and halo $C_{1-4}$alkyl. In some specific embodiments, one $R_3$' and one $R_4$' together with the atoms to which they are attached form $C_{4-6}$cycloalkyl, wherein the cycloalkyl is optionally substituted with 1-3 groups selected from halogen, OH, and $C_{1-4}$alkyl. In some specific embodiments, one $R_3$' and one $R_4$' together with the atoms to which they are attached form cyclobutyl, cyclopentyl, or cyclohexyl, wherein the cyclobutyl, cyclopentyl, or cyclohexyl is optionally substituted with 1-3 groups selected from F, Cl, Br, I, OH, methyl, ethyl, and propyl.

In some specific embodiments, when b is selected from 2, $R_3$' and $R_4$' are not both selected from H;

$R_c$ is H, $C_{1-4}$alkyl or $C_{3-6}$cycloalkyl; In some specific embodiments, $R_c$ is H, methyl, ethyl, propyl, isopropyl, cyclopropyl, cyclobutyl, cyclopentyl, or cyclohexyl;

$R_c$' is $C_{2-6}$alkyl, $C_{2-6}$alkenyl, $C_{2-6}$alkynyl, $-COR_e$, -OH, $-OC_{1-4}$alkyl or $-SO_2$Re. In some specific embodiments, $R_c$' is $C_{2-6}$alkyl, $C_{2-6}$alkenyl, $C_{2-6}$alkynyl, -OH or - $OC_{1-4}$alkyl. In some specific embodiments, $R_c$' is ethyl, propyl, isopropyl, butyl, isobutyl, tert-butyl, ethenyl, propenyl, allyl, ethynyl, propynyl, $-COR_e$, -OH, methoxy, ethoxy, propoxy, isopropyloxy or $-SO_2$Re;

Re is $C_{2-6}$alkyl, $C_{2-6}$alkenyl, $C_{2-6}$alkynyl, $C_{4-6}$cycloalkyl, $-C_{1-4}$alkyl$C_{3-6}$cycloalkyl, 5- to 6-membered heteroaryl containing 1-3 heteroatoms selected from N, S, or O, or 4- to 7-membered heterocycloalkyl containing 1-3 heteroatoms selected from N, S, or O, and the alkyl, alkenyl, alkynyl, cycloalkyl, heteroaryl and heterocycloalkyl are optionally substituted with 1-3 groups selected from halogen, OH, CN, $NH_2$, $C_{1-4}$alkyl and halo $C_{1-4}$alkyl. In some specific embodiments, Re is ethyl, propyl, isopropyl, butyl, isobutyl, tert-butyl, ethenyl, propenyl, allyl, ethynyl, propynyl, cyclobutyl, cyclopentyl, cyclohexyl, pyrazolyl, furyl, imidazolyl, thiazolyl, isothiazolyl, oxazolyl, isoxazolyl, pyrimidyl, pyridyl, tetrahydrofuryl, piperidyl or piperazinyl;

$R_d$ is $C_{1-4}$alkyl, $C_{1-4}$alkyl, halo $C_{1-4}$alkyl, deuterated $C_{1-4}$alkyl, or halo $C_{1-4}$alkyl substituted with $C_{3-6}$cycloalkyl, 5- to 6-membered heteroaryl containing 1-3 heteroatoms selected from N, S, or O, or 4- to 7-membered heterocycloalkyl containing 1-3 heteroatoms selected from N, S, or O, wherein the cycloalkyl, heteroaryl and heterocycloalkyl are optionally substituted with 1-3 groups selected from halogen, OH, CN, $NH_2$, $C_{1-4}$alkyl and halo $C_{1-4}$alkyl; furthermore, $R_d$ is $C_{1-4}$alkyl substituted with $C_{3-6}$cycloalkyl, 5- to 6-membered heteroaryl containing 1-3 heteroatoms selected from N, S, or O, or 4- to 7-membered heterocycloalkyl containing 1-3 heteroatoms selected from N, S, or O, wherein the cycloalkyl, heteroaryl and heterocycloalkyl are optionally substituted with 1-3 groups selected from halogen, OH, CN, $NH_2$, $C_{1-4}$alkyl and halo $C_{1-4}$alkyl. In some specific embodiments, $R_d$ is methyl, ethyl, propyl, isopropyl, butyl, isobutyl or tert-butyl substituted with cyclopropyl, cyclobutyl, cyclopentyl, cyclohexyl, pyrazolyl, furyl, imidazolyl, thiazolyl, isothiazolyl, oxazolyl, isoxazolyl, pyrimidyl, pyridyl, tetrahydrofuryl, piperidyl or piperazinyl, wherein the cyclopropyl, cyclobutyl, cyclopentyl, cyclohexyl, pyrazolyl, furyl, imidazolyl, thiazolyl, isothiazolyl, oxazolyl, isoxazolyl, pyrimidyl, pyridyl, tetrahydrofuryl, piperidyl or piperazinyl is optionally substituted with 1-3 groups selected from F, Cl, Br, I, OH, CN, $NH_2$, methyl, ethyl, propyl, isopropyl, butyl, halomethyl, haloethyl, halopropyl, haloisopropyl, and halobutyl. In some specific embodiments, $R_d$ is methyl, ethyl, propyl, isopropyl, monofluoromethyl, difluoromethyl, or trifluoromethyl;

$R_7$ and $R_7$' are H, $C_{1-4}$alkyl or $C_{3-6}$cycloalkyl, and the alkyl and cycloalkyl are optionally substituted with 1-3 groups selected from halogen, OH, SH, $C_{1-4}$alkyl and $C_{3-6}$cycloalkyl. In some specific embodiments, $R_7$ and $R_7$' are H or $C_{1-4}$alkyl. In some specific embodiments, $R_7$ and $R_7$' are H, methyl, ethyl, propyl, isopropyl, butyl, cyclopropyl, cyclobutyl, cyclopentyl or cyclohexyl, wherein the $R_7$ and $R_7$' are optionally substituted with 1-3 groups selected from F, Cl, Br, I, OH, SH, methyl, ethyl, propyl, isopropyl, butyl, cyclopropyl, cyclobutyl, cyclopentyl, and cyclohexyl;

$R_8$ and $R_8$' are independently $C_{1-6}$alkyl, $-OR_f$, $-SR_f$, $-NHR_f$, $C_{3-6}$cycloalkyl, or 4- to 7-membered heterocycloalkyl

containing 1-3 heteroatoms selected from N, S, or O, and the alkyl, cycloalkyl and heterocycloalkyl are optionally substituted with 1-3 groups selected from halogen, OH, CN, $NH_2$, $C_{1-4}$alkyl, -$C_{1-4}$alkyl-OH, $C_{3-6}$cycloalkyl, and 4- to 7-membered heterocycloalkyl containing 1-3 heteroatoms selected from N, S, or O. In some specific embodiments, $R_8$ and $R_8$' are independently $C_{1-4}$alkyl, -$OR_f$, -$NHR_f$, $C_{3-6}$cycloalkyl, or 5- to 6-membered heterocycloalkyl containing 1-3 heteroatoms selected from N, S, or O, wherein the alkyl, cycloalkyl and heterocycloalkyl are optionally substituted with 1-3 groups selected from halogen, OH, CN, $NH_2$, $C_{1-4}$alkyl, -$C_{1-4}$alkyl-OH, $C_{3-6}$cycloalkyl, and 5- to 6-membered heterocycloalkyl containing 1-3 heteroatoms selected from N, S, or O. In some specific embodiments, $R_8$ and $R_8$' are independently methyl, ethyl, propyl, isopropyl, butyl, -$OR_f$, -$NHR_f$, cyclopropyl, cyclobutyl, cyclopentyl, cyclohexyl, tetrahydrofuryl, piperidyl or piperazinyl, wherein the $R_8$ and $R_8$' are optionally substituted with 1-3 groups selected from F, Cl, Br, I, OH, CN, $NH_2$, methyl, ethyl, propyl, -$CH_2OH$, -$CH_2CH_2OH$, cyclopropyl, cyclobutyl, tetrahydrofuryl, piperidyl and piperazinyl;

$R_f$ is H, $C_{1-4}$alkyl, $C_{3-6}$cycloalkyl, or 4- to 7-membered heterocycloalkyl containing 1-3 heteroatoms selected from N, S, or O, and the alkyl, cycloalkyl and heterocycloalkyl are optionally substituted with 1-3 groups selected from halogen, OH, CN, $NH_2$, $C_{1-4}$alkyl, $C_{3-6}$cycloalkyl, and 4- to 7-membered heterocycloalkyl containing 1-3 heteroatoms selected from N, S, or O. In some specific embodiments, $R_f$ is $C_{1-4}$alkyl, $C_{3-6}$cycloalkyl, or 5- to 6-membered heterocycloalkyl containing 1-3 heteroatoms selected from N, S, or O, wherein the alkyl, cycloalkyl and heterocycloalkyl are optionally substituted with 1-3 groups selected from halogen, OH, CN, $NH_2$, $C_{1-4}$alkyl, $C_{3-6}$cycloalkyl, and 5- to 6-membered heterocycloalkyl containing 1-3 heteroatoms selected from N, S, or O. In some specific embodiments, $R_f$ is H, methyl, ethyl, propyl, cyclopropyl, cyclobutyl, cyclopentyl, cyclohexyl, tetrahydrofuryl, piperidyl or piperazinyl, which is optionally substituted with 1-3 groups selected from F, Cl, Br, I, OH, CN, $NH_2$, methyl, ethyl, propyl, cyclopropyl, cyclobutyl, cyclopentyl, cyclohexyl, tetrahydrofuryl, piperidyl and piperazinyl.

In some embodiments, the compound satisfies that:

(i) $R_3$ is -$NR_aSO_2R_b$, -$SO_2NR_a$, CN or halogen; or

(ii) $R_4$ is $SF_5$, -$NR_aSO_2R_B$, -$SO_2NR_a$, -P(=O)$(R_b)_2$ or -$NR_aP$(=O)$(R_b)_2$, wherein, $R_B$ is $C_{1-4}$alkyl, $C_{3-6}$cycloalkyl, phenyl, 5- to 6-membered heteroaryl containing 1-3 heteroatoms selected from N, S, or O, or 4- to 7-membered heterocycloalkyl containing 1-3 heteroatoms selected from N, S, or O; when $R_B$ is the alkyl, $R_B$ is further substituted with 1-3 groups selected from halogen, CN, $C_{3-6}$cycloalkyl, 5- to 6-membered heteroaryl containing 1-3 heteroatoms selected from N, S, or O, and 4-to 7-membered heterocycloalkyl containing 1-3 heteroatoms selected from N, S, or O; when $R_B$ is the cycloalkyl, phenyl, heteroaryl or heterocycloalkyl, $R_B$ is optionally substituted with 1-3 groups selected from halogen, CN, $NH_2$, OH and $C_{1-4}$alkyl; or

(iii) $R_5$ is CN or halogen; or

(iv) Rs is -$OR_f$, -$SR_f$, -$NHR_f$, $C_{1-6}$alkyl, $C_{3-6}$cycloalkyl, or 4- to 7-membered heterocycloalkyl containing 1-3 heteroatoms selected from N, S, or O, when Rs is the alkyl, Rs is further substituted with $C_{3-6}$cycloalkyl, 5- to 6-membered heteroaryl containing 1-3 heteroatoms selected from N, S, or O, or 4- to 7-membered heterocycloalkyl containing 1-3 heteroatoms selected from N, S, or O; when Rs is the cycloalkyl or heterocycloalkyl, Rs is optionally substituted with 1-3 groups selected from halogen, OH, CN, $NH_2$, $C_{1-4}$alkyl, $C_{3-6}$cycloalkyl, and 4- to 7-membered heterocycloalkyl containing 1-3 heteroatoms selected from N, S, or O; or

(v) at least one of $R_3$' and $R_4$' is -$NR_cR_c$', $OR_d$, $C_{2-6}$alkenyl, $C_{2-6}$alkynyl, 5- to 6-membered heteroaryl containing 1-3 heteroatoms selected from N, S, or O, or 4-to 7-membered heterocycloalkyl containing 1-3 heteroatoms selected from N, S, or O, the alkenyl, alkynyl, heteroaryl and heterocycloalkyl are optionally substituted with 1-3 groups selected from halogen, CN, $NH_2$, OH, $C_{1-4}$alkyl, $C_{3-6}$cycloalkyl, 5-to 6-membered heteroaryl containing 1-3 heteroatoms selected from N, S, or O, and 4- to 7-membered heterocycloalkyl containing 1-3 heteroatoms selected from N, S, or O, $R_c$' is $C_{2-6}$alkyl, $C_{2-6}$alkenyl, $C_{2-6}$alkynyl, -$OC_{1-4}$alkyl or OH;

optionally, one $R_3$' and one $R_4$' together with the atoms to which they are attached form $C_{3-8}$cycloalkyl, or 4- to 12-membered heterocycloalkyl containing 1-3 heteroatoms selected from N, S, or O, the cycloalkyl or heterocycloalkyl is optionally substituted with 1-3 groups selected from halogen, OH, $NH_2$, $C_{1-4}$alkyl and halo $C_{1-4}$alkyl; or

(vi) $R_8$' is -$OR_f$, $R_f$ is $C_{1-4}$alkyl, $C_{3-6}$cycloalkyl, or 4- to 7-membered heterocycloalkyl containing 1-3 heteroatoms selected from N, S, or O, the alkyl, cycloalkyl and heterocycloalkyl are optionally substituted with 1-3 groups selected from halogen, OH, CN, $NH_2$, $C_{1-4}$alkyl, $C_{3-6}$cycloalkyl, and 4- to 7-membered heterocycloalkyl containing 1-3 heteroatoms selected from N, S, or O.

In some specific embodiments, the compound satisfies that:

(i) $R_3$ is -$NR_aSO_2R_b$, -$SO_2NR_a$, CN or halogen; or

(ii) $R_4$ is $SF_5$, -$NR_aSO_2R_B$, -$SO_2NR_a$, -P(=O)$(R_b)_2$ or -$NR_aP$(=O)$(R_b)_2$, wherein,

$R_B$ is $C_{1-4}$alkyl, $C_{3-6}$cycloalkyl, phenyl, 5- to 6-membered heteroaryl containing 1-3 heteroatoms selected from N,

S, or O, or 5- to 6-membered heterocycloalkyl containing 1-3 heteroatoms selected from N, S, or O; when $R_B$ is the alkyl, $R_B$ is further substituted with 1-3 groups selected from halogen, CN, $C_{3-6}$cycloalkyl, 5- to 6-membered heteroaryl containing 1-3 heteroatoms selected from N, S, or O, and 5-to 6-membered heterocycloalkyl containing 1-3 heteroatoms selected from N, S, or O; when $R_B$ is the cycloalkyl, phenyl, heteroaryl or heterocycloalkyl, $R_B$ is optionally substituted with 1-3 groups selected from halogen, CN, $NH_2$, OH and $C_{1-4}$alkyl; or

(iii) $R_5$ is CN or halogen; or

(iv) $R_8$ is $-OR_f$, $-NHR_f$, $C_{1-4}$alkyl, $C_{3-6}$cycloalkyl, or 5- to 6-membered heterocycloalkyl containing 1-3 heteroatoms selected from N, S, or O, when Rs is the alkyl, Rs is further substituted with $C_{3-6}$cycloalkyl, 5- to 6-membered heteroaryl containing 1-3 heteroatoms selected from N, S, or O, or 5- to 6-membered heterocycloalkyl containing 1-3 heteroatoms selected from N, S, or O; when Rs is the cycloalkyl or heterocycloalkyl, Rs is optionally substituted with 1-3 groups selected from halogen, OH, CN, $NH_2$, $C_{1-4}$alkyl, $C_{3-6}$cycloalkyl, and 5- to 6-membered heterocycloalkyl containing 1-3 heteroatoms selected from N, S, or O; or

(v) at least one of $R_3$' and $R_4$' is $-NHR_c$', $OR_d$, $C_{2-6}$alkenyl, $C_{2-6}$alkynyl, 5-membered heteroaryl containing 1-3 heteroatoms selected from N, S, or O, or 5- to 6-membered heterocycloalkyl containing 1-3 heteroatoms selected from N, S, or O, the alkenyl, alkynyl, heteroaryl and heterocycloalkyl are optionally substituted with 1-3 groups selected from halogen, CN, $NH_2$, OH, $C_{1-4}$alkyl, $C_{3-6}$cycloalkyl, 5- to 6-membered heteroaryl containing 1-3 heteroatoms selected from N, S, or O, and 5- to 6-membered heterocycloalkyl containing 1-3 heteroatoms selected from N, S, or O, $R_c$' is $C_{2-6}$alkyl, $C_{2-6}$alkenyl, $C_{2-6}$alkynyl, $-OC_{1-4}$alkyl or OH;

optionally, one $R_3$' and one $R_4$' together with the atoms to which they are attached form $C_{3-8}$cycloalkyl, or 4- to 12-membered heterocycloalkyl containing 1-3 heteroatoms selected from N, S, or O, the cycloalkyl or heterocycloalkyl is optionally substituted with 1-3 groups selected from halogen, OH, $NH_2$, $C_{1-4}$alkyl and halo $C_{1-4}$alkyl; or

(vi) $R_8$' is $-OR_f$ or $C_{3-6}$cycloalkyl, the cycloalkyl is optionally substituted with 1-3 groups selected from halogen, OH, CN, $NH_2$, $C_{1-4}$alkyl, $-C_{1-4}$alkyl-OH, $C_{3-6}$cycloalkyl, and 4- to 7-membered heterocycloalkyl containing 1-3 heteroatoms selected from N, S, or O;

$R_f$ is $C_{1-4}$alkyl, $C_{3-6}$cycloalkyl, or 5-to 6-membered heterocycloalkyl containing 1-3 heteroatoms selected from N, S, or O, the alkyl, cycloalkyl and heterocycloalkyl are optionally substituted with 1-3 groups selected from halogen, OH, CN, $NH_2$, $C_{1-4}$alkyl, $C_{3-6}$cycloalkyl, and 5- to 6-membered heterocycloalkyl containing 1-3 heteroatoms selected from N, S, or O.

More specifically, as the first technical solution of the present invention, provided is a compound as represented by formula (I) or (I'), or the stereoisomer, solvate, deuterated compound, or pharmaceutically acceptable salt thereof, wherein, ring A is selected from I-a or I-b:

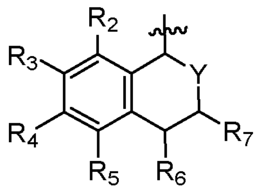

I-a ,

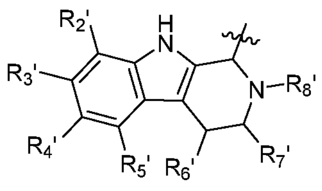

I-b ;

X is N or $CR_x$;

a is 0, 1, 2 or 3;

b is 1 or 2;

Y is $NR_8$ or $CHR_8$;

$R_1$ is $C_{1-6}$alkyl, $C_{2-6}$alkenyl or $C_{2-6}$alkynyl, the $R_1$ is optionally substituted with 1-3 groups selected from halogen, OH, $NH_2$, $-OC_{1-4}$alkyl, $-NHC_{1-4}$alkyl, $C_{3-6}$cycloalkyl, and 4- to 7-membered heterocycloalkyl containing 1-3 heteroatoms selected from N, S or O, and the cycloalkyl and heterocycloalkyl are optionally further substituted with 1-3 groups selected from halogen, OH, $C_{1-4}$alkyl and $-OC_{1-4}$alkyl;

each $R_x$ is independently H, $NH_2$, OH, halogen, $C_{1-4}$alkyl, halo $C_{1-4}$alkyl, $-N(C_{1-4}alkyl)_2$, $-NHC_{1-4}$alkyl or $-OC_{1-4}$alkyl;

$R_2$, $R_5$, $R_6$, $R_2$', $R_5$' and $R_6$' are independently H, CN, OH, $NH_2$, SH or halogen;

$R_3$ and $R_4$ are independently H, halogen, $SF_5$, CN, $-NR_aSO_2R_b$, $-SO_2NHR_a$, $-P(=O)(R_b)_2$, or $-NR_aP(=O)(R_b)_2$; $R_a$ is H or $R_b$;

$R_b$ is $C_{1-4}$alkyl, $C_{3-6}$cycloalkyl, phenyl, 5- to 6-membered heteroaryl containing 1-3 heteroatoms selected from N, S, or O, or 4- to 7-membered heterocycloalkyl containing 1-3 heteroatoms selected from N, S, or O, and the alkyl, cycloalkyl, phenyl, heteroaryl and heterocycloalkyl are optionally substituted with 1-3 groups selected from halogen, CN, $NH_2$, OH, $C_{1-4}$alkyl, $C_{3-6}$cycloalkyl, 5- to 6-membered heteroaryl containing 1-3 heteroatoms selected from N,

S, or O, and 4- to 7-membered heterocycloalkyl containing 1-3 heteroatoms selected from N, S, or O;

$R_3$' and $R_4$' are independently H, $-NR_cR_c$', $OR_d$, $C_{2-6}$alkenyl, $C_{2-6}$alkynyl, halogen, $SF_5$, CN, $C_{1-4}$alkyl, $C_{3-6}$cycloalkyl, phenyl, 5- to 6-membered heteroaryl containing 1-3 heteroatoms selected from N, S, or O, or 4- to 7-membered heterocycloalkyl containing 1-3 heteroatoms selected from N, S, or O, the alkyl, alkenyl, alkynyl, cycloalkyl, phenyl, heteroaryl and heterocycloalkyl are optionally substituted with 1-3 groups selected from halogen, CN, $NH_2$, OH, $C_{1-4}$alkyl, $C_{3-6}$cycloalkyl, 5- to 6-membered heteroaryl containing 1-3 heteroatoms selected from N, S, or O, and 4- to 7-membered heterocycloalkyl containing 1-3 heteroatoms selected from N, S, or O;

optionally, one $R_3$' and one $R_4$' together with the atoms to which they are attached form $C_{3-8}$cycloalkyl, or 4- to 12-membered heterocycloalkyl containing 1-3 heteroatoms selected from N, S, or O, the cycloalkyl or heterocycloalkyl is optionally substituted with 1-3 groups selected from halogen, OH, $NH_2$, $C_{1-4}$alkyl and halo $C_{1-4}$alkyl;

provided that, when b is selected from 2, $R_3$' and $R_4$' are not both selected from H;

$R_c$ is H, $C_{1-4}$alkyl or $C_{3-6}$cycloalkyl;

$R_c$' is $C_{2-6}$alkyl, $C_{2-6}$alkenyl, $C_{2-6}$alkynyl, $-COR_e$, -OH, $-OC_{1-4}$alkyl or $-SO_2$Re;

Re is $C_{2-6}$alkyl, $C_{2-6}$alkenyl, $C_{2-6}$alkynyl, $C_{4-6}$cycloalkyl, $-C_{1-4}$alkyl$C_{3-6}$cycloalkyl, 5- to 6-membered heteroaryl containing 1-3 heteroatoms selected from N, S, or O, or 4- to 7-membered heterocycloalkyl containing 1-3 heteroatoms selected from N, S, or O, and the alkyl, alkenyl, alkynyl, cycloalkyl, heteroaryl and heterocycloalkyl are optionally substituted with 1-3 groups selected from halogen, OH, CN, $NH_2$, $C_{1-4}$alkyl and halo $C_{1-4}$alkyl;

$R_d$ is $C_{1-4}$alkyl, $C_{1-4}$alkyl, halo $C_{1-4}$alkyl, deuterated $C_{1-4}$alkyl, or halo $C_{1-4}$alkyl substituted with $C_{3-6}$cycloalkyl, 5- to 6-membered heteroaryl containing 1-3 heteroatoms selected from N, S, or O, or 4- to 7-membered heterocycloalkyl containing 1-3 heteroatoms selected from N, S, or O, the cycloalkyl, heteroaryl and heterocycloalkyl are optionally substituted with 1-3 groups selected from halogen, OH, CN, $NH_2$, $C_{1-4}$alkyl and halo $C_{1-4}$alkyl;

$R_7$ and $R_7$' are H, $C_{1-4}$alkyl or $C_{3-6}$cycloalkyl, and the alkyl and cycloalkyl are optionally substituted with 1-3 groups selected from halogen, OH, SH, $C_{1-4}$alkyl and $C_{3-6}$cycloalkyl;

Rs and Rs' are independently $C_{1-6}$alkyl, $-OR_f$, $-SR_f$, $-NHR_f$, $C_{3-6}$cycloalkyl, or 4- to 7-membered heterocycloalkyl containing 1-3 heteroatoms selected from N, S, or O, and the alkyl, cycloalkyl and heterocycloalkyl are optionally substituted with 1-3 groups selected from halogen, OH, CN, $NH_2$, $C_{1-4}$alkyl, $-C_{1-4}$alkyl-OH, $C_{3-6}$cycloalkyl, and 4- to 7-membered heterocycloalkyl containing 1-3 heteroatoms selected from N, S, or O;

$R_f$ is H, $C_{1-4}$alkyl, $C_{3-6}$cycloalkyl, or 4- to 7-membered heterocycloalkyl containing 1-3 heteroatoms selected from N, S, or O, and the alkyl, cycloalkyl and heterocycloalkyl are optionally substituted with 1-3 groups selected from halogen, OH, CN, $NH_2$, $C_{1-4}$alkyl, $C_{3-6}$cycloalkyl, and 4- to 7-membered heterocycloalkyl containing 1-3 heteroatoms selected from N, S, or O.

More specifically, as the second technical solution of the present invention, provided is a compound as represented by formula (I) or (I'), or the stereoisomer, solvate, deuterated compound, or pharmaceutically acceptable salt thereof, wherein, ring A is selected from I-a or I-b:

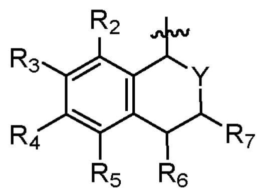

I-a ,

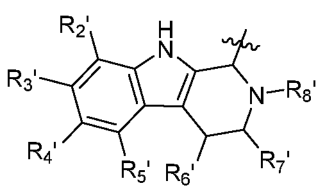

I-b ;

X is N or $CR_x$;

a is 0, 1, 2 or 3;

b is 1 or 2;

Y is $NR_8$ or $CHR_8$;

$R_1$ is $C_{1-6}$alkyl, $C_{2-6}$alkenyl or $C_{2-6}$alkynyl, the $R_1$ is optionally substituted with 1-3 groups selected from halogen, OH, $NH_2$, $-OC_{1-4}$alkyl, $-NHC_{1-4}$alkyl, $C_{3-6}$cycloalkyl, and 4- to 7-membered heterocycloalkyl containing 1-3 heteroatoms selected from N, S or O, and the cycloalkyl and heterocycloalkyl are optionally further substituted with 1-3 groups selected from halogen, OH, $C_{1-4}$alkyl and $-OC_{1-4}$alkyl;

each $R_x$ is independently H, $NH_2$, OH, halogen, $C_{1-4}$alkyl, halo $C_{1-4}$alkyl, $-N(C_{1-4}alkyl)_2$, $-NHC_{1-4}$alkyl or $-OC_{1-4}$alkyl;

$R_2$, $R_5$, $R_6$, $R_2$', $R_5$' and $R_6$' are independently H, CN, OH, $NH_2$, SH or halogen;

$R_3$ and $R_4$ are independently H, halogen, $SF_5$, CN, $-NR_aSO_2R_b$, $-SO_2NHR_a$, $-P(=O)(R_b)_2$, or $-NR_aP(=O)(R_b)_2$; $R_a$ is H or $R_b$;

$R_b$ is $C_{1-4}$alkyl, $C_{3-6}$cycloalkyl, phenyl, 5- to 6-membered heteroaryl containing 1-3 heteroatoms selected from N,

S, or O, or 4- to 7-membered heterocycloalkyl containing 1-3 heteroatoms selected from N, S, or O, and the alkyl, cycloalkyl, phenyl, heteroaryl and heterocycloalkyl are optionally substituted with 1-3 groups selected from halogen, CN, $NH_2$, OH, $C_{1-4}$alkyl, $C_{3-6}$cycloalkyl, 5- to 6-membered heteroaryl containing 1-3 heteroatoms selected from N, S, or O, and 4- to 7-membered heterocycloalkyl containing 1-3 heteroatoms selected from N, S, or O;

$R_3$' and $R_4$' are independently H, $-NR_cR_c$', $OR_d$, $C_{2-6}$alkenyl, $C_{2-6}$alkynyl, halogen, $SF_5$, CN, $C_{1-4}$alkyl, $C_{3-6}$cycloalkyl, phenyl, 5- to 6-membered heteroaryl containing 1-3 heteroatoms selected from N, S, or O, or 4- to 7-membered heterocycloalkyl containing 1-3 heteroatoms selected from N, S, or O, the alkyl, alkenyl, alkynyl, cycloalkyl, phenyl, heteroaryl and heterocycloalkyl are optionally substituted with 1-3 groups selected from halogen, CN, $NH_2$, OH, $C_{1-4}$alkyl, $C_{3-6}$cycloalkyl, 5- to 6-membered heteroaryl containing 1-3 heteroatoms selected from N, S, or O, and 4- to 7-membered heterocycloalkyl containing 1-3 heteroatoms selected from N, S, or O;

$R_c$ is H, $C_{1-4}$alkyl or $C_{3-6}$cycloalkyl;

$R_c$' is $C_{2-6}$alkyl, $C_{2-6}$alkenyl, $C_{2-6}$alkynyl, $-COR_e$, -OH, $-OC_{1-4}$alkyl or $-SO_2$Re;

Re is $C_{2-6}$alkyl, $C_{2-6}$alkenyl, $C_{2-6}$alkynyl, $C_{4-6}$cycloalkyl, $-C_{1-4}$alkyl$C_{3-6}$cycloalkyl, 5- to 6-membered heteroaryl containing 1-3 heteroatoms selected from N, S, or O, or 4- to 7-membered heterocycloalkyl containing 1-3 heteroatoms selected from N, S, or O, and the alkyl, alkenyl, alkynyl, cycloalkyl, heteroaryl and heterocycloalkyl are optionally substituted with 1-3 groups selected from halogen, OH, CN, $NH_2$, $C_{1-4}$alkyl and halo $C_{1-4}$alkyl;

$R_d$ is $C_{1-4}$alkyl substituted with $C_{3-6}$cycloalkyl, 5- to 6-membered heteroaryl containing 1-3 heteroatoms selected from N, S, or O, or 4- to 7-membered heterocycloalkyl containing 1-3 heteroatoms selected from N, S, or O, the cycloalkyl, heteroaryl and heterocycloalkyl are optionally substituted with 1-3 groups selected from halogen, OH, CN, $NH_2$, $C_{1-4}$alkyl and halo $C_{1-4}$alkyl; or $R_d$ is $C_{1-4}$alkyl, halo $C_{1-4}$alkyl, deuterated $C_{1-4}$alkyl, or halo $C_{1-4}$alkyl;

$R_7$ and $R_7$' are H, $C_{1-4}$alkyl or $C_{3-6}$cycloalkyl, and the alkyl and cycloalkyl are optionally substituted with 1-3 groups selected from halogen, OH, SH, $C_{1-4}$alkyl and $C_{3-6}$cycloalkyl;

$R_8$ and $R_8$' are independently $C_{1-6}$alkyl, $-OR_f$, $-SR_f$, $-NHR_f$, $C_{3-6}$cycloalkyl, or 4- to 7-membered heterocycloalkyl containing 1-3 heteroatoms selected from N, S, or O, the alkyl, cycloalkyl and heterocycloalkyl are optionally substituted with 1-3 groups selected from halogen, OH, CN, $NH_2$, $C_{1-4}$alkyl, $C_{3-6}$cycloalkyl, and 4- to 7-membered heterocycloalkyl containing 1-3 heteroatoms selected from N, S, or O;

$R_f$ is H, $C_{1-4}$alkyl, $C_{3-6}$cycloalkyl, or 4- to 7-membered heterocycloalkyl containing 1-3 heteroatoms selected from N, S, or O, and the alkyl, cycloalkyl and heterocycloalkyl are optionally substituted with 1-3 groups selected from halogen, OH, CN, $NH_2$, $C_{1-4}$alkyl, $C_{3-6}$cycloalkyl, and 4- to 7-membered heterocycloalkyl containing 1-3 heteroatoms selected from N, S, or O.

More specifically, as the third technical solution of the present invention, provided is a compound as represented by formula (I) or (I'), or the stereoisomer, solvate, deuterated compound, or pharmaceutically acceptable salt thereof, wherein, the compound satisfies that:

(i) $R_3$ is $-NR_aSO_2R_b$, $-SO_2NR_a$, CN or halogen; or

(ii) $R_4$ is $SF_5$, $-NR_aSO_2R_B$, $-SO_2NR_a$, $-P(=O)(R_b)_2$ or $-NR_aP(=O)(R_b)_2$, wherein, $R_B$ is $C_{1-4}$alkyl, $C_{3-6}$cycloalkyl, phenyl, 5- to 6-membered heteroaryl containing 1-3 heteroatoms selected from N, S, or O, or 4- to 7-membered heterocycloalkyl containing 1-3 heteroatoms selected from N, S, or O; when $R_B$ is the alkyl, $R_B$ is further substituted with 1-3 groups selected from halogen, CN, $C_{3-6}$cycloalkyl, 5- to 6-membered heteroaryl containing 1-3 heteroatoms selected from N, S, or O, and 4-to 7-membered heterocycloalkyl containing 1-3 heteroatoms selected from N, S, or O; when $R_B$ is the cycloalkyl, phenyl, heteroaryl or heterocycloalkyl, $R_B$ is optionally substituted with 1-3 groups selected from halogen, CN, $NH_2$, OH and $C_{1-4}$alkyl; or

(iii) $R_5$ is CN or halogen; or

(iv) Rs is $-OR_f$, $-SR_f$, $-NHR_f$, $C_{1-6}$alkyl, $C_{3-6}$cycloalkyl, or 4- to 7-membered heterocycloalkyl containing 1-3 heteroatoms selected from N, S, or O, when Rs is the alkyl, Rs is further substituted with $C_{3-6}$cycloalkyl, 5- to 6-membered heteroaryl containing 1-3 heteroatoms selected from N, S, or O, or 4- to 7-membered heterocycloalkyl containing 1-3 heteroatoms selected from N, S, or O; when Rs is the cycloalkyl or heterocycloalkyl, Rs is optionally substituted with 1-3 groups selected from halogen, OH, CN, $NH_2$, $C_{1-4}$alkyl, $C_{3-6}$cycloalkyl, and 4- to 7-membered heterocycloalkyl containing 1-3 heteroatoms selected from N, S, or O; or

(v) at least one of $R_3$' and $R_4$' is $-NR_cR_c$', $OR_d$, $C_{2-6}$alkenyl, $C_{2-6}$alkynyl, 5- to 6-membered heteroaryl containing 1-3 heteroatoms selected from N, S, or O, or 4-to 7-membered heterocycloalkyl containing 1-3 heteroatoms selected from N, S, or O, the alkenyl, alkynyl, heteroaryl and heterocycloalkyl are optionally substituted with 1-3 groups selected from halogen, CN, $NH_2$, OH, $C_{1-4}$alkyl, $C_{3-6}$cycloalkyl, 5-to 6-membered heteroaryl containing 1-3 heteroatoms selected from N, S, or O, and 4- to 7-membered heterocycloalkyl containing 1-3 heteroatoms selected from N, S, or O, $R_c$' is $C_{2-6}$alkyl, $C_{2-6}$alkenyl, $C_{2-6}$alkynyl, $-OC_{1-4}$alkyl or OH;

optionally, one $R_3$' and one $R_4$' together with the atoms to which they are attached form $C_{3-8}$cycloalkyl, or 4- to 12-membered heterocycloalkyl containing 1-3 heteroatoms selected from N, S, or O, the cycloalkyl or heterocycloalkyl

is optionally substituted with 1-3 groups selected from halogen, OH, $NH_2$, $C_{1-4}$alkyl and halo $C_{1-4}$alkyl; or
(vi) $R_8$' is $-OR_f$ or $C_{3-6}$cycloalkyl, the cycloalkyl is optionally substituted with 1-3 groups selected from halogen, OH, CN, $NH_2$, $C_{1-4}$alkyl, $-C_{1-4}$alkyl-OH, $C_{3-6}$cycloalkyl, and 4- to 7-membered heterocycloalkyl containing 1-3 heteroatoms selected from N, S, or O;

$R_f$ is $C_{1-4}$alkyl, $C_{3-6}$cycloalkyl, or 4- to 7-membered heterocycloalkyl containing 1-3 heteroatoms selected from N, S, or O, the alkyl, cycloalkyl and heterocycloalkyl are optionally substituted with 1-3 groups selected from halogen, OH, CN, $NH_2$, $C_{1-4}$alkyl, $C_{3-6}$cycloalkyl, and 4- to 7-membered heterocycloalkyl containing 1-3 heteroatoms selected from N, S, or O;
other groups are as described in the preceding first and second technical solutions.

More specifically, as the fourth technical solution of the present invention, provided is a compound as represented by formula (I) or (I'), or the stereoisomer, solvate, deuterated compound, or pharmaceutically acceptable salt thereof, wherein, the compound satisfies that:

(i) $R_3$ is $-NR_aSO_2R_b$, $-SO_2NR_a$, CN or halogen; or
(ii) $R_4$ is $SF_5$, $-NR_aSO_2R_B$, $-SO_2NR_a$, $-P(=O)(R_b)_2$ or $-NR_aP(=O)(R_b)_2$, wherein, $R_B$ is $C_{1-4}$alkyl, $C_{3-6}$cycloalkyl, phenyl, 5- to 6-membered heteroaryl containing 1-3 heteroatoms selected from N, S, or O, or 4- to 7-membered heterocycloalkyl containing 1-3 heteroatoms selected from N, S, or O; when $R_B$ is the alkyl, $R_B$ is further substituted with 1-3 groups selected from halogen, CN, $C_{3-6}$cycloalkyl, 5- to 6-membered heteroaryl containing 1-3 heteroatoms selected from N, S, or O, and 4-to 7-membered heterocycloalkyl containing 1-3 heteroatoms selected from N, S, or O; when $R_B$ is the cycloalkyl, phenyl, heteroaryl or heterocycloalkyl, $R_B$ is optionally substituted with 1-3 groups selected from halogen, CN, $NH_2$, OH and $C_{1-4}$alkyl; or
(iii) $R_5$ is CN or halogen; or
(iv) Rs is $-OR_f$, $-SR_f$, $-NHR_f$, $C_{1-6}$alkyl, $C_{3-6}$cycloalkyl, or 4- to 7-membered heterocycloalkyl containing 1-3 heteroatoms selected from N, S, or O, when Rs is the alkyl, Rs is further substituted with $C_{3-6}$cycloalkyl, 5- to 6-membered heteroaryl containing 1-3 heteroatoms selected from N, S, or O, or 4- to 7-membered heterocycloalkyl containing 1-3 heteroatoms selected from N, S, or O; when Rs is the cycloalkyl or heterocycloalkyl, Rs is optionally substituted with 1-3 groups selected from halogen, OH, CN, $NH_2$, $C_{1-4}$alkyl, $C_{3-6}$cycloalkyl, and 4- to 7-membered heterocycloalkyl containing 1-3 heteroatoms selected from N, S, or O; or
(v) at least one of $R_3$' and $R_4$' is $-NR_cR_c$', $OR_d$, $C_{2-6}$alkenyl, $C_{2-6}$alkynyl, 5- to 6-membered heteroaryl containing 1-3 heteroatoms selected from N, S, or O, or 4-to 7-membered heterocycloalkyl containing 1-3 heteroatoms selected from N, S, or O, the alkenyl, alkynyl, heteroaryl and heterocycloalkyl are optionally substituted with 1-3 groups selected from halogen, CN, $NH_2$, OH, $C_{1-4}$alkyl, $C_{3-6}$cycloalkyl, 5-to 6-membered heteroaryl containing 1-3 heteroatoms selected from N, S, or O, and 4- to 7-membered heterocycloalkyl containing 1-3 heteroatoms selected from N, S, or O, $R_c$' is $C_{2-6}$alkyl, $C_{2-6}$alkenyl, $C_{2-6}$alkynyl, $-OC_{1-4}$alkyl or OH; or
(vi) $R_8$' is $-OR_f$, $R_f$ is $C_{1-4}$alkyl, $C_{3-6}$cycloalkyl, or 4- to 7-membered heterocycloalkyl containing 1-3 heteroatoms selected from N, S, or O, the alkyl, cycloalkyl and heterocycloalkyl are optionally substituted with 1-3 groups selected from halogen, OH, CN, $NH_2$, $C_{1-4}$alkyl, $C_{3-6}$cycloalkyl, and 4- to 7-membered heterocycloalkyl containing 1-3 heteroatoms selected from N, S, or O;

other groups are as described in any of the preceding technical solutions.
More specifically, as the fifth technical solution of the present invention, provided is a compound as represented by formula (I) or (I'), or the stereoisomer, solvate, deuterated compound, or pharmaceutically acceptable salt thereof, wherein, the compound has a structure of formula (II), (II'), (III') or (III),

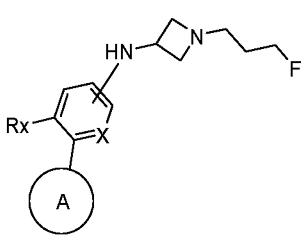
(II')

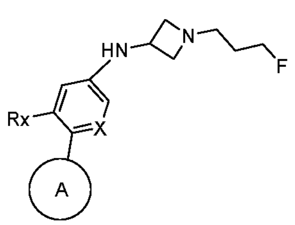
(III')

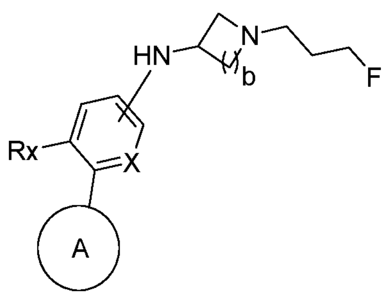

(II)

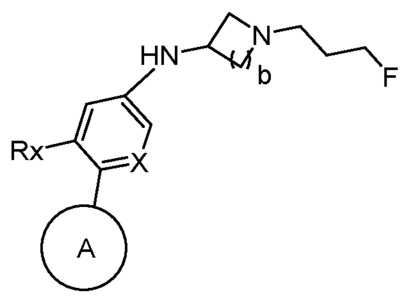

(III)

wherein, ring A is selected from II-a or II-b:

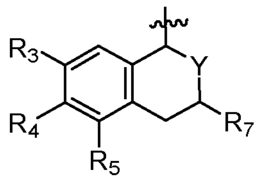

II-a

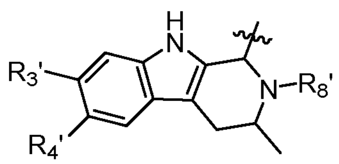

II-b

X is N or $CR_x$;
Y is $NR_8$ or $CHR_8$;
each $R_x$ is independently H or halogen;
other groups are as described in any of the preceding technical solutions.

More specifically, as the sixth technical solution of the present invention, provided is a compound as represented by formula (II'), (II), (III') or (III), or the stereoisomer, solvate, deuterated compound, or pharmaceutically acceptable salt thereof,

wherein, $R_3$' and $R_4$' are independently H, -$NR_cR_c$', $OR_d$, $C_{2-6}$alkenyl, $C_{2-6}$alkynyl, halogen, $SF_5$, CN, $C_{1-4}$alkyl, $C_{3-6}$cycloalkyl, 5-membered heteroaryl containing 1-3 heteroatoms selected from N, S, or O, or 5- to 6-membered heterocycloalkyl containing 1-3 heteroatoms selected from N, S, or O, the alkyl, alkenyl, alkynyl, cycloalkyl, heteroaryl and heterocycloalkyl are optionally substituted with 1-3 groups selected from halogen, CN, $NH_2$, OH, $C_{1-4}$alkyl, $C_{3-6}$cycloalkyl, 5- to 6-membered heteroaryl containing 1-3 heteroatoms selected from N, S, or O, and 5- to 6-membered heterocycloalkyl containing 1-3 heteroatoms selected from N, S, or O;
optionally, one $R_3$' and one $R_4$' together with the atoms to which they are attached form $C_{4-6}$cycloalkyl, the cycloalkyl is optionally substituted with 1-3 groups selected from halogen, OH, and $C_{1-4}$alkyl;
$R_c$ is H, $C_{1-4}$alkyl or $C_{3-6}$cycloalkyl;
$R_c$' is $C_{2-6}$alkyl, $C_{2-6}$alkenyl, $C_{2-6}$alkynyl, -OH or -$OC_{1-4}$alkyl;
$R_d$ is $C_{1-4}$alkyl, $C_{1-4}$alkyl, halo $C_{1-4}$alkyl, deuterated $C_{1-4}$alkyl, or halo $C_{1-4}$alkyl substituted with $C_{3-6}$cycloalkyl, 5- to 6-membered heteroaryl containing 1-3 heteroatoms selected from N, S, or O, or 5- to 6-membered heterocycloalkyl containing 1-3 heteroatoms selected from N, S, or O, the cycloalkyl, heteroaryl and heterocycloalkyl are optionally substituted with 1-3 groups selected from halogen, OH, $C_{1-4}$alkyl and halo $C_{1-4}$alkyl;
Rs is independently H, CN, OH or halogen;
$R_7$ is H or $C_{1-4}$alkyl;
$R_8$ and $R_8$' are independently $C_{1-4}$alkyl, -$OR_f$, -$NHR_f$, $C_{3-6}$cycloalkyl, or 5- to 6-membered heterocycloalkyl containing 1-3 heteroatoms selected from N, S, or O, the alkyl, cycloalkyl and heterocycloalkyl are optionally substituted with 1-3 groups selected from halogen, OH, CN, $NH_2$, $C_{1-4}$alkyl, -$C_{1-4}$alkyl-OH, $C_{3-6}$cycloalkyl, and 5- to 6-membered heterocycloalkyl containing 1-3 heteroatoms selected from N, S, or O;
$R_f$ is $C_{1-4}$alkyl, $C_{3-6}$cycloalkyl, or 5-to 6-membered heterocycloalkyl containing 1-3 heteroatoms selected from N, S, or O, the alkyl, cycloalkyl and heterocycloalkyl are optionally substituted with 1-3 groups selected from halogen, OH, CN, $NH_2$, $C_{1-4}$alkyl, $C_{3-6}$cycloalkyl, and 5- to 6-membered heterocycloalkyl containing 1-3 heteroatoms selected from N, S, or O;
provided that, the compound satisfies that:

(i) $R_3$ is $-NR_aSO_2R_b$, $-SO_2NR_a$, CN or halogen; or
(ii) $R_4$ is $SF_5$, $-NR_aSO_2R_B$, $-SO_2NR_a$, $-P(=O)(R_b)_2$ or $-NR_aP(=O)(R_b)_2$, wherein,

$R_B$ is $C_{1-4}$alkyl, $C_{3-6}$cycloalkyl, phenyl, 5- to 6-membered heteroaryl containing 1-3 heteroatoms selected from N, S, or O, or 5- to 6-membered heterocycloalkyl containing 1-3 heteroatoms selected from N, S, or O; when $R_B$ is the alkyl, $R_B$ is further substituted with 1-3 groups selected from halogen, CN, $C_{3-6}$cycloalkyl, 5- to 6-membered heteroaryl containing 1-3 heteroatoms selected from N, S, or O, and 5-to 6-membered heterocycloalkyl containing 1-3 heteroatoms selected from N, S, or O; when $R_B$ is the cycloalkyl, phenyl, heteroaryl or heterocycloalkyl, $R_B$ is optionally substituted with 1-3 groups selected from halogen, CN, $NH_2$, OH and $C_{1-4}$alkyl; or
(iii) $R_5$ is CN or halogen; or
(iv) Rs is $-OR_f$, $-NHR_f$, $C_{1-4}$alkyl, $C_{3-6}$cycloalkyl, or 5- to 6-membered heterocycloalkyl containing 1-3 heteroatoms selected from N, S, or O, when Rs is the alkyl, Rs is further substituted with $C_{3-6}$cycloalkyl, 5- to 6-membered heteroaryl containing 1-3 heteroatoms selected from N, S, or O, or 5- to 6-membered heterocycloalkyl containing 1-3 heteroatoms selected from N, S, or O; when Rs is the cycloalkyl or heterocycloalkyl, Rs is optionally substituted with 1-3 groups selected from halogen, OH, CN, $NH_2$, $C_{1-4}$alkyl, $C_{3-6}$cycloalkyl, and 5- to 6-membered heterocycloalkyl containing 1-3 heteroatoms selected from N, S, or O; or
(v) at least one of $R_3$' and $R_4$' is $-NHR_c$', $OR_d$, $C_{2-6}$alkenyl, $C_{2-6}$alkynyl, 5-membered heteroaryl containing 1-3 heteroatoms selected from N, S, or O, or 5- to 6-membered heterocycloalkyl containing 1-3 heteroatoms selected from N, S, or O, the alkenyl, alkynyl, heteroaryl and heterocycloalkyl are optionally substituted with 1-3 groups selected from halogen, CN, $NH_2$, OH, $C_{1-4}$alkyl, $C_{3-6}$cycloalkyl, 5- to 6-membered heteroaryl containing 1-3 heteroatoms selected from N, S, or O, and 5- to 6-membered heterocycloalkyl containing 1-3 heteroatoms selected from N, S, or O, $R_c$' is $C_{2-6}$alkyl, $C_{2-6}$alkenyl, $C_{2-6}$alkynyl, $-OC_{1-4}$alkyl or OH;
optionally, one $R_3$' and one $R_4$' together with the atoms to which they are attached form $C_{3-8}$cycloalkyl, or 4- to 12-membered heterocycloalkyl containing 1-3 heteroatoms selected from N, S, or O, the cycloalkyl or heterocycloalkyl is optionally substituted with 1-3 groups selected from halogen, OH, $NH_2$, $C_{1-4}$alkyl and halo $C_{1-4}$alkyl; or
(vi) $R_8$' is $-OR_f$ or $C_{3-6}$cycloalkyl, the cycloalkyl is optionally substituted with 1-3 groups selected from halogen, OH, CN, $NH_2$, $C_{1-4}$alkyl, $-C_{1-4}$alkyl-OH, $C_{3-6}$cycloalkyl, and 4- to 7-membered heterocycloalkyl containing 1-3 heteroatoms selected from N, S, or O;
$R_f$ is $C_{1-4}$alkyl, $C_{3-6}$cycloalkyl, or 5-to 6-membered heterocycloalkyl containing 1-3 heteroatoms selected from N, S, or O, the alkyl, cycloalkyl and heterocycloalkyl are optionally substituted with 1-3 groups selected from halogen, OH, CN, $NH_2$, $C_{1-4}$alkyl, $C_{3-6}$cycloalkyl, and 5- to 6-membered heterocycloalkyl containing 1-3 heteroatoms selected from N, S, or O;
other groups are as described in any of the preceding technical solutions.

More specifically, as the seventh technical solution of the present invention, provided is a compound as represented by formula (II), (II'), (III') or (III), or the stereoisomer, solvate, deuterated compound, or pharmaceutically acceptable salt thereof,

wherein, $R_3$' and $R_4$' are independently H, $-NR_cR_c$', $OR_d$, $C_{2-6}$alkenyl, $C_{2-6}$alkynyl, halogen, $SF_5$, CN, $C_{1-4}$alkyl, $C_{3-6}$cycloalkyl, 5-membered heteroaryl containing 1-3 heteroatoms selected from N, S, or O, or 5- to 6-membered heterocycloalkyl containing 1-3 heteroatoms selected from N, S, or O, the alkyl, alkenyl, alkynyl, cycloalkyl, heteroaryl and heterocycloalkyl are optionally substituted with 1-3 groups selected from halogen, CN, $NH_2$, OH, $C_{1-4}$alkyl, $C_{3-6}$cycloalkyl, 5- to 6-membered heteroaryl containing 1-3 heteroatoms selected from N, S, or O, and 5- to 6-membered heterocycloalkyl containing 1-3 heteroatoms selected from N, S, or O;
$R_c$ is H, $C_{1-4}$alkyl or $C_{3-6}$cycloalkyl;
$R_c$' is $C_{2-6}$alkyl, $C_{2-6}$alkenyl, $C_{2-6}$alkynyl, -OH or $-OC_{1-4}$alkyl;
$R_d$ is $C_{1-4}$alkyl substituted with $C_{3-6}$cycloalkyl, 5- to 6-membered heteroaryl containing 1-3 heteroatoms selected from N, S, or O, or 5- to 6-membered heterocycloalkyl containing 1-3 heteroatoms selected from N, S, or O, the cycloalkyl, heteroaryl and heterocycloalkyl are optionally substituted with 1-3 groups selected from halogen, OH, $C_{1-4}$alkyl and halo $C_{1-4}$alkyl; or $R_d$ is $C_{1-4}$alkyl, halo $C_{1-4}$alkyl, deuterated $C_{1-4}$alkyl, or halo $C_{1-4}$alkyl;
Rs is independently H, CN, OH or halogen;
$R_7$ is H or $C_{1-4}$alkyl;
$R_8$ and $R_8$' are independently $C_{1-4}$alkyl, $-OR_f$, $-NHR_f$, $C_{3-6}$cycloalkyl, or 5- to 6-membered heterocycloalkyl containing 1-3 heteroatoms selected from N, S, or O, the alkyl, cycloalkyl and heterocycloalkyl are optionally substituted with 1-3 groups selected from halogen, OH, CN, $NH_2$, $C_{1-4}$alkyl, $C_{3-6}$cycloalkyl, and 5- to 6-membered heterocycloalkyl containing 1-3 heteroatoms selected from N, S, or O;
$R_f$ is $C_{1-4}$alkyl, $C_{3-6}$cycloalkyl, or 5-to 6-membered heterocycloalkyl containing 1-3 heteroatoms selected from N, S, or O, the alkyl, cycloalkyl and heterocycloalkyl are optionally substituted with 1-3 groups selected from halogen,

OH, CN, $NH_2$, $C_{1-4}$alkyl, $C_{3-6}$cycloalkyl, and 5- to 6-membered heterocycloalkyl containing 1-3 heteroatoms selected from N, S, or O;
provided that, the compound satisfies that:

(i) $R_3$ is $-NR_aSO_2R_b$, $-SO_2NR_a$, CN or halogen; or
(ii) $R_4$ is $SF_5$, $-NR_aSO_2R_B$, $-SO_2NR_a$, $-P(=O)(R_b)_2$ or $-NR_aP(=O)(R_b)_2$, wherein, $R_B$ is $C_{1-4}$alkyl, $C_{3-6}$cycloalkyl, phenyl, 5- to 6-membered heteroaryl containing 1-3 heteroatoms selected from N, S, or O, or 5- to 6-membered heterocycloalkyl containing 1-3 heteroatoms selected from N, S, or O; when $R_B$ is the alkyl, $R_B$ is further substituted with 1-3 groups selected from halogen, CN, $C_{3-6}$cycloalkyl, 5- to 6-membered heteroaryl containing 1-3 heteroatoms selected from N, S, or O, and 5-to 6-membered heterocycloalkyl containing 1-3 heteroatoms selected from N, S, or O; when $R_B$ is the cycloalkyl, phenyl, heteroaryl or heterocycloalkyl, $R_B$ is optionally substituted with 1-3 groups selected from halogen, CN, $NH_2$, OH and $C_{1-4}$alkyl; or
(iii) $R_5$ is CN or halogen; or
(iv) Rs is $-OR_f$, $-NHR_f$, $C_{1-4}$alkyl, $C_{3-6}$cycloalkyl, or 5- to 6-membered heterocycloalkyl containing 1-3 heteroatoms selected from N, S, or O, when Rs is the alkyl, Rs is further substituted with $C_{3-6}$cycloalkyl, 5- to 6-membered heteroaryl containing 1-3 heteroatoms selected from N, S, or O, or 5- to 6-membered heterocycloalkyl containing 1-3 heteroatoms selected from N, S, or O; when Rs is the cycloalkyl or heterocycloalkyl, Rs is optionally substituted with 1-3 groups selected from halogen, OH, CN, $NH_2$, $C_{1-4}$alkyl, $C_{3-6}$cycloalkyl, and 5- to 6-membered heterocycloalkyl containing 1-3 heteroatoms selected from N, S, or O; or
(v) at least one of $R_3$' and $R_4$' is $-NHR_c$', $OR_d$, $C_{2-6}$alkenyl, $C_{2-6}$alkynyl, 5-membered heteroaryl containing 1-3 heteroatoms selected from N, S, or O, or 5- to 6-membered heterocycloalkyl containing 1-3 heteroatoms selected from N, S, or O, the alkenyl, alkynyl, heteroaryl and heterocycloalkyl are optionally substituted with 1-3 groups selected from halogen, CN, $NH_2$, OH, $C_{1-4}$alkyl, $C_{3-6}$cycloalkyl, 5- to 6-membered heteroaryl containing 1-3 heteroatoms selected from N, S, or O, and 5- to 6-membered heterocycloalkyl containing 1-3 heteroatoms selected from N, S, or O, $R_c$' is $C_{2-6}$alkyl, $C_{2-6}$alkenyl, $C_{2-6}$alkynyl, $-OC_{1-4}$alkyl or OH; or
(vi) $R_8$' is $-OR_f$, $R_f$ is $C_{1-4}$alkyl, $C_{3-6}$cycloalkyl, or 5- to 6-membered heterocycloalkyl containing 1-3 heteroatoms selected from N, S, or O, the alkyl, cycloalkyl and heterocycloalkyl are optionally substituted with 1-3 groups selected from halogen, OH, CN, $NH_2$, $C_{1-4}$alkyl, $C_{3-6}$cycloalkyl, and 5- to 6-membered heterocycloalkyl containing 1-3 heteroatoms selected from N, S, or O;

other groups are as described in any of the preceding technical solutions.

More specifically, as the eighth technical solution of the present invention, provided is a compound as represented by formula (I) or (I'), or the stereoisomer, solvate, deuterated compound, or pharmaceutically acceptable salt thereof, wherein, the compound has a structure of formula (III'-1) or (II'-1),

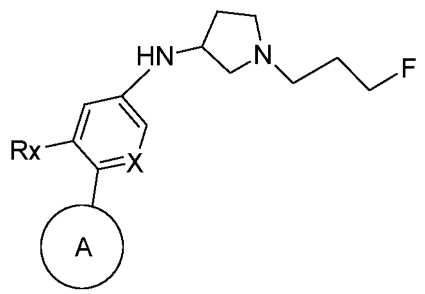

(III'-1)

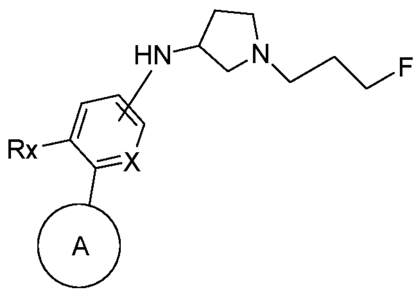

(II'-1)

wherein, ring A is selected from II-a or II-b:

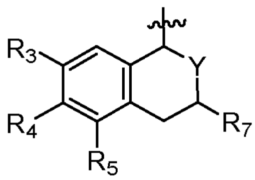

II-a

,

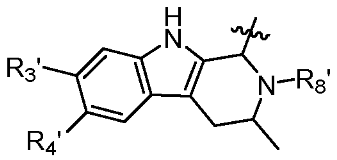

II-b

X is N or $CR_x$;
Y is $NR_8$ or $CHR_8$;
each $R_x$ is independently H or halogen;
provided that, $R_3$' and $R_4$' are not both selected from H;
other groups are as described in any of the preceding technical solutions.

More specifically, as the ninth technical solution of the present invention, provided is a compound as represented by formula (I) or (I'), or the stereoisomer, solvate, deuterated compound, or pharmaceutically acceptable salt thereof, wherein, the compound has a structure of formula (II'-a) or (II'-a-1),

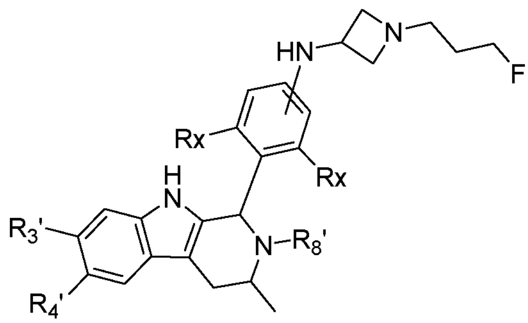

(II'-a)

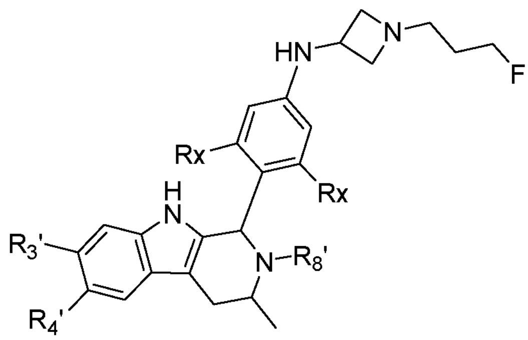

(II'-a-1)

each Rx is independently H or halogen;

$R_3$' and $R_4$' are independently H, $C_{1-6}$alkyl, $C_{1-6}$alkoxy, -O-$CH_2$-$C_{3-6}$cycloalkyl, 5- to 6-membered heteroaryl containing 1-3 heteroatoms selected from N, S, or O, $C_{2-6}$alkenyl or $C_{2-6}$alkynyl, or $R_3$' and $R_4$' form 4-membered cycloalkyl or 5-membered cycloalkyl;

provided that, $R_3$' and $R_4$' are not both selected from H;

$R_8$' is independently $C_{1-6}$alkyl, $C_{3-6}$cycloalkyl, or 4- to 7-membered heterocycloalkyl containing 1-3 heteroatoms selected from N, S, or O, the alkyl, cycloalkyl and heterocycloalkyl are optionally substituted with 1-3 groups selected from halogen, OH, CN, $NH_2$, $C_{1-4}$alkyl, -$C_{1-4}$alkyl-OH, $C_{3-6}$cycloalkyl, and 4- to 7-membered heterocycloalkyl containing 1-3 heteroatoms selected from N, S, or O;

further,

each Rx is independently H or halogen;

$R_3$' and $R_4$' are independently H, $C_{2-6}$alkenyl, or $C_{2-6}$alkynyl, provided that, $R_3$' and $R_4$' are not both selected from H;

$R_8$' is independently $C_{1-6}$alkyl, $C_{3-6}$cycloalkyl, or 4- to 7-membered heterocycloalkyl containing 1-3 heteroatoms selected from N, S, or O, the alkyl, cycloalkyl and heterocycloalkyl are optionally substituted with 1-3 groups selected from halogen, OH, CN, $NH_2$, $C_{1-4}$alkyl, -$C_{1-4}$alkyl-OH, $C_{3-6}$cycloalkyl, and 4- to 7-membered heterocycloalkyl containing 1-3 heteroatoms selected from N, S, or O.

More specifically, as the tenth technical solution of the present invention, provided is a compound as represented by formula (II'-a) or (II'-a-1), or the stereoisomer, solvate, deuterated compound, or pharmaceutically acceptable salt thereof, wherein,

each Rx is independently H, F or Cl. In some embodiments, each Rx is independently F or Cl. In some embodiments, each Rx is independently F;

$R_3$' and $R_4$' are independently H, $C_{1-4}$alkyl, $C_{1-4}$alkoxy, -O-$CH_2$-$C_{3-6}$cycloalkyl, 5- to 6-membered heteroaryl containing 1-3 heteroatoms selected from N, S, or O, $C_{2-4}$alkenyl, or $C_{2-4}$alkynyl. In some embodiments, $R_3$' and $R_4$' are independently H, $C_{2-4}$alkenyl or $C_{2-4}$alkynyl. In some embodiments, $R_3$' and $R_4$' are independently H, ethenyl, propenyl, butenyl, ethynyl, propynyl, or butynyl; provided that, $R_3$' and $R_4$' are not both selected from H;

$R_8$' is independently $C_{1-6}$alkyl, $C_{3-6}$cycloalkyl, or 4- to 7-membered heterocycloalkyl containing 1-3 heteroatoms selected from N, S, or O, the alkyl, cycloalkyl and heterocycloalkyl are optionally substituted with 1-3 groups selected from halogen, OH, CN, $NH_2$, $C_{1-4}$alkyl, -$C_{1-4}$alkyl-OH, $C_{3-6}$cycloalkyl, and 4- to 7-membered heterocycloalkyl containing 1-3 heteroatoms selected from N, S, or O. In some embodiments, $R_8$' is independently $C_{1-4}$alkyl, $C_{3-4}$cycloalkyl, or 4-, 5- or 6-membered heterocycloalkyl containing 1, 2, or 3 heteroatoms selected from N, S, or O, wherein the alkyl, cycloalkyl and heterocycloalkyl are optionally substituted with 1, 2, or 3 groups selected from halogen, OH, CN, $NH_2$, $C_{1-2}$alkyl, -$C_{1-2}$alkyl-OH, $C_{3-4}$cycloalkyl, and 4-, 5- or 6-membered heterocycloalkyl containing 1, 2, or 3 heteroatoms selected from N, S, or O. In some embodiments, $R_8$' is independently $C_{1-4}$alkyl, wherein the alkyl is optionally substituted with 1, 2, or 3 groups selected from halogen, OH, CN, $NH_2$, $C_{1-2}$alkyl, -$C_{1-2}$alkyl-OH, $C_{3-4}$cycloalkyl, and 4-, 5- or 6-membered heterocycloalkyl containing 1, 2, or 3 heteroatoms selected from N, S, or O. In some embodiments, $R_8$' is independently methyl, ethyl, propyl, isopropyl, butyl, or isobutyl, and is optionally substituted with 1, 2, or 3 groups selected from halogen, OH, CN, $NH_2$, $C_{1-2}$alkyl, -$C_{1-2}$alkyl-OH, $C_{3-4}$cycloalkyl, and 4-, 5- or 6-membered heterocycloalkyl containing 1, 2, or 3 heteroatoms selected from N, S, or O. In some embodiments, $R_8$' is independently $C_{1-4}$alkyl, wherein the alkyl is substituted with 1, 2, or 3 groups selected from F, Cl, OH, CN, $NH_2$, methyl, hydroxymethyl, cyclopropyl, azetidinyl, or oxetanyl. In some embodiments, $R_8$' is independently $-CH_3$, $-CH_2F$, $-CHF_2$, $-CF_3$, $-CH_3$, $-CH_2Cl$, $-CHCl_2$, $-CCl_3$, $-CH_2CH_3$, $-CH_2CH_2F$, $-CH_2CHF_2$, $-CH_2CF_3$, $-CH_2CH_2Cl$, $-CH_2CHCl_2$, $-CH_2CCl_3$, - $CH_2CH_2CH_3$, $-CH_2CF_2CH_3$, $-CH_2CF_2CH_2OH$, $-CH_2CF_2CH_2F$, $-CH_2CF_2CHF_2$, - $CH_2CF_2CF_3$, $-CF_2CF_2CH_2OH$, $-CH(F)CF_2CH_2OH$, $-CH(F)CH(F)CH_2OH$, - $CH_2CCl_2CH_3$, $-CH_2CCl_2CH_2OH$, $-CH_2CCl_2CH_2Cl$, $-CH_2CF_2CH_2NH_2$, - $CH_2CF_2CH_2CN$, $-CH_2C(CH_3)_2F$, $-CH_2CH_2CH_2CH_3$, $-CH_2C(CH_3)_2F$, - $CH_2C(CH_3)_2Cl$,

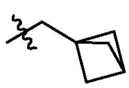, or 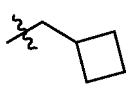.

More specifically, as the eleventh technical solution of the present invention, provided is a compound as represented by formula (I) or (I'), or the stereoisomer, solvate, deuterated compound, or pharmaceutically acceptable salt thereof, wherein, the compound has a structure of formula (II'-1a) or (II'-1a-1),

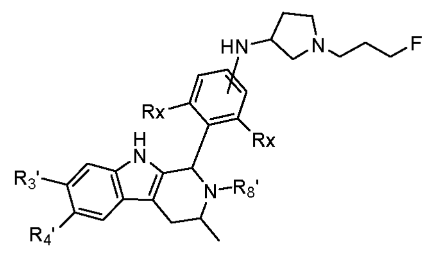

(II'-1a)

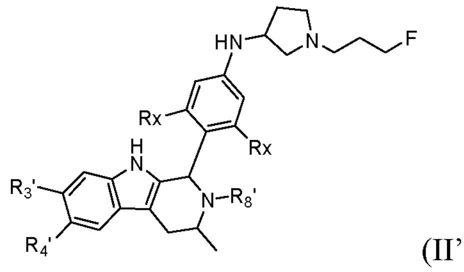

(II'-1a-1)

each Rx is independently H or halogen;

$R_3$' and $R_4$' are independently H, halo $C_{1-4}$alkyl, $C_{1-6}$alkyl, $C_{2-6}$alkenyl, $C_{2-6}$alkynyl or halogen; or

$R_3$' and $R_4$' together form cyclobutyl;

$R_8$' is independently $C_{1-6}$alkyl, $C_{3-6}$cycloalkyl, or 4- to 7-membered heterocycloalkyl containing 1-3 heteroatoms selected from N, S, or O, the alkyl, cycloalkyl and heterocycloalkyl are optionally substituted with 1-3 groups selected from halogen, OH, CN, $NH_2$, $C_{1-4}$alkyl, -$C_{1-4}$alkyl-OH, $C_{3-6}$cycloalkyl, and 4- to 7-membered heterocycloalkyl containing 1-3 heteroatoms selected from N, S, or O;

provided that, $R_3$' and $R_4$' are not both selected from H;

further,

each Rx is independently H or halogen;

$R_3$' and $R_4$' are independently H, $C_{2-6}$alkenyl, $C_{2-6}$alkynyl, or halogen;

$R_8$' is independently $C_{1-6}$alkyl, $C_{3-6}$cycloalkyl, or 4- to 7-membered heterocycloalkyl containing 1-3 heteroatoms selected from N, S, or O, the alkyl, cycloalkyl and heterocycloalkyl are optionally substituted with 1-3 groups selected from halogen, OH, CN, $NH_2$, $C_{1-4}$alkyl, -$C_{1-4}$alkyl-OH, $C_{3-6}$cycloalkyl, and 4- to 7-membered heterocycloalkyl containing 1-3 heteroatoms selected from N, S, or O;

provided that, $R_3$' and $R_4$' are not both selected from H.

More specifically, as the twelfth technical solution of the present invention, provided is a compound as represented by formula (II'-1a) or (II'-1a-1), or the stereoisomer, solvate, deuterated compound, or pharmaceutically acceptable salt thereof, wherein,

each Rx is independently H, F or Cl. In some embodiments, each Rx is independently F or Cl. In some embodiments, each Rx is independently F;

$R_3$' and $R_4$' are independently H, $C_{2-4}$alkenyl, $C_{2-4}$alkynyl, or halogen. In some embodiments, $R_3$' and $R_4$' are independently H, ethenyl, propenyl, butenyl, ethynyl, propynyl, butynyl, F, or Cl; provided that, $R_3$' and $R_4$' are not both selected from H;

$R_8$' is independently $C_{1-6}$alkyl, $C_{3-6}$cycloalkyl, or 4- to 7-membered heterocycloalkyl containing 1-3 heteroatoms selected from N, S, or O, the alkyl, cycloalkyl and heterocycloalkyl are optionally substituted with 1-3 groups selected from halogen, OH, CN, $NH_2$, $C_{1-4}$alkyl, -$C_{1-4}$alkyl-OH, $C_{3-6}$cycloalkyl, and 4- to 7-membered heterocycloalkyl containing 1-3 heteroatoms selected from N, S, or O. In some embodiments, $R_8$' is independently $C_{1-4}$alkyl, $C_{3-4}$cycloalkyl, or 4-, 5- or 6-membered heterocycloalkyl containing 1, 2, or 3 heteroatoms selected from N, S, or O, wherein the alkyl, cycloalkyl and heterocycloalkyl are optionally substituted with 1, 2, or 3 groups selected from halogen, OH, CN, $NH_2$, $C_{1-2}$alkyl, -$C_{1-2}$alkyl-OH, $C_{3-4}$cycloalkyl, and 4-, 5- or 6-membered heterocycloalkyl containing 1, 2, or 3 heteroatoms selected from N, S, or O. In some embodiments, $R_8$' is independently $C_{1-4}$alkyl, wherein the alkyl is optionally substituted with 1, 2, or 3 groups selected from halogen, OH, CN, $NH_2$, $C_{1-2}$alkyl, -$C_{1-2}$alkyl-OH, $C_{3-4}$cycloalkyl, and 4-, 5- or 6-membered heterocycloalkyl containing 1, 2, or 3 heteroatoms selected from N, S, or O. In some embodiments, $R_8$' is independently methyl, ethyl, propyl, isopropyl, butyl, or isobutyl, and is optionally substituted with 1, 2, or 3 groups selected from halogen, OH, CN, $NH_2$, $C_{1-2}$alkyl, -$C_{1-2}$alkyl-OH, $C_{3-4}$cycloalkyl, and

4-, 5- or 6-membered heterocycloalkyl containing 1, 2, or 3 heteroatoms selected from N, S, or O. In some embodiments, $R_8$' is independently $C_{1-4}$alkyl, wherein the alkyl is substituted with 1, 2, or 3 groups selected from F, Cl, OH, CN, $NH_2$, methyl, hydroxymethyl, cyclopropyl, azetidinyl, or oxetanyl. In some embodiments, $R_8$' is independently $-CH_3$, $-CH_2F$, $-CHF_2$, $-CF_3$, $-CH_3$, $-CH_2Cl$, $-CHCl_2$, $-CCl_3$, $-CH_2CH_3$, $-CH_2CH_2F$, $-CH_2CHF_2$, $-CH_2CF_3$, $-CH_2CH_2Cl$, $-CH_2CHCl_2$, $-CH_2CCl_3$, $-CH_2CH_2CH_3$, $-CH_2CF_2CH_3$, $-CH_2CF_2CH_2OH$, $-CH_2CF_2CH_2F$, $-CH_2CF_2CHF_2$, $-CH_2CF_2CF_3$, $-CF_2CF_2CH_2OH$, $-CH(F)CF_2CH_2OH$, $-CH(F)CH(F)CH_2OH$, $-CH_2CCl_2CH_3$, $-CH_2CCl_2CH_2OH$, $-CH_2CCl_2CH_2Cl$, $-CH_2CF_2CH_2NH_2$, $-CH_2CF_2CH_2CN$, $-CH_2C(CH_3)_2F$, $-CH_2CH_2CH_2CH_3$, $-CH_2C(CH_3)_2F$, $-CH_2C(CH_3)_2Cl$,

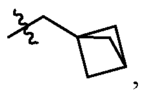, or 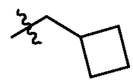.

More specifically, as the thirteenth technical solution of the present invention, provided is a compound as represented by formula (II'-1a) or (II'-1a-1), or the stereoisomer, solvate, deuterated compound, or pharmaceutically acceptable salt thereof, wherein,

each Rx is independently F;
$R_3$' and $R_4$' are independently H, difluoromethyl, trifluoromethyl, ethenyl, propenyl, ethynyl, propynyl, F or Cl. In some embodiments, $R_3$' and $R_4$' are independently H, ethenyl, propenyl, ethynyl, propynyl, F or Cl;
$R_8$' is independently methyl, ethyl or propyl, the methyl, ethyl or propyl is substituted with 1, 2, or 3 groups selected from F, Cl, OH, CN, $NH_2$, methyl, hydroxymethyl, cyclopropyl, azetidinyl or oxetanyl;
provided that, $R_3$' and $R_4$' are not both selected from H.

More specifically, as the fourteenth technical solution of the present invention, provided is a compound as represented by formula (I), (I'), (II), (II'), (IF-1), (III'-1), (III'), (III), (II'-a), (II'-a-1), (II'-1a) or (II'-1a-1), or the stereoisomer, solvate, deuterated compound, or pharmaceutically acceptable salt thereof, wherein the compound is selected from one of the following structures:

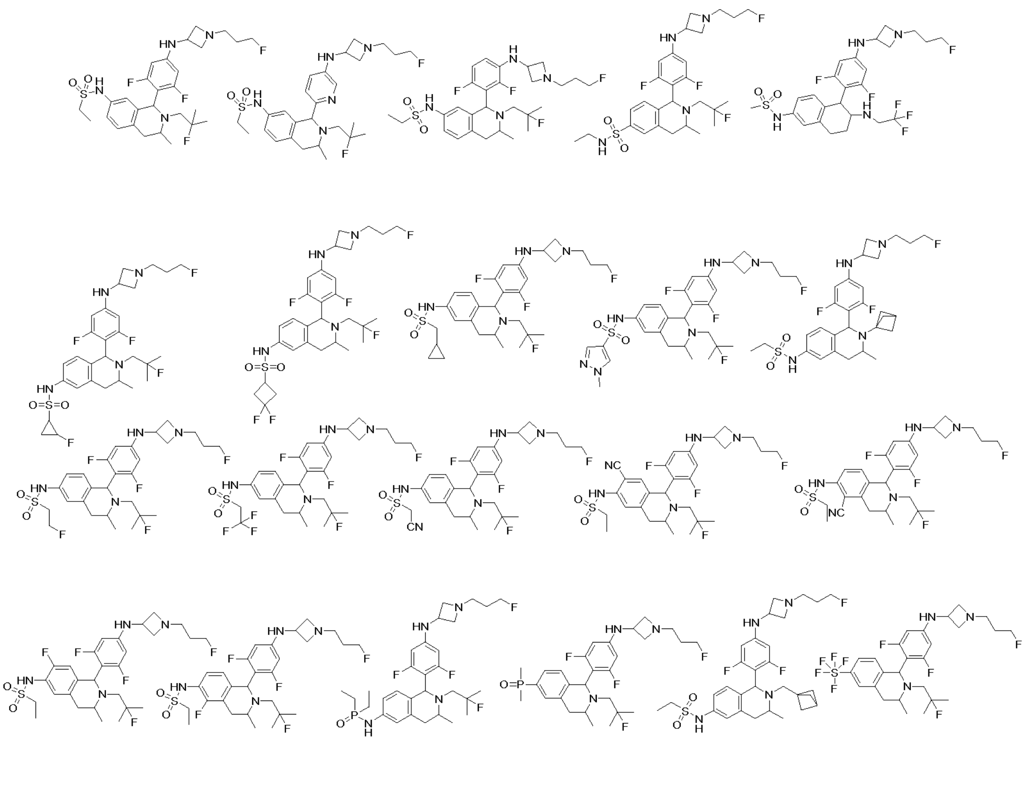

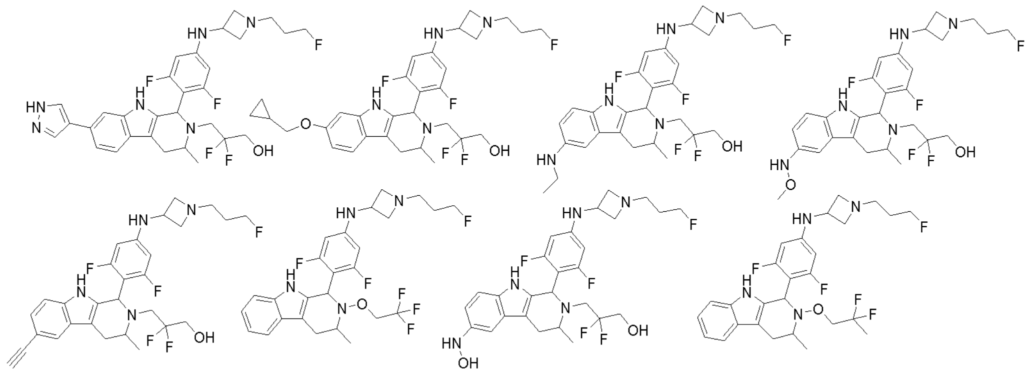

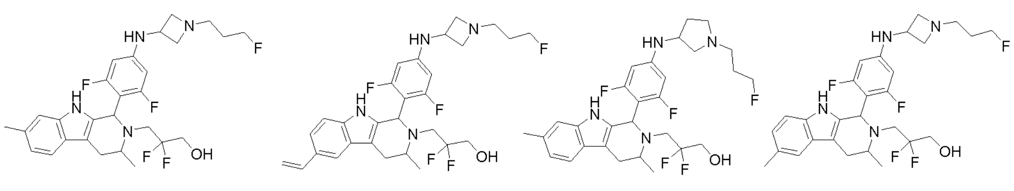

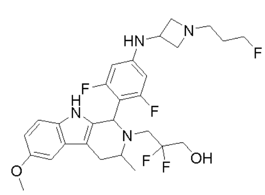

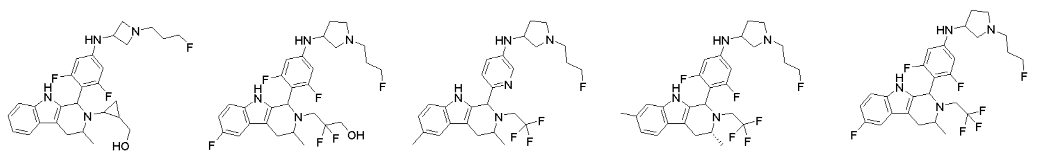

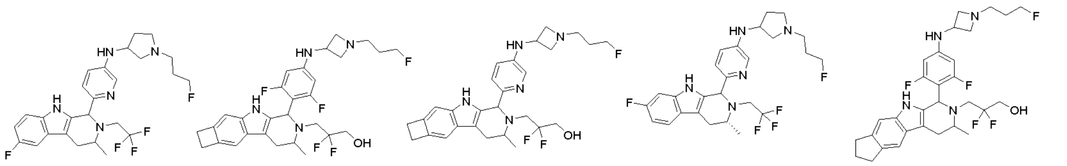

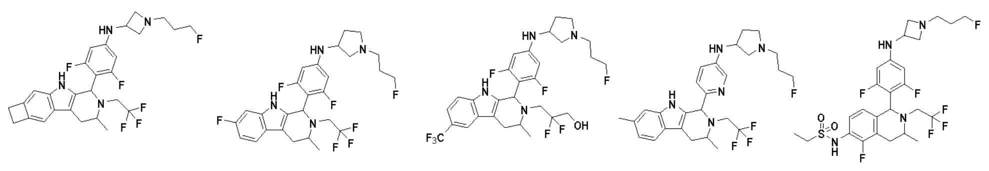

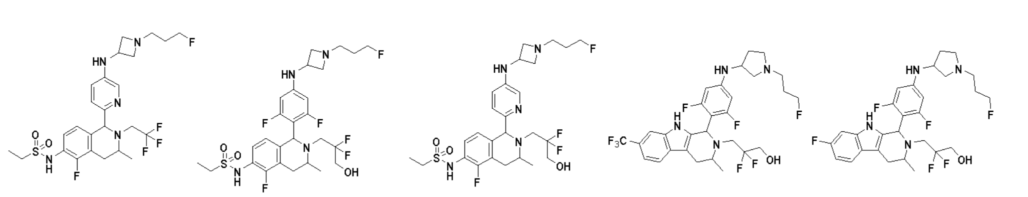

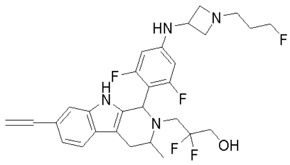
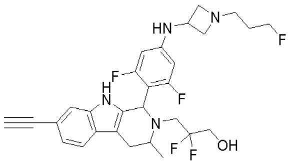

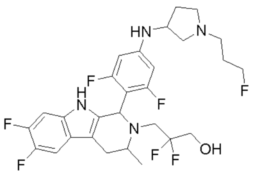
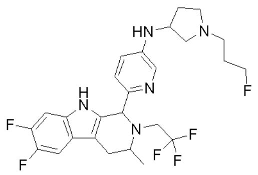
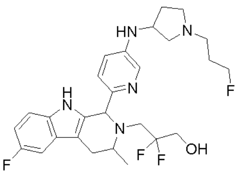
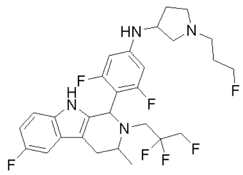

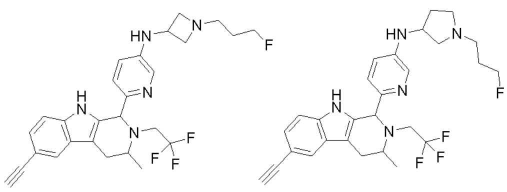
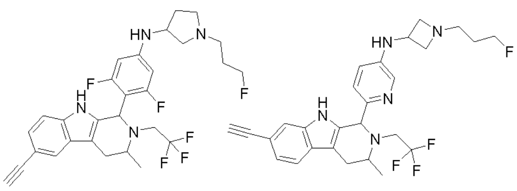

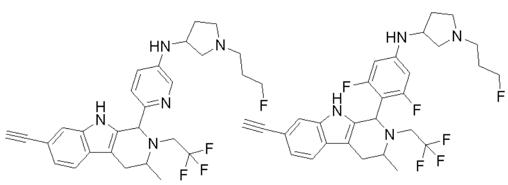

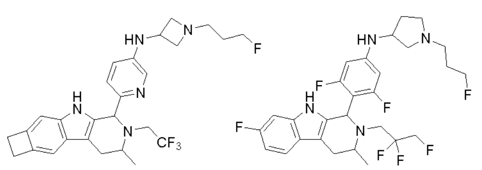
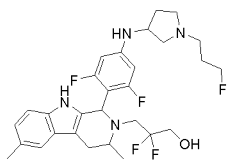
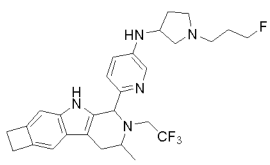

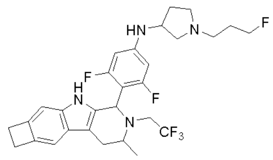
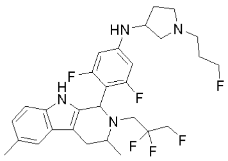

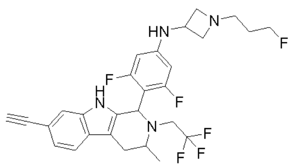
.

More specifically, as the fifteenth technical solution of the present invention, provided is a compound as represented by formula (I), (I'), (II), (II'), (II'-1), (III'-1), (III'), (III), (II'-a), (II'-a-1), (II'-1a) or (II'-1a-1), or the stereoisomer, solvate, deuterated compound, or pharmaceutically acceptable salt thereof, wherein the compound is selected from one of the following structures:

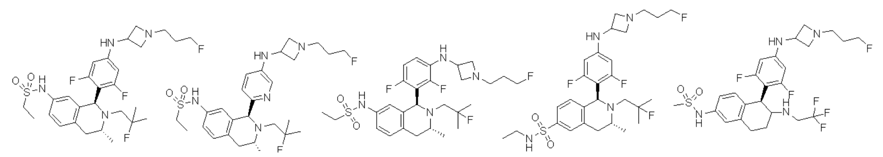

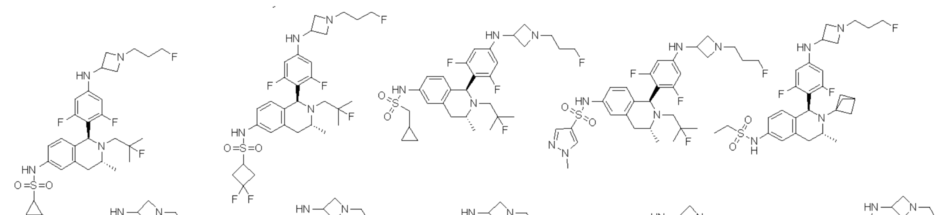

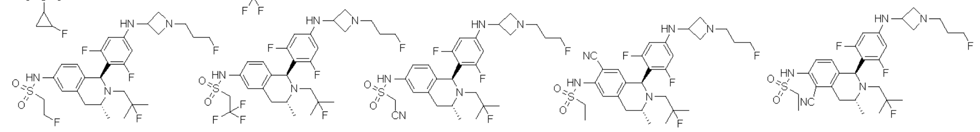

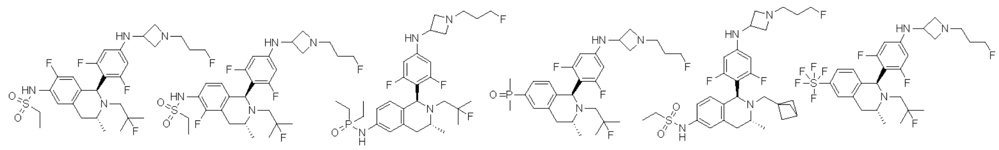

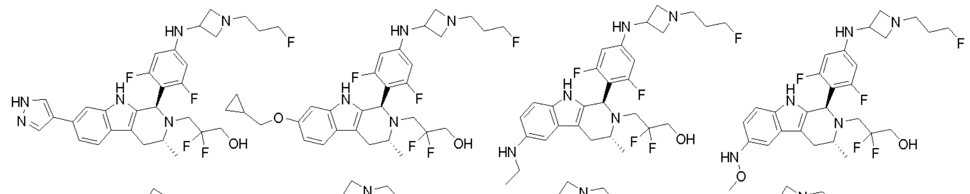

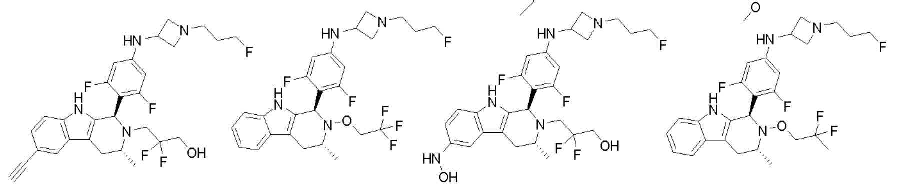

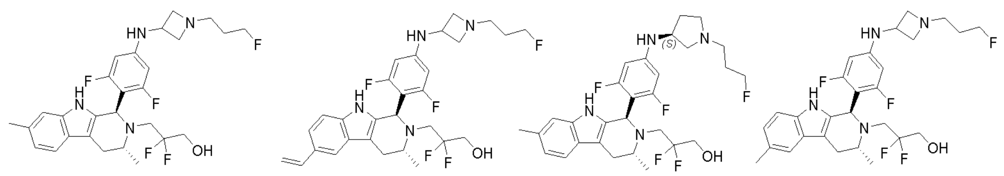

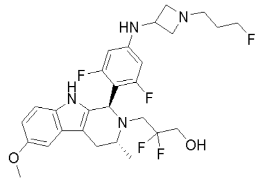

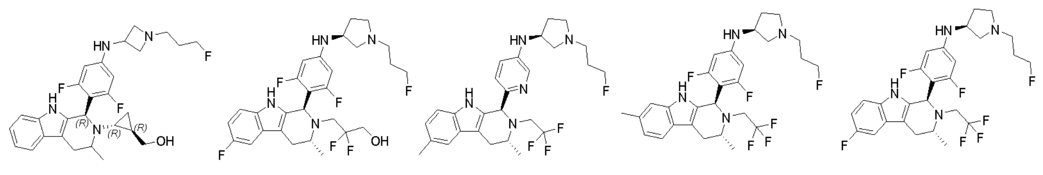

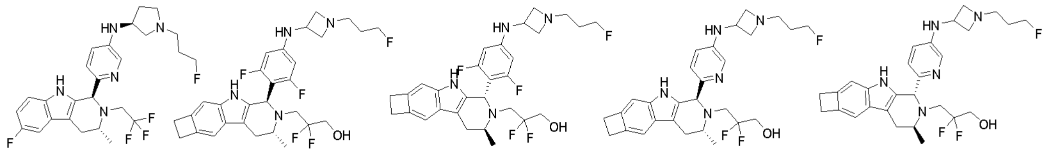

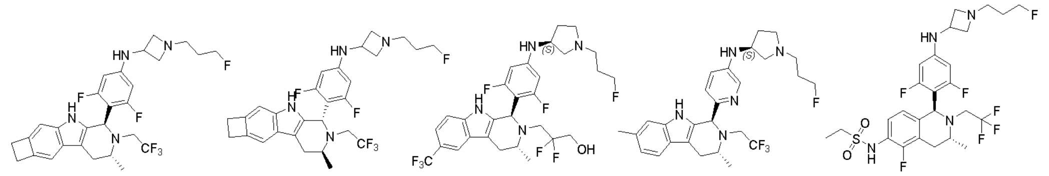

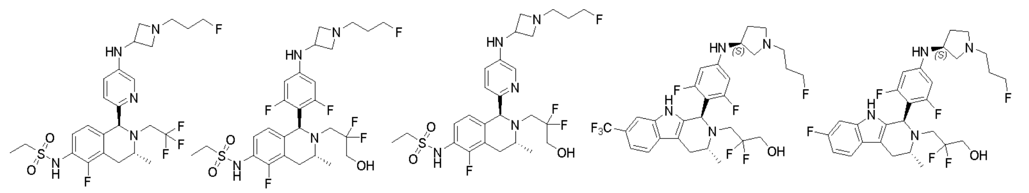

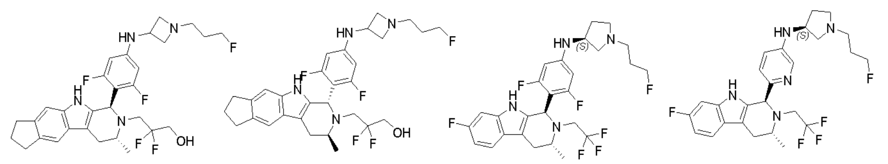

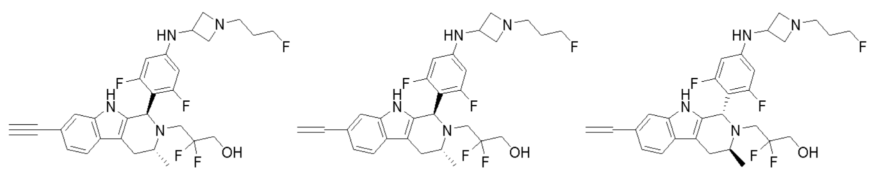

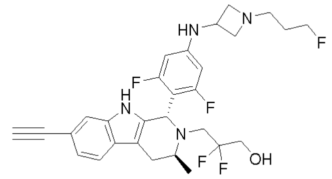

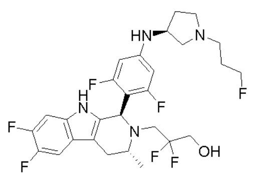

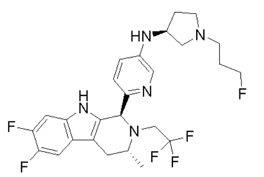

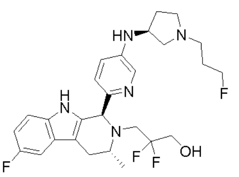

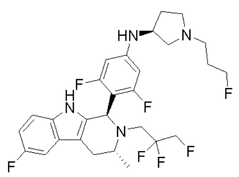

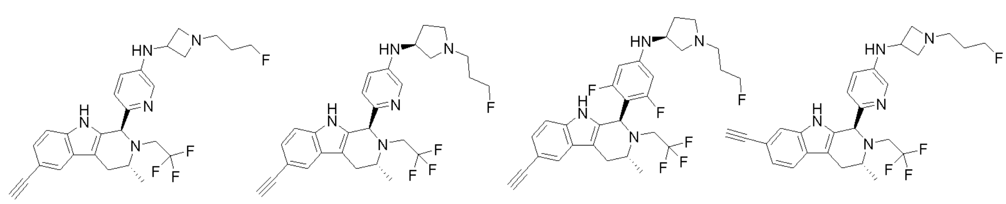

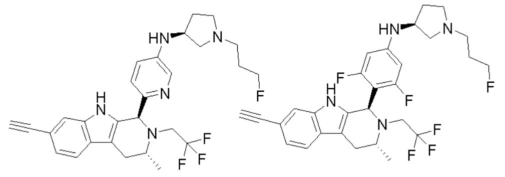

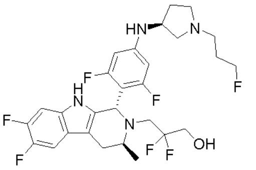
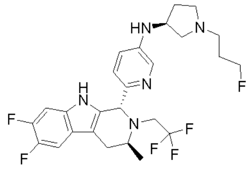
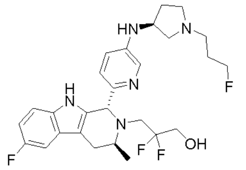
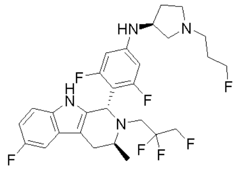

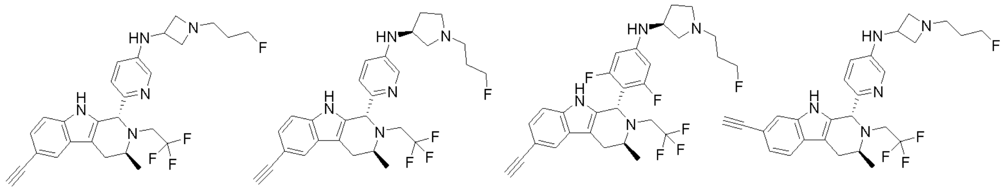

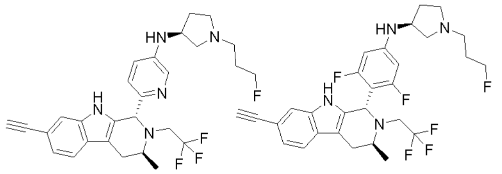

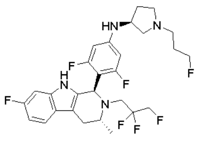
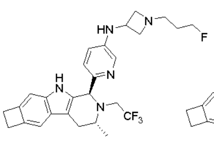
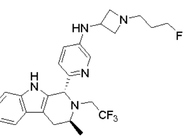
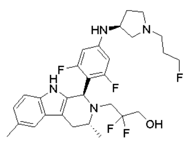
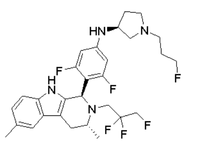

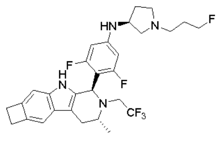
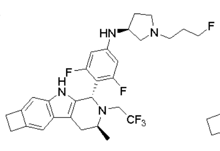
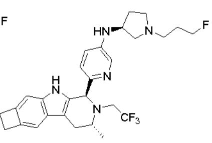
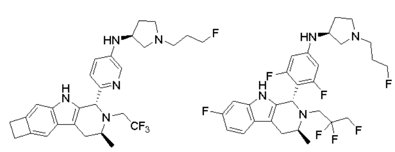

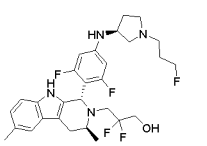
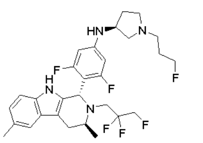

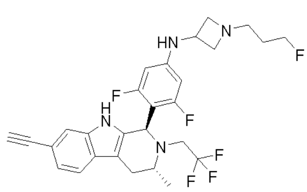
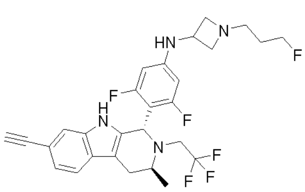
.

The present invention further provides a pharmaceutical composition comprising the compound, or the stereoisomer, solvate, deuterated compound, or pharmaceutically acceptable salt thereof according to any one of the preceding first to fifteenth technical solutions, and a pharmaceutically acceptable adjuvant and/or carrier.

Furthermore, the pharmaceutical composition comprises 1-600 mg of the compound, or the stereoisomer,

deuterated compound, solvate, or pharmaceutically acceptable salt thereof according to any one of the preceding technical solutions, and a pharmaceutically acceptable adjuvant and/or carrier.

Furthermore, the present invention further provides the use of the compound, or the stereoisomer, solvate, deuterated compound, or pharmaceutically acceptable salt thereof according to any one of the preceding embodiments, or the pharmaceutical composition containing same, in the preparation of a drug for treating/preventing an ER-mediated disease.

Furthermore, the ER-mediated disease includes, but is not limited to, breast cancer, ovarian cancer, uterine cancer or cervical cancer.

The present invention relates to a pharmaceutical composition or pharmaceutical preparation, wherein the pharmaceutical composition or pharmaceutical preparation comprises a therapeutically effective amount of the compound, or the stereoisomer, solvate, deuterated compound, or pharmaceutically acceptable salt thereof described in the present invention, and a pharmaceutically acceptable adjuvant and/or carrier. The pharmaceutical composition may be in the form of a unit preparation (the amount of the main drug in a unit preparation is also referred to as "preparation specification").

The present invention further provides a method for treating diseases in mammals, which method comprises administering to a subject a therapeutically effective amount of the compound, or the stereoisomer, solvate, deuterated compound, or pharmaceutically acceptable salt thereof according to any one of the previous technical solutions, and a pharmaceutically acceptable adjuvant and/or carrier, wherein the therapeutically effective amount is preferably 1-600 mg; preferably, the disease is an ER-mediated disease, and more preferably, the disease is breast cancer, ovarian cancer, uterine cancer or cervical cancer.

The present invention further provides a method for treating diseases in mammals, which method comprises administering to the mammals a therapeutically effective amount of the compound, or the stereoisomer, deuterated compound, solvate, pharmaceutically acceptable salt or pharmaceutical composition thereof described in the present invention. In some embodiments, the mammals described in the present invention include human.

The "effective amount" or "therapeutically effective amount" as described in the present application refers to administration of a sufficient amount of the compound disclosed in the present application that will alleviate to some extent one or more symptoms of the diseases or conditions being treated (e.g., cancer). In some embodiments, the result is reduction and/or alleviation of signs, symptoms, or causes of the disease, or any other desired change in a biological system. For example, the "effective amount" for therapeutic use is the amount of the composition comprising the compound disclosed in the present application that is required to provide a clinically significant reduction in disease symptoms. Examples of therapeutically effective amounts include, but are not limited to, 1-600 mg, 2-600 mg, 3-600 mg, 4-600 mg, 5-600 mg, 6-600 mg, 10-600 mg, 20-600 mg, 25-600 mg, 30-600 mg, 40-600 mg, 50-600 mg, 60-600 mg, 70-600 mg, 75-600 mg, 80-600 mg, 90-600 mg, 100-600 mg, 200-600 mg, 1-500 mg, 2-500 mg, 3-500 mg, 4-500 mg, 5-500 mg, 6-500 mg, 10-500 mg, 20-500 mg, 25-500 mg, 30-500 mg, 40-500 mg, 50-500 mg, 60-500 mg, 70-500 mg, 75-500 mg, 80-500 mg, 90-500 mg, 100-500 mg, 125-500 mg, 150-500 mg, 200-500 mg, 250-500 mg, 300-500 mg, 400-500 mg, 5-400 mg, 10-400 mg, 20-400 mg, 25-400 mg, 30-400 mg, 40-400 mg, 50-400 mg, 60-400 mg, 70-400 mg, 75-400 mg, 80-400 mg, 90-400 mg, 100-400 mg, 125-400 mg, 150-400 mg, 200-400 mg, 250-400 mg, 300-400 mg, 1-300 mg, 2-300 mg, 5-300 mg, 10-300 mg, 20-300 mg, 25-300 mg, 30-300 mg, 40-300 mg, 50-300 mg, 60-300 mg, 70-300 mg, 75-300 mg, 80-300 mg, 90-300 mg, 100-300 mg, 125-300 mg, 150-300 mg, 200-300 mg, 250-300 mg, 1-200 mg, 2-200 mg, 5-200 mg, 10-200 mg, 20-200 mg, 25-200 mg, 30-200 mg, 40-200 mg, 50-200 mg, 60-200 mg, 70-200 mg, 75-200 mg, 80-200 mg, 90-200 mg, 100-200 mg, 125-200 mg, and 150-200 mg.

The present invention relates to a pharmaceutical composition or pharmaceutical preparation, wherein the pharmaceutical composition or pharmaceutical preparation comprises a therapeutically effective amount of the compound, or the stereoisomer, deuterated compound, solvate, or pharmaceutically acceptable salt thereof described in the present invention, and an adjuvant and/or a carrier. The pharmaceutical composition may be in the form of a unit preparation (the amount of the main drug in a unit preparation is also referred to as "preparation specification"). In some embodiments, the pharmaceutical composition comprises the compound, or the stereoisomer, deuterated compound, solvate, or pharmaceutically acceptable salt thereof of the present invention in an amount including not limited to 1 mg, 1.25 mg, 2.5 mg, 5 mg, 10 mg, 12.5 mg, 15 mg, 20 mg, 25 mg, 30 mg, 35 mg, 40 mg, 45 mg, 50 mg, 60 mg, 70 mg, 80 mg, 90 mg, 100 mg, 120 mg, 125 mg, 150 mg, 200 mg, 250 mg, 300 mg, 400 mg, 500 mg, and 600 mg.

Provided is a method for treating a disease in mammals, the method comprising administering to a subject a therapeutically effective amount of the compound, or the stereoisomer, deuterated compound, solvate, or pharmaceutically acceptable salt thereof of the present invention, wherein the therapeutically effective amount is preferably 1-600 mg, and the disease is preferably breast cancer, ovarian cancer, uterine cancer or cervical cancer.

Provided is a method for treating a disease in mammals, the method comprising administering to a subject the pharmaceutical compound, or the stereoisomer, deuterated compound, solvate, or pharmaceutically acceptable salt thereof of the present invention at a daily dose of 1-600 mg/day, wherein the daily dose may be a single dose or divided doses. In some embodiments, the daily dose includes, but is not limited to: 1-600 mg/day, 1-500 mg/day, 1-400 mg/day,

1-300 mg/day, 1-250 mg/day, 1-200 mg/day, 1-150 mg/day, 1-125 mg/day, 1-100 mg/day, 1-80 mg/day, 1-60 mg/day, 1-40 mg/day, 1-20 mg/day, 5-600 mg/day, 5-500 mg/day, 5-400 mg/day, 5-300 mg/day, 5-250 mg/day, 5-200 mg/day, 5-150 mg/day, 5-125 mg/day, 5-100 mg/day, 5-80 mg/day, 5-60 mg/day, 5-40 mg/day, 5-20 mg/day, 5-90 mg/day, 5-70 mg/day, 5-50 mg/day, 5-30 mg/day, 10-600 mg/day, 10-500 mg/day, 10-400 mg/day, 10-300 mg/day, 10-250 mg/day, 10-200 mg/day, 10-150 mg/day, 10-125 mg/day, 10-100 mg/day, 10-80 mg/day, 10-60 mg/day, 10-40 mg/day, 10-20 mg/day, 10-90 mg/day, 10-70 mg/day, 10-50 mg/day, 10-30 mg/day, 20-600 mg/day, 20-500 mg/day, 20-400 mg/day, 20-300 mg/day, 20-200 mg/day, 20-100 mg/day, 20-80 mg/day, 20-60 mg/day, 20-40 mg/day, 50-600 mg/day, 50-500 mg/day, 50-450 mg/day, 50-400 mg/day, 50-350 mg/day, 50-50-300 mg/day, 50-250 mg/day, 50-200 mg/day, 50-150 mg/day, 50-100 mg/day, 100-600 mg/day, 100-500 mg/day, 100-400 mg/day, 100-300 mg/day, or 100-200 mg/day.

In some embodiments, the daily dose includes, but is not limited to: 1 mg/day, 2.5 mg/day, 5 mg/day, 10 mg/day, 12.5 mg/day, 15 mg/day, 20 mg/day, 25 mg/day, 30 mg/day, 35 mg/day, 40 mg/day, 45 mg/day, 50 mg/day, 60 mg/day, 70 mg/day, 80 mg/day, 90 mg/day, 100 mg/day, 120 mg/day, 150 mg/day, 200 mg/day, 250 mg/day, 300 mg/day, 400 mg/day, 500 mg/day, and 600 mg/day.

The present invention relates to a kit, which may include a composition in single-dose or multiple-dose form, wherein the kit comprises the compound, or the stereoisomer, deuterated compound, solvate, or pharmaceutically acceptable salt thereof of the present invention, and the amount of the compound, or the stereoisomer, deuterated compound, solvate, or pharmaceutically acceptable salt thereof of the present invention is the same as that in the above-mentioned pharmaceutical composition.

The amount of the compound, or the stereoisomer, deuterated compound, solvate, or pharmaceutically acceptable salt thereof of the present invention is converted in the form of free bases in each case.

"Preparation specification" refers to the weight of the main drug contained in each tube, tablet or other unit preparation.

**Synthetic route**

Those skilled in the art would have been able to prepare the compounds of the present invention according to known organic synthesis techniques, and the starting materials used therein are commercially available chemicals and (or) compounds described in chemical documents. "Commercially available chemicals" are obtained from regular commercial sources, and suppliers include: Titan Technology Co., Ltd., Energy Chemical Co., Ltd., Shanghai Demo Co., Ltd., Chengdu Kelong Chemical Co., Ltd., Accela ChemBio Co., Ltd., PharmaBlock Sciences (Nanjing), Inc., WuXi Apptec Co., Ltd., J&K Scientific Co., Ltd., etc.

References and monographs in the art introduce in detail the synthesis of reactants that can be used to prepare the compounds described herein, or provide articles describing the preparation method for reference. The references and monographs include: "Synthetic Organic Chemistry", John Wiley & Sons, Inc., New York; S. R. Sandler et al., "Organic Functional Group Preparations," 2nd Ed., Academic Press, New York, 1983; H. O. House, "Modern Synthetic Reactions", 2nd Ed., W. A. Benjamin, Inc. Menlo Park, Calif. 1972; T. L. Gilchrist, "Heterocyclic Chemistry", 2nd Ed., John Wiley & Sons, New York, 1992; J. March, "Advanced Organic Chemistry: Reactions, Mechanisms and Structure", 4th Ed., Wiley-Interscience, New York, 1992; Fuhrhop, J. and Penzlin G. "Organic Synthesis: Concepts, Methods, Starting Materials", Second, Revised and Enlarged Edition (1994) John Wiley & Sons ISBN: 3-527-29074-5; Hoffman, R.V. "Organic Chemistry, An Intermediate Text" (1996) Oxford University Press, ISBN 0-19-509618-5; Larock, R. C. "Comprehensive Organic Transformations: A Guide to Functional Group Preparations" 2nd Edition (1999) Wiley-VCH, ISBN: 0-471-19031-4; March, J. "Advanced Organic Chemistry: Reactions, Mechanisms, and Structure" 4th Edition (1992) John Wiley & Sons, ISBN: 0-471-60180-2; Otera, J. (editor) "Modern Carbonyl Chemistry" (2000) Wiley-VCH, ISBN: 3-527-29871-1; Patai, S. "Patai's 1992 Guide to the Chemistry of Functional Groups" (1992) Interscience ISBN: 0-471-93022-9; Solomons, T. W. G. "Organic Chemistry" 7th Edition (2000) John Wiley & Sons, ISBN: 0-471-19095-0; Stowell, J.C., "Intermediate Organic Chemistry" 2nd Edition (1993) Wiley-Interscience, ISBN: 0-471-57456-2; "Industrial Organic Chemicals: Starting Materials and Intermediates: An Ullmann's Encyclopedia" (1999) John Wiley & Sons, ISBN: 3-527-29645-X, in 8 volumes; "Organic Reactions" (1942-2000) John Wiley & Sons, in over 55 volumes; and "Chemistry of Functional Groups" John Wiley & Sons, in 73 volumes.

Specific and similar reactants can be selectively identified by the indexes of known chemicals prepared by the Chemical Abstracts Service of the American Chemical Society, wherein the indexes are available in most public libraries or university libraries and online. Chemicals that are known but not commercially available in the catalog are optionally prepared by custom chemical synthesis plants, wherein many of standard chemical supply plants (for example, those listed above) provide custom synthesis services. Reference document for the preparation and selection of the pharmaceutically acceptable salts of the compounds described herein is P. H. Stahl & C. G. Wermuth "Handbook of Pharmaceutical Salts", Verlag Helvetica Chimica Acta, Zurich, 2002.

**Term**

Unless otherwise specified, the terms of the present invention have the following meanings.

The carbon, hydrogen, oxygen, sulfur, nitrogen and halogen involved in the groups and compounds of the present invention all comprise isotopes thereof, and are optionally further substituted with one or more of the corresponding isotopes thereof, wherein the isotopes of carbon comprise $^{12}C$, $^{13}C$ and $^{14}C$; the isotopes of hydrogen comprise protium (H), deuterium (deuterium, also known as heavy hydrogen), and tritium (T, also known as superheavy hydrogen); the isotopes of oxygen comprise $^{16}O$, $^{17}O$ and $^{18}O$; the isotopes of sulfur comprise $^{32}S$, $^{33}S$, $^{34}S$ and $^{36}S$; the isotopes of nitrogen comprise $^{14}N$ and $^{15}N$; the isotope of fluorine comprises $^{19}F$; the isotopes of chlorine comprise $^{35}Cl$ and $^{37}Cl$; and the isotopes of bromine comprise $^{79}Br$ and $^{81}Br$.

The term "halogen" herein refers to F, Cl, Br, I, or isotopes thereof.

The term "halo" or "substituted with halogen" refers to being substituted with one or more groups selected from F, Cl, Br, I, or isotopes thereof, wherein the upper limit of the number of halogen substituents is equal to the sum of the number of hydrogens that can be substituted in the group to be substituted. Without particular limitation, the number of halogen substituents is any integer between 1 and the upper limit, and when the number of halogen substituents is greater than 1, the group to be substituted can be substituted with the same or different halogen. Generally, the circumstances of being substituted with 1-5 halogen, 1-3 halogen, 1-2 halogen, and 1 halogen are included.

The term "deuterium" refers to the isotope deuterium of hydrogen (H).

The term "deuterated" or "deuterated compound" refers to the case where a hydrogen atom on a group, such as alkyl, cycloalkyl, alkylene, aryl, heteroaryl, mercapto, heterocycloalkyl, alkenyl and alkynyl is substituted with at least one deuterium atom, wherein the upper limit of the number of deuterium substituents is equal to the sum of the number of hydrogens that can be substituted in the group to be substituted. Without particular limitation, the number of deuterium substituents is any integer between 1 and the upper limit, for example, 1-20 deuterium atoms, 1-10 deuterium atoms, 1-6 deuterium atoms, 1-3 deuterium atoms, 1-2 deuterium atoms or 1 deuterium atom.

Group "$C_{x-y}$" refers to a group comprising x to y carbon atoms, for example, "$C_{1-6}$ alkyl" refers to alkyl comprising 1-6 carbon atoms.

"Alkyl" refers to a monovalent linear or branched saturated aliphatic hydrocarbon group, usually an alkyl group with 1 to 20 carbon atoms, or an alkyl group with 1 to 8 carbon atoms, or an alkyl group with 1 to 6 carbon atoms, or an alkyl group with 1 to 4 carbon atoms. Non-limiting examples of alkyl include methyl, ethyl, n-propyl, isopropyl, n-butyl, sec-butyl, neobutyl, tert-butyl, n-pentyl, isoamyl, neopentyl, n-hexyl, etc., and alkyl may be further substituted with a substituent.

The term "haloalkyl" refers to an alkyl group in which one or more hydrogens are substituted with one or more halogen atoms (e.g., fluorine, chlorine, bromine, iodine, or isotopes thereof), wherein the upper limit of the number of halogen substituents is equal to the sum of the number of hydrogens that can be substituted in the alkyl group. Without particular limitation, the number of halogen substituents is any integer between 1 and the upper limit. Generally, the alkyl group is substituted with 1-5 halogen, 1-3 halogen, 1-2 halogen or 1 halogen; and when the number of halogen substituents is greater than 1, the group to be substituted can be substituted with the same or different halogen. specific examples include, but are not limited to, $-CF_3$, $-CH_2Cl$, $-CH_2CF_3$, $-CCl_2$, $CF_3$, etc.

The term "alkoxy" or "alkyloxy" refers to -O-alkyl. such as -O-$C_{1-8}$ alkyl, -O-$C_{1-6}$ alkyl, -O-$C_{1-4}$ alkyl or -O-$C_{1-2}$ alkyl. Non-limiting and specific examples of alkoxy or alkyloxy include methoxy, ethoxy, n-propoxy, isopropoxy, n-butoxy, secbutoxy, tert-butoxy, n-pentoxy, n-hexyloxy, cyclopropoxy, cyclobutoxy, etc. The alkoxy may be optionally substituted with a substituent.

The term "haloalkoxy" refers to -O-haloalkyl. such as -O-halo $C_{1-8}$ alkyl, -O-halo $C_{1-6}$ alkyl, -O-halo $C_{1-4}$ alkyl or -O-halo $C_{1-2}$ alkyl; and the upper limit of the number of halogen substituents is equal to the sum of the number of hydrogens that can be substituted in the group to be substituted. Without particular limitation, the number of halogen substituents is any integer between 1 and the upper limit, preferably 1-5 halogen, 1-3 halogen, 1-2 halogen, and 1 halogen; and when the number of halogen substituents is greater than 1, the group to be substituted can be substituted with the same or different halogen. Non-limiting examples of haloalkoxy include monofluoromethoxy, difluoromethoxy, trifluoromethoxy, difluoroethyloxy, *etc.*

The term "alkenyl" refers to a straight or branched hydrocarbon group comprising at least one carbon-carbon double bond (C=C) and generally comprises 2 to 18 carbon atoms, such as 2 to 8 carbon atoms, further such as 2 to 6 carbon atoms, and more further such as 2 to 4 carbon atoms. Examples of alkenyl include, but are not limited to, vinyl, allyl, 1-propenyl, 2-propenyl, 1-butenyl, 2-butenyl, 3-butenyl, 1-pentenyl, 2-pentenyl, 3-pentenyl, 4-pentenyl, 1-methyl-1-butenyl, 2-methyl-1-butenyl, 2-methyl-3-butenyl, 1-hexenyl, 2-hexenyl, 3-hexenyl, 4-hexenyl, 5-hexenyl, 1-methyl-1-pentenyl, 2-methyl-1-pentenyl, 1-heptenyl, 2-heptenyl, 3-heptenyl, 4-heptenyl, 1-octenyl, 3-octenyl, 1-nonenyl, 3-nonenyl, 1-decenyl, 4-decenyl, 1,3-butadiene, 1,3-pentadiene, 1,4-pentadiene, 1,4-hexadiene, etc.; and the alkenyl may further be optionally substituted with a substituent.

The term "alkynyl" refers to a straight or branched hydrocarbon group containing at least one carbon-carbon

triple bond (C=C) and generally comprises 2 to 18 carbon atoms; further, alkynyl comprises 2 to 8 carbon atoms; further, alkynyl comprises 2 to 6 carbon atoms, and more further, alkynyl comprises 2 to 4 carbon atoms. Examples of alkynyl include, but are not limited to ethynyl, 1-propynyl, 2-propynyl, butynyl, 2-butynyl, 3-butynyl, 1-methyl-2-propynyl, 4-pentynyl, 3-pentynyl, 1-methyl-2-butynyl, 2-hexynyl, 3-hexynyl, 2-heptynyl, 3-heptynyl, 4-heptynyl, 3-octynyl, 3-nonynyl, 4-decynyl, etc.; and the alkynyl may be optionally substituted with a substituent.

The term "cycloalkyl" refers to a saturated or partially unsaturated, non-aromatic carbocyclic hydrocarbon group containing no ring heteroatoms. The cycloalkyl may be monocyclic, bicyclic or polycyclic, the bicyclic or polycyclic cycloalkyl may be in the form of a fused ring, a spiro ring, a bridged ring or a combination thereof, and may comprise one or more aryl rings, but the ring system is non-aromatic as a whole, and the connecting site may be on an aromatic ring or a non-aromatic ring. Generally, the cycloalkyl contains 3 to 20 carbon atoms; further, the cycloalkyl contains 3-8 carbon atoms; and still further, the cycloalkyl contains 3-6 carbon atoms; when the cycloalkyl is monocyclic cycloalkyl, the cycloalkyl contains 3-15 carbon atoms, or 3-10 carbon atoms, or 3-8 carbon atoms, or 3-6 carbon atoms; when the cycloalkyl is bicyclic or polycyclic cycloalkyl, the cycloalkyl contains 5-12 carbon atoms, or 5-11 carbon atoms, or 6-10 carbon atoms; and non-limiting examples of cycloalkyl include cyclopropyl, cyclobutyl, cyclopentyl, cyclohexyl, cycloheptyl, butenyl, cyclopentenyl, cyclohexenyl,

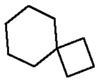

, 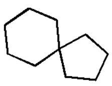, 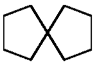, 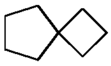, 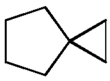, 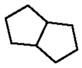, 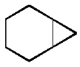, 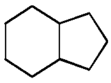, 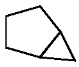, 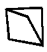, 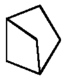,

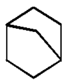, 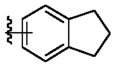,

etc., and cycloalkyl may be optionally substituted with a substituent.

"Aryl" refers to an aromatic carbocyclic ring that does not contain heteroatoms, including monocyclic aryl and fused aryl. Typically, it contains 6 to 13 carbon atoms, and further contains 6 to 9 carbon atoms, and it is further phenyl. The non-limiting examples thereof include phenyl, naphthyl, anthryl, and phenanthryl. The aryl may be optionally substituted with a substituent.

"Carbocyclic ring" or "carbocyclyl" refers to a saturated, partially unsaturated, or aromatic carbocyclic ring, and its meaning includes aryl and cycloalkyl. The carbocyclic ring can be a single ring, a bicyclic ring or a polycyclic ring, and the bicyclic ring or polycyclic ring includes bridged rings, fused rings, spiro rings and combinations thereof. Carbocyclic ring typically has 3 to 12 carbon atoms, or 3-10 carbon atoms, or 3-6 carbon atoms. In non-limiting embodiments, a monocyclic carbocyclic ring includes cyclopropyl, cyclobutyl, cyclopentyl, cyclohexyl, cycloheptyl or phenyl, *etc*., a bicyclic bridged ring includes

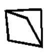, 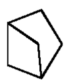, 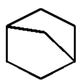,

*etc.,* a bicyclic fused ring includes

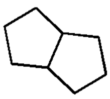, 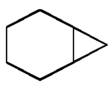, 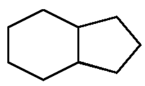, 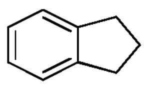,

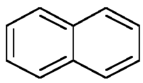 and 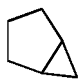,

*etc*., and a bicyclic spiro ring includes

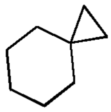, 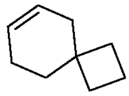,

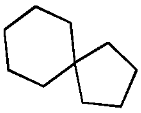, 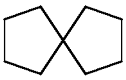, 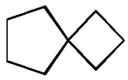 and 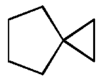,

*etc.* and carbocyclic ring may be optionally substituted with a substituent.

"Heterocycloalkyl" refers to a saturated or partially unsaturated non-aromatic carbocyclic ring containing 1, 2, 3, or 4 heteroatoms selected from N, S, or O. The heterocycloalkyl may be monocyclic, bicyclic or polycyclic, the bicyclic or polycyclic heterocycloalkyl may be in the form of a bridged ring, a fused ring, a spiro ring or a combination thereof, and may comprise one or more aromatic rings or heteroaromatic rings, but the ring system is non-aromatic as a whole, and the connecting site may be on an aromatic ring or a non-aromatic ring. Usually the heterocycloalkyl group is a 3-20-membered ring. When it is a monocyclic heterocycloalkyl group, it is usually a 3- to 15-membered ring, or a 3- to 10-membered ring, or a 3-8-membered ring, or a 3-6-membered ring; when it is a bicyclic or polycyclic heterocycloalkyl group, it is usually a 5-12 membered ring, a 5-11 membered ring, or a 6-9 membered ring. The heteroatoms N and S include their oxidation states. Non-limiting examples of heterocycloalkyl include azetidinyl, morpholinyl, piperazinyl, piperidyl, tetrahydropyranyl, oxetanyl, pyranyl, azacyclopentenyl, azacyclohexenyl, oxacyclopentenyl, oxacyclohexenyl, etc., and heterocycloalkyl may be optionally substituted with a substituent.

"Heteroaromatic ring" or "heteroaryl", unless otherwise specified, refers to an aromatic ring containing 1 to 4 heteroatoms selected from N, O or S and their oxidation states, which can be monocyclic, bicyclic, or polycyclic, wherein the bicyclic or polycyclic heteroaromatic ring or heteroaryl can be bridged rings, fused rings, spiro rings and combinations thereof. Bicyclic or polycyclic heteroaromatic ring or heteroaryl can be formed by fusion of heteroaryl to aryl, or of heteroaryl to heteroaryl, wherein the heteroaryl or aryl may be the connection site. Non-limiting examples of heteroaromatic ring or heteroaryl include furyl, thienyl, pyrrolyl, oxazolyl, thiazolyl, imidazolyl, pyrazolyl, pyridyl, pyrimidinyl, pyridazinyl, pyrazinyl, indolyl, purinyl,

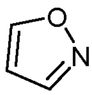, 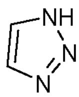, 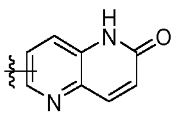, 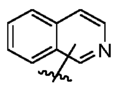, 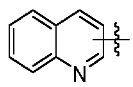,

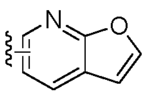, 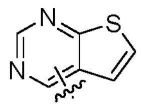, 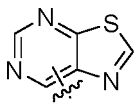, 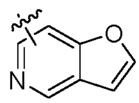,

etc. The heteroaryl can be optionally substituted with a substituent.

The definition of "aromatic ring" includes aryl and heteroaryl groups, and the aromatic ring may be optionally substituted with a substituent.

"Heterocycle" or "heterocyclyl" refers to a saturated or unsaturated, aromatic or non-aromatic ring containing 1 to 4 heteroatoms selected from N, O or S and their oxidation states, and its meaning includes heteroaryl and heterocycloalkyl. Heterocycles include monocyclic heterocycles, bicyclic bridged heterocycles, bicyclic fused heterocycles and bicyclic spiroheterocycles or combinations thereof. The heterocycle is usually a 3- to 12-membered heterocycle, a 5- to 12-membered heterocycle, or a 5- to 7-membered heterocycle. Heterocyclyl can be connected to a heteroatom or carbon atom. Non-limiting examples include oxiranyl, azacyclopropyl, oxetanyl, azetidinyl, 1,3-dioxolanyl, 1,4-dioxolanyl, 1,3-dioxanyl, piperazinyl, azacycloheptyl, pyridyl, furanyl, thienyl, pyranyl, N-alkylpyrrolyl, pyrimidinyl, pyrazinyl, pyrazolyl, pyridazinyl, imidazolyl, piperidyl, piperadinyl, morpholinyl, thiomorpholinyl, 1,3-dithianyl, dihydrofuranyl, dihydropyranyl, dithiolanyl, tetrahydrofuryl, tetrahydropyrrolyl, tetrahydroimidazolyl, oxazolyl, dihydrooxazolyl, tetrahydrooxazolyl, tetrahydrothiazolyl, tetrahydropyranyl, benzimidazolyl, benzopyridyl, pyrrolopyridyl, benzodihydrofuranyl, azabicyclo[3.2.1]octyl, azabicyclo[5.2.0]nonyl, oxatricyclic[5.3.1.1]dodecyl, azaadamantyl and oxaspiro[3.3]heptyl,

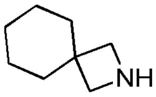, 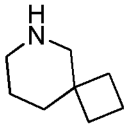, 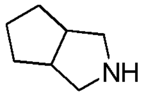,

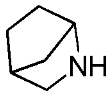, 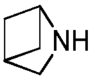, 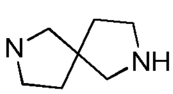, 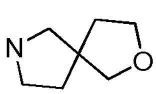, 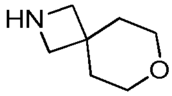, 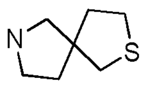,

*etc.*, and the heterocycle may be optionally substituted with a substituent.

The term "heterocyclene" refers to a substituted or unsubstituted, saturated or unsaturated, aromatic or non-aromatic, divalent heterocyclyl group. Non-limiting examples of heterocyclene include

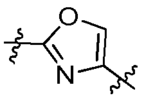, 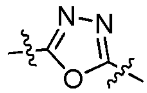, 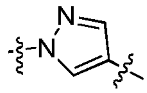,

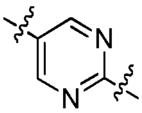, 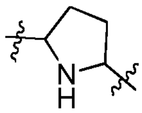,

*etc.*

The term "spiro ring" refers to a polycyclic group sharing one carbon atom (referred to as a spiro atom) between rings, which may contain 0 or at least 1 double bond or triple bond, and may contain 0 to 5 heteroatoms selected from N, O, S, P, Si and an oxidation state thereof. Generally, a spiro ring is a 6- to 14-membered ring, or a 6- to 12-membered ring, or a 6- to 10-membered ring. Generally, a spiro ring is a spiro ring formed by a three-membered ring and a three-membered ring, a three-membered ring and a four-membered ring, a three-membered ring and a five-membered ring, a three-membered ring and a six-membered ring, a four-membered ring and a four-membered ring, a four-membered ring and a five-membered ring, a four-membered ring and a six-membered ring, a five-membered ring and a five-membered ring or a five-membered ring and a six-membered ring. Non-limiting examples of spiro ring include:

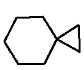 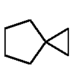 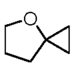 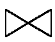 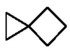 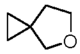 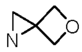 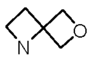 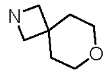

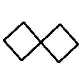 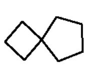 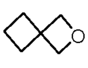 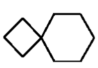 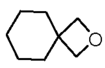 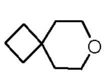 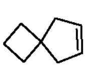 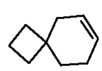

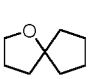 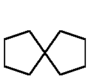 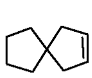 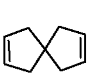 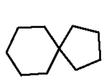 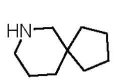 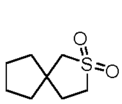 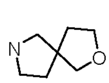

, and the spiro ring may be optionally substituted with a substituent.

The term "fused ring" refers to a polycyclic group in which the rings share two adjacent ring atoms and one chemical bond, and may contain one or more double or triple bonds and a fused ring may contain 0 to 5 heteroatoms selected from N, S, O, P, Si and an oxidation state thereof. Generally, a fused ring is a 5- to 20-membered ring, or a 5- to 14-membered ring, or a 5- to 12-membered ring or a 5- to 10-membered ring. Generally, a fused ring is in the form of a three-membered ring fused a four-membered ring (indicating a fused ring formed by a three-membered ring and a four-membered ring, and either the three-membered ring or the four-membered ring may be possibly used as the basic ring according to the IUPC nomenclature; similarly hereinafter), a three-membered ring fused a five-membered ring, a three-membered ring fused a six-membered ring, a four-membered ring fused a four-membered ring, a four-membered ring fused a five-membered ring, a four-membered ring fused a six-membered ring, a five-membered ring fused a five-membered ring, a five-membered ring fused a six-membered ring, and a six-membered ring fused a six-membered ring.

Non-limiting examples of fused ring include purine, quinoline, isoquinoline, benzopyran, benzofuran, benzothiophene,

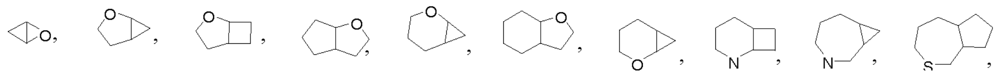

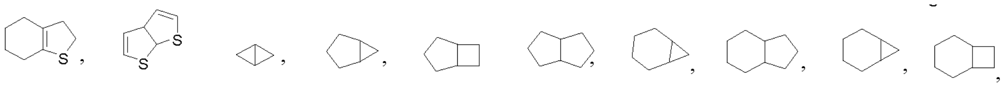

and

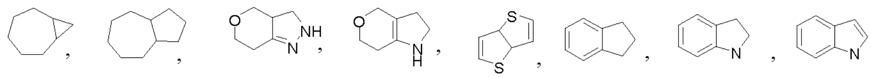

; and the fused ring may be optionally substituted with a substituent.

The term "bridged ring" refers to a ring system in which two non-adjacent ring atoms are shared between two rings and a bridged ring may contain 1 or more double or triple bonds. The bridged ring may contain 0 to 5 heteroatoms selected from N, S, O, P, Si and an oxidation state thereof. Generally, the bridged ring has 5 to 20, 5 to 14, 5 to 12, or 5 to 10 ring atoms. Non-limiting examples of bridged ring include adamantane,

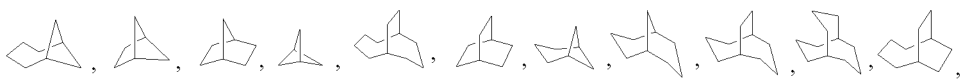

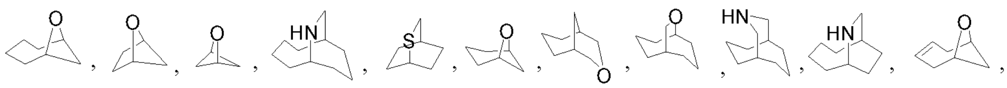

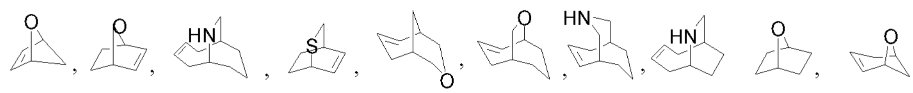

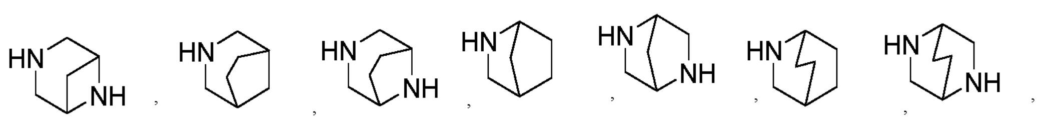

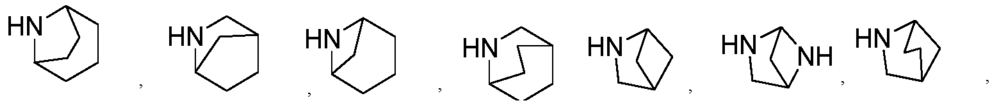

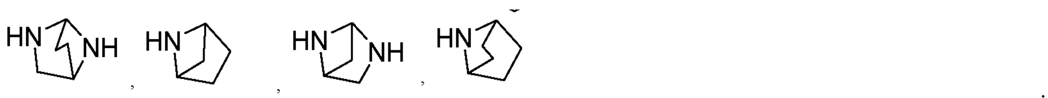

.

Unless otherwise specified, the term "substitute" or "substituent" refers to any substitution at a position allowed by chemical theory, and the number of substituents conforms to the rules of chemical bonding. Exemplary substituents include, but are not limited to: $C_{1-6}$ alkyl, $C_{2-6}$ alkenyl, $C_{2-6}$ alkynyl, $C_{3-8}$ heteroalkyl, $C_{5-12}$ aryl, 5- to 12-membered heteroaryl, hydroxyl, $C_{1-6}$ alkoxy, $C_{5-12}$ aryloxy, thiol, $C_{1-6}$ alkylthio, cyano, halogen, $C_{1-6}$ alkylthiocarbonyl, $C_{1-6}$ alkylcarbamoyl, N-carbamoyl, nitro, silyl, sulfinyl, sulfonyl, sulfoxide, halo $C_{1-6}$ alkyl, halo $C_{1-6}$ alkoxy, amino, phosphonic acid, $-CO_2(C_{1-6}$ alkyl), $-OC(=O)(C_{1-6}$ alkyl), $-OCO_2(C_{1-6}$ alkyl), - $C(=O)NH_2$, $-C(=O)N(C_{1-6}$ alkyl$)_2$, $-OC(=O)NH(C_{1-6}$ alkyl), $-NHC(=O)(C_{1-6}$ alkyl), $-N(C_{1-6}$ alkyl)$C(=O)(C_{1-6}$ alkyl), $-NHCO_2(C_{1-6}$ alkyl) , $-NHC(=O)N(C_{1-6}$ alkyl$)_2$, - $HC(=O)NH(C_{1-6}$ alkyl), $-NHC(=O)NH_2$, $-NHSO_2(C_{1-6}$ alkyl), $-SO_2N(C_{1-6}$ alkyl$)_2$, - $SO_2NH(C_{1-6}$ alkyl), -SOzNHz, $-SO_2C_{1-6}$ alkyl, *etc.*

The term "optional" or "optionally" refers to that the events or circumstances subsequently described may but

not necessarily occur, and the description includes the occasions where the events or circumstances occur or do not occur. For example, "alkyl optionally substituted with F" means that the alkyl may but not necessarily be substituted by F, and the description includes the case where the alkyl is substituted with F and the case where the alkyl is not substituted with F.

The term "pharmaceutically acceptable salt" refers to a salt of the compound of the present invention, which salt maintains the biological effectiveness and characteristics of a free acid or a free base and is obtained by reacting the free acid with a non-toxic inorganic base or organic base, or reacting the free base with a non-toxic inorganic acid or organic acid.

The term "pharmaceutical composition" refers to a mixture of one or more compounds described herein, or stereoisomers, solvates, deuterated compounds, pharmaceutically acceptable salts or cocrystals thereof, and other components, wherein the other components comprise physiologically/pharmaceutically acceptable carriers and/ excipients.

The term "carrier" refers to: a system that does not cause significant irritation to the organism and does not eliminate the biological activity and characteristics of the administered compound, and can change the way the drug enters the human body and the distribution of the drug in the body, control the release rate of the drug and delivery the drug to targeted organs. Non-limiting examples of the carrier include microcapsule, microsphere, nanoparticle, liposome, etc.

The term "excipient" refers to: a substance that is not a therapeutic agent per se, but used as a diluent, adjuvant, adhesive and/or vehicle for addition to a pharmaceutical composition, thereby improving the disposal or storage properties thereof, or allowing to or promoting the formation of a compound or a pharmaceutical composition into a unit dosage form for administration. As is known to those skilled in the art, a pharmaceutically acceptable excipient can provide various functions and can be described as a wetting agent, a buffer, a suspending agent, a lubricant, an emulsifier, a disintegrating agent, an absorbent, a preservative, a surfactant, a colorant, a flavoring agent and a sweetening agent. Examples of pharmaceutically acceptable excipients include, but are not limited to: (1) sugars, such as lactose, glucose and sucrose; (2) starch, such as corn starch and potato starch; (3) cellulose and its derivatives, such as sodium carboxymethyl cellulose, ethyl cellulose, cellulose acetate, hydroxypropyl methylcellulose, hydroxypropyl cellulose, microcrystalline cellulose and croscarmellose (such as croscarmellose sodium); (4) tragacanth powder; (5) malt; (6) gelatin; (7) talc; (8) excipients, such as cocoa butter or suppository wax; (9) oils, such as peanut oil, cottonseed oil, safflower oil, sesame oil, olive oil, corn oil and soybean oil; (10) diols, such as propylene glycol; (11) polyols, such as glycerol, sorbitol, mannitol and polyethylene glycol; (12) esters, such as ethyl oleate and ethyl laurate; (13) agar; (14) buffers, such as magnesium hydroxide and aluminum hydroxide; (15) alginic acid; (16) pyrogen-free water; (17) isotonic saline; (18) Ringer’s solution; (19) ethanol; (20) pH buffered solution; (21) polyester, polycarbonate and/or polyanhydride; and (22) other non-toxic compatible substances used in a pharmaceutical preparation.

The term "stereoisomer" refers to an isomer produced as a result of different spatial arrangement of atoms in molecules, including cis-trans isomers, enantiomers and conformational isomers.

The compounds of the present invention also include tautomers thereof, for example, when the present invention describes the left side compound in which the pyrimidine ring is substituted with OH, the right side tautomer compound is also included.

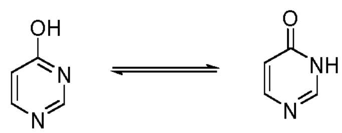

The term "solvate" refers to a substance formed by the compound of the present invention or the salt thereof and a stoichiometric or non-stoichiometric solvent bound by intermolecular non-covalent forces. When the solvent is water, the solvate is a hydrate.

The term "co-crystal" refers to a crystal formed by the combination of active pharmaceutical ingredient (API) and co-crystal former (CCF) under the action of hydrogen bonds or other non-covalent bonds. The pure state of API and CCF are both solid at room temperature, and there is a fixed stoichiometric ratio between various components. The co-crystal is a multi-component crystal, which includes both a binary co-crystal formed between two neutral solids and a multi-element co-crystal formed between a neutral solid and a salt or solvate.

Brief Description of the Drawings

Figure 1 shows the intracranial fluorescence signal changes in the mouse brain orthotopic MCF-7 model.
Figure 2 shows the rate of change in mouse body weight in the mouse brain orthotopic MCF-7 model.

Detailed Description of Embodiments

The content of the present invention is described in detail with the following examples. If a specific condition is not indicated in the examples, a conventional condition is used in an experimental method. The listed examples are intended to better illustrate the content of the present invention, but should not be construed as limiting the content of the present invention. According to the above-mentioned content of the invention, those skilled in the art can make unsubstantial modifications and adjustments to the embodiments, which still fall within the protection scope of the present invention.

**Detection method**

The structure of the compound is determined by nuclear magnetic resonance (NMR) or (and) mass spectrometry (MS). The NMR shift (δ) is given in the unit of 10-6 (ppm). NMR is determined with (Bruker Avance III 400 and Bruker Avance 300) nuclear magnetic resonance instrument; the solvents for determination are deuterated dimethyl sulfoxide (DMSO-$d_6$), deuterated chloroform ($CDCl_3$) and deuterated methanol ($CD_3OD$); and the internal standard is tetramethylsilane (TMS).

MS is determined with Agilent 6120B (ESI) and Agilent 6120B (APCI);
HPLC is determined with Agilent 1260DAD high pressure liquid chromatograph (Zorbax SB-C18 100 × 4.6 mm, 3.5 μM);
Yantai Huanghai HSGF254 or Qingdao GF254 silica gel plate is used as a thin layer chromatography silica plate, and the silica gel plate for the thin layer chromatography (TLC) is of the specification of 0.15 mm-0.20 mm, and the specification when separating and purifying a product by thin layer chromatography is 0.4 mm - 0.5 mm.
and for the column chromatography, Yantai Huanghai silica gel of 200-300 mesh silica gel is generally used as a carrier.

Abbreviations or terms

PE: Petroleum ether
EA: Ethyl acetate
MeOH: Methanol
THF: Tetrahydrofuran
n-BuLi: Butyl lithium
DCM: Dichloromethane
DMF: N,N-dimethylformamide
DIPEA: N,N-diisopropylethylamine
BINAP: Binaphthyl diphenylphosphine
$Pd_2$(dba)3: Tris(dibenzylidene acetone)dipalladium
t-BuONa: Sodium tert-butoxide
Brettphos G3 Pd: methanesulfonic acid (2-dicyclohexylphosphino-3,6-dimethoxy-2',4',6'-triisopropyl-1,1'-biphenyl)(2'-amino-1,1'-biphenyl-2-yl)palladium(II)

**Preparation of compounds**

**Intermediate 1: (S)-2,6-difluoro-4-((1-(3-fluoropropyl)pyrrolidin-3-yl)amino)benzaldehyde (intermediate 1)**

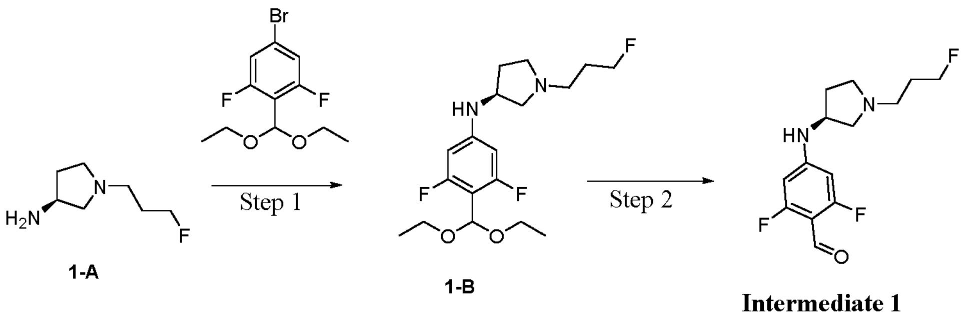


1-A

1-B

Intermediate 1

Step 1: ((S)-N-(4-(diethoxymethyl)-3,5-difluorophenyl)-1-(3-fluoropropyl)pyrrolidin-3-amine **(1-B)**

**1-A** (5.0 g, 34 mmol) (referring to WO 2021139756 for the synthetic step), 5-bromo-2-(diethoxymethyl)-1,3-difluorobenzene (11.8 g, 40 mmol) (referring to WO 2019245974 for the synthetic step), cesium carbonate (22.8 g, 70 mmol), XantPhos (2.9 g, 50 mmol), and $Pd_2(dba)_3$ (2.3 g, 25 mmol) were mixed in a single-neck bottle, 1,4-dioxane (100 mL) was added, and the reaction system was subjected to nitrogen replacement three times, and reacted at 100°C for 16 hours. After the reaction was completed, the mixture was cooled to room temperature, concentrated, and diluted with water (80 mL), and then extracted with ethyl acetate (60 mL × 3). The organic phases were combined, washed with saturated brine, dried over anhydrous sodium sulfate, concentrated, and then separated by silica gel column chromatography (methanol: dichloromethane (v/v) = 0:1-1:20) to obtain the target compound **1-B** (5.5 g, 45%).

Step 2: (S)-2,6-difluoro-4-((1-(3-fluoropropyl)pyrrolidin-3-yl)amino) benzaldehyde **(intermediate1)**

**1-B** (5.5 g, 15 mmol) was dissolved in toluene (60 mL), trifluoroacetic acid (8 mL) was added, and same was stirred at room temperature for 2 hours. 60 mL of water was added into the system, stirred for ten minutes, and liquid-liquid separation was performed. The aqueous phase was adjusted to pH = 8-9 with a saturated sodium bicarbonate solution, then extracted with ethyl acetate (50 mL ×3), the organic phases were combined, washed with saturated brine, dried over anhydrous sodium sulfate, and concentrated to obtain **intermediate 1** (light yellow solid, 4.0 g, 93%).

**Intermediate 2: 5-((1-(3-fluoropropyl)azetidin-3-yl)amino)picolinaldehyde (intermediate2)**

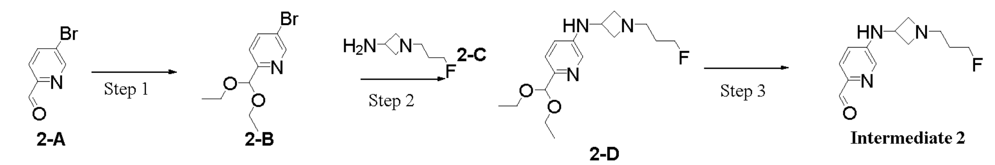


2-A

2-B

2-D

Intermediate 2

Step 1: 5-bromo-2-(diethoxymethyl)pyridine **(2-B)**

To a 250 mL three-neck bottle were added **2-A** (10.00 g, 53.76 mmol) and triethyl orthoformate (15.94 g, 107.52 mmol), same was dissolved in toluene (100 mL), and subjected to nitrogen replacement three times, p-toluenesulfonic acid (0.93 g, 5.38 mmol) was added, stirred at room temperature for 1 h, quenched with sodium bicarbonate solution (1M, 100 mL), stirred at room temperature for 0.5 h, and extracted with EA (2×100 mL), the organic phases were combined, dried over anhydrous sodium sulfate for 0.5 h, and concentrated to obtain the title compound **2-B** (12 g, crude), which was directly used in the next reaction.

$^1H$ NMR (400 MHz, CDCl3) $\delta$8.65 (s, 1H), 4.54 (t, 1H), 7.85(dd, 1H),7.51(d, 1H), 3.56-3.74 (m, 4H), 1.24 (t, 6H).

Step 2: 6-(diethoxymethyl)-N-(1-(3-fluoropropyl)azetidin-3-yl)pyridin-3-amine **(2-D)**

To a 100 mL single-neck bottle was added **2-B** (2 g, crude), **2-C** (1.22 g, 9.23 mmol), cesium carbonate (7.52 g, 23.07 mmol), Xantphos (0.44 g, 0.77 mmol) and $Pd_2(dba)_3$ (0.35 g, 0.38 mmol), same was dissolved in toluene (40 mL) and reacted at 100°C for 12 h. The mixture was filtered, concentrated and purified by column chromatography (dichloromethane: methanol (v/v) = 100:1-10: 1) to obtain the title compound **2-D** (1.3 g, 54%).

Step 3: 5-((1-(3-fluoropropyl)azetidin-3-yl)amino)picolinaldehyde **(intermediate 2)**

To a 100 mL single-neck bottle was added **2-D** (1.30 g, 4.17 mmol), same was dissolved in ethyl acetate (20 mL), 2N hydrochloric acid (20 mL) was added, stirred at room temperature for 1 h, the mixture was allowed to stand for liquid-liquid separation, the aqueous phase was adjusted to pH 9 with 1N sodium hydroxide solution, and extracted with ethyl acetate (20 mL×2), and the organic phases were combined, and concentrated to obtain the title compound **intermediate 2** (0.8 g, 80%).

LC-MS (ESI): m/z = 238.1 $[M+H]^+$.

$^1H$ NMR (400 MHz, CDCl3) $\delta$ 9.87 (s, 1H),8.05 (d, 1H), 7.82 (d, 1H), 6.81-6.84 (m, 1H), 4.80 (d, 1H),4.55(t, 1H), 4.44 (t, 1H), 4.15-4.23 (m, 1H), 3.76 (t, 2H), 3.01 (t, 2H), 2.63 (t, 2H), 1.70-1.83 (m, 2H).

**Intermediate 3: 5-((1-(3-fluoropropyl)pyrrolidin-3-yl)amino)picolinaldehyde (intermediate 3)**

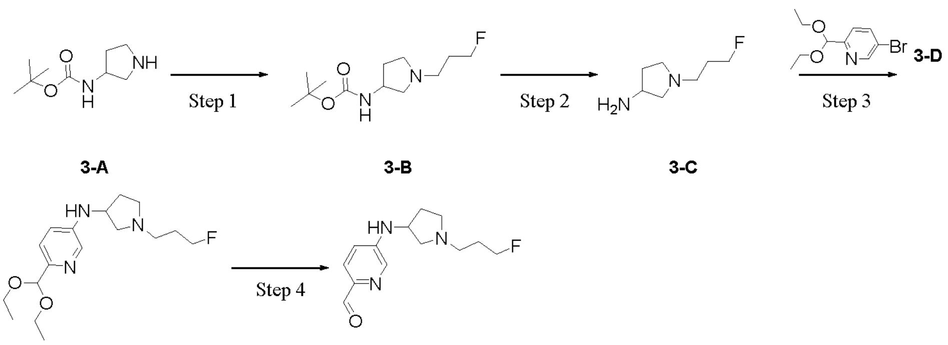

3-A
3-B
3-C
3-E
Intermediate 3

Step 1: tert-butyl (1-(3-fluoropropyl)pyrrolidin-3-yl)carbamate **(3-B)**

To a 100 mL three-neck bottle was added **3-A** (4.00 g, 21.48 mmol), fluoroiodopropane (6.06 g, 32.22 mmol), and DIPEA (8.33 g, 64.44 mmol), same was dissolved in tetrahydrofuran (40 mL), and subjected to nitrogen replacement three times, stirred at room temperature for 12 h, quenched with water (50 mL), stirred at room temperature for 0.5 h, extracted with EA (2 × 50 mL), and the organic phases were combined, dried over anhydrous sodium sulfate for 0.5 h, concentrated and purified by column chromatography (petroleum ether: ethyl acetate (v/v) = 100 : 1-3 : 1) to obtain the title compound **3-B** (4 g, 75.5%).

$^1H$ NMR (400 MHz, CDCl3) $\delta$ 4.86 (s, 1H), 4.57 (t, 1H), 4.44 (t, 1H), 4.17(s, 1H),2.54-2.58 (m, 4H), 2.28 (s, 2H), 1.81-1.95 (m, 2H), 1.60 (s, 1H),1.44 (s, 9H).

Step 2: 1-(3-fluoropropyl)pyrrolidin-3-amine **(3-C)**

To a 100 mL three-neck bottle was added **3-B** (3.00 g, 12.18 mmol), same was dissolved in dichloromethane (30 mL), and subjected to nitrogen replacement three times, trifluoroacetic acid (10 mL) was added, stirred at room temperature for 1 h and concentrated to obtain the title compound **3-C** (5 g, crude), which was directly used in the next reaction.

Step 3: 6-(diethoxymethyl)-N-(1-(3-fluoropropyl)pyrrolidin-3-yl)pyridin-3-amine **(3-E)**

To a 250 mL single-neck bottle was added **3-C** (5 g, crude), **3-D** (8 g, 30.75 mmol), cesium carbonate (30.06 g, 92.25 mmol), Xantphos (1.78 g, 3.08 mmol) and $Pd_2(dba)_3$ (1.41 g, 1.54 mmol), same was dissolved in toluene (100 mL) and reacted at 100°C for 12 h. The mixture was filtered, concentrated and purified by column chromatography (dichloromethane: methanol (v/v) = 100:1-10: 1) to obtain the title compound **3-E** (0.8 g, 21%).

Step 4: 5-((1-(3-fluoropropyl)pyrrolidin-3-yl)amino)picolinaldehyde **(intermediate 3)**

To a 100 mL single-neck bottle was added **3-E** (0.8 g, 2.46 mmol), same was dissolved in ethyl acetate (20 mL), 2N hydrochloric acid (20 mL) was added, stirred at room temperature for 1 h, the mixture was allowed to stand for liquid-liquid separation, the aqueous phase was adjusted to pH 9 with 1N sodium hydroxide solution, extracted with ethyl

acetate (20mL × 2), the organic phases were combined, and concentrated to obtain the title compound **intermediate 3** (0.35 g, 56%).

LC-MS (ESI): m/z = 252.2 $[M+H]^+$.

$^1H$ NMR (400 MHz, CDCl3) $\delta$ 9.87 (s, 1H),8.07 (d, 1H), 7.82 (d, 1H), 6.86-6.89 (m, 1H), 4.80 (d, 1H),4.59(t, 1H), 4.47 (t, 1H), 4.10 (s, 1H), 2.97-2.99 (m, 1H), 2.71-2.79 (m, 2H),2.66 (t, 2H),2.36-2.46 (m, 2H), 1.87-2.00 (m, 2H),1.72-1.79 (m, 1H).

**Example 1: 1-cyclopropyl-N-((1S,3R)-1-(2,6-difluoro-4-((1-(3-fluoropropyl)azetidin-3-yl)amino)phenyl)-2-(2-fluoro-2-methylpropyl)-3-methyl-1,2,3,4-tetrahydroisoquinolin-6-yl)methanesulfonamide (compound 1)**

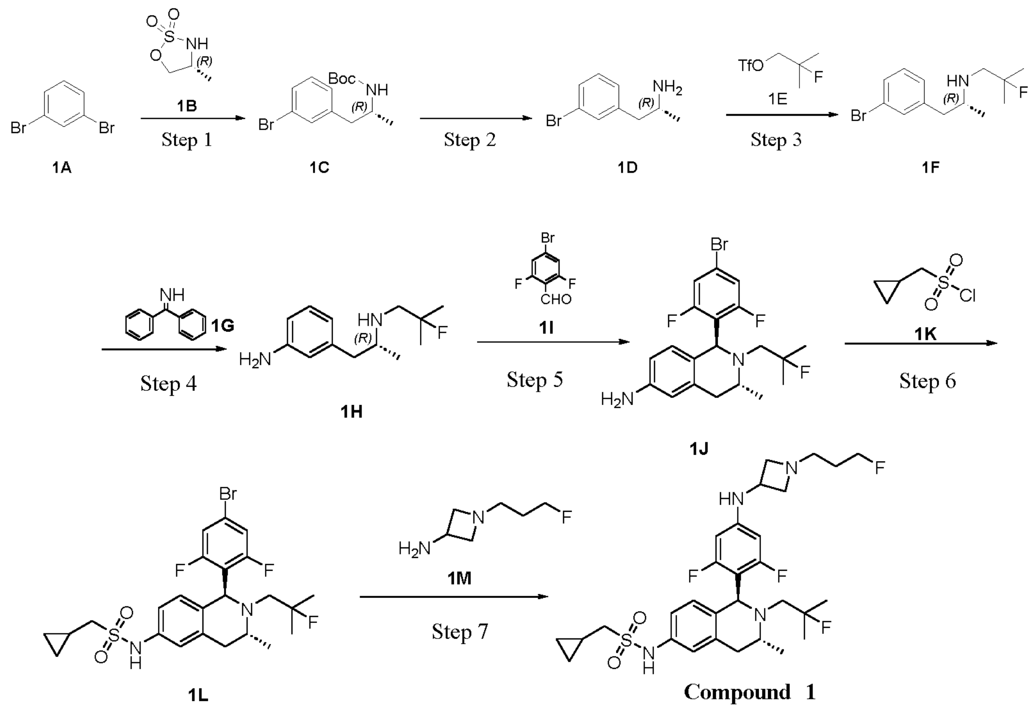


Step 1: tert-butyl (R)-(1-(3-bromophenyl)propan-2-yl)carbamate **(1C)**

To a 250 mL three-neck bottle was added **1A** (5.00 g, 21.20 mmol), same was dissolved in THF (25 mL), the system was cooled to -60°C in a dry ice ethanol bath, and subjected to nitrogen replacement three times, n-BuLi (2.5 M, 8.5 mL, 21.20 mmol) was added dropwise, stirred for 1 h while maintaining this temperature constant, a solution of **1B** (5.30 g, 22.26 mmol) in THF (25 mL) was added dropwise, warmed to 0°C and reacted for 2 h, quenched with citric acid solution (1M, 50 mL), stirred at room temperature for 0.5 h, extracted with EA (2 × 50 mL), the organic phases were combined, dried over anhydrous sodium sulfate for 0.5 h, and concentrated to obtain the title compound **1C** (11.0 g, crude), which was directly used for the next reaction.

Step 2: (R)-1-(3-bromophenyl)propan-2-amine **(1D)**

To a 250 mL single-neck bottle was added **1C** (11.00 g, crude), same was dissolved in MeOH (25 mL), a solution of hydrogen chloride in dioxane (4 M, 25 mL) was added, reacted at room temperature for 2 h, concentrated, diluted hydrochloric acid (2 M, 50 mL) was added for dissolution, same was extracted with EA (2× 50 mL) to remove impurities, the aqueous phase was collected, the reaction system was adjusted to pH>10 with NaOH (20%), extracted with DCM (2× 50 mL), the organic phases were combined, dried over anhydrous sodium sulfate for 0.5 h, concentrated, and purified by column chromatography (dichloromethane: methanol (v/v) = 20: 1-10: 1) to obtain the title compound **1D** (0.91 g, 20%).

LC-MS (ESI): m/z = 214.0 $[M+H]^+$.

Step 3: (R)-N-(1-(3-bromophenyl)propan-2-yl)-2-fluoro-2-methylpropan-1-amine **(1F)**

To a 50 mL single-neck bottle was added **1D** (0.50 g, 2.34 mmol), same was dissolved in dioxane (10 mL), DIPEA (0.53 g, 4.09 mmol) and **1E** (0.60 g, 2.69 mmol) were added, reacted at 85°C for 18 h, concentrated and purified by column chromatography (petroleum ether : ethyl acetate (v/v) = 10 : 1-4 : 1) to obtain the title compound **1F** (0.50 g, 74%).

LC-MS (ESI): m/z = 288.1 $[M+H]^+$.

Step 4: (R)-3-(2-((2-fluoro-2-methylpropyl)amino)propyl)aniline **(1H)**

To a 50 mL single-neck bottle was added **1F** (0.50 g, 1.73 mmol), same was dissolved in dioxane (10 mL), **1G** (0.31 g, 1.73 mmol), t-BuONa (0.25 g, 2.59 mmol), BINAP (54 mg, 0.087 mmol) and $Pd_2(dba)_3$ (25 mg, 0.043 mmol) were added, the reaction system was subjected to nitrogen replacement three times, reacted at 90°C for 4 h, cooled to room temperature, dissolved in HCl (1 M, 20 mL), extracted with DCM (2 × 20 mL) to remove impurities, the aqueous phase was collected, the reaction system was adjusted to pH > 10 with NaOH (2 M), extracted with EA (2 × 20 mL), the organic phases were combined, dried over anhydrous sodium sulfate for 0.5 h, concentrated and purified by column chromatography (petroleum ether : ethyl acetate (v/v) = 10 : 1-2 : 1) to obtain the title compound **1H** (0.35 g, 90%).

$^1H$ NMR (400 MHz, $CD_3Cl$) $\delta$ 7.11 - 7.02 (m, 1H), 6.59 (dt, 1H), 6.55-6.52 (m, 2H), 3.60 (s, 2H), 2.95-2.88 (m, 1H), 2.84 - 2.58 (m, 3H), 2.51 (dd, 1H), 1.38 (s, 3H), 1.32 (s, 3H), 1.06 (d, 3H).

Step 5: (1S,3R)-1-(4-bromo-2,6-difluorophenyl)-2-(2-fluoro-2-methylpropyl)-3-methyl-1,2,3,4-tetrahydroisoquinolin-6-amine **(1J)**

To a 50 mL single-neck bottle was added **1H** (0.35 g, 1.56 mmol), same was dissolved in acetic acid (5mL) and water (0.2 mL), **1I** (0.69 g, 3.12 mmol) was added, reacted at 60°C for 18 h, concentrated, dissolved in DCM (20 mL), washed with HCl (1 M, 20 mL), the aqueous phase was collected, the reaction system was adjusted to pH > 10 with NaOH (2 M), extracted with DCM (2 × 20 mL), the organic phases were combined, dried over anhydrous sodium sulfate for 0.5 h, concentrated and purified by column chromatography (petroleum ether : ethyl acetate (v/v) = 3 : 1-1 : 1) to obtain the title compound **1J** (0.31 g, 47%).

LC-MS (ESI): m/z = 427.1 $[M+H]^+$.

Step 6: N-((1S,3R)-1-(4-bromo-2,6-difluorophenyl)-2-(2-fluoro-2-methylpropyl)-3-methyl-1,2,3,4-tetrahydroisoquinolin-6-yl)-1-cyclopropylmethanesulfonamide **(1L)**

To a 50 mL single-neck bottle was added **1J** (0.31 g, 0.73 mmol), dissolved in DCM (10 mL), pyridine (0.23 g, 2.92 mmol) and **1K** (0.34 g, 2.19 mmol) were added, reacted at room temperature for 18 h, concentrated and purified by column chromatography (petroleum ether : ethyl acetate (v/v) = 3 : 1-1 : 1) to obtain the title compound **1L** (0.21 g, 53%).

LC-MS (ESI): m/z = 545.1 $[M+H]^+$.

Step 7: 1-cyclopropyl-N-((1S,3R)-1-(2,6-difluoro-4-((1-(3-fluoropropyl)azetidin-3-yl)amino)phenyl)-2-(2-fluoro-2-methylpropyl)-3-methyl-1,2,3,4-tetrahydroisoquinolin-6-yl)methanesulfonamide **(compound 1)**

To a 50 mL single-neck bottle was added **1L** (0.21 g, 0.38 mmol), same was dissolved in dioxane (10 mL), **1M** (75 mg, 0.57 mmol), $Cs_2CO_3$ (0.43 g, 1.33 mmol), xantphos (44 mg, 0.076 mmol) and $Pd_2(dba)_3$ (38 mg, 0.038 mmol) were added, the reaction system was subjected to nitrogen replacement three times, reacted at 110°C for 5 h, filtered over celite, concentrated, and subjected to preparative HPLC to obtain the title **compound 1** (15 mg, 6%).

Preparation method: instrument: waters 2767 (preparative liquid phase chromatographic instrument); chromatographic column: SunFire@ Prep C18 (19 mm × 250 mm); the sample was dissolved in DMF and filtered with a 0.45 μm filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 40%-70%; c. flow rate: 15 mL/min; d. elution time: 20 min; retention time: 10.12 min.

$^1H$ NMR (400 MHz, $CD_3Cl$) $\delta$ 6.93 (d, 1H), 6.87 (dd, 1H), 6.69 (d, 1H), 6.50 (s, 1H), 6.00 (d, 2H), 5.00 (s, 1H), 4.57 (t, 1H), 4.45 (t, 1H), 4.17 (s, 1H), 3.97 (s, 2H), 3.64 (s, 2H), 3.48 (s, 2H), 3.29 (dd, 1H), 3.03 (d, 2H), 2.86-2.82 (m, 2H), 2.51 (dd, 1H), 2.34 - 2.18 (m, 2H), 1.35-1.26 (m, 3H), 1.20 - 1.10 (m, 6H), 0.96 (d, 3H), 0.69 - 0.61 (m, 2H), 0.31-0.28 (m, 2H).

LC-MS (ESI): m/z = 597.2 $[M+H]^+$.

**Example 2: N-(2-(bicyclo[1.1.1]pentan-1-yl)-1-(2,6-difluoro-4-((1-(3-fluoropropyl)azetidin-3-yl)amino)phenyl)-3-methyl-1,2,3,4-tetrahydroisoquinolin-6-yl)ethanesulfonamide (compound 2)**

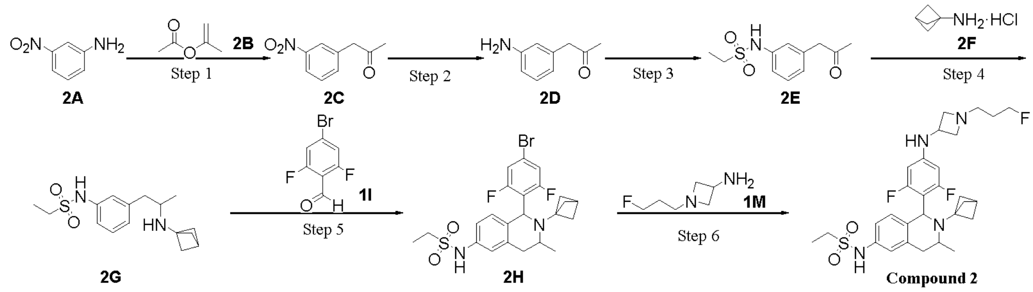


Step 1: 1-(3-nitrophenyl)propan-2-one **(2C)**

Under nitrogen protection, compound **2A** (25 g, 181 mmol) was dissolved in acetonitrile (250 mL) and water (25 mL), and salicylic acid (2.5 g, 18.1 mmol) and compound **2B** (18.12 g, 181 mmol) were added. The mixture was vigorously stirred for five minutes to a homogeneous phase, tert-butyl nitrite (28 g, 271.5 mmol) was then added, and the mixture was reacted for three hours while maintaining the temperature below 20°C. Water (200 ml) was added to quench the reaction, and the organic phase was extracted with ethyl acetate (100 mL × 3). The organic phases were combined and separated by column chromatography (PE : EA = 2 : 1) to obtain compound **2C** (11.8 g, 36.39%).

LC-MS (ESI): m/z =180.1 $[M+H]^+$.

Step 2: 1-(3-aminophenyl)propan-2-one **(2D)**

Compound **2C** (11.8 g, 65.8 mmol) was dissolved in a mixed solvent of toluene (50 mL) and methanol (50 mL), and 10% palladium on carbon (2.5 g) was added. The hydrogenation reaction was carried out at room temperature and normal pressure overnight. The mixture was filtered to remove insoluble matter, and dried by rotary evaporation to obtain compound **2D** (7.1 g, 72.26%).

LC-MS (ESI): m/z =150.1 $[M+H]^+$.

Step 3: N-(3-(2-oxopropyl)phenyl)ethanesulfonamide **(2E)**

Compound **2D** (7.1 g, 47.59 mmol) was dissolved in dichloromethane (70 mL), and pyridine (5.65 g, 71.39 mmol) and ethyl sulfonyl chloride (6.73 g, 52.35 mmol) were added. The mixture was warmed to 50°C and reacted overnight. Water (50 ml) was added to quench the reaction, and the organic phase was extracted with ethyl acetate (50 mL × 3). The organic phases were combined and separated by column chromatography (PE : EA = 2 : 1) to obtain compound **2E** (11.8 g, 36.39%).

LC-MS (ESI): m/z =242.1 $[M+H]^+$.

Step 4: N-(3-(2-(bicyclo[1.1.1]pentan-1-ylamino)propyl)phenyl) ethanesulfonamide **(2G)**

Compound **2F** (4.64 g, 38.78 mmol) was dissolved in methanol (50 mL), triethylamine (5 mL) was added, and the mixture was warmed to 50°C and reacted for half an hour. Compound **2E** (7.8 g, 32.32 mmol) was added. The reaction was continued at this temperature for 6 hours. Sodium cyanoborohydride (3.05 g, 48.48 mmol) was then added, and the mixture was reacted at room temperature for two hours. Water (50 ml) was added to quench the reaction, and the organic phase was extracted with ethyl acetate (50 mL × 3). The organic phases were combined and separated by column chromatography (PE : EA = 2 : 1) to obtain compound **2G** (8.9 g, 89.28%).

$^1H$ NMR (400 MHz, Chloroform-d) $\delta$ 7.26 (t, 2H), 7.11 - 7.02 (m, 3H), 6.98 (t, 1H), 3.13 (d, 2H), 3.04 (p, 1H), 2.77 (dd, 1H), 2.57 (dd, 1H), 1.88 - 1.77 (m, 6H), 1.37 (t, 4H), 1.29 - 1.21 (m, 1H), 1.06 (d, 3H).

Step 5: N-(2-(bicyclo[1.1.1]pentan-1-yl)-1-(4-bromo-2,6-difluorophenyl)-3-methyl-1,2,3,4-tetrahydroisoquinolin-6-yl)ethanesulfonamide **(2H)**

To a 50 mL single-neck bottle was added compound **2G** (2.7 g, 8.75 mmol) and compound **1I** (1.93 g, 8.75

mmol), same was dissolved in toluene (30 mL), and acetic acid (6 mL) was added. The mixture was then warmed to 110°C and reacted for 4 hours. The solvent was removed by distillation under reduced pressure, and the residue was separated by column chromatography (PE : EA = 3 : 1) to obtain the compound **2H** (0.513 g, 11.46%).

LC-MS (ESI): m/z = 511.1$[M+H]^+$.

Step 6: N-(2-(bicyclo[1.1.1]pentan-1-yl)-1-(2,6-difluoro-4-((1-(3-fluoropropyl) azetidin-3-yl)amino)phenyl)-3-methyl-1,2,3,4-tetrahydroisoquinolin-6-yl)ethanesulfonamide **(compound 2)**

To a 100 mL single-neck bottle was added compound **2H** (0.58 g, 1.13 mmol) and compound **1M** (0.22 g, 1.69 mmol), same was dissolved in 1,4-dioxane (10 mL), and XantPhos (0.13 g, 0.23 mmol), $Pd_2(dba)_3$ (0.1 g, 0.11 mmol) and cesium carbonate (1.1 g, 3.39 mmol) were added. The mixture was then warmed to 110°C and reacted for 4 hours. The solvent was removed by distillation under reduced pressure, and the residue was separated by preparative chromatography to obtain **Compound 2** (4 mg, 0.63%).

Preparation method: instrument: waters 2767 (preparative liquid phase chromatographic instrument); chromatographic column: SunFire@ Prep C18 (19 mm × 250 mm); the sample was dissolved in DMF and filtered with a 0.45 μm filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 40%-70%; c. flow rate: 15 mL/min; d. elution time: 20 min; retention time: 13.12 min.

LC-MS (ESI): m/z = 563.3$[M+H]^+$.

$^1$H NMR (400 MHz, DMSO-$d_6$) δ 9.58 (s, 1H), 6.93 - 6.85 (m, 1H), 6.67 (d, 1H), 6.55 (d, 1H), 6.06 (d, 2H), 5.04 (s, 1H), 4.50 (t, 1H), 4.39 (t, 1H), 3.91 (q, 1H), 3.65 - 3.56 (m, 2H), 3.56 - 3.46 (m, 1H), 3.11 (dd, 1H), 3.03 (q, 2H), 2.77 - 2.70 (m, 2H), 2.48 - 2.41 (m, 3H), 2.23 (s, 1H), 1.73 (dd, 3H), 1.64 (t, 2H), 1.53 (dd, 3H), 1.24 (s, 1H), 1.18 (t, 3H), 0.92 (d, 3H).

**Example 3: ((1S,3R)-1-(2,6-difluoro-4-((1-(3-fluoropropyl)azetidin-3-yl)amino)phenyl)-2-(2-fluoro-2-methylpropyl)-3-methyl-1,2,3,4-tetrahydroisoquinolin-6-yl)dimethylphosphine oxide (compound 3)**

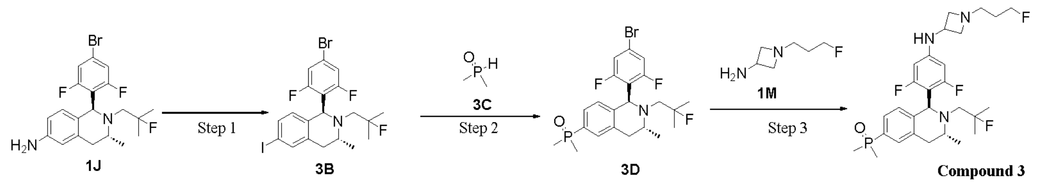

1J 3B 3D Compound 3

Step 1: (1S,3R)-1-(4-bromo-2,6-difluorophenyl)-2-(2-fluoro-2-methylpropyl)-6-iodo-3-methyl-1,2,3,4-tetrahydroisoquinoline **(3B)**

To a 100 mL three-neck bottle was added **1J** (0.50 g, 1.17 mmol), same was dissolved in acetonitrile (10 mL), p-toluenesulfonic acid monohydrate (0.67 g, 3.51 mmol) was added, cooled to 0°C in an ice bath, an aqueous solution (2 mL) of sodium nitrite (0.16 g, 2.34 mmol) and potassium iodide (0.49 g, 2.92 mmol) was added dropwise, then stirred for 0.5 h while maintaining this temperature constant, naturally warmed to room temperature and reacted for 2 h, concentrated to remove acetonitrile, washed with a saturated sodium bicarbonate solution (20 mL), extracted with dichloromethane (2 × 20 mL), the organic phases were combined, dried over anhydrous sodium sulfate, concentrated and separated by column chromatography (petroleum ether : ethyl acetate (v/v) = 10 : 1-4 : 1) to obtain the title compound **3B** (0.4 g, 64%).

LC-MS (ESI): m/z = 538.0 $[M+H]^+$.

Step 2: ((1S,3R)-1-(4-bromo-2,6-difluorophenyl)-2-(2-fluoro-2-methylpropyl)-3-methyl-1,2,3,4-tetrahydroisoquinolin-6-yl)dimethylphosphine oxide **(3D)**

To a 50 mL single-neck bottle was added **3B** (0.40 g, 0.74 mmol), same was dissolved in dioxane (10 mL), **3C** (58 mg, 0.74mmol), triethylamine (75 mg, 0.74 mmol), xantphos (86 mg, 0.15 mmol) and $Pd_2(dba)_3$ (68 mg, 0.074mmol) were added, the reaction system was subjected to nitrogen replacement three times, reacted at room temperature for 3 h, same was dissolved in HCl (1 M, 20 mL), extracted with dichloromethane (2 × 20 mL) to remove impurities, the aqueous phase was collected, the reaction system was adjusted to pH > 10 with NaOH (2 M), extracted with ethyl acetate (2 × 20 mL), the organic phases were combined, dried over anhydrous sodium sulfate for 0.5 h, concentrated and

purified by column chromatography (dichloromethane : methanol (v/v) = 20 : 1-10: 1) to obtain the title compound **3D** (0.11 g, 30%).

LC-MS (ESI): m/z = 488.0 $[M+H]^+$.

Step 3: ((1S,3R)-1-(2,6-difluoro-4-((1-(3-fluoropropyl)azetidin-3-yl)amino)phenyl)-2-(2-fluoro-2-methylpropyl)-3-methyl-1,2,3,4-tetrahydroisoquinolin-6-yl)dimethylphosphine oxide **(compound 3)**

To a 50 mL single-neck bottle was added **3D** (0.09 g, 0.18 mmol), same was dissolved in dioxane (10 mL), **1M** (48 mg, 0.36 mmol), $Cs_2CO_3$ (0.47 g, 1.44 mmol), Brettphos G3 Pd (33 mg, 0.036 mmol) were added, the reaction system was subjected to nitrogen replacement three times, reacted at 105°C for 5 h, filtered over celite, concentrated, and subjected to preparative HPLC to obtain the title **compound 3** (30 mg, 31%).

Preparation method: instrument: waters 2767 (preparative liquid phase chromatographic instrument); chromatographic column: SunFire@ Prep C18 (19 mm × 250 mm); the sample was dissolved in DMF and filtered with a 0.45 μm filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 40%-70%; c. flow rate: 15 mL/min; d. elution time: 20 min; retention time: 10.80 min.

$^1H$ NMR (400 MHz, DMSO-d6) $\delta$ 7.53 - 7.45 (m, 1H), 7.44 - 7.32 (m, 1H), 6.82 (dd, 1H), 6.64 (d, 1H), 6.09 (d, 2H), 4.97 (s, 1H), 4.50 (t, 1H), 4.38 (t, 1H), 3.91 (q, 1H), 3.65 - 3.50 (m, 3H), 3.18 (dd, 1H), 2.83 (t, 1H), 2.73 (t, 2H), 2.67 - 2.59 (m, 1H), 2.45 (t, 2H), 2.33-2.18 (m, 2H), 1.70-1.64 (m, 1H), 1.62 (s, 3H), 1.59 (s, 3H), 1.11 (dd, 6H), 0.92 (d, 3H).

LC-MS (ESI): m/z = 540.3 [M+H]+.

**Example 4: N-((1S,3R)-1-(2,6-difluoro-4-((1-(3-fluoropropyl)azetidin-3-yl)amino)phenyl)-5-fluoro-2-(2-fluoro-2-methylpropyl)-3-methyl-1,2,3,4-tetrahydroisoquinolin-6-yl)ethanesulfonamide (compound 4)**

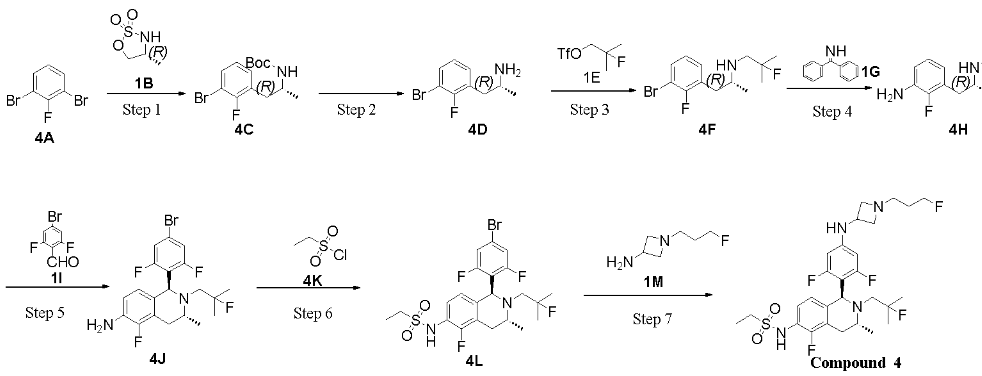


Step 1: tert-butyl (R)-(1-(3-bromo-2-fluorophenyl)propan-2-yl)carbamate **(4C)**

To a 500 mL three-neck bottle was added **4A** (10.00 g, 39.39 mmol), same was dissolved in THF (100 mL), the system was cooled to -60°C in a dry ice ethanol bath, and subjected to nitrogen replacement three times, n-BuLi (2.5 M, 17.33 mL, 43.33 mmol) was added dropwise, stirred for 1 h while maintaining this temperature constant, a solution of **1B** (5.94 g, 43.33 mmol) in THF (100 mL) was added dropwise, warmed to 0°C and reacted for 2 h, quenched with citric acid solution (1M, 100 mL), stirred at room temperature for 0.5 h, extracted with EA (2 × 100 mL), the organic phases were combined, dried over anhydrous sodium sulfate for 0.5 h, and concentrated to obtain the title compound **4C** (15.0 g, crude), which was directly used for the next reaction.

Step 2: (R)-1-(3-bromo-2-fluorophenyl)propan-2-amine **(4D)**

To a 250 mL single-neck bottle was added **4C** (15.00 g, crude), same was dissolved in MeOH (50 mL), a solution of hydrogen chloride in dioxane (4 M, 50 mL) was added, reacted at room temperature for 2 h, concentrated, diluted hydrochloric acid (2 M, 50 mL) was added for dissolution, extracted with EA (2 × 50 mL) to remove impurities, the aqueous phase was collected, the reaction system was adjusted to pH > 10 with NaOH (20%), extracted with DCM (2 × 50 mL), the organic phases were combined, dried over anhydrous sodium sulfate for 0.5 h, concentrated, and purified by column chromatography (dichloromethane : methanol (v/v) = 20 : 1-10 : 1) to obtain the title compound **4D** (2.6 g, 28%).

LC-MS (ESI): m/z = 232.0 $[M+H]^+$.

Step 3: (R)-N-(1-(3-bromo-2-fluorophenyl)propan-2-yl)-2-fluoro-2-methylpropan-1-amine **(4F)**

To a 50 mL single-neck bottle was added **4D** (2.60 g, 11.20 mmol), same was dissolved in dioxane (30 mL), DIPEA (4.36 g, 33.6 mmol) and **1E** (2.57 g, 12.32 mmol) were added, reacted at 85°C for 18 h, concentrated and purified by column chromatography (petroleum ether : ethyl acetate (v/v) = 10 : 1-4 : 1) to obtain the title compound **4F** (1.20 g, 35%).

LC-MS (ESI): m/z = 306.1 $[M+H]^+$.

Step 4: (R)-2-fluoro-3-(2-((2-fluoro-2-methylpropyl)amino)propyl)aniline **(4H)**

To a 100 mL single-neck bottle was added **4F** (1.20 g, 3.92 mmol), same was dissolved in dioxane (20 mL), **1G** (1.42 g, 7.84 mmol), t-BuONa (2.94 g, 11.76 mmol), BINAP (486.6 mg, 0.784 mmol) and $Pd_2(dba)_3$ (227.9 mg, 0.392 mmol) were added, the reaction system was subjected to nitrogen replacement three times, reacted at 90°C for 4 h, cooled to room temperature, dissolved in HCl (1 M, 40 mL), extracted with DCM (2 × 40 mL) to remove impurities, the aqueous phase was collected, the reaction system was adjusted to pH > 10 with NaOH (2 M), extracted with EA (2 × 40 mL), the organic phases were combined, dried over anhydrous sodium sulfate for 0.5 h, concentrated and purified by column chromatography (petroleum ether: ethyl acetate (v/v) = 10 : 1-2 : 1) to obtain the title compound **4H** (0.85 g, 89%).

LC-MS (ESI): m/z = 243.2 $[M+H]^+$.

Step 5: (1S,3R)-1-(4-bromo-2,6-difluorophenyl)-5-fluoro-2-(2-fluoro-2-methylpropyl)-3-methyl-1,2,3,4-tetrahydroisoquinolin-6-amine **(4J)**

To a 100 mL single-neck bottle was added **4H** (0.85 g, 3.51 mmol), same was dissolved in acetic acid (20 mL) and water (0.5 mL), **1I** (1.55 g, 7.02 mmol) was added, reacted at 65°C for 18 h, concentrated, dissolved in DCM (20 mL), washed with HCl (1 M, 20 mL), the aqueous phase was collected, the reaction system was adjusted to pH > 10 with NaOH (2 M), extracted with DCM (2 × 20 mL), the organic phases were combined, dried over anhydrous sodium sulfate for 0.5 h, concentrated and purified by column chromatography (petroleum ether : ethyl acetate (v/v) = 3 : 1-1 : 1) to obtain the title compound **4J** (0.5 g, 32%).

LC-MS (ESI): m/z = 445.1 $[M+H]^+$.

Step 6: N-((1S,3R)-1-(4-bromo-2,6-difluorophenyl)-5-fluoro-2-(2-fluoro-2-methylpropyl)-3-methyl-1,2,3,4-tetrahydroisoquinolin-6-yl)ethanesulfonamide **(4L)**

To a 100 mL single-neck bottle was added **4J** (0.26 g, 0.58 mmol), same was dissolved in DCM (10 mL), pyridine (0.18 g, 2.32 mmol)) and **4K** (0.22 g, 1.74 mmol) were added, reacted at room temperature for 18 h, concentrated and purified by column chromatography (petroleum ether : ethyl acetate (v/v) = 3 : 1-1 : 1) to obtain the title compound **4L** (0.22 g, 70%).

LC-MS (ESI): m/z = 537.1 $[M+H]^+$.

Step 7: N-((1S,3R)-1-(2,6-difluoro-4-((1-(3-fluoropropyl)azetidin-3-yl)amino)phenyl)-5-fluoro-2-(2-fluoro-2-methylpropyl)-3-methyl-1,2,3,4-tetrahydroisoquinolin-6-yl)ethanesulfonamide **(compound 4)**

To a 50 mL single-neck bottle was added **4L** (0.22 g, 0.41 mmol), same was dissolved in dioxane (10 mL), **1M** (160 mg, 1.23 mmol), $Cs_2CO_3$ (0.53 g, 1.64 mmol), xantphos (47.5 mg, 0.082 mmol) and $Pd_2(dba)_3$ (41 mg, 0.041 mmol) were added, the reaction system was subjected to nitrogen replacement three times, reacted at 110°C for 5 h, filtered over celite, concentrated, and subjected to preparative HPLC to obtain the title **compound 4** (30 mg, 12%).

Preparation method: instrument: waters 2767 (preparative liquid phase chromatographic instrument); chromatographic column: SunFire@ Prep C18 (19 mm × 250 mm); the sample was dissolved in DMF and filtered with a 0.45 $\mu$m filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 40%-80%; c. flow rate: 15 mL/min; d. elution time: 20 min; retention time: 10.38 min.

$^1H$ NMR (400 MHz, DMSO-d6) $\delta$ 9.40 (s, 1H), 7.05 (t, 1H), 6.64 (d, 1H), 6.52 (d, 1H), 6.08 (d, 2H), 4.95 (s, 1H), 4.50 (t, 1H), 4.38 (t, 1H), 3.95-3.85 (m, 1H), 3.60 (t, 2H), 3.55-3.47 (s, 1H), 3.04 (dd, 2H), 2.90-2.79 (m, 2H), 2.72 (t, 2H), 2.65-2.60 (m, 1H), 2.45 (t, 2H), 2.30 - 2.18 (m, 1H), 1.71-1.57 (m, 2H), 1.25 (t, 3H), 1.12 (dd, 6H), 0.94 (d, 3H).

LC-MS (ESI): m/z = 589.2 $[M+H]^+$.

**Example 5: 3-((1R,3R)-1-(2,6-difluoro-4-((1-(3-fluoropropyl)azetidin-3-yl)amino)phenyl)-6-ethynyl-3-methyl-1,3,4,9-tetrahydro-2H-pyrido[3,4-b]indol-2-yl)-2,2-difluoropropan-1-ol (compound 5)**

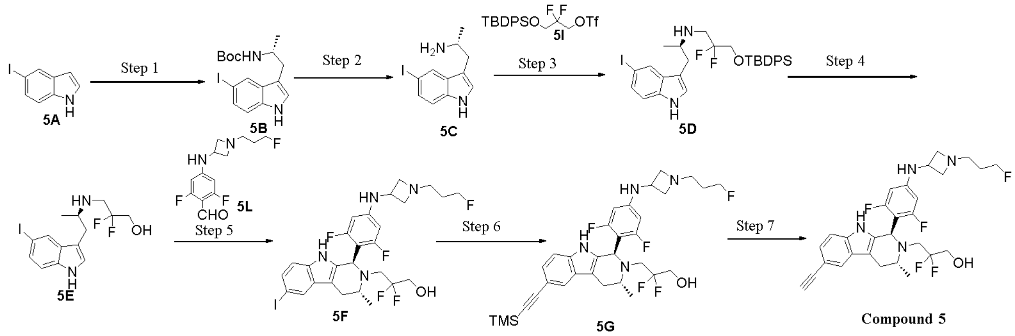


Step 1: tert-butyl (R)-(1-(5-iodo-1H-indol-3-yl)propan-2-yl)carbamate (compound **5B**)

Raw materials 5-iodoindole (10 g, 41.16 mmol) and cuprous chloride (3.5 g,35.7 mmol) were added into 150 mL of dichloromethane, placed at 0°C and under nitrogen protection, a solution of methylmagnesium chloride (14 mL, 1.5 mol/l) in THF was added dropwise at this temperature, and then reacted for 1 hour. Tert-butyl (R)-4-methyl-1,2,3-oxathiazolidine-3-carboxylate 2,2-dioxide (6.5 g, 27.42 mmol) was then added at -20°C and reacted for 20 hours. A saturated ammonium chloride solution was added to quench the reaction, extracted with EA, dried over anhydrous sodium sulfate, and filtered, the filtrate was concentrated and then same was purified and separated by silica gel column chromatography (petroleum ether : ethyl acetate (v/v) = 1 : 0-5 : 1) to obtain the title compound **5B** (7.76 g, yield: 54%).

LCMS m/z = 301.1 [M-100].

Step 2: (R)-1-(5-iodo-1H-indol-3-yl)propan-2-amine (compound **5C**)

Raw material 5**B** (3.0 g, 7.5 mmol) was added into dichloromethane (20 mL), trifluoroacetic acid (5 mL) was then added, stirred at room temperature for 1 hour, the filtrate was concentrated to obtain the title compound **5C** (5 g, yield: 90%).

LCMS m/z = 301.1 [M+1].

Step 3: (R)-3-((tert-butyldiphenylsilyl)oxy)-2,2-difluoro-N-(1-(5-iodo-1H-indol-3-yl)propan-2-yl)propan-1-amine (compound **5D**)

Reagent 3-((tert-butyldiphenylsilyl)oxy)-2,2-difluoropropyl trifluoromethanesulfonate (6.74 g, 13.98 mmol), raw materials **5C** (4 g, 7.5 mmol) and DIPEA (3.64 g, 28.00 mmol) were added into 1,4-dioxane solvent (80 mL), heated to 100°C and stirred for 16 hours, cooled, and directly purified and separated by silica gel column chromatography (petroleum ether : ethyl acetate (v/v) = 1 : 0-5 : 1) to obtain the title compound **5D** (2 g, yield: 43%).

LCMS m/z =633.1 [M+1].

Step 4: (R)-2,2-difluoro-3-((1-(5-iodo-1H-indol-3-yl)propan-2-yl)amino)propan-1-ol (compound **5E**)

Raw material **5D** (1.26 g, 1.99 mmol) was dissolved in THF (20 mL) solvent, TBAF solution (3.98 mL, 3.98 mmol, 1M) was then added, stirred at room temperature for 1 hour, diluted with water, extracted with EA, dried over anhydrous sodium sulfate, and filtered, the filtrate was concentrated and then purified and separated by silica gel column chromatography (dichloromethane : anhydrous methanol (v/v) = 1 : 0-10 : 1) to obtain the title compound **5E** (0.48 g, yield: 61%).

LCMS m/z = 395.1 [M+1].

Step 5: 3-((1R,3R)-1-(2,6-difluoro-4-((1-(3-fluoropropyl)azetidin-3-yl)amino)phenyl)-6-iodo-3-methyl-1,3,4,9-tetrahydro-2H-pyrido[3,4-b]indol-2-yl)-2,2-difluoropropan-1-ol (compound **5F**)

Raw material **5D** (250 mg, 0.63 mmol) and raw material **5L (**obtained by the synthetic method in WO 2019/245974) (190 mg, 0.69 mmol) were added into a mixed solvent of toluene (4 mL) and acetic acid (1 mL), then heated to 95°C and reacted for 1 hour, directly concentrated to dryness, and purified by TLC (DCM : MeOH = 5 : 1) to obtain the title compound (200 mg, yield: 49%).

LCMS m/z = 649.1 [M+1].

Step 6: 3-((1R,3R)-1-(2,6-difluoro-4-((1-(3-fluoropropyl)azetidin-3-yl)amino) phenyl)-3-methyl-6-((trimethylsilyl)ethynyl)-1,3,4,9-tetrahydro-2H-pyrido[3,4-b]indol-2-yl)-2,2-difluoropropan-1-ol (compound **5G**)

Raw materials **5F** (250 mg, 0.39 mmol), trimethylethynylsilane (110 mg, 1.17 mmol), triethylamine (390 mg, 0.39 mmol), tetrakis(triphenylphosphine)palladium (45 mg, 0.039 mmol) and cuprous iodide (15 mg, 0.078 mmol) were added into anhydrous DMF (10 mL) solvent, heated under nitrogen protection to 65°C and stirred for 5 hours, cooled, and filtered, the filtrate was concentrated and then subjected to silica gel column chromatography (dichloromethane : anhydrous methanol (v/v) = 1 : 0-10 : 1) to obtain the title compound **5G** (200 mg, yield: 83%).
LCMS m/z = 619.3 [M+1].

Step 7: 3-((1R,3R)-1-(2,6-difluoro-4-((1-(3-fluoropropyl)azetidin-3-yl)amino) phenyl)-6-ethynyl-3-methyl-1,3,4,9-tetrahydro-2H-pyrido[3,4-b]indol-2-yl)-2,2-difluoropropan-1-ol (compound **5**)

Raw material **5G** (40 mg, 0.065 mmol) was dissolved in anhydrous methanol (5 mL) solvent, potassium carbonate (18 mg, 0.13 mmol) was added at room temperature, reacted for 1 hours, and filtered, and the filtrate was concentrated and then purified and separated by silica gel column chromatography (dichloromethane : anhydrous methanol (v/v) = 1 : 0-5 : 1) to obtain the title compound **5** (5 mg, yield: 14%).
LCMS m/z = 547.3 [M+1].
$^1$H NMR (400 MHz, $CD_3OD$) δ 7.54 (s, 1H), 7.10-7.16 (m, 2H), 6.08-6.13 (m, 2H), 5.15 (s, 1H), 4.51 (t, 1H), 4.39 (t, 1H), 4.01-4.07 (m, 1H), 3.73-3.80 (m, 3H), 3.58-3.63 (m, 1H), 3.42-3.52 (m, 1H), 3.20 (s, 1H), 3.09-3.17 (m, 1H), 2.94-2.99 (m, 3H), 2.71-2.82 (m, 1H), 2.56-2.65 (m, 3H), 1.69-1.82 (m, 2H), 1.14 (d, 3H).

**Example 6: 3-((1R,3R)-1-(2,6-difluoro-4-((1-(3-fluoropropyl)azetidin-3-yl)amino)phenyl)-3-methyl-6-vinyl-1,3,4,9-tetrahydro-2H-pyrido[3,4-b]indol-2-yl)-2,2-difluoropropan-1-ol (compound 6)**

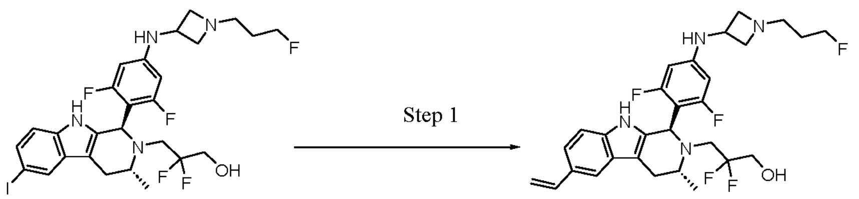

5F
Compound 6

Raw materials **5F** (150 mg, 0.23 mmol), tributyl(vinyl)tin (110 mg, 0.35 mmol), potassium carbonate (48 mg, 0.35 mmol), bis(triphenylphosphine)palladium(II) dichloride (16 mg, 0.023 mmol) and tetraethyl ammonium chloride (76 mg, 0.46 mmol) were added to anhydrous DMF (10 mL) solvent, heated under nitrogen protection to 100°C and stirred for 3 hours, cooled, and filtered, and the filtrate was concentrated and then subjected to silica gel column chromatography (dichloromethane : anhydrous methanol (v/v) = 1 : 0-10 : 1) to obtain the title compound **6** (60 mg, yield: 48%).
LCMS m/z = 549.3 [M+1].
$^1$H NMR (400 MHz, $CD_3OD$) δ 7.42 (s, 1H), 7.13-7.19 (m, 2H), 6.76-6.83 (m, 1H), 6.08-6.13 (m, 2H), 5.60-5.65 (m, 1H), 5.14 (s, 1H), 5.01-5.04 (m, 1H), 4.51 (t, 1H), 4.39 (t, 1H), 4.01-4.07 (m, 1H), 3.73-3.84 (m, 3H), 3.58-3.63 (m, 1H), 3.42-3.52 (m, 1H), 3.09-3.19 (m, 1H), 2.95-2.99 (m, 3H), 2.71-2.82 (m, 1H), 2.58-2.66 (m, 3H), 1.69-1.82 (m, 2H), 1.15 (d, 3H).

**Example 7: 3-((1R,3R)-1-(2,6-difluoro-4-((1-(3-fluoropropyl)azetidin-3-yl)amino)phenyl)-3,7-dimethyl-1,3,4,9-tetrahydro-2H-pyrido[3,4-b]indol-2-yl)-2,2-difluoropropan-1-ol (compound 7)**

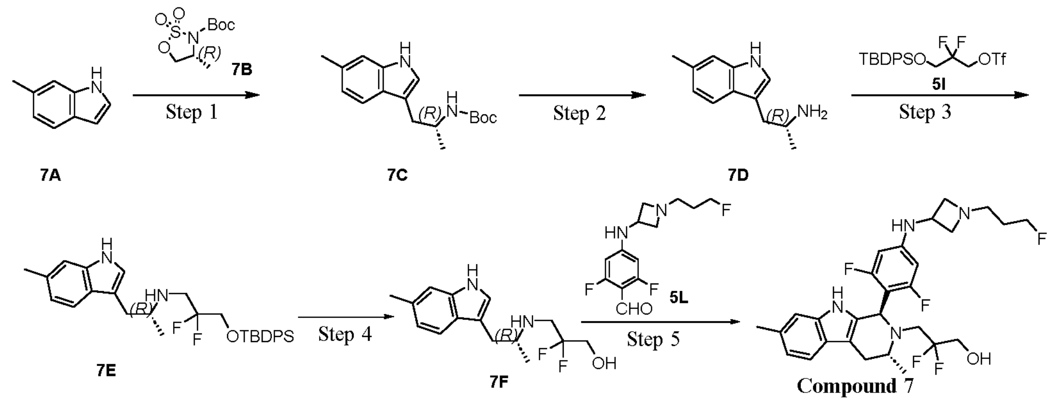


Step 1: tert-butyl (R)-(1-(6-methyl-1H-indol-3-yl)propan-2-yl)carbamate **(7C)**

To a 500 mL three-neck bottle were added **7A** (8.0 g, 60.99 mmol) and cuprous chloride (5.25 g, 53.06 mmol), same was dissolved in anhydrous dichloromethane (80 mL), cooled to 0°C, methylmagnesium bromide (17.7 mL, 3 M) was added dropwise, the reaction was carried out while maintaining this temperature constant for 1 h, cooled to -20°C, a solution **of 7B** (9.6 g, 40.25 mmol) in dichloromethane (20 mL) was added dropwise, stirred overnight while maintaining this temperature constant, quenched with 10% citric acid solution (200 mL), stirred for 0.5 h, filtered through celite, and extracted with dichloromethane (2 × 100 mL), and the organic phases were combined, dried over anhydrous sodium sulfate, concentrated and purified by column chromatography (petroleum ether : ethyl acetate = 10 : 1-5 : 1) to obtain the title compound **7C** (8.0 g, 45%).

LC-MS (ESI): m/z = 289.3 $[M+H]^+$.

Step 2: (R)-1-(6-methyl-1H-indol-3-yl)propan-2-amine **(7D)**

To a 100 mL three-neck bottle was added **7C** (4.0 g, 13.87 mmol), same was dissolved in anhydrous methanol (20 mL), hydrogen chloride in dioxane (20 mL) was added, stirred at room temperature for 2 h, and concentrated to obtain the title compound **7D** (2.61 g, 99%).

LC-MS (ESI): m/z = 189.3 $[M+H]^+$.

Step 3: (R)-3-((tert-butyldiphenylsilyl)oxy)-2,2-difluoro-N-(1-(6-methyl-1H-indol-3-yl)propan-2-yl)propan-1-amine **(7E)**

To a 100 mL three-neck bottle was added **7D** (2.6 g, 13.81 mmol), and same was dissolved in dioxane (26 mL) and DMF (2 mL), and **5I** (8.0 g, 16.57 mmol) and N,N-diisopropylethylamine (5.35 g, 41.43 mmol) were added. The mixture was stirred at 90°C for 18 h, concentrated, and purified by column chromatography (petroleum ether : ethyl acetate = 10 : 1-5 : 1) to obtain the title compound **7E** (1.3 g, 18%).

LC-MS (ESI): m/z = 521.3 $[M+H]^+$.

Step 4: (R)-2,2-difluoro-3-((1-(6-methyl-1H-indol-3-yl)propan-2-yl)amino)propan-1-ol **(7F)**

To a 100 mL three-neck bottle was added **7E** (1.3 g, 2.5 mmol), same was dissolved in tetrahydrofuran (10 mL), tetrabutylammonium fluoride (5 mL, 1 M) was added, stirred at room temperature for 3 h, washed with water (20 mL), extracted with ethyl acetate (2 × 20 mL), and the organic phases were combined, dried over anhydrous sodium sulfate, concentrated, and purified by column chromatography (petroleum ether : ethyl acetate = 2 : 1) to obtain the title compound **7F** (0.56 g, 79%).

LC-MS (ESI): m/z = 283.2 $[M+H]^+$.

Step 5: 3-((1R,3R)-1-(2,6-difluoro-4-((1-(3-fluoropropyl)azetidin-3-yl)amino) phenyl)-3,7-dimethyl-1,3,4,9-tetrahydro-2H-pyrido[3,4-b]indol-2-yl)-2,2-difluoropropan-1-ol (compound 7)

To a 50 mL single-neck bottle were added **7F** (0.1 g, 0.35 mmol) and **5L** (obtained by the synthetic method in WO 2019/245974) (95 mg, 0.35 mmol), same was dissolved in toluene (8 mL), acetic acid (2 mL) was added, stirred under nitrogen protection at 100°C for 3 h, concentrated, and subjected to preparative HPLC to obtain the title compound **7** (45 mg, 24%).

Preparation method: instrument: waters 2767 (preparative liquid phase chromatographic instrument); chromatographic column: SunFire@ Prep C18 (19 mm × 250 mm); the sample was dissolved in DMF and filtered with a 0.45 μm filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 40%-70%; c. flow rate: 15 mL/min; d. elution time: 20 min; retention time: 10.60 min.

$^1$H NMR (400 MHz, DMSO-d6) δ 10.33 (s, 1H), 7.24 (d, 1H), 6.97 (s, 1H), 6.76 (dd, 1H), 6.67 (d, 1H), 6.09 (d, 2H), 5.22 (s, 1H), 5.03 (s, 1H), 4.51 (t, 1H), 4.39 (t, 1H), 3.93 (q, 1H), 3.75 - 3.53 (m, 4H), 3.51 - 3.38 (m, 2H), 3.16 - 2.99 (m, 1H), 2.82 - 2.71 (m, 3H), 2.62 (q, 1H), 2.46 (t, 2H), 2.34 (s, 3H), 1.66 (dq, 2H), 1.06 (d, 3H).

LC-MS (ESI): m/z = 537.3 $[M+H]^+$.

**Example 8: 3-((1R,3R)-1-(2,6-difluoro-4-(((S)-1-(3-fluoropropyl) pyrrolidin-3-yl)amino)phenyl)-3,7-dimethyl-1,3,4,9-tetrahydro-2H-pyrido[3,4-b]indol-2-yl)-2,2-difluoropropan-1-ol (compound 8)**

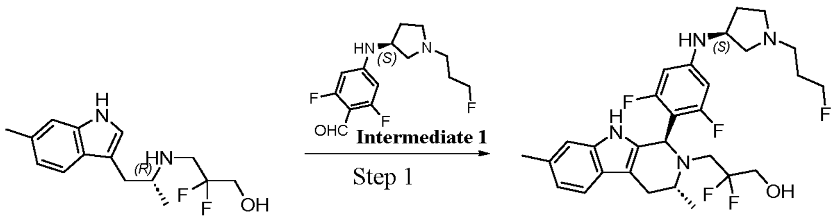


7F

Compound 8

Step 1: 3-((1R,3R)-1-(2,6-difluoro-4-(((S)-1-(3-fluoropropyl)pyrrolidin-3-yl)amino)phenyl)-3,7-dimethyl-1,3,4,9-tetrahydro-2H-pyrido[3,4-b]indol-2-yl)-2,2-difluoropropan-1-ol **(compound 8)**

To a 50 mL single-neck bottle were added **7F** (0.13 g, 0.46 mmol) and **intermediate 1** (0.13 g, 0.35 mmol), same was dissolved in toluene (8 mL), acetic acid (2 mL) was added, stirred under nitrogen protection at 100°C for 3 h, concentrated, and subjected to preparative HPLC to obtain the title compound **8** (100 mg, 39%).

Preparation method: instrument: waters 2767 (preparative liquid phase chromatographic instrument); chromatographic column: SunFire@ Prep C18 (19 mm × 250 mm); the sample was dissolved in DMF and filtered with a 0.45 μm filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 40%-70%; c. flow rate: 15 mL/min; d. elution time: 20 min; retention time: 11.20 min.

$^1$H NMR (400 MHz, DMSO-d6) δ 10.33 (s, 1H), 7.24 (d, 1H), 6.97 (s, 1H), 6.76 (dd, 1H), 6.37 (d, 1H), 6.17 - 6.08 (m, 2H), 5.21 (s, 1H), 5.03 (s, 1H), 4.54 (t, 1H), 4.42 (t, 1H), 3.83 (qd, 1H), 3.66 (q, 1H), 3.42 (dt, 2H), 3.15 - 2.96 (m, 1H), 2.84 - 2.73 (m, 2H), 2.71 - 2.57 (m, 2H), 2.48 - 2.38 (m, 4H), 2.34 (s, 4H), 2.24-2.15 (m, 1H), 1.87 - 1.72 (m, 2H), 1.62 - 1.47 (m, 1H), 1.06 (d, 3H).

LC-MS (ESI): m/z = 551.3 $[M+H]^+$.

**Example 9: 3-((1R,3R)-1-(2,6-difluoro-4-((1-(3-fluoropropyl)azetidin-3-yl)amino)phenyl)-3,6-dimethyl-1,3,4,9-tetrahydro-2H-pyrido[3,4-b]indol-2-yl)-2,2-difluoropropan-1-ol (compound 9)**

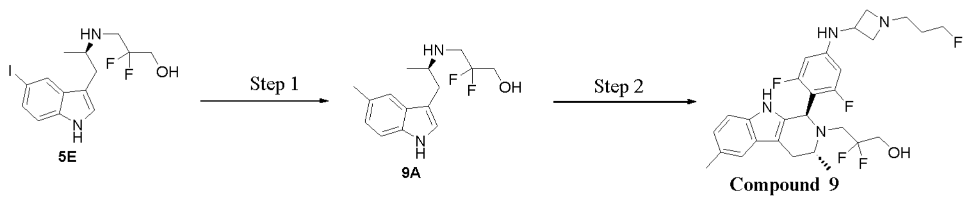


5E 9A Compound 9

Step 1: (R)-2,2-difluoro-3-((1-(5-methyl-1H-indol-3-yl)propan-2-yl)amino) propan-1-ol (compound **9A**)

Raw materials **5E** (100 mg, 0.25 mmol), trimethylboroxine (78 mg, 0.63 mmol), tetrakis(triphenylphosphine)palladium (35 mg, 0.030 mmol) and potassium carbonate (140 mg, 1.0 mmol) were added into anhydrous DMF (10 mL)

solvent, under nitrogen protection, the mixture was heated to 65°C and stirred for 5 hours, cooled, and filtered, and the filtrate was concentrated and subjected to silica gel column chromatography (dichloromethane : anhydrous methanol (v/v) = 1 : 0-10 : 1) to obtain the title compound **9A** (50 mg, yield: 28%).

LCMS m/z = 283.2 [M+1].

Step 2: 3-((1R,3R)-1-(2,6-difluoro-4-((1-(3-fluoropropyl)azetidin-3-yl)amino) phenyl)-3,6-dimethyl-1,3,4,9-tetrahydro-2H-pyrido[3,4-b]indol-2-yl)-2,2-difluoropropan-1-ol (compound **9**)

Raw materials **9A** (200 mg, 0.71 mmol) and **5L** (190 mg, 0.71 mmol) were added into a mixed solvent of toluene (4 mL) and acetic acid (1 mL), then heated to 95°C and reacted for 1 hour, directly concentrated to dryness, and then purified by TLC (DCM : MeOH = 5 : 1) to obtain the title compound **9** (5 mg, yield: 1%).

LCMS m/z = 537.3 [M+1].

$^1$H NMR (400 MHz, DMSO) δ 10.35 (s, 1H), 7.15 (s, 1H), 7.06 (d, 1H), 6.81 (d, 1H), 6.68 (d, 1H), 6.10 (d, 2H), 5.02 (s, 1H), 4.51 (t, 1H), 4.39 (t, 1H), 3.90-3.97 (m, 1H), 3.61-3.71 (m, 3H), 3.39-3.46 (m, 2H), 3.01-3.12 (m, 1H), 2.72-2.80 (m, 3H), 2.58-2.67 (m, 2H), 2.43-2.46 (m, 2H), 2.35 (s, 3H), 1.97-2.01 (m, 1H), 1.59-1.70 (m, 2H), 1.06 (d, 3H).

**Example 10: 3-((1R,3R)-1-(2,6-difluoro-4-((1-(3-fluoropropyl)azetidin-3-yl)amino)phenyl)-6-methoxy-3-methyl-1,3,4,9-tetrahydro-2H-pyrido[3,4-b]indol-2-yl)-2,2-difluoropropan-1-ol (compound 10)**

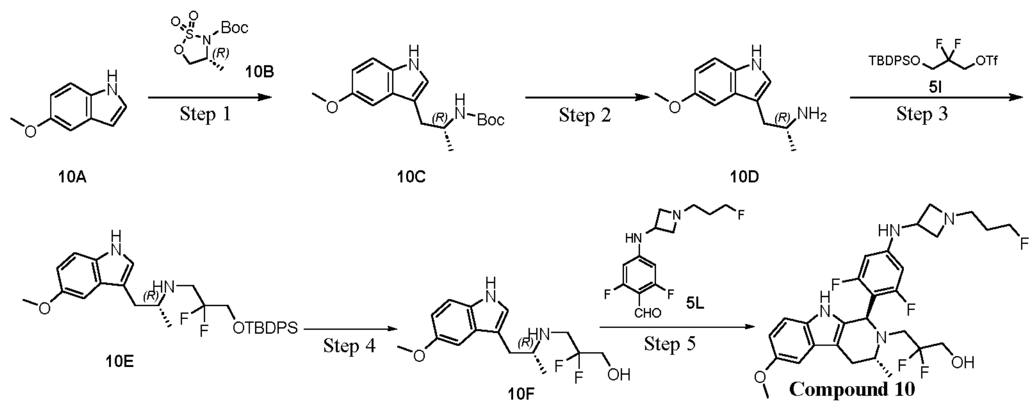


Step 1: tert-butyl (R)-(1-(5-methoxy-1H-indol-3-yl)propan-2-yl)carbamate **(10C)**

To a 250 mL three-neck bottle were added **10A** (5.0 g, 33.97 mmol) and cuprous chloride (2.69 g, 27.18 mmol), same was dissolved in anhydrous dichloromethane (50 mL), cooled to 0°C, methylmagnesium bromide (11.32 mL, 3 M) was added dropwise, the reaction was carried out with the temperature maintained constant for 1 h, cooled to -20°C, a solution of **10B** (5.64 g, 23.78 mmol) in dichloromethane (20 mL) was added dropwise, stirred overnight while maintaining this temperature constant, quenched with 10% citric acid solution (100 mL), stirred for 0.5 h, filtered through celite, and extracted with dichloromethane (2 × 100 mL), and the organic phases were combined, dried over anhydrous sodium sulfate, concentrated and purified by column chromatography (petroleum ether : ethyl acetate = 10 : 1-5 : 1) to obtain the title compound **10C** (4.0 g, 38.5%).

LC-MS (ESI): m/z = 305.2[M+H]$^+$.

Step 2: (R)-1-(5-methoxy-1H-indol-3-yl)propan-2-amine **(10D)**

To a 100 mL three-neck bottle was added **10C** (4.0 g, 13.14 mmol), same was dissolved in anhydrous methanol (20 mL), hydrogen chloride in dioxane (20 mL) was added, stirred at room temperature for 2 h, and concentrated to obtain the title compound **10D** (3.0 g, crude).

LC-MS (ESI): m/z = 205.3 [M+H]$^+$.

Step 3: (R)-3-((tert-butyldiphenylsilyl)oxy)-2,2-difluoro-N-(1-(5-methoxy-1H-indol-3-yl)propan-2-yl)propan-1-amine **(10E)**

To a 100 mL three-neck bottle was added **10D** (3.0 g, 14.69 mmol), same was dissolved in dioxane (40 mL),

5I (8.51 g, 17.63 mmol) and N,N-diisopropyl ethylamine (5.70 g, 44.07 mmol) were added, stirred at 90°C for 12 h, concentrated, and purified by column chromatography (petroleum ether : ethyl acetate = 10 : 1-5 : 1) to obtain the title compound **10E** (3.5 g, 44.39%).

LC-MS (ESI): m/z = 537.3 $[M+H]^+$.

Step 4: (R)-2,2-difluoro-3-((1-(5-methoxy-1H-indol-3-yl)propan-2-yl)amino) propan-1-ol **(10F)**

To a 10 mL three-neck bottle was added **10E** (0.70 g, 1.30 mmol), same was dissolved in tetrahydrofuran (20 mL), tetrabutylammonium fluoride (3.9 mL, 1 M) was added, stirred at room temperature for 3 h, washed with water (20 mL), extracted with ethyl acetate (2 × 20 mL), and the organic phases were combined, dried over anhydrous sodium sulfate, concentrated, and purified by column chromatography (petroleum ether : ethyl acetate = 2 : 1) to obtain the title compound **10F** (0.32 g, 82%).

LC-MS (ESI): m/z = 299.2 $[M+H]^+$.

Step 5: 3-((1R,3R)-1-(2,6-difluoro-4-((1-(3-fluoropropyl)azetidin-3-yl)amino) phenyl)-6-methoxy-3-methyl-1,3,4,9-tetrahydro-2H-pyrido[3,4-b]indol-2-yl)-2,2-difluoropropan-1-ol **(compound 10)**

To a 50 mL single-neck bottle were added **10F** (0.1 g, 0.34 mmol) and **5L** (110 mg, 0.41 mmol), same was dissolved in toluene (8 mL), acetic acid (2 mL) was added, stirred under nitrogen protection at 100°C for 3 h, concentrated, and subjected to preparative HPLC to obtain the title compound **10** (51 mg, 27.1%).

Preparation method: instrument: waters 2767 (preparative liquid phase chromatographic instrument); chromatographic column: SunFire@ Prep C18 (19 mm × 250 mm); the sample was dissolved in methanol and filtered with a 0.45 μm filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 30%-65%; c. flow rate: 15 mL/min; d. elution time: 15 min; retention time: 8.40 min.

$^1$H NMR (400 MHz, DMSO-d6) δ 10.32 (s, 1H), 7.06 (d, 1H), 6.87 (d, 1H), 6.67 (d, 1H), 6.62 (dd, 1H), 6.10 (d, 2H), 5.22 (t, 1H), 5.02 (s, 1H), 4.51 (t, 1H), 4.39 (t, 1H), 3.91-3.96 (m, 1H), 3.74 (s, 3H), 3.60 - 3.63 (m, 3H), 3.37 - 3.47 (m, 2H), 3.01 - 3.13 (m, 1H), 2.72 - 2.83 (m, 3H), 2.56 - 2.66 (m, 1H), 2.46 (t, 3H), 1.58 - 1.69 (m, 2H), 1.06 (d, 3H).

LC-MS (ESI): m/z = 553.3 $[M+H]^+$.

**Example 11: 3-((1R,3R)-7-(cyclopropylmethoxy)-1-(2,6-difluoro-4-((1-(3-fluoropropyl)azetidin-3-yl)amino)phenyl)-3-methyl-1,3,4,9-tetrahydro-2H-pyrido[3,4-b]indol-2-yl)-2,2-difluoropropan-1-ol (compound 11)**

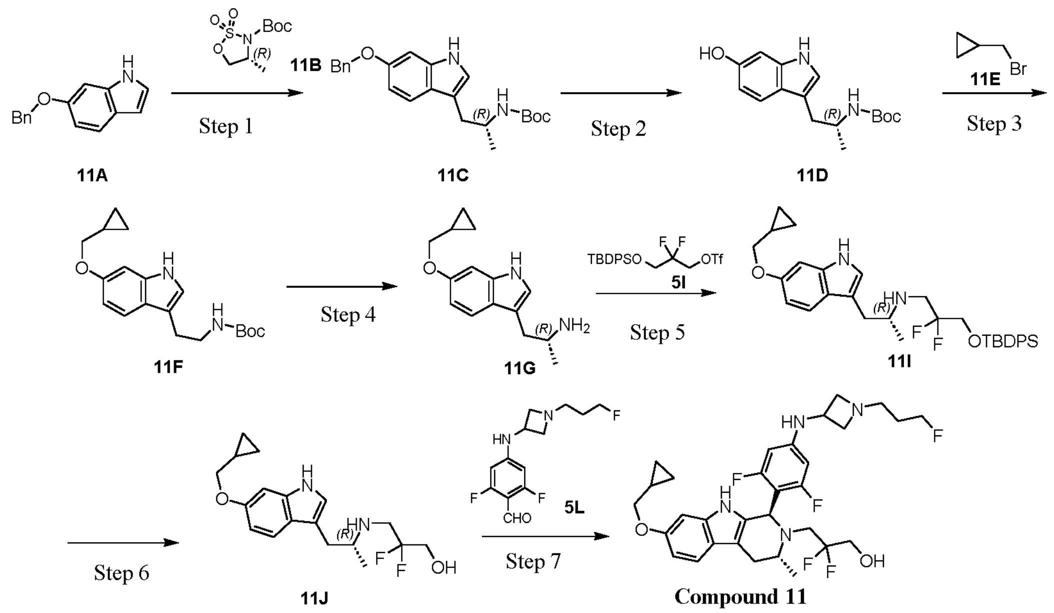

Step 1: tert-butyl (R)-(1-(6-(benzyloxy)-1H-indol-3-yl)propan-2-yl)carbamate (11C)

To a 250 mL three-neck bottle was added **11A** (5.00 g, 22.39 mmol) and cuprous chloride (2.35 g, 23.7 mmol), same was dissolved in THF (50 mL), the system was cooled to 0°C in a dry ice ethanol bath, and subjected to nitrogen replacement three times, methylmagnesium bromide (2.5 M, 9.48 mL, 23.70 mmol) was added dropwise, stirred for 1 h while maintaining this temperature constant, cooled to -20°C, a solution of **11B** (2.5 g, 18.23 mmol) in THF (20 mL) was added dropwise, stirred for 12 h while maintaining this temperature constant, quenched with citric acid solution (1M, 100 mL), stirred at room temperature for 0.5 h, extracted with EA (2 × 100 mL), and the organic phases were combined, dried over anhydrous sodium sulfate for 0.5 h, and concentrated to obtain the title compound 11C (2.5 g, crude).
LC-MS (ESI): m/z = 381.2 $[M+H]^+$.

Step 2: tert-butyl (R)-(1-(6-hydroxy-1H-indol-3-yl)propan-2-yl)carbamate **(11D)**

To a 100 mL single-neck bottle was added **11C** (2.5 g, crude), same was dissolved in MeOH (30 mL), wet palladium carbon (140 mg) was added, reacted at room temperature for 12 h, filtered, concentrated, and purified by column chromatography (petroleum ether : ethyl acetate (v/v) = 20 : 1-1 : 1) to obtain the title compound **11D** (1.6 g, 83%).
$^1$H NMR (400 MHz, DMSO-d6) $\delta$ 10.34 (s, 1H), 8.77 (s, 1H), 7.29 (d, 1H), 6.85 (d, 1H), 6.67 (d, 1H), 6.65 (s, 1H), 6.48-6.61 (t, 1H), 3.66-3.71 (t, 1H), 2.76-2.81 (m, 1H), 2.52-2.57 (t, 1H), 1.37 (s, 9H), 0.99 (d, 3H).

Step 3: tert-butyl (R)-(1-(6-(cyclopropylmethoxy)-1H-indol-3-yl)propan-2-yl)carbamate **(11F)**

To a 50 mL single-neck bottle was added **11D** (1.20 g, 4.13 mmol), same was dissolved in DMF (20 mL), cesium carbonate (4.04 g, 12.39 mmol) and **11E** (0.56 g, 4.13 mmol) were added, reacted at 25°C for 12 h, concentrated and purified by column chromatography (petroleum ether : ethyl acetate (v/v) = 10 : 1-4 : 1) to obtain the title compound **11F** (0.80 g, 56.2%).
LC-MS (ESI): m/z = 345.2$[M+H]^+$.

Step 4: (R)-1-(6-(cyclopropylmethoxy)-1H-indol-3-yl)propan-2-amine **(11G)**

To a 100 mL single-neck bottle was added **11F** (0.80 g, 2.32 mmol), same was dissolved in dichloromethane (15 mL), trifluoroacetic acid (5 mL) was added, reacted at 25°C for 2 h, and concentrated to obtain the title compound **11G** (0.90 g, crude).
LC-MS (ESI): m/z = 245.2 $[M+H]^+$.

Step 5: (R)-3-((tert-butyldiphenylsilyl)oxy)-N-(1-(6-(cyclopropylmethoxy)-1H-indol-3-yl)propan-2-yl)-2,2-difluoropropan-1-amine **(11I)**

To a 100 mL three-neck bottle was added **11G** (0.90 g, 3.68 mmol), same was dissolved in dioxane (20 mL), 5I (2.66 g, 5.52 mmol) and N,N-diisopropyl ethylamine (1.43, 11.04 mmol) were added, stirred at 90°C for 12 h, concentrated, and purified by column chromatography (petroleum ether : ethyl acetate = 10 : 1-5 : 1) to obtain the title compound **11I** (0.5 g, 24%).
LC-MS (ESI): m/z = 577.1 $[M+H]^+$.

Step 6: (R)-3-((1-(6-(cyclopropylmethoxy)-1H-indol-3-yl)propan-2-yl)amino) -2,2-difluoropropan-1-ol **(11J)**

To a 100 mL three-neck bottle was added **11I** (0.5 g, 0.87 mmol), same was dissolved in tetrahydrofuran (10 mL), tetrabutylammonium fluoride (2.61 mL, 1 M) was added, stirred at room temperature for 3 h, washed with water (20 mL), extracted with ethyl acetate (2 × 20 mL), the organic phases were combined, dried over anhydrous sodium sulfate, concentrated, and purified by column chromatography (petroleum ether : ethyl acetate = 2 : 1) to obtain the title compound **11J** (0.20 g, 67.9%).
LC-MS (ESI): m/z = 338.2 $[M+H]^+$.

Step 7: 3-((1R,3R)-7-(cyclopropylmethoxy)-1-(2,6-difluoro-4-((1-(3-fluoropropyl)azetidin-3-yl)amino)phenyl)-3-methyl-1,3,4,9-tetrahydro-2H-pyrido[3,4-b]indol-2-yl)-2,2-difluoropropan-1-ol **(compound 11)**

To a 50 mL single-neck bottle were added **11J** (50.00 mg, 0.15 mmol) and **5L** (49 mg, 0.18 mmol), same was dissolved in toluene (4 mL), acetic acid (1 mL) was added, stirred under nitrogen protection at 100°C for 3 h, concentrated, and subjected to preparative HPLC to obtain the title compound **11** (7 mg, 7.9%).

Preparation method: instrument: waters 2767 (preparative liquid phase chromatographic instrument); chromatographic column: SunFire@ Prep C18 (19 mm × 250 mm); the sample was dissolved in DMF and filtered with a 0.45 μm filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 40%-80%; c. flow rate: 15 mL/min; d. elution time: 20 min; retention time: 10.38 min.

$^1H$ NMR (400 MHz, DMSO-d6) δ 10.28 (s, 1H), 7.22 (d, 1H), 6.65-6.68 (m, 2H), 6.59 (dd, 1H), 6.09 (d, 2H), 5.21 (s, 1H), 5.01 (s, 1H),4.51 (t, 1H), 4.39 (t, 1H), 3.89-3.97 (m, 1H), 3.76 (d, 2H), 3.58-3.68 (m, 3H), 3.36-3.44 (m, 3H), 3.00-3.11 (m, 1H), 2.71-2.78 (m, 3H), 2.59-2.66 (m, 1H), 2.43-2.47 (m, 2H), 1.58 - 1.71 (m, 2H), 1.15-1.25 (m, 1H), 1.05 (d, 3H), 0.52-0.57 (m, 2H), 0.28-0.32(m, 2H).

LC-MS (ESI): m/z = 592.2 $[M+H]^+$.

**Example 12: 3-((1R,3R)-1-(2,6-difluoro-4-((1-(3-fluoropropyl)azetidin-3-yl)amino)phenyl)-3-methyl-7-(1H-pyrazol-4-yl)-1,3,4,9-tetrahydro-2H-pyrido[3,4-b]indol-2-yl)-2,2-difluoropropan-1-ol(compound 12)**

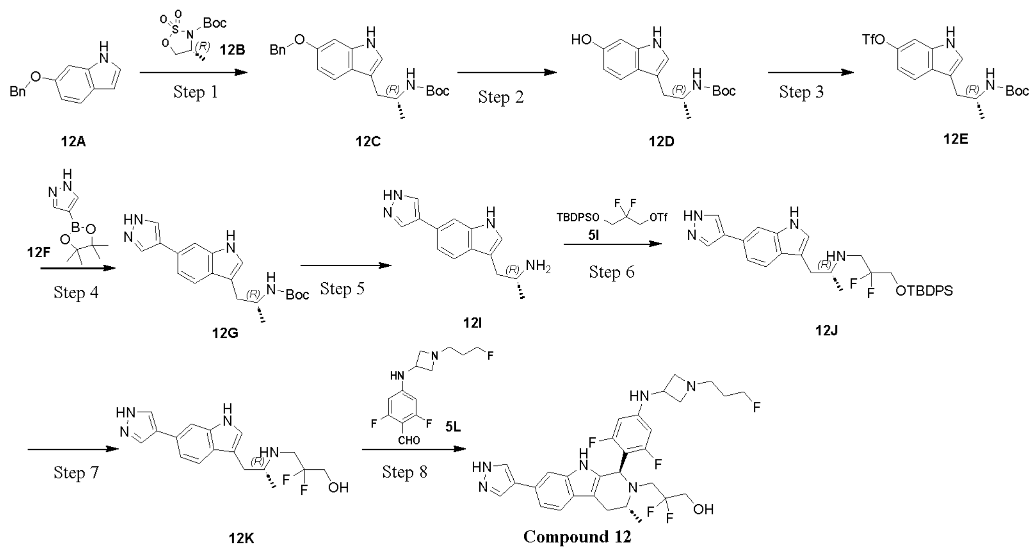


Step 1: tert-butyl (R)-(1-(6-(benzyloxy)-1H-indol-3-yl)propan-2-yl)carbamate **(12C)**

To a 250 mL three-neck bottle was added **12A** (5.00 g, 22.39 mmol) and cuprous chloride (2.35 g, 23.7 mmol), same was dissolved in THF (50 mL), the system was cooled to 0°C in a dry ice ethanol bath, and subjected to nitrogen replacement three times, methylmagnesium bromide (2.5 M, 9.48 mL, 23.70 mmol) was added dropwise, stirred for 1 h while maintaining this temperature constant, cooled to -20°C, a solution of **12B** (2.5 g, 18.23 mmol) in THF (20 mL) was added dropwise, stirred for 12 h while maintaining this temperature constant, quenched with citric acid solution (1M, 100 mL), stirred at room temperature for 0.5 h, extracted with EA (2 × 100 mL), and the organic phases were combined, dried over anhydrous sodium sulfate for 0.5 h, and concentrated to obtain the title compound **12C** (2.5 g, crude).

LC-MS (ESI): m/z = 381.2 $[M+H]^+$.

Step 2: tert-butyl (R)-(1-(6-hydroxy-1H-indol-3-yl)propan-2-yl)carbamate **(12D)**

To a 100 mL single-neck bottle was added **12C** (2.5 g, crude), same was dissolved in MeOH (30 mL), wet palladium carbon (140 mg) was added, reacted at room temperature for 12 h, filtered, concentrated, and purified by column chromatography (petroleum ether : ethyl acetate (v/v) = 20 : 1-1 : 1) to obtain the title compound **12D** (1.7 g, 85%).

$^1H$ NMR (400 MHz, DMSO-d6) δ 10.34 (s, 1H), 8.77 (s, 1H), 7.29 (d, 1H), 6.85 (d, 1H), 6.67 (d, 1H), 6.65 (s, 1H), 6.48-6.61 (t, 1H), 3.66-3.71 (t, 1H), 2.76-2.81 (m, 1H), 2.52-2.57 (t, 1H), 1.37 (s, 9H), 0.99 (d, 3H).

Step 3: (R)-3-(2-((tert-butoxycarbonyl)amino)propyl)-1H-indol-6-yl trifluoromethanesulfonate **(12E)**

To a 50 mL single-neck bottle was added **12D** (1.70 g, 5.85 mmol), same was dissolved in dichloromethane (20 mL), N-phenylbis(trifluoromethanesulfonyl)imide (4.18 g, 11.7 mmol) and **triethylamine** (1.18 g, 11.7 mmol) were

added, reacted at 25°C for 12 h, concentrated, and purified by column chromatography (petroleum ether : ethyl acetate (v/v) = 10 : 1-4 : 1) to obtain the title compound **12E** (1.40 g, 56.6%).
LC-MS (ESI): m/z = 423.2[M+H]+.

Step 4: tert-butyl (R)-(1-(6-(1H-pyrazol-4-yl)-1H-indol-3-yl)propan-2-yl)carbamate **(12G)**

To a 100 mL single-neck bottle was added **12E** (1.30 g, 3.08 mmol), **12F** (0.32 g, 3.70 mmol) and [1,1'-bis(diphenylphosphino)ferrocene]palladium(II) dichloride (0.23 g, 0.31 mmol), same was dissolved in 1,4-dioxane (20 mL), water (4 mL) was added, reacted at 90°C for 12 h, concentrated, and purified by column chromatography (petroleum ether : ethyl acetate (v/v) = 10 : 1-3 : 1) to obtain the title compound **12G** (0.70 g, 66.8%).
LC-MS (ESI): m/z = 341.2 [M+H]+.

Step 5: (R)-1-(6-(1H-pyrazol-4-yl)-1H-indol-3-yl)propan-2-amine **(12I)**

To a 100 mL single-neck bottle was added **12G** (0.70 g, 2.05 mmol), same was dissolved in dichloromethane (15 mL), trifluoroacetic acid (5 mL) was added, reacted at 25°C for 2 h, and concentrated to obtain the title compound **12I** (0.50 g, crude).
LC-MS (ESI): m/z = 240.1 [M+H]+.

Step 6: (R)-N-(1-(6-(1H-pyrazol-4-yl)-1H-indol-3-yl)propan-2-yl)-3-((tert-butyldiphenylsilyl)oxy)-2,2-difluoropropan-1-amine **(12J)**

To a 100 mL three-neck bottle was added **12I** (0.40 g, 1.66 mmol), same was dissolved in dioxane (20 mL), 5I (1.60 g, 3.32 mmol) and N,N-diisopropyl ethylamine (0.64 g, 4.98 mmol) were added, stirred at 90°C for 12 h, concentrated, and purified by column chromatography (petroleum ether : ethyl acetate = 10 : 1-5 : 1) to obtain the title compound **12J** (0.3 g, 31.6%).
LC-MS (ESI): m/z = 573.2 [M+H]+.

Step 7: (R)-3-((1-(6-(1H-pyrazol-4-yl)-1H-indol-3-yl)propan-2-yl)amino)-2,2-difluoropropan-1-ol **(12K)**

To a 100 mL three-neck bottle was added **12J** (0.30 g, 0.52 mmol), same was dissolved in tetrahydrofuran (10 mL), tetrabutylammonium fluoride (0.43 mL, 1 M) was added, stirred at room temperature for 3 h, washed with water (20 mL), extracted with ethyl acetate (2 × 20 mL), and the organic phases were combined, dried over anhydrous sodium sulfate, concentrated, and purified by column chromatography (petroleum ether : ethyl acetate = 2 : 1) to obtain the title compound **12K** (0.16 g, 92.0%).
LC-MS (ESI): m/z = 334.2 [M+H]+.

Step 7: 3-((1R,3R)-1-(2,6-difluoro-4-((1-(3 -fluoropropyl)azetidin-3-yl)amino) phenyl)-3-methyl-7-(1H-pyrazol-4-yl)-1,3,4,9-tetrahydro-2H-pyrido[3,4-b]indol-2-yl)-2,2-difluoropropan-1-ol **(compound 12)**

To a 50 mL single-neck bottle were added **12K** (110.0 mg, 0.33 mmol) and **5L** (110 mg, 0.40 mmol), same was dissolved in toluene (4 mL), acetic acid (1 mL) was added, stirred under nitrogen protection at 100°C for 3 h, concentrated, and subjected to preparative HPLC to obtain the title compound **12** (35 mg, 18.5%).
Preparation method: instrument: waters 2767 (preparative liquid phase chromatographic instrument); chromatographic column: SunFire@ Prep C18 (19 mm × 250 mm); the sample was dissolved in DMF and filtered with a 0.45 μm filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 40%-80%; c. flow rate: 15 mL/min; d. elution time: 20 min; retention time: 10.38 min.
$^1$H NMR (400 MHz, DMSO-d6) δ 10.48 (s, 1H), 7.92 (s, 2H),7.34 (d, 2H), 7.19 (d, 1H), 6.67 (d, 1H), 6.11 (d, 2H), 5.22 (s, 1H), 5.05 (s, 1H),4.51 (t, 1H), 4.39 (t, 1H), 3.91-3.97 (m, 1H), 3.60-3.64 (m, 3H), 3.36-3.49 (m, 3H), 3.03-3.113(m, 1H), 2.32-2.81 (m, 4H), 2.46(t, 3H), 1.58-1.70 (m, 2H), 1.07 (d, 3H).
LC-MS (ESI): m/z = 589.2 [M+H]+.

**Example 13: ((1R,2R)-2-((1R)-1-(2,6-difluoro-4-((1-(3-fluoropropyl) azetidin-3-yl)amino)phenyl)-3-methyl-1,3,4,9-tetrahydro-2H-pyrido[3,4-b]indol-2-yl)cyclopropyl)methanol (compound 13)**

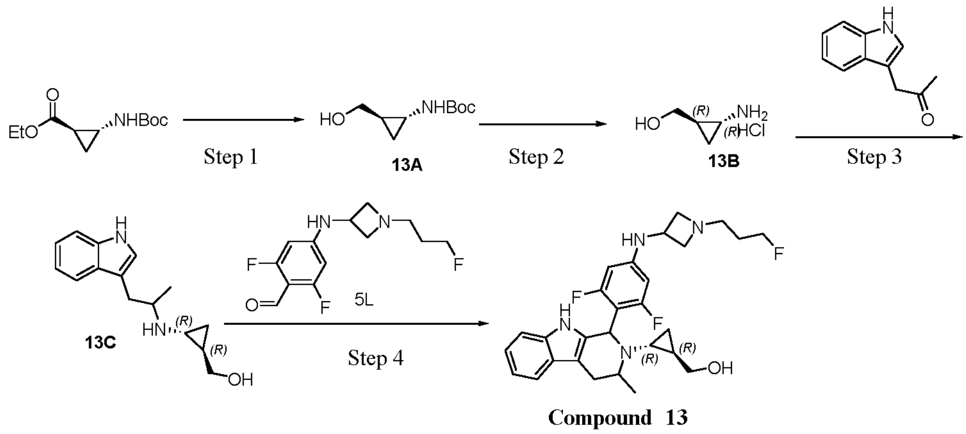


Step 1: tert-butyl ((1R,2R)-2-(hydroxymethyl)cyclopropyl)carbamate **(13A)**

Compound tert-butyl (1R,2R)-2-((tert-butoxycarbonyl)amino)cyclopropane-1-carboxylate (2.0 g, 8.72 mmol) was dissolved in ethanol (40 ml), anhydrous calcium chloride (2.90 g, 26.16 mmol) was added, and sodium borohydride (1.98 g, 52.32mmol) was added in batches; after the addition, the mixture was reacted at room temperature for 16 hours, and after the reaction was completed, water was added to quench the reaction, extracted with ethyl acetate, washed, dried, concentrated, and separated and purified by column chromatography (eluent: PE/EA = 0%-50%) to obtain the target compound **13A** (1.6 g, 98.0%).
LC-MS (ESI): m/z = 210.1 $[M+Na]^+$.

Step 2: ((1R,2R)-2-aminocyclopropyl)methanol hydrochloride **(13B)**

13A (1.60 g, 8.55 mmol) and dichloromethane (20 ml) were added into a flask, HCl/dioxane (8 ml) was then added, and then reacted at room temperature for 3 hours. Upon complete depletion of raw materials monitored by LCMS, a product was generated. The product was concentrated to obtain the crude target compound **13B** (2.1 g).
LC-MS (ESI): m/z = 88.2 $[M+H]^+$.

Step 3: ((1R,2R)-2-((1-(1H-indol-3-yl)propan-2-yl)amino)cyclopropyl) methanol **(13C)**

**13B** (1.8 g, 10.39 mmol) and indole-3-acetone (0.8 g, 8.31 mmol) were dissolved in methanol (30 mL), glacial acetic acid (0.62 g, 10.39 mmol) was added dropwise, and reacted at room temperature for 10 minutes. Sodium cyanoborohydride (1.96 g, 31.17 mmol) was slowly added and reacted at room temperature overnight. After the reaction was completed, a saturated potassium carbonate solution (10 mL) and water (60 ml) were added to the reaction solution, the aqueous phase was extracted with ethyl acetate (100 mL × 3), the organic phases were combined and washed with water (100 mL × 2), dried over anhydrous sodium sulfate, filtered, concentrated and passed through a silica gel column (DCM : MeOH = 0 : 1-10 : 1) to obtain the target compound **13C** (0.51 g, 20.1%).
LC-MS (ESI): m/z = 245.1 $[M+H]^+$.

Step 4: ((1R,2R)-2-((1R)-1-(2,6-difluoro-4-((1-(3-fluoropropyl)azetidin-3-yl)amino)phenyl)-3-methyl-1,3,4,9-tetrahydro-2H-pyrido[3,4-b]indol-2-yl)cyclopropyl)methanol **(compound 13)**

**13C** (100 mg, 0.41 mmol) and **5L** (111 mg, 0.41 mmol) were added into a flask, toluene (8 mL) and acetic acid (2 mL) were then added, and then reacted at 100°C for 0.5 hours. A product was generated as monitored by LCMS. After concentration, same was subjected to preparative HPLC. Preparative HPLC separation method: 1. Instrument: waters 2767 (preparative liquid phase chromatographic instrument); chromatographic column: SunFire@ Prep C18 (19 mm × 250 mm). 2. The sample was filtered with a 0.45 μm filter to prepare a sample solution. 3. Preparative chromatography conditions: a. composition of mobile phases A and B: mobile phase A: acetonitrile; mobile phase B: water (containing 0.1% ammonium acetate). b. gradient elution, mobile phase A: 5%-50%. c. flow rate: 25 mL/min. d. elution time: 20 min. retention time: 8.57 min. and the preparative liquid was concentrated and lyophilized to obtain the target **compound 13** (39 mg, 19.08%).
LC-MS (ESI): m/z = 499.2 $[M+H]^+$.
1H NMR (400 MHz, DMSO-d6) δ 10.46 (s, 1H), 7.35 (d, 1H), 7.17 (d, 1H), 6.98-6.89(m, 2H), 6.61 (d, 1H), 6.09 (d, 2H), 5.17 (s, 1H), 4.51 (t, 1H), 4.39 (t, 1H), 4.23 (t, 1H), 3.94 (q, 1H), 3.63 (s, 2H), 3.41 (t, 1H), 3.23-3.18 (m, 1H),

2.92 -2.79 (m, 4H), 2.76 -2.51 (m, 2H), 1.91 (s, 1H), 1.78 -1.61 (m, 2H), 1.13 (d, 3H), 0.71 (s, 1H), 0.50-0.42 (m, 2H).

**Example 14: 3-((1R,3R)-1-(2,6-difluoro-4-((1-(3-fluoropropyl)azetidin-3-yl)amino)phenyl)-7-ethynyl-3-methyl-1,3,4,9-tetrahydro-2H-pyrido[3,4-b]indol-2-yl)-2,2-difluoropropan-1-ol (compound 14)**

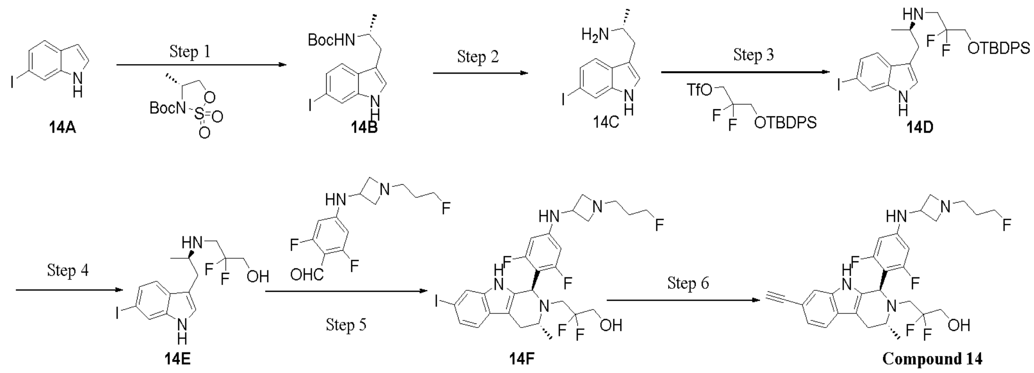


Step 1: tert-butyl (R)-(1-(6-iodo-1H-indol-3-yl)propan-2-yl)carbamate (compound **14B**)

Raw materials 6-iodo-1H-indole (5.54 g, 22.80 mmol) and cuprous chloride (1.61 g, 21.38 mmol) were added into dichloromethane (100 mL), and under nitrogen protection and at 0°C, methylmagnesium chloride (7 mL, 21.28 mmmol, 1.5 mol/L) was slowly added dropwise and reacted for 1 hour. The temperature was then reduced to -20°C, at this temperature, tert-butyl (R)-4-methyl-1,2,3-oxathiazolidine-3-carboxylate 2,2-dioxide (3.6 g, 15.19 mmol) was added and reacted for 16 hours. At 0°C, the reaction was quenched with 1 mol/L sodium citrate solution and stirred for 0.5 hours, extracted with dichloromethane, dried over anhydrous sodium sulfate, and filtered, and the filtrate was concentrated and then purified and separated by silica gel column chromatography (petroleum ether : ethyl acetate (v/v) = 1 : 0-10 : 1) to obtain the title compound **14B** (4.0 g, yield: 66%).
LCMS m/z = 345.0 [M-55].

Step 2: (R)-1-(6-iodo-1H-indol-3-yl)propan-2-amine (compound **14C**)

Raw material **14B** (2 g) was added into dichloromethane (20 mL), trifluoromethanoic acid (5mL) was then added, stirred at room temperature for 1 hour, and the reaction solution was directly concentrated to obtain the title compound **14C** (2 g, yield: 100%).
LCMS m/z = 301.0 [M-100].

Step 3: (R)-3-((tert-butyldiphenylsilyl)oxy)-2,2-difluoro-N-(1-(6-iodo-1H-indol-3-yl)propan-2-yl)propan-1-amine **(compound 14D)**

Raw materials **14C** (2 g, 6.67 mmol), 3-((tert-butyldiphenylsilyl)oxy)-2,2-difluoropropyl trifluoromethanesulfonate (4.82 g, 10.00 mmol) and N,N-diisopropylethylamine (3.47 g, 26.69mmol) were dissolved in dioxane (60 mL) solvent, and reacted at 100°C for 16 hours. After the reaction was completed, the mixture was poured into water (50 ml), extracted with EA, dried over anhydrous sodium sulfate, filtered, and the filtrate was concentrated and purified and separated by silica gel column chromatography (petroleum ether : ethyl acetate (v/v) = 1 : 0-5 : 1) to obtain the title compound **14D** (2.0 g, yield: 48%).
LCMS m/z = 633.1 [M+1].

Step 4: (R)-2,2-difluoro-3-((1-(6-iodo-1H-indol-3-yl)propan-2-yl)amino)propan-1-ol **(compound 14E)**

Raw material **14D** (1 g, 1.58 mmol) was added into tetrahydrofuran (20 mL), tetrabutylammonium fluoride (8 ml, 3.16 mmol) was then added, and stirred at room temperature for 1 hour. After the reaction was completed, the mixture was poured into water (50 ml), extracted with EA, dried over anhydrous sodium sulfate, filtered, and the filtrate was concentrated and purified and separated by silica gel column chromatography (petroleum ether : ethyl acetate (v/v) = 1 : 0-5 : 1) to obtain the title compound **14E** (350 mg, yield: 48%).
LCMS m/z = 273.2 [M-121].

Step 5: 3-((1R,3R)-1-(2,6-difluoro-4-((1-(3-fluoropropyl)azetidin-3-yl)amino) phenyl)-7-iodo-3-methyl-1,3,4,9-tetrahydro-2H-pyrido[3,4-b]indol-2-yl)-2,2-difluoropropan-1-ol **(compound 14F)**

Raw materials **14E** (350 mg, 0.89 mmol) and 2,6-difluoro-4-((1-(3-fluoropropyl)azetidin-3-yl)amino)benzaldehyde 5L (350 mg, 1.20 mmol) were added into a solution of toluene (4 mL) and acetic acid (1 mL), and reacted at 90°C for 3 hours. The reaction solution was directly concentrated and purified and separated by silica gel column chromatography (dichloromethane : methanol (v/v) = 1 : 0-10 : 1) to obtain the title compound **14F** (300 mg, yield: 52%).
LCMS m/z = 512.0 [M-136].

Step 6: 3 -((1R,3 R)-1-(2,6-difluoro-4-((1 -(3 -fluoropropyl)azetidin-3 -yl)amino) phenyl)-7-ethynyl-3-methyl-1,3,4,9-tetrahydro-2H-pyrido[3,4-b]indol-2-yl)-2,2-difluoropropan-1-ol **(compound 14)**

Raw materials **14F** (300 mg, 0.46 mmol), trimethylsilylacetylene (182 mg, 1.86 mmol), cuprous iodide (9.0 mg, 0.05 mmol), and triethylamine (10mg, 0.1 mmol) were added into N,N-dimethylformamide solution (5 ml), the reaction system was subjected to nitrogen replacement 3 times, a catalyst bis(triphenylphosphine)palladium(II) dichloride (60 mg, 0.1 mmol) was then added, and the mixture was reacted at 75°C for 16 hours. After the reaction was completed, the mixture was poured into water (25 ml), extracted with EA, washed twice with saturated brine, dried over anhydrous sodium sulfate, and filtered, the filtrate was concentrated, and then poured into 5 ml of methanol, potassium carbonate was then added, and the mixture was stirred at room temperature for 1 hour. Firstly, the reaction solution was purified and separated by silica gel column chromatography (dichloromethane : methanol (v/v) = 1 : 0-10 : 1) to obtain the title compound **14** (20 mg, yield: 8%).
LCMS m/z = 410.1 [M-136].
$^1$H NMR (400 MHz, DMSO) δ 10.73 (s, 1H), 7.37 (d, *J* = 8.1 Hz, 1H), 7.30 (s, 1H), 7.03 (dd, *J* = 8.1, 1.1 Hz, 1H), 6.70 (d, *J* = 6.8 Hz, 1H), 6.11 (d, *J* = 12.1 Hz, 2H), 5.22 (t, *J* = 5.7 Hz, 1H), 5.06 (s, 1H), 4.51 (t, *J* = 6.1 Hz, 1H), 4.39 (t, *J* = 6.1 Hz, 1H), 3.99 - 3.91 (m, 1H), 3.63 (dd, *J* = 14.4, 7.8 Hz, 3H), 3.51 - 3.34 (m, 2H), 3.08 (q, *J* = 14.6 Hz, 1H), 2.82 (dd, *J* = 15.0, 4.7 Hz, 1H), 2.74 (t, *J* = 6.3 Hz, 2H), 2.69 - 2.52 (m, 2H), 2.46 (t, *J* = 7.0 Hz, 3H), 1.75 - 1.48 (m, 2H), 1.06 (d, *J* = 6.5 Hz, 3H).

**Example 15: 3-((1R,3R)-1-(2,6-difluoro-4-(((S)-1-(3-fluoropropyl) pyrrolidin-3-yl)amino)phenyl)-5-fluoro-3-methyl-1,3,4,9-tetrahydro-2H-pyrido[3,4-b]indol-2-yl)-2,2-difluoropropan-1-ol (compound 15)**

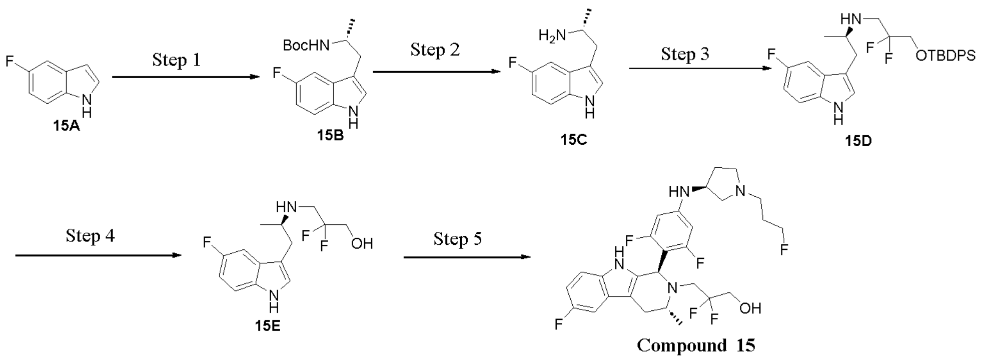


Step 1: tert-butyl (R)-(1-(5-fluoro-1H-indol-3-yl)propan-2-yl)carbamate (compound **15B**)

Raw materials 5-fluoroindole (10 g, 74.0 mmol) and cuprous chloride (5.86 g, 59.2 mmol) were added into 150 mL of dichloromethane, placed at 0°C and under nitrogen protection, a solution of methylmagnesium chloride (59.2 mL, 1 mol/l) in THF was added dropwise at this temperature, and then reacted for 1 hour. Tert-butyl (R)-4-methyl-1,2,3-oxathiazolidine-3-carboxylate 2,2-dioxide (11.76 g, 49.58 mmol) was then added at -20°C and reacted for 20 hours. A saturated citric acid solution was added to quench the reaction, then a saturated ammonium chloride solution was added, extracted with EA, dried over anhydrous sodium sulfate, and filtered, and the filtrate was concentrated and then same was purified and separated by silica gel column chromatography (petroleum ether : ethyl acetate (v/v) = 1 : 0-5 : 1) to obtain the title compound **15B** (11.5 g, yield: 53%).
LCMS m/z = 293.2 [M+1].

Step 2: (R)-1-(5-fluoro-1H-indol-3-yl)propan-2-amine (compound **15C**)

Raw material 15**B** (1.0 g, 3.42 mmol) was added into dichloromethane (5 mL), trifluoroacetic acid (5 mL) was then added, stirred at room temperature for 1 hour, and the filtrate was concentrated to obtain the title compound **15C** (1 g, yield: 100%).
LCMS m/z = 193.2 [M+1].

Step 3: (R)-3-((tert-butyldiphenylsilyl)oxy)-2,2-difluoro-N-(1-(5-fluoro-1H-indol-3-yl)propan-2-yl)propan-1-amine (compound **15D**)

Reagent 3-((tert-butyldiphenylsilyl)oxy)-2,2-difluoropropyl trifluoromethanesulfonate (2.26 g, 4.68 mmol), raw materials **15C** (0.6 g, 3.12 mmol) and DIPEA (1.21 g, 9.36 mmol) were added into 1,4-dioxane solvent (50 mL), heated to 100°C and stirred for 6 hours, cooled, and directly purified and separated by silica gel column chromatography (petroleum ether : ethyl acetate (v/v) = 1 : 0-5 : 1) to obtain the title compound **15D** (0.6 g, yield: 37%).
LCMS m/z = 525.2 [M+1].

Step 4: (R)-2,2-difluoro-3-((1-(5-fluoro-1H-indol-3-yl)propan-2-yl)amino) propan-1-ol (compound **15E**)

Raw material **15D** (0.6 g, 1.14 mmol) was dissolved in THF (20 mL) solvent, TBAF solution (2.28 mL, 2.28 mmol, 1M) was then added, stirred at room temperature for 2 hours, diluted with water, extracted with EA, dried over anhydrous sodium sulfate, and filtered, and the filtrate was concentrated and then purified and separated by silica gel column chromatography (petroleum ether : ethyl acetate (v/v) = 1 : 0-1 : 4) to obtain the title compound **15E** (0.3 g, yield: 92%).
LCMS m/z = 287.1 [M+1].

Step 5: 3-((1R,3R)-1-(2,6-difluoro-4-(((S)-1-(3-fluoropropyl)pyrrolidin-3-yl)amino)phenyl)-5-fluoro-3-methyl-1,3,4,9-tetrahydro-2H-pyrido[3,4-b]indol-2-yl)-2,2-difluoropropan-1-ol (compound **15**)

Raw materials **15D** (80 mg, 0.28 mmol) and **intermediate 1** (80 mg, 0.28 mmol) were added into a mixed solvent of toluene (4 mL) and acetic acid (1 mL), then heated to 95°C and reacted for 1 hour, directly concentrated to dryness, and then purified by TLC (DCM : MeOH = 10 : 1) to obtain the title compound **15** (30 mg, yield: 19%).
LCMS m/z = 555.2 [M+1].
$^1$H NMR (400 MHz, $CD_3OD$) δ7.11-7.14 (m, 1H),7.02-7.05 (m, 1H), 6.72-6.77 (m, 1H), 6.13-6.16 (m, 2H), 5.14 (s, 1H), 4.54 (t, 1H), 4.42 (t, 1H), 3.92-3.98 (m, 1H), 3.73-3.84 (m, 1H), 3.58-3.64 (m, 1H), 3.45-3.51 (m, 1H), 3.09-3.19 (m, 1H), 2.92-2.96 (m, 2H), 2.74-2.82 (m, 2H), 2.51-2.71 (m, 5H), 2.27-2.36 (m, 1H), 1.84-1.87(m, 2H), 1.66-1.74 (m, 1H), 1.14 (d, 3H).

**Example 16: 6-((1S,3R)-3,6-dimethyl-2-(2,2,2-trifluoroethyl)-2,3,4,9-tetrahydro-1H-pyrido [3,4-b] indol-1-yl)-N-((S)-1-(3-fluoropropyl)pyrrolidin-3-yl)pyridin-3-amine (compound 16)**

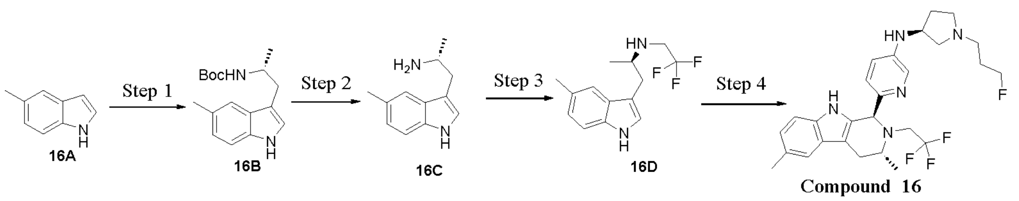

16A 16B 16C 16D Compound 16

Step 1: tert-butyl (R)-(1-(5-methyl-1H-indol-3-yl)propan-2-yl)carbamate (compound **16B**)

Raw materials 5-methylindole (5 g, 38.12 mmol) and cuprous chloride (3.02 g, 30.50 mmol) were added into 200 mL of dichloromethane, placed at 0°C and under nitrogen protection, a solution of methylmagnesium chloride (20.33 mL, 1.5 mol/l) in THF was added dropwise at this temperature, and then reacted for 1 hour. Tert-butyl (R)-4-methyl-1,2,3-oxathiazolidine-3-carboxylate 2,2-dioxide (6.06 g, 25.54 mmol) was then added at -20°C and reacted for 20 hours. A saturated citric acid solution was added to quench the reaction, then a saturated ammonium chloride solution was added, extracted with EA, dried over anhydrous sodium sulfate, and filtered, and the filtrate was concentrated and then

same was purified and separated by silica gel column chromatography (petroleum ether : ethyl acetate (v/v) = 1 : 0-10 : 1) to obtain the title compound **16B** (6.5 g, yield: 59%).
LCMS m/z = 289.2 [M+1].

Step 2: (R)-1-(5-methyl-1H-indol-3-yl)propan-2-amine (compound **16C**)

Raw material 16**B** (1.0 g, 3.47 mmol) was added into dichloromethane (5 mL), trifluoroacetic acid (5 mL) was then added, stirred at room temperature for 1 hour, and the filtrate was concentrated to obtain the title compound **16C** (1 g, yield: 100%).
LCMS m/z = 189.1 [M+1].

Step 3: (R)-1-(5-methyl-1H-indol-3-yl)-N-(2,2,2-trifluoroethyl) propan-2-amine (compound **16D**)

Reagent 2,2,2-trifluoroethyl trifluoromethanesulfonate (2.46 g, 10.62 mmol), raw materials **16C** (1 g, 5.31 mmol) and DIPEA (2.06 g, 15.93 mmol) were added into 1,4-dioxane solvent (50 mL), heated to 100°C, stirred for 8 hours, cooled, and directly purified and separated by silica gel column chromatography (petroleum ether : ethyl acetate (v/v) = 1 : 0-5 : 1) to obtain the title compound **16D** (0.43 g, yield: 30%).
LCMS m/z = 271.1 [M+1].

Step 4: 6-((1S,3R)-3,6-dimethyl-2-(2,2,2-trifluoroethyl)-2,3,4,9-tetrahydro-1H-pyrido[3,4-b]indol-1-yl)-N-((S)-1-(3-fluoropropyl)pyrrolidin-3-yl)pyridin-3-amine (compound **16**)

Raw materials **16D** (80 mg, 0.3 mmol) and **intermediate 3** (75 mg, 0.3 mmol) were added into a mixed solvent of toluene (4 mL) and acetic acid (1 mL), then heated to 95°C and reacted for 3 hour, directly concentrated to dryness, and then purified by TLC (DCM : MeOH = 10 : 1) to obtain the title compound 16 (20 mg, yield: 13%).
LCMS m/z = 508.2 [M+1].
$^1$H NMR (400 MHz, $CD_3OD$) δ 7.84 (d, 1H), 7.22 (s, 1H), 7.17 (d, 1H), 7.11 (d, 1H), 6.97-7.00 (m, 1H), 6.86-6.88 (m, 1H), 4.91 (s, 1H), 4.53 (t, 1H), 4.42 (t, 1H), 3.97-4.02 (m, 1H), 3.34-3.49 (m, 2H), 2.86-3.04 (m, 3H), 2.74-2.80 (m, 1H), 2.56-2.66 (m, 4H), 2.50-2.54 (m, 1H), 2.39 (s, 3H), 2.28-2.35 (m, 1H), 1.83-1.96 (m, 2H), 1.67-1.75 (m, 1H), 1.17 (d, 3H).

**Example 17: (S)-N-(4-((1R,3R)-3,7-dimethyl-2-(2,2,2-trifluoroethyl)-2,3,4,9-tetrahydro-1H-pyrido[3,4-b]indol-1-yl)-3,5-difluorophenyl)-1-(3-fluoropropyl)pyrrolidin-3-amine (compound 17)**

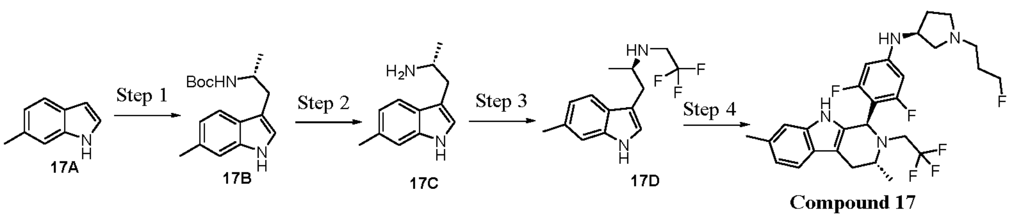


Step 1: tert-butyl (R)-(1-(5-methyl-1H-indol-3-yl)propan-2-yl)carbamate (compound **17B**)

Raw materials 6-methylindole (5 g, 38.12 mmol) and cuprous chloride (3.02 g, 30.50 mmol) were added into 200 mL of dichloromethane, placed at 0°C and under nitrogen protection, a solution of methylmagnesium chloride (20.33 mL, 1.5 mol/l) in THF was added dropwise at this temperature, and then reacted for 1 hour. Tert-butyl (R)-4-methyl-1,2,3-oxathiazolidine-3-carboxylate 2,2-dioxide (6.06 g, 25.54 mmol) was then added at -20°C and reacted for 20 hours. A saturated citric acid solution was added to quench the reaction, then a saturated ammonium chloride solution was added, extracted with EA, dried over anhydrous sodium sulfate, and filtered, and the filtrate was concentrated and then same was purified and separated by silica gel column chromatography (petroleum ether : ethyl acetate (v/v) = 1 : 0-10 : 1) to obtain the title compound **17B** (6.5 g, yield: 59%).
LCMS m/z = 289.2 [M+1].

Step 2: (R)-1-(5-methyl-1H-indol-3-yl)propan-2-amine (compound **17C**)

Raw material 17**B** (1.0 g, 3.47 mmol) was added into dichloromethane (5 mL), trifluoroacetic acid (5 mL) was

then added, stirred at room temperature for 1 hour, the filtrate was concentrated to obtain the title compound **17C** (1 g, yield: 100%).

LCMS m/z = 189.1 [M+1].

Step 3: (R)-1-(5-methyl-1H-indol-3-yl)-N-(2,2,2-trifluoroethyl) propan-2-amine (compound **17D**)

Reagent 2,2,2-trifluoroethyl trifluoromethanesulfonate (2.46 g, 10.62 mmol), raw materials **17C** (1 g, 5.31 mmol) and DIPEA (2.06 g, 15.93 mmol) were added into 1,4-dioxane solvent (50 mL), heated to 100°C and stirred for 8 hours, cooled, and directly purified and separated by silica gel column chromatography (petroleum ether : ethyl acetate (v/v) = 1 : 0-5 : 1) to obtain the title compound **17D** (0.48 g, yield: 33%).

LCMS m/z = 271.1 [M+1].

Step 4: (S)-N-(4-((1R,3R)-3,7-dimethyl-2-(2,2,2-trifluoroethyl)-2,3,4,9-tetrahydro-1H-pyrido[3,4-b]indol-1-yl)-3,5-difluorophenyl)-1-(3-fluoropropyl)pyrrolidin-3-amine (compound **17**)

Raw materials **17D** (80 mg, 0.3 mmol) and **intermediate** 1 (86 mg, 0.3 mmol) were added into a mixed solvent of toluene (4 mL) and acetic acid (1 mL), then heated to 95°C and reacted for 3 hour, directly concentrated to dryness, and then purified by TLC (DCM : MeOH = 10 : 1) to obtain the title compound **17** (30 mg, yield: 19%).

LCMS m/z = 539.3 [M+1].

$^1$H NMR (400 MHz, $CD_3OD$) δ7.27 (d, 1H), 7.01(s, 1H), 6.80 (d, 1H),6.10-6.15 (m, 2H), 5.18 (s, 1H), 4.54 (t, 1H), 4.42 (t, 1H), 3.92-3.98 (m, 1H), 3.50-3.57 (m, 1H), 3.22-3.34 (m, 1H), 2.91-3.03 (m, 3H), 2.75-2.78 (m, 1H), 2.50-2.69 (m, 5H), 2.37 (s, 3H), 2.27-2.36 (m, 1H), 1.84-1.97 (m, 2H), 1.66-1.74 (m, 1H), 1.16 (d, 3H).

**Example 18: (S)-N-(3,5-difluoro-4-((1R,3R)-6-fluoro-3-methyl-2-(2,2,2-trifluoroethyl)-2,3,4,9-tetrahydro-1H-pyrido[3,4-b]indol-1-yl)phenyl)-1-(3-fluoropropyl)pyrrolidin-3-amine (compound 18)**

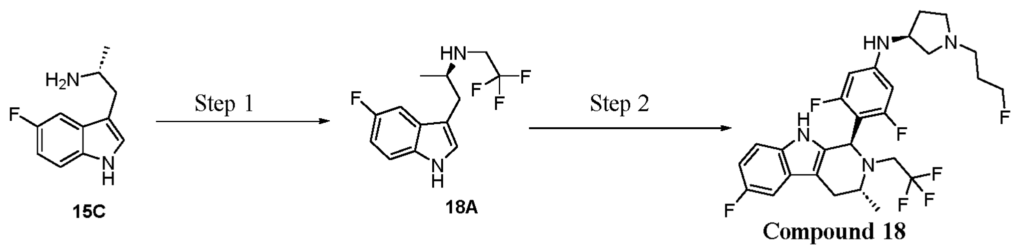


Step 1: (R)-1-(5-fluoro-1H-indol-3-yl)-N-(2,2,2-trifluoroethyl) propan-2-amine (compound **18A**)

Reagent 2,2,2-trifluoroethyl trifluoromethanesulfonate (2.66 g, 11.48 mmol), raw materials **15C** (1.1 g, 5.74 mmol) and DIPEA (2.23 g, 17.22 mmol) were added into 1,4-dioxane solvent (50 mL), heated to 100°C, stirred for 8 hours, cooled, and directly purified and separated by silica gel column chromatography (petroleum ether : ethyl acetate (v/v) = 1 : 0-5 : 1) to obtain the title compound **18A** (0.43 g, yield: 27%).

LCMS m/z = 275.1 [M+1].

Step 2: (S)-N-(3,5-difluoro-4-((1R,3R)-6-fluoro-3-methyl-2-(2,2,2-trifluoroethyl)-2,3,4,9-tetrahydro-1H-pyrido[3,4-b]indol-1-yl)phenyl)-1-(3-fluoropropyl)pyrrolidin-3-amine (compound **18**)

Raw materials **18A** (80 mg, 0.29 mmol) and **intermediate 1** (83 mg, 0.29 mmol) were added into a mixed solvent of toluene (4 mL) and acetic acid (1 mL), then heated to 95°C and reacted for 3 hour, directly concentrated to dryness, and then purified by TLC (DCM : MeOH = 10 : 1) to obtain the title compound **18** (30 mg, yield: 18%).

LCMS m/z = 543.2 [M+1].

$^1$H NMR (400 MHz, $CD_3OD$) δ7.12-7.15 (m, 1H), 7.03-7.06 (m, 1H), 6.73-6.78 (m, 1H),6.11-6.16 (m, 2H), 5.20 (s, 1H), 4.54 (t, 1H), 4.42 (t, 1H), 3.92-3.98 (m, 1H), 3.53-3.57 (m, 1H), 3.25-3.36 (m, 1H), 2.91-3.02 (m, 3H), 2.74-2.80 (m, 1H), 2.50-2.67 (m, 5H), 2.29-2.36 (m, 1H), 1.84-1.97 (m, 2H), 1.67-1.74 (m, 1H), 1.16 (d, 3H).

**Example 19: 6-((1S,3R)-6-fluoro-3-methyl-2-(2,2,2-trifluoroethyl)-2,3,4,9-tetrahydro-1H-pyrido [3,4-b] indol-1-yl)-N-((S)-1-(3-fluoropropyl)pyrrolidin-3-yl)pyridin-3-amine (compound 19)**

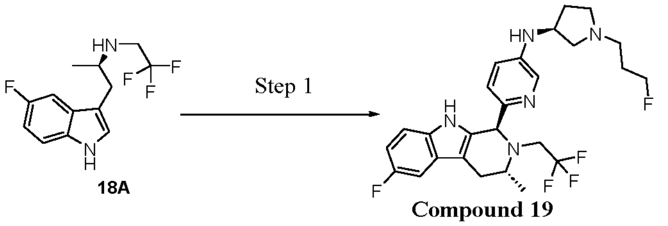


18A

Compound 19

Step 1: 6-((1S,3R)-6-fluoro-3-methyl-2-(2,2,2-trifluoroethyl)-2,3,4,9-tetrahydro-1H-pyrido[3,4-b]indol-1-yl)-N-((S)-1-(3-fluoropropyl)pyrrolidin-3-yl)pyridin-3-amine (compound **19**)

Raw materials **18A** (80 mg, 0.29 mmol) and **intermediate 3** (73 mg, 0.29 mmol) were added into a mixed solvent of toluene (4 mL) and acetic acid (1 mL), then heated to 95°C and reacted for 3 hour, directly concentrated to dryness, and then purified by TLC (DCM : MeOH = 10 : 1) to obtain the title compound **19** (30 mg, yield: 20%).
LCMS m/z = 508.2 [M+1].
$^1$H NMR (400 MHz, $CD_3OD$) δ 7.84 (d, 1H), 7.16-7.20 (m, 2H), 7.07-7.10 (m, 1H), 6.98-7.01(m, 1H),6.76-6.82 (m, 1H), 4.93 (s, 1H), 4.54 (t, 1H), 4.42 (t, 1H), 3.97-4.03 (m, 1H), 3.35-3.47 (m, 2H), 2.94-3.06 (m, 2H), 2.85-2.90 (m, 1H), 2.77-2.81 (m, 1H), 2.52-2.68 (m, 5H),2.29-2.38 (m, 1H),1.84-1.97 (m, 2H), 1.67-1.75 (m, 1H), 1.18 (d, 3H).

**Example 20: 3-((1R,3R)-1-(2,6-difluoro-4-((1-(3-fluoropropyl)azetidin-3-yl)amino)phenyl)-3-methyl-1,3,4,6,7,9-hexahydro-2H-cyclobuta[f]pyrido[3,4-b]indol-2-yl)-2,2-difluoropropan-1-ol and** 3-((1S,3S)-1-(2,6-difluoro-4-((1-(3-fluoropropyl)azetidin-3-yl)amino)phenyl)-3-methyl-1,3,4,6,7,9-hexahydro-2H-cyclobuta[f]pyrido[3,4-b]indol-2-yl)-2,2-difluoropropan-1-ol **(compounds 20-1** and **20-2)**

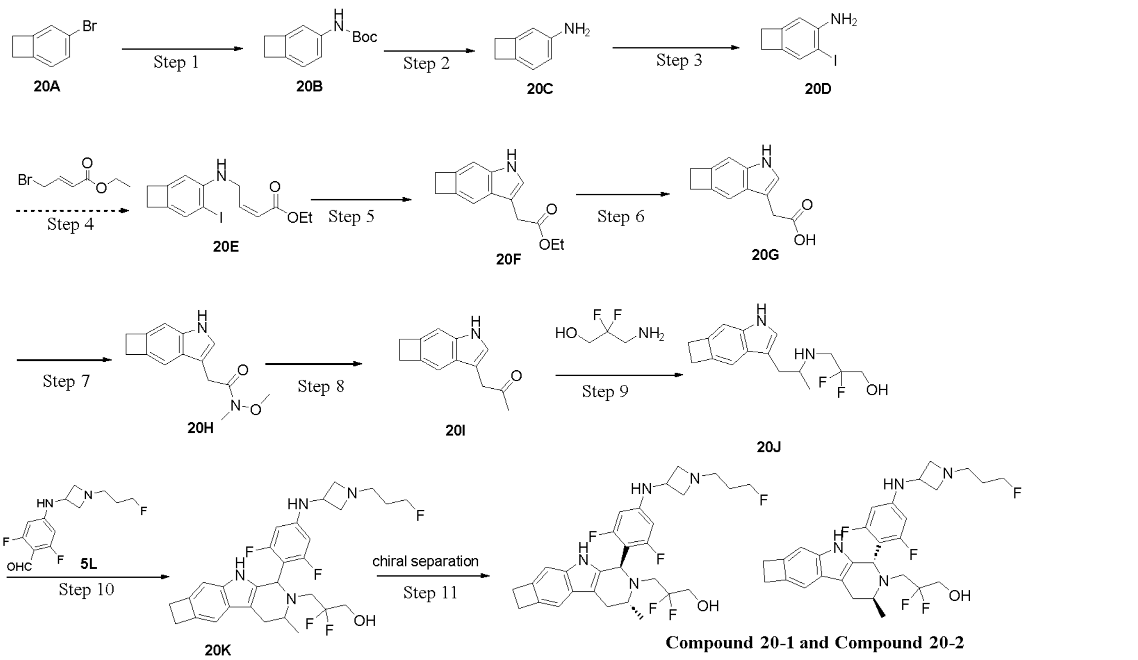


Compound 20-1 and Compound 20-2

Step 1: tert-butyl bicyclo[4.2.0]octa-1,3,5-trien-3-ylcarbamate **(20B)**

To a 500 mL single-neck bottle was added **20A** (21.0 g, 114.7 mmol), same was dissolved in dioxane (200 mL), tert-butyl carbamate (16.1 g, 137.7 mmol), cesium carbonate (59.8 g, 183.6 mmol), Xantphos (3.3 g, 5.74 mmol)

and $Pd_2(dba)_3$ (2.6 g, 2.9 mmol) were added, under nitrogen protection, the mixture was stirred at 100°C for 18 h, filtered through celite, concentrated, and separated by column chromatography (PE : EA = 20 : 1) to obtain the title compound **20B** (20.0 g, 80%).

LC-MS (ESI): m/z = 164.1[M-55]$^+$.

Step 2: bicyclo[4.2.0]octa-1,3,5-trien-3-amine **(20C)**

To a 500 mL round bottom flask was added **20B** (20.0 g, 91.2 mmol), same was dissolved in dichloromethane (100 mL), trifluoroacetic acid (25 mL) was added, stirred at room temperature for 18 h, washed with a saturated sodium bicarbonate solution to pH = 8-9, extracted with dichloromethane (2 × 200 mL), the organic phases were combined, dried over anhydrous sodium sulfate, and concentrated to obtain the title compound **20C** (10.1 g, 93%).

LC-MS (ESI): m/z = 120.2 [M+H]$^+$.

Step 3: 4-iodobicyclo[4.2.0]octa-1,3,5-trien-3-amine **(20D)**

To a 250 mL round bottom flask was added **20C** (9.0 g, 75.53 mmol), same was dissolved in acetonitrile (45 mL), a solution of NIS (17.0 g, 75.53 mmol) in acetonitrile (90 mL) was added dropwise, stirred at room temperature for 3 h, concentrated, same was dissolved in dichloromethane (200 mL), washed with a saturated sodium thiosulfate solution, the organic phase was dried over anhydrous sodium sulfate, and separated and purified by column chromatography (PE : EA = 20 : 1) to obtain the title compound **20D** (9.0 g, 49%).

LC-MS (ESI): m/z = 246.0 [M+H]$^+$.

Step 4: ethyl (E)-4-((4-iodobicyclo[4.2.0]octa-1,3,5-trien-3-yl)amino)but-2-enoate **(20E)**

To a 250 mL round bottom flask was added **20D** (9.0 g, 36.73 mmol), same was dissolved in DMF (70 mL), ethyl 4-butenoate (11.3 g, 58.77 mmol) and potassium carbonate (15.2 g, 110.19 mmol) were added, stirred at 60°C for 18 h, water (150 mL) was added, same was extracted with ethyl acetate (2 × 100 mL), the organic phases were combined, washed with saturated brine, dried over anhydrous sodium sulfate, and separated and purified by column chromatography (PE : EA = 20 : 1) to obtain the title compound **20E** (7.4 g, 56%).

LC-MS (ESI): m/z = 358.0 [M+H]$^+$.

Step 5: ethyl 2-(5,6-dihydro-1H-cyclobuta[f]indol-3-yl)acetate **(20F)**

To a 250 mL round bottom flask was added **20E** (7.4 g, 20.72 mmol), same was dissolved in DMF (50 mL), palladium acetate (0.2 g, 1.04 mmol), potassium carbonate (5.7 g, 41.44 mmol) and triphenylphosphine (0.5 g, 2.07 mmol) were added, under nitrogen protection, the mixture was stirred at 75°C for 18 h, water (100 mL) was added, extracted with ethyl acetate (2 × 80 mL), the organic phases were combined, washed with saturated brine, dried over anhydrous sodium sulfate, and separated and purified by column chromatography (PE : EA = 10 : 1-5 : 1) to obtain the title compound **20F** (3.6 g, 76%).

LC-MS (ESI): m/z = 230.1[M+H]$^+$.

Step 6: 2-(5,6-dihydro-1H-cyclobuta[f]indol-3-yl)acetic acid **(20G)**

To a 100 mL round bottom flask was added **20F** (3.6 g, 15.70 mmol), same was dissolved in methanol (30 mL), lithium hydroxide (1.1 g, 47.10 mmol) and water (20 mL) were added, stirred at room temperature for 5 h, concentrated to remove excess methanol, HCl (2 M) was added to adjust to pH = 5-6, extracted with ethyl acetate (2 × 80 mL), the organic phases were combined, and concentrated to obtain the title compound **20G** (2.9 g, 92%).

LC-MS (ESI): m/z = 202.2[M+H]$^+$.

Step 7: 2-(5,6-dihydro-1H-cyclobuta[f]indol-3-yl)-N-methoxy-N-methylacetamide **(20H)**

To a 100 mL round bottom flask was added **20G** (2.9 g, 14.41 mmol), same was dissolved in THF (30 mL) and DMF (6 mL), dimethylhydroxylamine hydrochloride (2.8 g, 28.82mmol) and diisopropylethylamine (9.3 g, 72.05 mmol) were added, 1-propyl phosphate anhydride (13.8 g, 21.62 mmol) was added dropwise in an ice bath, stirred at room temperature for 18 h, water (50 mL) was added, extracted with ethyl acetate (2 × 50 mL), the organic phases were combined, washed with saturated brine, dried over anhydrous sodium sulfate, and separated by column chromatography (PE : EA = 4 : 1) to obtain the title compound **20H** (2.9 g, 82%).

LC-MS (ESI): m/z = 245.2[M+H]$^+$.

Step 8: 1-(5,6-dihydro-1H-cyclobuta[f]indol-3-yl)propan-2-one **(201)**

To a 100 mL round bottom flask was added **20H** (2.9 g, 11.87 mmol), same was dissolved in THF (20 mL), methylmagnesium bromide (3 M, 14.4 mL) was added dropwise in an ice bath, stirred for 1 h while maintaining in the ice bath, saturated ammonium chloride (50 mL) was added, the reaction was quenched, extracted with ethyl acetate (2 × 50 mL), the organic phases were combined, dried over anhydrous sodium sulfate, and separated by column chromatography (PE : EA = 10 : 1) to obtain the title compound **201** (1.6 g, 68%).
LC-MS (ESI): m/z = 200.2[M+H]$^+$.

Step 9: 3-((1-(5,6-dihydro-1H-cyclobuta[f]indol-3-yl)propan-2-yl)amino)-2,2-difluoropropan-1-ol **(20J)**

To a 100 mL round bottom flask was added **201** (0.6 g, 3.01 mmol), same was dissolved in anhydrous methanol (10 mL), 3-amino-2,2-difluoropropyl-1-ol (0.5 g, 4.51 mmol) and acetic acid (0.18 g, 3.01 mmol) were added, stirred at room temperature for 1 h, sodium cyanoborohydride (0.28 g, 4.51 mmol) was added, stirring was continued at room temperature overnight, and same was concentrated, and separated by column chromatography (PE : EA = 1 : 1-EA) to obtain the title compound **20J** (0.85 g, 96%).
LC-MS (ESI): m/z = 295.2[M+H]$^+$.

Step 10: 3-(1-(2,6-difluoro-4-((1-(3-fluoropropyl)azetidin-3-yl)amino)phenyl) -3-methyl-1,3,4,6,7,9-hexahydro-2H-cyclobuta[f]pyrido[3,4-b]indol-2-yl)-2,2-difluoropropan-1-ol **(20K)**

To a 100 mL round bottom flask was added **20J** (0.2 g, 0.68 mmol), and same was dissolved in dioxane (10 mL), **5L** (0.22 g, 0.82 mmol) and trifluoroacetic acid (78 mg, 0.68 mmol) were added, stirred at 70°C for 10 h, concentrated, and separated by preparative HPLC to obtain the title compound **20K** (0.15 g, 40%).
Preparation method: instrument: waters 2767 (preparative liquid phase chromatographic instrument); chromatographic column: SunFire@ Prep C18 (19 mm × 250 mm); the sample was dissolved in DMF and filtered with a 0.45 μm filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 40%-70%; c. flow rate: 15 mL/min; d. elution time: 20 min; retention time: 12.0 min.
$^1$H NMR (400 MHz, DMSO-d6) δ 10.32 (s, 1H), 7.04 (d, 1H), 6.89 (d, 1H), 6.65 (d, 1H), 6.09 (d, 2H), 5.20 (t, 1H), 5.02 (s, 1H), 4.51 (t, 1H), 4.39 (t, 1H), 3.96-3.91 (m, 1H), 3.63-3.60 (m, 3H), 3.48 - 3.33 (m, 2H), 3.09-3.05 (m, 5H), 2.79-2.71 (m, 3H), 2.69-2.60 (m, 1H), 2.48-2.46 (m, 3H), 1.69-1.60 (m, 2H), 1.05 (d, 3H).
LC-MS (ESI): m/z = 549.2 [M+H]$^+$.

Step 11: 3-((1R,3R)-1-(2,6-difluoro-4-((1-(3-fluoropropyl)azetidin-3-yl)amino)phenyl)-3-methyl-1,3,4,6,7,9-hexahydro-2H-cyclobuta[f]pyrido[3,4-b]indol-2-yl)-2,2-difluoropropan-1-ol and 3-((1S,3S)-1-(2,6-difluoro-4-((1-(3-fluoropropyl)azetidin-3-yl)amino)phenyl)-3-methyl-1,3,4,6,7,9-hexahydro-2H-cyclobuta[f]pyrido[3,4-b]indol-2-yl)-2,2-difluoropropan-1-ol (compound **20-1** and compound **20-2)**

Compound **20K** (0.15 g, 0.27 mmol) was subjected to chiral preparation to obtain the title compound **20-1** (46 mg, retention time 1.085 min) and compound **20-2** (60 mg, retention time 1.558 min), the absolute configurations of the two compounds have not been determined, and NMR and MS are consistent with the racemate.
instrument: Waters 150 MGM; chromatographic column: Chiralpak Column; mobile phase: A: carbon dioxide, and B: isopropanol; isocratic elution: 50% mobile phase B; flow rate: 100 mL/min; back pressure: 100 bar; column temperature: 35°C; wavelength: 220 nm; elution time: 5 min.

**Example 21: 2,2-difluoro-3-((1S,3R)-1-(5-((1-(3-fluoropropyl)azetidin-3-yl)amino)pyridin-2-yl)-3-methyl-1,3,4,6,7,9-hexahydro-2H-cyclobuta[f]pyrido[3,4-b]indol-2-yl)propan-1-ol and** 2,2-difluoro-3-((1R,3S)-1-(5-((1-(3-fluoropropyl)azetidin-3-yl)amino)pyridin-2-yl)-3-methyl-1,3,4,6,7,9-hexahydro-2H-cyclobuta[f]pyrido[3,4-b]indol-2-yl)propan-1-ol **(compound 21-1** and **compound 21-2)**

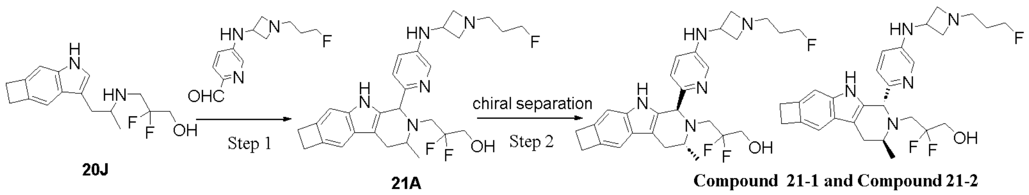


20J

21A

Compound 21-1 and Compound 21-2

Step 1: 2,2-difluoro-3-(1-(5-((1-(3-fluoropropyl)azetidin-3-yl)amino)pyridin-2-yl)-3-methyl-1,3,4,6,7,9-hexahydro-2H-cyclobuta[f]pyrido[3,4-b]indol-2-yl)propan-1-ol **(21A)**

To a 100 mL round bottom flask was added **20J** (0.15 g, 0.51 mmol), and same was dissolved in dioxane (10 mL), **intermediate 2** (0.15 g, 0.61 mmol) and trifluoroacetic acid (58 mg, 0.51 mmol) were added, stirred at 70°C for 2 h, concentrated, and separated by preparative HPLC to obtain the title compound **21A** (36 mg, 14%).

Preparation method: instrument: waters 2767 (preparative liquid phase chromatographic instrument); chromatographic column: SunFire@ Prep C18 (19 mm × 250 mm); the sample was dissolved in DMF and filtered with a 0.45 μm filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 40%-70%; c. flow rate: 15 mL/min; d. elution time: 20 min; retention time: 11.2 min.

$^1$H NMR (400 MHz, DMSO-d6) δ 10.42 (s, 1H), 7.83 (d, 1H), 7.07 (s, 1H), 6.98 - 6.91 (m, 2H), 6.84 (dd, 1H), 6.23 (d, 1H), 5.48 (t, 1H), 4.92 (s, 1H), 4.50 (t, 1H), 4.39 (t, 1H), 4.00 - 3.88 (m, 1H), 3.78-3.74 (m, 2H), 3.63 (t, 2H), 3.26 - 3.17 (m, 1H), 3.11 (brs, 5H), 2.74 (t, 2H), 2.70 - 2.53 (m, 2H), 2.46 (t, 3H), 1.69-1.60 (m, 2H), 1.09 (d, 3H).

LC-MS (ESI): m/z = 514.2 $[M+H]^+$.

Step 2: 2,2-difluoro-3-((1S,3R)-1-(5-((1-(3-fluoropropyl)azetidin-3-yl)amino) pyridin-2-yl)-3-methyl-1,3,4,6,7,9-hexahydro-2H-cyclobuta[f]pyrido[3,4-b]indol-2-yl)propan-1-ol and 2,2-difluoro-3-((1R,3S)-1-(5-((1-(3-fluoropropyl)azetidin-3-yl)amino)pyridin-2-yl)-3-methyl-1,3,4,6,7,9-hexahydro-2H-cyclobuta[f]pyrido[3,4-b]indol-2-yl)propan-1-ol (**compound 21-1** and **compound 21-2)**

Compound **21A** (36 mg, 0.070 mmol) was subjected to chiral preparation to obtain the title compound **21-1** (10 mg, retention time 1.129 min) and compound **21-2** (11 mg, retention time 1.652 min), the absolute configurations of the two compounds have not been determined, and NMR and MS are consistent with the racemate.

Instrument: Waters 150 MGM

Chromatographic column: Chiralpak Column

Mobile phase: A: carbon dioxide, and B: methanol +acetonitrile (0.1% aqueous ammonia); isocratic elution: 50% mobile phase B; flow rate: 100 mL/min; back pressure: 100 bar; column temperature: 35°C; wavelength: 220 nm; elution time: 6 min.

**Example 22: N-(3,5-difluoro-4-((1R,3R)-3-methyl-2-(2,2,2-trifluoroethyl)-2,3,4,6,7,9-hexahydro-1H-cyclobuta[f]pyrido[3,4-b]indol-1-yl)phenyl)-1-(3-fluoropropyl)azetidin-3-amine and** N-(3,5-difluoro-4-((1S,3S)-3-methyl-2-(2,2,2-trifluoroethyl)-2,3,4,6,7,9-hexahydro-1H-cyclobuta[f]pyrido[3,4-b]indol-1-yl)phenyl)-1-(3-fluoropropyl)azetidin-3-amine **(compound 22-1** and **compound 22-2)**

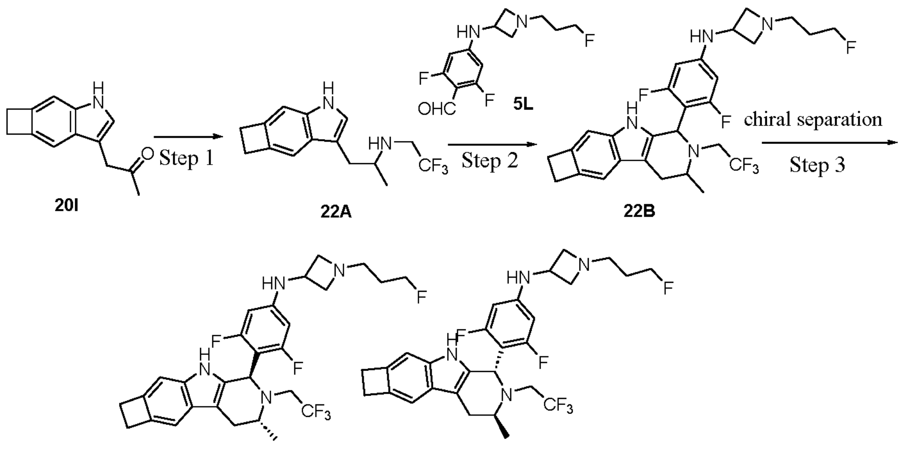


20I 22A 5L 22B

Compound 22-1 and Compound 22-2

Step 1: 1-(5,6-dihydro-1H-cyclobuta[f]indol-3-yl)-N-(2,2,2-trifluoroethyl)propan-2-amine **(22A)**

To a 100 mL round bottom flask was added **201** (0.3 g, 1.51 mmol), same was dissolved in anhydrous dichloromethane (10 mL), trifluoroethylamine (0.3 g, 3.02 mmol) and acetic acid (91 mg, 1.51 mmol) were added, stirred at room temperature for 1 h, sodium triacetylborohydride (0.64 g, 3.02 mmol) was added, stirring was continued at room temperature overnight, quenched with water (10 mL), extracted with dichloromethane (2 × 10 mL), the organic phases were combined, and separated by column chromatography (PE : EA = 5 : 1) to obtain the title compound **22A** (0.30 g, 70%).

LC-MS (ESI): m/z = 283.2[M+H]$^+$.

Step 2: N-(3,5-difluoro-4-(3-methyl-2-(2,2,2-trifluoroethyl)-2,3,4,6,7,9-hexahydro-1H-cyclobuta[f]pyrido[3,4-b]indol-1-yl)phenyl)-1-(3-fluoropropyl)azetidin-3-amine **(22B)**

To a 100 mL round bottom flask was added **22A** (0.25 g, 0.89 mmol), and same was dissolved in dioxane (10 mL), **5L** (0.29 g, 1.07 mmol) and trifluoroacetic acid (100 mg, 0.89 mmol) were added, stirred at 70°C for 18 h, concentrated, and separated by preparative HPLC to obtain the title compound **22B** (0.26 g, 54%).

Preparation method: instrument: waters 2767 (preparative liquid phase chromatographic instrument); chromatographic column: SunFire@ Prep C18 (19 mm × 250 mm); the sample was dissolved in DMF and filtered with a 0.45 μm filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 40%-70%; c. flow rate: 15 mL/min; d. elution time: 20 min; retention time: 11.6 min.

$^1$H NMR (400 MHz, DMSO-d6) δ 10.38 (s, 1H), 7.05 (d, 1H), 6.90 (d, 1H), 6.68 (d, 1H), 6.15 - 6.01 (m, 2H), 5.08 (s, 1H), 4.51 (t, 1H), 4.39 (t, 1H), 3.96-3.91 (m, 1H), 3.66 - 3.55 (m, 2H), 3.45-3.36 (m, 2H), 3.10 (s, 4H), 3.00 - 2.84 (m, 1H), 2.80-2.71 (m, 3H), 2.55 (d, 1H), 2.46 (t, 2H), 1.71-1.60 (m, 2H), 1.09 (d, 3H).

LC-MS (ESI): m/z = 537.2 [M+H]$^+$.

Step 3: N-(3,5-difluoro-4-((1R,3R)-3-methyl-2-(2,2,2-trifluoroethyl)-2,3,4,6,7,9-hexahydro-1H-cyclobuta[f]pyrido[3,4-b]indol-1-yl)phenyl)-1-(3-fluoropropyl)azetidin-3-amine and N-(3,5-difluoro-4-((1S,3S)-3-methyl-2-(2,2,2-trifluoroethyl)-2,3,4,6,7,9-hexahydro-1H-cyclobuta[f]pyrido[3,4-b]indol-1-yl)phenyl)-1-(3-fluoropropyl)azetidin-3-amine (**compound 22-1** and **compound 22-2)**

Compound **22B** (0.26 g, 0.27 mmol) was subjected to chiral preparation to obtain the title compound **22-1** (100 mg, retention time 1.691 min) and compound **22-2** (90 mg, retention time 1.929 min), the absolute configurations of the two compounds have not been determined, and NMR and MS are consistent with the racemate. instrument: Waters 150 MGM; chromatographic column: Chiralpak Column; mobile phase: A: carbon dioxide, and B: isopropanol (0.1% aqueous ammonia); isocratic elution: 25% mobile phase B; flow rate: 100 mL/min; back pressure: 100 bar; column temperature: 35°C; wavelength: 220 nm; elution time: 6.2 min.

**Example 23: 3-((1R,3R)-1-(2,6-difluoro-4-(((S)-1-(3-fluoropropyl) pyrrolidin-3-yl)amino)phenyl)-3-methyl-6-(trifluoromethyl)-1,3,4,9-tetrahydro-2H-pyrido[3,4-b]indol-2-yl)-2,2-difluoropropan-1-ol (compound 23)**

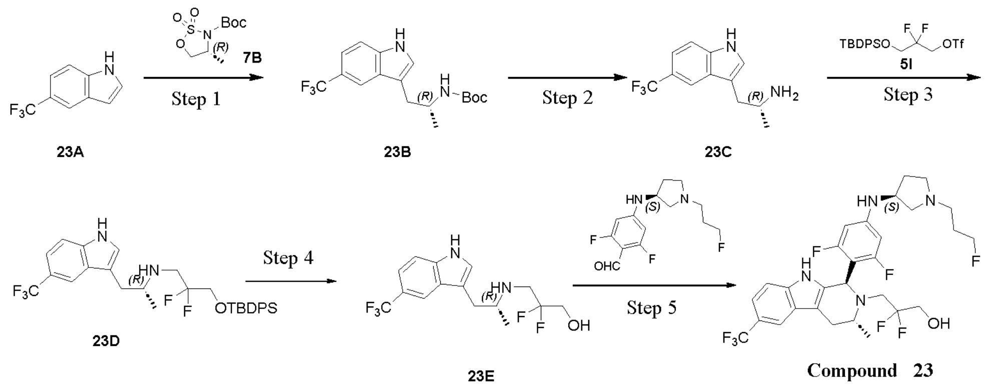


Step 1: tert-butyl (R)-(1-(5-(trifluoromethyl)-1H-indol-3-yl)propan-2-yl)carbamate **(23B)**

To a 500 mL three-neck bottle were added **23A** (5.1 g, 27.34 mmol) and cuprous chloride (2.35 g, 23.70 mmol), same was dissolved in anhydrous dichloromethane (80 mL), cooled to 0°C, MeMgCl (7.9 mL, 3 M) was added dropwise, the reaction was carried out with the temperature maintained constant for 1 h, cooled to -20°C, a solution of 7B (2.5 g, 18.23 mmol) in dichloromethane (20 mL) was added dropwise, stirred overnight while maintaining this temperature constant, quenched with 10% citric acid solution (200 mL), stirred for 0.5 h, filtered through celite, and extracted with dichloromethane (2 × 100 mL), the organic phases were combined, dried over anhydrous sodium sulfate, concentrated and purified by column chromatography (PE : EA = 10 : 1-5 : 1) to obtain the title compound **23B** (2.7 g, 43%).
$^1$H NMR (400 MHz, $CD_3Cl$) δ 8.47 - 8.30 (m, 1H), 7.90 (s, 1H), 7.42 (d, 2H), 7.12 (s, 1H), 4.43 (s, 1H), 4.02 (s, 1H), 2.92 (d, 2H), 1.40 (s, 9H), 1.15 (d, 3H).

Step 2: (R)-1-(5-(trifluoromethyl)-1H-indol-3-yl)propan-2-amine **(23C)**

To a 100 mL three-neck bottle was added **23B** (2.7 g, 7.89 mmol), same was dissolved in anhydrous methanol (15 mL), hydrogen chloride in dioxane (16 mL) was added, stirred at room temperature for 2 h, and concentrated to obtain the title compound **23C** (1.9 g, 99%), which was directly used in the next reaction.

Step 3: (R)-3-((tert-butyldiphenylsilyl)oxy)-2,2-difluoro-N-(1-(5-(trifluoromethyl)-1H-indol-3-yl)propan-2-yl)propan-1-amine **(23D)**

To a 100 mL three-neck bottle was added **23C** (1.90 g, 7.84 mmol), same was dissolved in dioxane (20 mL) and DMF (2 mL), 5I (4.54 g, 9.41 mmol) and DIPEA (3.04 g, 23.52 mmol) were added, stirred at 90°C for 18 h, concentrated, and purified by column chromatography (PE : EA = 10 : 1-5 : 1) to obtain the title compound **23D** (1.3 g, 29%).
LC-MS (ESI): m/z = 575.3 $[M+H]^+$.

Step 4: (R)-2,2-difluoro-3-((1-(5-(trifluoromethyl)-1H-indol-3-yl)propan-2-yl)amino)propan-1-ol **(23E)**

To a 100 mL three-neck bottle was added **23D** (1.3 g, 2.26 mmol), same was dissolved in tetrahydrofuran (10 mL), tetrabutylammonium fluoride (4.52 mL, 1 M) was added, stirred at room temperature for 3 h, washed with water (20 mL), extracted with ethyl acetate (2 × 20 mL), the organic phases were combined, dried over anhydrous sodium sulfate, concentrated, and purified by column chromatography (PE : EA = 2 : 1) to obtain the title compound **23E** (0.60 g, 79%).
LC-MS (ESI): m/z = 337.1 $[M+H]^+$.

Step 5: 3-((1R,3R)-1-(2,6-difluoro-4-(((S)-1-(3-fluoropropyl)pyrrolidin-3-yl)amino)phenyl)-3-methyl-6-(trifluoromethyl)-1,3,4,9-tetrahydro-2H-pyrido[3,4-b]indol-2-yl)-2,2-difluoropropan-1-ol **(compound 23)**

To a 10 mL of microwave tube was added **23E** (0.10 g, 0.30 mmol) and **intermediate 1** (100 mg, 0.36 mmol),

same was dissolved in toluene (4 mL), acetic acid (2 mL) was added, stirred at 120°C under microwave for 2 h, concentrated, and separated by HPLC to obtain the title compound **23** (10 mg, 5%).

Preparation method: instrument: waters 2767 (preparative liquid phase chromatographic instrument); chromatographic column: SunFire@ Prep C18 (19 mm × 250 mm); the sample was dissolved in DMF and filtered with a 0.45 μm filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 45%-70%; c. flow rate: 15 mL/min; d. elution time: 20 min; retention time: 10.1 min.

$^1$H NMR (400 MHz, DMSO-d6) δ 11.06 (s, 1H), 7.76 (s, 1H), 7.42 - 7.24 (m, 2H), 6.42 (d, 1H), 6.15 (d, 2H), 5.24 (t, 1H), 5.08 (s, 1H), 4.54 (t, 1H), 4.42 (t, 1H), 3.84 (s, 1H), 3.69-3.64 (m, 1H), 3.52 - 3.36 (m, 2H), 3.15-3.04 (m, 1H), 2.92 - 2.70 (m, 2H), 2.67-2.59 (m, 3H), 2.35 - 2.26 (m, 1H), 2.24-2.17 (m, 1H), 1.86-1.73 (m, 2H), 1.57-1.51 (m, 1H), 1.38 - 1.23 (m, 2H), 1.07 (d, 3H), 0.87 (t, 1H).

LC-MS (ESI): m/z = 605.2 $[M+H]^+$.

**Example 24: 6-((1S,3R)-3,7-dimethyl-2-(2,2,2-trifluoroethyl)-2,3,4,9-tetrahydro-1H-pyrido [3,4-b] indol-1-yl)-N-((S)-1-(3-fluoropropyl)pyrrolidin-3-yl)pyridin-3-amine (compound 24)**

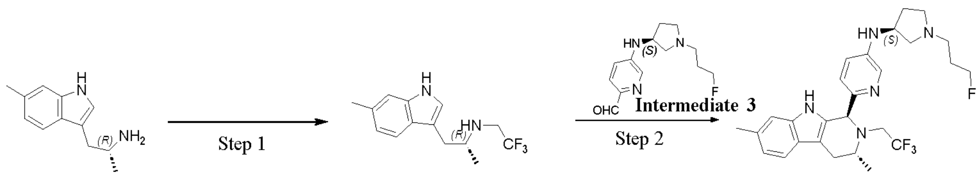


24A 24B Compound 24

Step 1: (R)-1-(6-methyl-1H-indol-3-yl)-N-(2,2,2-trifluoroethyl)propan-2-amine **(24B)**

To a 100 mL three-neck bottle was added **24A** (1.25 g, 6.64 mmol), same was dissolved in dioxane (15 mL), 2,2,2-trifluoroethyl trifluoromethanesulfonate (1.85 g, 7.97 mmol) and DIPEA (1.72 g, 13.28 mmol) were added, stirred at 90°C for 18 h, concentrated, and subjected to column chromatography (PE : EA = 10 : 1-5 : 1) to obtain the title compound **24B** (1.0 g, 56%).

LC-MS (ESI): m/z = 537.2 $[M+H]^+$.

Step 2: 6-((1S,3R)-3,7-dimethyl-2-(2,2,2-trifluoroethyl)-2,3,4,9-tetrahydro-1H-pyrido[3,4-b]indol-1-yl)-N-((S)-1-(3-fluoropropyl)pyrrolidin-3-yl)pyridin-3-amine **(compound 24)**

To a 100 mL single-neck bottle was added 24B (81 mg, 0.30 mmol) and **intermediate 3** (110 mg, 0.45 mmol), same was dissolved in toluene (4 mL), acetic acid (2 mL) was added, stirred at 100°C for 2 h, concentrated, and separated by HPLC to obtain the title compound **24** (12 mg, 8%).

Preparation method: instrument: waters 2767 (preparative liquid phase chromatographic instrument); chromatographic column: SunFire@ Prep C18 (19 mm × 250 mm); the sample was dissolved in DMF and filtered with a 0.45 μm filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 40%-80%; c. flow rate: 15 mL/min; d. elution time: 20 min; retention time: 11.5 min.

$^1$H NMR (400 MHz, DMSO-d6) δ 10.40 (s, 1H), 7.86 (s, 1H), 7.28 (d, 1H), 7.04 (d, 2H), 6.91 (d, 1H), 6.79 (d, 1H), 5.95 (d, 1H), 4.88 (s, 1H), 4.54 (t, J = 6.0 Hz, 1H), 4.43 (t, J = 6.0 Hz, 1H), 3.86 (s, 1H), 3.55-3.51 (m, 1H), 3.04-2.98 (m, 2H), 2.83-2.79 (t, 1H), 2.67-2.60 (m , 2H), 2.37-2.33 (m, 5H), 2.20-2.17 (m, 2H), 1.85-1.76 (m, 2H), 1.56 (s, 1H), 1.25 (s, 1H), 1.13 (d, 3H).

LC-MS (ESI): m/z = 504.3 $[M+H]^+$.

**Example 25: N-((1S,3R)-1-(2,6-difluoro-4-((1-(3-fluoropropyl)azetidin-3-yl)amino)phenyl)-5-fluoro-3-methyl-2-(2,2,2-trifluoroethyl)-1,2,3,4-tetrahydroisoquinolin-6-yl)ethanesulfonamide(compound 25)**

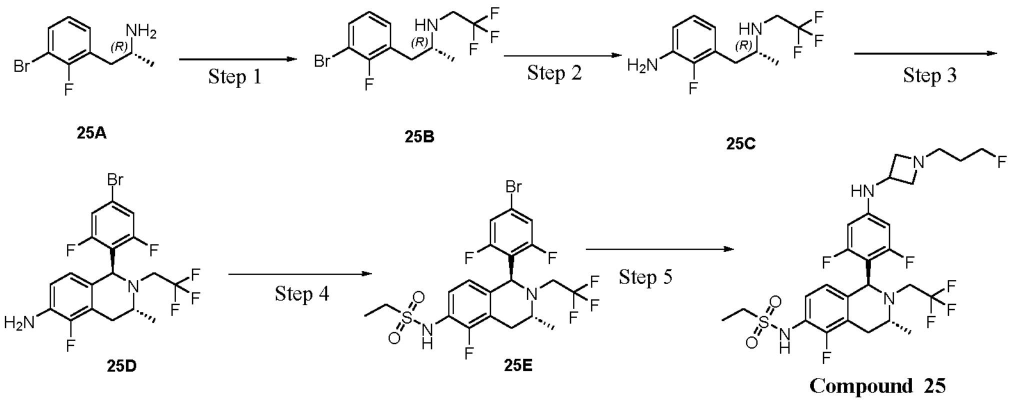


25A 25B 25C 25D 25E Compound 25

Step 1: (R)-1-(3-bromo-2-fluorophenyl)-N-(2,2,2-trifluoroethyl)propan-2-amine **(25B)**

To a 250 mL three-neck bottle was added **25A** (5.0 g, 21.54 mmol), same was dissolved in dioxane (50 mL), 2,2,2-trifluoroethyl trifluoromethanesulfonate (6.25 g, 26.92 mmol) and DIPEA (4.18 g, 32.31 mmol) were added, stirred at 90°C for 18 h, concentrated, and subjected to column chromatography (PE : EA = 30 : 1-20 : 1) to obtain the title compound **25B** (5.5 g, 81%).
LC-MS (ESI): m/z = 314.1 $[M+H]^+$.

Step 2: (R)-2-fluoro-3-(2-((2,2,2-trifluoroethyl)amino)propyl)aniline **(25C)**

To a 100 mL single-neck bottle was added **25B** (5.5 g, 17.51mmol), same was dissolved in toluene (36 mL), benzhydrylamine (6.4 g, 35.02 mmol), sodium tert-butoxide (5.1 g, 52.53 mmol), BINAP (1.1 g, 1.75 mmol) and $Pd_2(dba)_3$ (0.8 g, 0.88 mmol) were added, under nitrogen protection and at 90°C, the mixture was stirred for 18 h, HCl (2 M, 20 mL) was added, stirred for 2 h, filtered through celite, concentrated, and separated by column chromatography (PE : EA = 20 : 1) to obtain the title compound **25C** (3.3 g, 75%).
LC-MS (ESI): m/z = 251.1 $[M+H]^+$.

Step 3: (1S,3R)-1-(4-bromo-2,6-difluorophenyl)-5-fluoro-3-methyl-2-(2,2,2-trifluoroethyl)-1,2,3,4-tetrahydroisoquinolin-6-amine **(25D)**

To a 100 mL single-neck bottle was added **25C** (1.0 g, 4.00 mmol), same was dissolved in dioxane (20 mL), 4-bromo-2,6-difluorobenzaldehyde (1.77 g, 8.00 mmol) and trifluoroacetic acid (0.46 g, 4.00 mmol) were added, under nitrogen protection and at 70°C, the mixture was stirred for 18 h, concentrated, and separated by column chromatography (PE : EA = 10 : 1) to obtain the title compound **25D** (0.84 g, 46%).
LC-MS (ESI): m/z = 453.0 $[M+H]^+$.

Step 4: N-((1S,3R)-1-(4-bromo-2,6-difluorophenyl)-5-fluoro-3-methyl-2-(2,2,2-trifluoroethyl)-1,2,3,4-tetrahydroisoquinolin-6-yl)ethanesulfonamide **(25E)**

To a 100 mL single-neck bottle was added **25D** (0.77 g, 1.70 mmol), same was dissolved in dichloromethane (10 mL), pyridine (0.67 g, 8.50 mmol) and ethylsulfonyl chloride (0.66 g, 5.10 mmol) were added, stirred at 30°C for 18 h, concentrated, and separated by column chromatography (PE : EA = 5 : 1) to obtain the title compound **25E** (0.56 g, 60%).
LC-MS (ESI): m/z = 545.1 $[M+H]^+$.

Step 5: N-((1S,3R)-1-(2,6-difluoro-4-((1-(3-fluoropropyl)azetidin-3-yl)amino)phenyl)-5-fluoro-3-methyl-2-(2,2,2-trifluoroethyl)-1,2,3,4-tetrahydroisoquinolin-6-yl)ethanesulfonamide **(compound 25)**

To a 50 mL single-neck bottle was added **25E** (0.10 g, 0.18 mmol), same was dissolved in dioxane (10 mL), **1M** (48 mg, 0.36 mmol), cesium carbonate (0.15 g, 0.45 mmol) and Brettphos G3 Pd (16 mg, 0.018 mmol) were added, the reaction system was subjected to nitrogen replacement three times, reacted at 105°C for 4 h, filtered through celite, concentrated, purified by preparative HPLC, and lyophilized to obtain the title compound **25** (50 mg, 46%).
Preparation method: instrument: waters 2767 (preparative liquid phase chromatographic instrument); chromatographic column: SunFire@ Prep C18 (19 mm × 250 mm); the sample was dissolved in DMF and filtered with a 0.45

μm filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 40%-80%; c. flow rate: 15 mL/min; d. elution time: 20 min; retention time: 10.5 min.

$^1$H NMR (400 MHz, DMSO-d6) δ 6.97 (t, 1H), 6.63 (d, 1H), 6.27 (d, 1H), 6.07 (d, 2H), 4.98 (s, 1H), 4.50 (t, 1H), 4.39 (t, 1H), 3.93-3.89 (m, 1H), 3.66 - 3.55 (m, 2H), 3.40-3.30 (m, 2H), 2.89 - 2.63 (m, 6H), 2.59-2.55 (m, 1H), 2.47-2.43 (m, 2H), 1.70-1.58 (m, 2H), 1.11 (t, 3H), 1.02 (d, 3H).

LC-MS (ESI): m/z = 597.2 $[M+H]^+$.

**Example 26: N-((1S,3R)-5-fluoro-1-(5-((1-(3-fluoropropyl)azetidin-3-yl)amino)pyridin-2-yl)-3-methyl-2-(2,2,2-trifluoroethyl)-1,2,3,4-tetrahydroisoquinolin-6-yl)ethanesulfonamid(compound 26)**

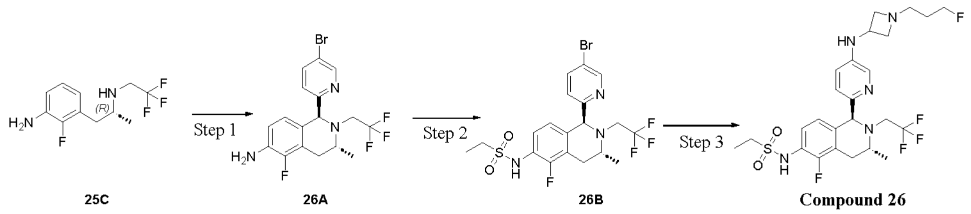


25C 26A 26B Compound 26

Step 1: (1S,3R)-1-(5-bromopyridin-2-yl)-5-fluoro-3-methyl-2-(2,2,2-trifluoroethyl)-1,2,3,4-tetrahydroisoquinolin-6-amine **(26A)**

To a 100 mL single-neck bottle was added **25C** (1.0 g, 4.00 mmol), same was dissolved in dioxane (20 mL), 5-bromopyridine-2-formaldehyde (1.49 g, 8.00 mmol) and trifluoroacetic acid (0.46 g, 4.00 mmol) were added, under nitrogen protection and at 70°C, the mixture was stirred for 18 h, concentrated, and separated by column chromatography (PE : EA = 10 : 1-5 : 1) to obtain the title compound **26A** (1.5 g, 90%).

LC-MS (ESI): m/z = 418.1 $[M+H]^+$.

Step 2: N-((1S,3R)-1-(5-bromopyridin-2-yl)-5-fluoro-3-methyl-2-(2,2,2-trifluoroethyl)-1,2,3,4-tetrahydroisoquinolin-6-yl)ethanesulfonamide **(26B)**

To a 100 mL single-neck bottle was added **26A** (0.80 g, 1.91 mmol), same was dissolved in dichloromethane (10 mL), pyridine (0.76 g, 9.55 mmol) and ethylsulfonyl chloride (0.74 g, 5.73 mmol) were added, stirred at 30°C for 18 h, concentrated, and separated by column chromatography (PE : EA = 5 : 1) to obtain the title compound 2**6B** (0.27 g, 28%).

LC-MS (ESI): m/z = 510.0 $[M+H]^+$.

Step 3: N-((1S,3R)-5-fluoro-1-(5-((1-(3-fluoropropyl)azetidin-3-yl)amino) pyridin-2-yl)-3-methyl-2-(2,2,2-trifluoroethyl)-1,2,3,4-tetrahydroisoquinolin-6-yl)ethanesulfonamid **(compound 26)**

To a 50 mL single-neck bottle was added **26B** (0.10 g, 0.20 mmol), same was dissolved in dioxane (10 mL), **1M** (53 mg, 0.40 mmol), cesium carbonate (0.16 g, 0.50 mmol) and Brettphos G3 Pd (18 mg, 0.020 mmol) were added, the reaction system was subjected to nitrogen replacement three times, reacted at 105°C for 4 h, filtered through celite, concentrated, purified by preparative HPLC, and lyophilized to obtain the title compound **26** (18 mg, 16%).

Preparation method: instrument: waters 2767 (preparative liquid phase chromatographic instrument); chromatographic column: SunFire@ Prep C18 (19 mm × 250 mm); the sample was dissolved in DMF and filtered with a 0.45 μm filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 40%-80%; c. flow rate: 15 mL/min; d. elution time: 20 min; retention time: 11.2 min.

$^1$H NMR (400 MHz, DMSO-d6) δ 10.01 (d, 1H), 9.54 (s, 1H), 7.83 (dd, 1H), 7.27 (d, 1H), 7.16 - 7.04 (m, 2H), 6.72 (d, 1H), 5.03 (s, 1H), 4.60-4.55 (m, 2H), 4.49 - 4.27 (m, 3H), 4.10-4.04 (m, 1H), 3.88-3.86 (m, 1H), 3.58-3.51 (m, 1H), 3.38 - 3.26 (m, 3H), 3.08 (q, J = 7.3 Hz, 2H), 3.03-2.92 (m, 1H), 2.82-2.77 (m, 1H), 2.62-2.56 (m, 1H), 2.00 - 1.83 (m, 2H), 1.27 (t, 3H), 1.06 (d, 3H).

LC-MS (ESI): m/z = 562.2 $[M+H]^+$.

**Example 27: N-((1S,3R)-1-(2,6-difluoro-4-((1-(3-fluoropropyl)azetidin-3-yl)amino)phenyl)-5-fluoro-2-(2-fluoro-2-methylpropyl)-3-methyl-1,2,3,4-tetrahydroisoquinolin-6-yl)ethanesulfonamide(compound 27)**

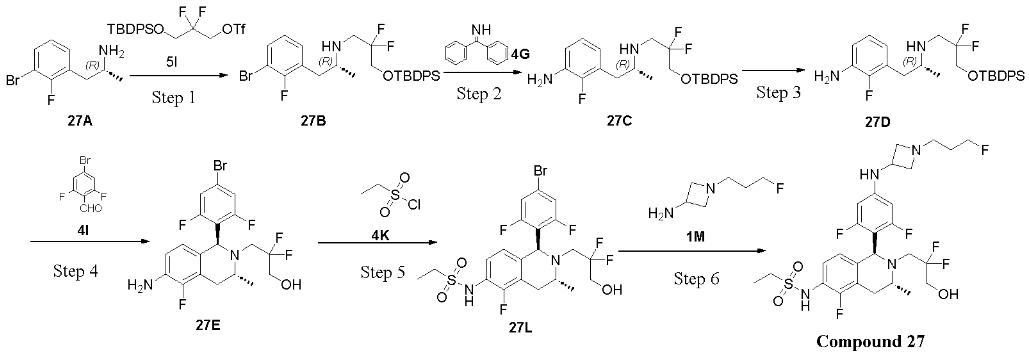


Step 1: (R)-N-(1-(3-bromo-2-fluorophenyl)propan-2-yl)-3-((tert-butyldiphenylsilyl)oxy)-2,2-difluoropropan-1-amine **(27B)**

To a 250 mL single-neck bottle was added **27A** (10 g, 43.09 mmol), same was dissolved in dioxane (100 mL), DIPEA (16.71 g, 129.27 mmol) and **5I** (2.57 g, 12.32 mmol) were added, reacted at 90°C for 12 h, concentrated and purified by column chromatography (petroleum ether : ethyl acetate (v/v) = 10 : 1-4 : 1) to obtain the title compound **27B** (8 g, 32.89%).
LC-MS (ESI): m/z = 564.1 $[M+H]^+$.

Step 2: (R)-3-(2-((3-((tert-butyldiphenylsilyl)oxy)-2,2-difluoropropyl)amino) propyl)-2-fluoroaniline **(27C)**

To a 100 mL single-neck bottle was added **27B** (4 g, 7.08 mmol), same was dissolved in dioxane (40 mL), **4G** (3.85 g, 21.24 mmol), t-BuONa (5.31 g, 21.24 mmol), BINAP (486.6 mg, 0.78 mmol) and $Pd_2(dba)_3$ (227.9 mg, 0.39 mmol) were added, the reaction system was subjected to nitrogen replacement three times, reacted at 90°C for 4 h, cooled to room temperature, dissolved in HCl (1 M, 40 mL), extracted with DCM (2 × 40 mL) to remove impurities, the aqueous phase was collected, the reaction system was adjusted to pH > 10 with NaOH (2 M), extracted with EA (2 × 40 mL), the organic phases were combined, dried over anhydrous sodium sulfate for 0.5 h, concentrated and purified by column chromatography (petroleum ether : ethyl acetate (v/v) = 10 : 1-2 : 1) to obtain the title compound **27C** (0.2 g, crude).
LC-MS (ESI): m/z = 501.2 $[M+H]^+$.

Step 3: (R)-3-((1-(3-amino-2-fluorophenyl)propan-2-yl)amino)-2,2-difluoropropan-1-ol **(27D)**

To a 10 mL three-neck bottle was added **27C** (2 g, 3.99 mmol), same was dissolved in tetrahydrofuran (20 mL), tetrabutylammonium fluoride (3.29 mL, 1 M) was added, stirred at room temperature for 3 h, washed with water (20 mL), extracted with ethyl acetate (2 × 20 mL), the organic phases were combined, dried over anhydrous sodium sulfate, concentrated, and purified by column chromatography (petroleum ether : ethyl acetate = 2 : 1) to obtain the title compound **27D** (0.5 g, 47.8%).
LC-MS (ESI): m/z = 263.2 $[M+H]^+$.

Step 4: 3-((1S,3R)-6-amino-1-(4-bromo-2,6-difluorophenyl)-5-fluoro-3-methyl-3,4-dihydroisoquinolin-2(1H)-yl)-2,2-difluoropropan-1-ol **(27E)**

To a 100 mL single-neck bottle was added **27D** (0.35 g, 1.33 mmol), same was dissolved in acetic acid (2 mL) and 1,4-dioxane (20 mL), **41** (0.59 g, 2.66 mmol) was added, reacted at 100°C for 2 h, concentrated, dissolved in DCM (20 mL), washed with HCl (1 M, 20 mL), the aqueous phase was collected, the reaction system was adjusted to pH > 10 with NaOH (2 M), extracted with DCM (2 × 20 mL), the organic phases were combined, dried over anhydrous sodium sulfate for 0.5 h, concentrated and purified by column chromatography (petroleum ether : ethyl acetate (v/v) = 3 : 1-1 : 1) to obtain the title compound **27E** (0.13 g, 21%).
LC-MS (ESI): m/z = 465.1 $[M+H]^+$.

Step 5: N-((1S,3R)-1-(4-bromo-2,6-difluorophenyl)-2-(2,2-difluoro-3-hydroxypropyl)-5-fluoro-3-methyl-1,2,3,4-tetrahydroisoquinolin-6-yl)ethanesulfonamide **(27L)**

To a 50 mL single-neck bottle was added **27D** (0.11 g, 0.24 mmol), same was dissolved in DCM (10 mL), pyridine (0.076 g, 0.96 mmol)) and **4K** (0.093 g, 0.72 mmol) were added, reacted at room temperature for 18 h, concentrated and purified by column chromatography (petroleum ether : ethyl acetate (v/v) = 3 : 1-1 : 1) to obtain the title compound **27L** (70 mg, 52.3%).

LC-MS (ESI): m/z = 557.1 $[M+H]^+$.

Step 6: N-((1S,3R)-2-(2,2-difluoro-3-hydroxypropyl)-1-(2,6-difluoro-4-((1-(3-fluoropropyl)azetidin-3-yl)amino)phenyl)-5-fluoro-3-methyl-1,2,3,4-tetrahydroisoquinolin-6-yl)ethanesulfonamide (compound 27)

To a 50 mL single-neck bottle was added **27L** (0.06 g, 0.11 mmol), same was dissolved in dioxane (10 mL), **1M** (40 mg, 0.3 mmol), $Cs_2CO_3$ (77 mg, 0.3 mmol), Brettphos G3 Pd (10 mg, 0.01 mmol) were added, the reaction system was subjected to nitrogen replacement three times, reacted at 100°C for 5 h, filtered over celite, concentrated, and subjected to preparative HPLC to obtain the title compound **27** (15 mg, 22.4%).

Preparation method: instrument: waters 2767 (preparative liquid phase chromatographic instrument); chromatographic column: SunFire@ Prep C18 (19 mm × 250 mm); the sample was dissolved in DMF and filtered with a 0.45 μm filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 40%-80%; c. flow rate: 15 mL/min; d. elution time: 20 min; retention time: 10.38 min.

$^1H$ NMR (400 MHz, DMSO-d6) $\delta$7.07 (t, 1H), 6.68 (d, 1H), 6.58 (d, 1H), 6.09 (d, 2H), 5.23 (s, 1H), 5.00 (s, 1H), 4.51 (t, 1H), 4.39 (t, 1H), 3.89-3.94 (m, 1H), 3.57-3.63 (m, 3H), 3.36-3.45 (m, 2H), 3.02-3.08 (m, 3H), 2.81-2.86 (m, 1H),2.73 (t, 2H), 2.54-2.66 (m, 2H), 2.45 (t, 2H), 1.91 (s, 2H), 1.59-1.69 (m, 2H), 1.25 (t, 3H), 1.00(d, 3H).

LC-MS (ESI): m/z = 609.2 $[M+H]^+$.

**Example 28: N-((1S,3R)-2-(2,2-difluoro-3-hydroxypropyl)-5-fluoro-1-(5-((1-(3-fluoropropyl)azetidin-3-yl)amino)pyridin-2-yl)-3-methyl-1,2,3,4-tetrahydroisoquinolin-6-yl)ethanesulfonamide(compound 28)**

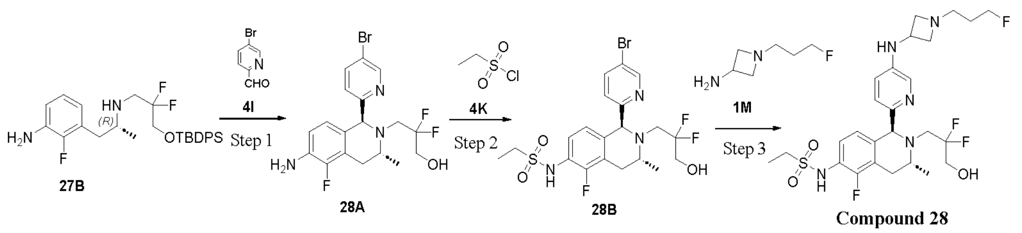


Step 1: 3-((1S,3R)-6-amino-1-(5-bromopyridin-2-yl)-5-fluoro-3-methyl-3,4-dihydroisoquinolin-2(1H)-yl)-2,2-difluoropropan-1-ol **(28A)**

To a 100 mL single-neck bottle was added **27B** (0.4 g, 1.53 mmol), same was dissolved in acetic acid (2 mL) and toluene (20 mL), **41** (0.57 g, 3.06 mmol) was added, reacted at 100°C for 2 h, concentrated, dissolved in DCM (20 mL), washed with HCl (1 M, 20 mL), the aqueous phase was collected, the reaction system was adjusted to pH > 10 with NaOH (2 M), extracted with DCM (2 × 20 mL), the organic phases were combined, dried over anhydrous sodium sulfate for 0.5 h, concentrated and purified by column chromatography (petroleum ether : ethyl acetate (v/v) = 3 : 1-1 : 1) to obtain the title compound **28A** (0.4 g, 61%).

LC-MS (ESI): m/z = 430.1 $[M+H]^+$.

Step 6: N-((1S,3R)-1-(5-bromopyridin-2-yl)-2-(2,2-difluoro-3-hydroxypropyl) -5-fluoro-3-methyl-1,2,3,4-tetrahydroisoquinolin-6-yl)ethanesulfonamide **(28B)**

To a 50 mL single-neck bottle was added **28A** (0.25 g, 0.58 mmol), same was dissolved in DCM (10 mL), pyridine (0.23 g, 2.9 mmol)) and **4K** (0.22 g, 1.74 mmol) were added, reacted at room temperature for 18 h, concentrated and purified by column chromatography (petroleum ether : ethyl acetate (v/v) = 3 : 1-1 : 1) to obtain the title compound **28B** (100 mg, 33%).

LC-MS (ESI): m/z = 522.1 $[M+H]^+$.

Step 7: N-((1S,3R)-2-(2,2-difluoro-3-hydroxypropyl)-5-fluoro-1-(5-((1-(3-fluoropropyl)azetidin-3-yl)amino)pyridin-2-yl)-3-methyl-1,2,3,4-tetrahydroisoquinolin-6-yl)ethanesulfonamide **(compound 28)**

To a 50 mL single-neck bottle was added **28B** (0.1 g, 0.19 mmol), same was dissolved in dioxane (10 mL), **1M** (75 mg, 0.57 mmol), $Cs_2CO_3$ (110 mg, 0.57 mmol), and Brettphos G3 Pd (17 mg, 0.019 mmol) were added, the reaction system was subjected to nitrogen replacement three times, reacted at 100°C for 5 h, filtered over celite, concentrated, and subjected to preparative HPLC to obtain the title compound **28** (15 mg, 13.8%).

Preparation method: instrument: waters 2767 (preparative liquid phase chromatographic instrument); chromatographic column: SunFire@ Prep C18 (19 mm × 250 mm); the sample was dissolved in DMF and filtered with a 0.45 μm filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 20%-70%; c. flow rate: 15 mL/min; d. elution time: 20 min; retention time: 10.38 min.

$^1$H NMR (400 MHz, DMSO-d6) δ8.80 (s, 1H), 7.74 (d, 1H), 7.08 (t, 1H), 6.99 (d, 1H), 6.83 (dd, 1H), 6.65 (d, 1H), 6.23 (d, 1H), 5.42 (s, 1H), 4.88 (s, 1H), 4.51 (t, 1H), 4.39 (t, 1H), 3.88-3.96 (m, 1H), 3.61-3.69 (m, 4H), 2.98-3.14 (m, 4H), 2.73 (t, 2H), 2.58-2.69 (m, 2H), 2.46 (t, 3H), 1.58-1.69 (m, 2H), 1.24 (t, 3H), 1.01(d, 3H).

LC-MS (ESI): m/z = 574.2 $[M+H]^+$.

**Example 29: 3-((1R,3R)-1-(2,6-difluoro-4-(((S)-1-(3-fluoropropyl)pyrrolidin-3-yl)amino)phenyl)-3-methyl-7-(trifluoromethyl)-1,3,4,9-tetrahydro-2H-pyrido[3,4-b]indol-2-yl)-2,2-difluoropropan-1-ol (compound 29)**

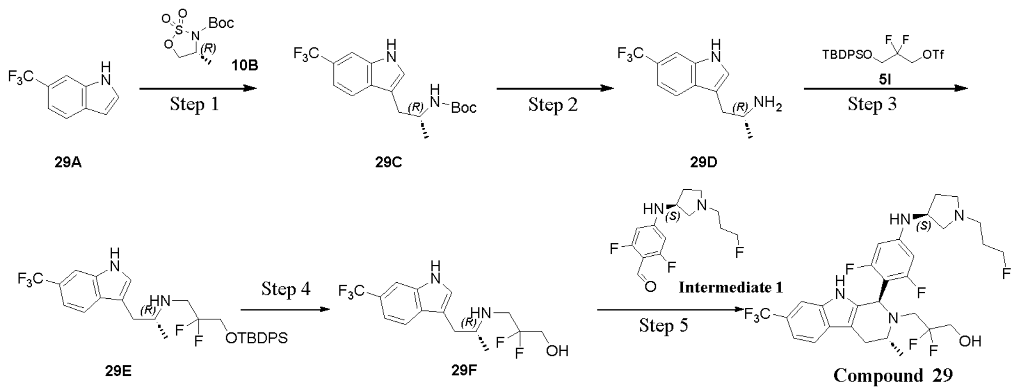


Step 1: tert-butyl (R)-(1-(6-(trifluoromethyl)-1H-indol-3-yl)propan-2-yl)carbamate **(29C)**

To a 250 mL three-neck bottle were added **29A** (5.0 g, 27.01 mmol) and cuprous chloride (2.14 g, 21.61 mmol), same was dissolved in anhydrous dichloromethane (50 mL), cooled to 0°C, methylmagnesium bromide (9.00 mL, 3 M) was added dropwise, the reaction was carried out with the temperature maintained constant for 1 h, cooled to -20°C, a solution of 10B (4.49, 18.91 mmol) in dichloromethane (20 mL) was added dropwise, stirred overnight while maintaining this temperature constant, quenched with 10% citric acid solution (100 mL), stirred for 0.5 h, filtered through celite, and extracted with dichloromethane (2 × 100 mL), the organic phases were combined, dried over anhydrous sodium sulfate, concentrated and purified by column chromatography (petroleum ether : ethyl acetate = 10 : 1-5 : 1) to obtain the title compound 29C (1.8 g, 19.5%).

LC-MS (ESI): m/z = 343.2$[M+H]^+$.

Step 2: (R)-1-(6-(trifluoromethyl)-1H-indol-3-yl)propan-2-amine **(29D)**

To a 100 mL three-neck bottle was added **29C** (1.8 g, 13.14 mmol), same was dissolved in anhydrous methanol (10 mL), hydrogen chloride in dioxane (10 mL) was added, stirred at room temperature for 2 h, and concentrated to obtain the title compound **29D** (1.8 g, crude).

Step 3: (R)-3-((tert-butyldiphenylsilyl)oxy)-2,2-difluoro-N-(1-(6-(trifluoromethyl)-1H-indol-3-yl)propan-2-yl)propan-1-amine **(29E)**

To a 100 mL three-neck bottle was added **29D** (1.8 g, 7.43 mmol), same was dissolved in dioxane (40 mL), 5I (4.3 g, 8.92 mmol) and N,N-diisopropyl ethylamine (2.88 g, 22.29 mmol) were added, stirred at 90°C for 12 h, concentrated, and purified by column chromatography (petroleum ether : ethyl acetate = 10 : 1-5 : 1) to obtain the title compound 29**E** (1.2 g, 28.1%).

LC-MS (ESI): m/z = 575.1 $[M+H]^+$.

Step 4: (R)-2,2-difluoro-3-((1-(6-(trifluoromethyl)-1H-indol-3-yl)propan-2-yl)amino)propan-1-ol **(29F)**

To a 100 mL three-neck bottle was added **29E** (1.2 g, 2.09 mmol), same was dissolved in tetrahydrofuran (20 mL), tetrabutylammonium fluoride (6.27 mL, 1 M) was added, stirred at room temperature for 3 h, washed with water (50 mL), extracted with ethyl acetate (2 × 20 mL), the organic phases were combined, dried over anhydrous sodium sulfate, concentrated, and purified by column chromatography (petroleum ether : ethyl acetate = 2 : 1) to obtain the title compound **29F** (0.6 g, 85%).

LC-MS (ESI): m/z = 337.2 $[M+H]^+$.

Step 5: 3-((1R,3R)-1-(2,6-difluoro-4-(((S)-1-(3-fluoropropyl)pyrrolidin-3-yl)amino)phenyl)-3-methyl-7-(trifluoromethyl)-1,3,4,9-tetrahydro-2H-pyrido[3,4-b]indol-2-yl)-2,2-difluoropropan-1-ol **(compound 29)**

To a 50 mL single-neck bottle were added **29F** (0.1 g, 0.3 mmol) and **intermediate 1** (100 mg, 0.3 mmol), same was dissolved in toluene (8 mL), acetic acid (2 mL) was added, stirred under nitrogen protection at 100°C for 3 h, concentrated, and subjected to preparative HPLC to obtain the title compound **29** (30 mg, 16.5%).

Preparation method: instrument: waters 2767 (preparative liquid phase chromatographic instrument); chromatographic column: SunFire@ Prep C18 (19 mm × 250 mm); the sample was dissolved in methanol and filtered with a 0.45 μm filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 30%-65%; c. flow rate: 15 mL/min; d. elution time: 15 min; retention time: 8.40 min.

$^1$H NMR (400 MHz, DMSO-d6) δ 11.04 (s, 1H), 7.58 (d, 1H), 7.51 (s, 1H), 7.23 (d, 1H), 6.43 (d, 1H), 6.16 (d, 2H), 5.11 (s, 1H), 4.54 (t, 1H), 4.42 (t, 1H), 3.85 (s, 1H),3.60-3.70 (m, 1H), 3.38 - 3.50 (m, 2H), 3.04- 3.15 (m, 1H), 2.84 -2.88 (m, 1H), 2.75 (t, 1H), 2.56 - 2.65 (m, 3H), 2.43 - 2.46(m, 2H), 2.33 - 2.36 (m, 1H), 2.15 - 2.23 (m, 1H), 1.89 (s, 2H), 1.73- 1.87 (m, 2H), 1.08 (d, 3H).

LC-MS (ESI): m/z = 605.1 $[M+H]^+$.

**Example 30: 3-((1R,3R)-1-(2,6-difluoro-4-((1-(3-fluoropropyl)azetidin-3-yl)amino)phenyl)-3-methyl-1,3,4,6,7,9-hexahydro-2H-cyclobuta[f]pyrido[3,4-b]indol-2-yl)-2,2-difluoropropan-1-ol and** 3-((1S,3S)-1-(2,6-difluoro-4-((1-(3-fluoropropyl)azetidin-3-yl)amino)phenyl)-3-methyl-1,3,4,6,7,9-hexahydro-2H-cyclobuta[f]pyrido[3,4-b]indol-2-yl)-2,2-difluoropropan-1-ol **(compound 30-1** and **compound 30-2)**

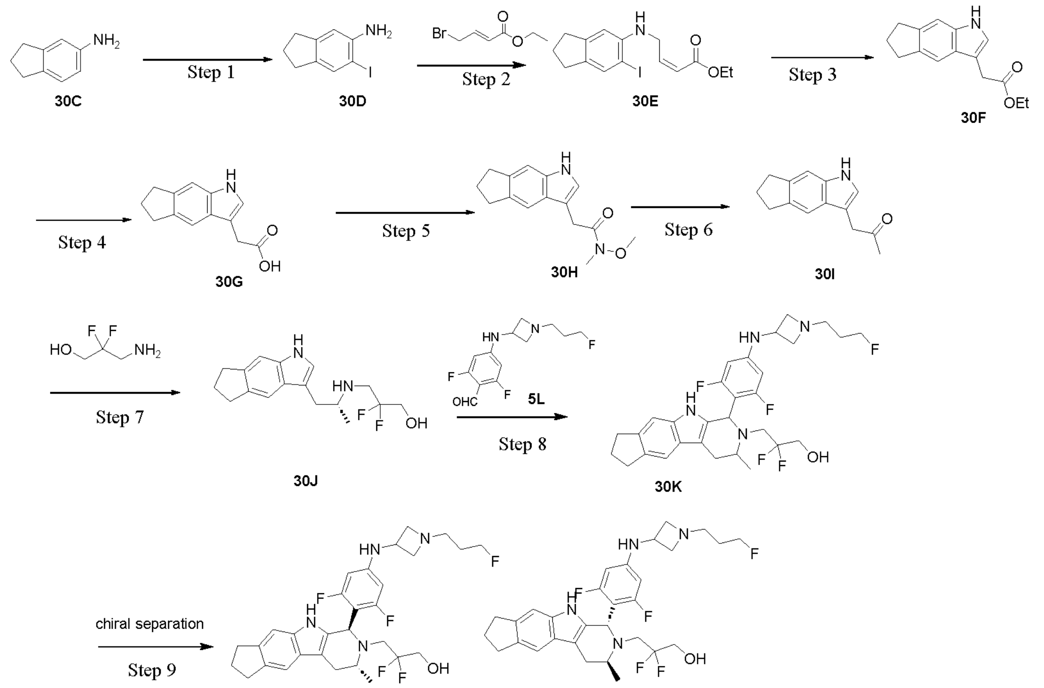

30C 30D 30E 30F 30G 30H 30I 30J 5L 30K

Compound 30-1 and Compound 30-2

Step 1: 6-iodo-2,3-dihydro-1H-inden-5-amine **(30D)**

To a 250 mL round bottom flask was added **30C** (5.0 g, 37.54 mmol), same was dissolved in acetonitrile (50 mL), NIS (8.45 g, 37.54 mmol) was added, stirred at room temperature for 3 h, concentrated, same was dissolved in dichloromethane (50 mL), washed with a saturated sodium thiosulfate solution, the organic phase was dried over anhydrous sodium sulfate, and separated and purified by column chromatography (PE : EA = 20 : 1) to obtain the title compound **30D** (5.0 g, 51%).

$^1$H NMR (400 MHz, CDCl3) $\delta$ 7.25 (s, 1H), 6.67(s, 1H), 2.75-2.95 (m, 4H), 2.01-2.04 (m, 2H).

Step 2: ethyl (Z)-4-((6-iodo-2,3-dihydro-1H-inden-5-yl)amino)but-2-enoate **(30E)**

To a 250 mL round bottom flask was added **30D** (5 g, 19.3 mmol), same was dissolved in DMF (50 mL), ethyl 4-butenoate (5.59 g, 28.95 mmol) and potassium carbonate (8.0 g, 57.9 mmol) were added, stirred at 70°C for 18 h, water (100 mL) was added, extracted with ethyl acetate (2 $\times$ 100 mL), the organic phases were combined, washed with saturated brine, dried over anhydrous sodium sulfate, and separated and purified by column chromatography (PE : EA = 20 : 1) to obtain the title compound **30E** (4 g, 55.8%).

$^1$H NMR (400 MHz, CDCl3) $\delta$ 7.29 (s, 1H), 7.00-7.05 (m, 1H), 6.50 (s, 1H), 6.03 (d, 1H),4.16-4.21 (m, 2H), 3.99-4.01 (m, 2H), 3.78-2.86 (m, 4H) ,2.00-2.07 (m, 2H), 1.28 (t, 3H).

Step 3: ethyl 2-(1,5,6,7-tetrahydrocyclopenta[f]indol-3-yl)acetate **(30F)**

To a 250 mL round bottom flask was added **30E** (4 g, 10.78 mmol), same was dissolved in DMF (50 mL), palladium acetate (0.12 g, 0.54 mmol), potassium carbonate (4.47 g, 32.34 mmol) and triphenylphosphine (0.28 g, 1.08 mmol) were added, under nitrogen protection, the mixture was stirred at 75°C for 18 h, water (100 mL) was added, extracted with ethyl acetate (2 $\times$ 80 mL), the organic phases were combined, washed with saturated brine, dried over anhydrous sodium sulfate, and separated and purified by column chromatography (PE : EA = 10 : 1-5 : 1) to obtain the title compound **30F** (2.0 g, 72.2%).

$^1$H NMR (400 MHz, CDCl3) $\delta$ 7.88 (s, 1H), 7.43 (s, 1H), 7.17 (s, 1H), 7.07 (s, 1H), 4.13-4.19 (m, 2H) , 3.72 (s, 2H) ,2.95-3.29 (m, 4H) ,2.04-2.17 (m, 2H) , 1.25 (t, 3H).

Step 4: 2-(1,5,6,7-tetrahydrocyclopenta[f]indol-3-yl)acetic acid **(30G)**

To a 100 mL round bottom flask was added **30F** (2 g, 8.22 mmol), same was dissolved in methanol (20 mL),

lithium hydroxide (0.59 g, 24.66 mmol) and water (20 mL) were added, stirred at room temperature for 5 h, concentrated to remove excess methanol, HCl (2 M) was added to adjust to pH = 5-6, extracted with ethyl acetate (2 × 80 mL), the organic phases were combined, and concentrated to obtain the title compound **30G** (1.5 g, 85%).

LC-MS (ESI): m/z = 216.2$[M+H]^+$.

Step 5: N-methoxy-N-methyl-2-(1,5,6,7-tetrahydrocyclopenta[f]indol-3-yl)acetamide **(30H)**

To a 100 mL round bottom flask was added **30G** (1.5 g, 6.97 mmol), same was dissolved in THF (20 mL), dimethylhydroxylamine hydrochloride (0.85 g, 13.94mmol) and diisopropylethylamine (2.70 g, 27.91 mmol) were added, 1-propyl phosphate anhydride (6.65 g, 10.46 mmol) was added dropwise in an ice bath, stirred at room temperature for 18 h, water (50 mL) was added, extracted with ethyl acetate (2 × 50 mL), the organic phases were combined, washed with saturated brine, dried over anhydrous sodium sulfate, and separated by column chromatography (PE : EA = 4 : 1) to obtain the title compound **30H** (1.8 g, 99%).

LC-MS (ESI): m/z = 259.2$[M+H]^+$.

Step 6: 1-(1,5,6,7-tetrahydrocyclopenta[f]indol-3-yl)propan-2-one **(30I)**

To a 100 mL round bottom flask was added **30H** (1.8 g, 6.97 mmol), same was dissolved in THF (20 mL), methylmagnesium bromide (3 M, 4.65 mL) was added dropwise in an ice bath, stirred for 1 h while maintaining in the ice bath, saturated ammonium chloride (50 mL) was added, the reaction was quenched, extracted with ethyl acetate (2 × 50 mL), the organic phases were combined, dried over anhydrous sodium sulfate, and separated by column chromatography (PE : EA = 10 : 1) to obtain the title compound **301** (1.6 g, 68%).

LC-MS (ESI): m/z = 214.2$[M+H]^+$.

Step 7: 2,2-difluoro-3-((1-(1,5,6,7-tetrahydrocyclopenta[f]indol-3 -yl)propan-2-yl)amino)propan-1-ol **(30J)**

To a 100 mL round bottom flask was added **301** (1.3 g, 6.1 mmol), same was dissolved in anhydrous methanol (20 mL), 3-amino-2,2-difluoropropyl-1-ol (1.02 g, 9.15 mmol) and acetic acid (0.37 g, 6.1 mmol) were added, stirred at room temperature for 1 h, sodium cyanoborohydride (0.57 g, 9.15 mmol) was added, stirring was continued at room temperature overnight, concentrated, and separated by column chromatography (PE : EA = 1 : 1-EA) to obtain the title compound **30J** (0.8 g, 42.5%).

LC-MS (ESI): m/z = 309.2$[M+H]^+$.

Step 8: 3-(1-(2,6-difluoro-4-((1-(3-fluoropropyl)azetidin-3-yl)amino)phenyl)-3-methyl-3,4,6,7,8,10-hexahydrocyclopenta[f]pyrido[3,4-b]indol-2(1H)-yl)-2,2-difluoropropan-1-ol **(30K)**

To a 100 mL round bottom flask was added **30J** (0.8 g, 2.59 mmol), and same was dissolved in dioxane (10 mL), **5L** (0.71 g, 2.59 mmol) and trifluoroacetic acid (0.2 mL) were added, stirred at 70°C for 10 h, concentrated, and separated by preparative HPLC to obtain the title compound **30K** (0.3 g, 20%).

Preparation method: instrument: waters 2767 (preparative liquid phase chromatographic instrument); chromatographic column: SunFire@ Prep C18 (19 mm × 250 mm); the sample was dissolved in DMF and filtered with a 0.45 μm filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 40%-70%; c. flow rate: 15 mL/min; d. elution time: 20 min; retention time: 12.0 min.

$^1$H NMR (400 MHz, DMSO-d6) δ 10.22 (s, 1H), 7.16 (s, 1H), 7.00 (s, 1H), 6.66 (d, 1H), 6.09 (d, 2H), 5.21 (t, 1H), 5.01 (s, 1H), 4.51 (t, 1H), 4.39 (t, 1H), 3.89-3.96 (m, 1H), 3.59-3.70 (m, 3H), 3.34 - 3.45 (m, 2H), 3.00-3.11 (m, 1H), 2.86 (t, 4H), 2.71-2.81 (m, 3H), 2.56-2.66 (m, 1H), 2.46 (t, 3H), 1.97-2.04 (m, 2H), 1.58-1.71 (m, 2H), 1.05 (d, 3H).

LC-MS (ESI): m/z = 563.2 $[M+H]^+$.

Step 9: 3-((1R,3R)-1-(2,6-difluoro-4-((1-(3-fluoropropyl)azetidin-3-yl)amino) phenyl)-3-methyl-3,4,6,7,8,10-hexahydrocyclopenta[f]pyrido[3,4-b]indol-2(1H)-yl)-2,2-difluoropropan-1-ol and 3-((1S,3S)-1-(2,6-difluoro-4-((1-(3-fluoropropyl) azetidin-3-yl)amino)phenyl)-3-methyl-3,4,6,7,8,10-hexahydrocyclopenta[f]pyrido [3,4-b]indol-2(1H)-yl)-2,2-difluoropropan-1-ol**(compound 30-1** and **compound 30-2)**

Compound **30K** (0.15 g, 0.27 mmol) was subjected to chiral preparation to obtain the title compound **30-1** (70 mg, retention time 10.0 min) and compound **30-2** (50 mg, retention time 12.0 min), the absolute configurations of the two compounds have not been determined, and NMR and MS are consistent with the racemate.

Preparation method: instrument: waters 2767 (preparative liquid phase chromatographic instrument); chromatographic column: SunFire@ Prep C18 (19 mm × 250 mm); the sample was dissolved in DMF and filtered with a 0.45 μm filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 30%-75%; c. flow rate: 15 mL/min; d. elution time: 20 min; retention time: 10.0 min. instrument: Waters 150 MGM; chromatographic column: Chiralpak Column; mobile phase: A: carbon dioxide, and B: isopropanol; isocratic elution: 50% mobile phase B; flow rate: 100 mL/min; back pressure: 100 bar; column temperature: 35°C; wavelength: 220 nm; elution time: 5 min; retention time: 12.0 min.

**Example 31: 3-((1R,3R)-1-(2,6-difluoro-4-(((S)-1-(3-fluoropropyl) pyrrolidin-3-yl)amino)phenyl)-7-fluoro-3-methyl-1,3,4,9-tetrahydro-2H-pyrido[3,4-b]indol-2-yl)-2,2-difluoropropan-1-ol(compound 31)**

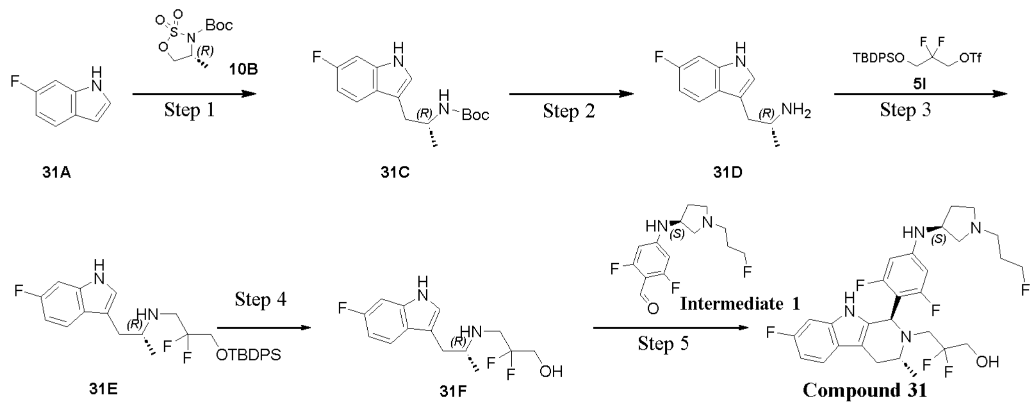

31A 31C 31D 31E 31F Compound 31

Step 1: tert-butyl (R)-(1-(6-fluoro-1H-indol-3-yl)propan-2-yl)carbamate (**31C**)

To a 250 mL three-neck bottle were added **31A** (5.0 g, 37 mmol) and cuprous chloride (2.93 g, 29.6 mmol), same was dissolved in anhydrous dichloromethane (50 mL), cooled to 0°C, methylmagnesium bromide (12.33 mL, 3 M) was added dropwise, the reaction was carried out with the temperature maintained constant for 1 h, cooled to -20°C, a solution of 10B (6.15, 25.9 mmol) in dichloromethane (20 mL) was added dropwise, stirred overnight while maintaining this temperature constant, quenched with 10% citric acid solution (100 mL), stirred for 0.5 h, filtered through celite, and extracted with dichloromethane (2 × 100 mL), the organic phases were combined, dried over anhydrous sodium sulfate, concentrated and purified by column chromatography (petroleum ether : ethyl acetate = 10 : 1-5 : 1) to obtain the title compound **31C** (5.4 g, 46%).
LC-MS (ESI): m/z = 293.2[M+H]$^+$.

Step 2: (R)-1-(6-fluoro-1H-indol-3-yl)propan-2-amine **(31D)**

To a 100 mL three-neck bottle was added **31C** (1 g, 3.42 mmol), same was dissolved in anhydrous methanol (10 mL), hydrogen chloride in dioxane (10 mL) was added, stirred at room temperature for 2 h, and concentrated to obtain the title compound **31D** (1 g, crude).

Step 3: (R)-3-((tert-butyldiphenylsilyl)oxy)-2,2-difluoro-N-(1-(6-fluoro-1H-indol-3-yl)propan-2-yl)propan-1-amine **(31E)**

To a 100 mL three-neck bottle was added **31D** (1 g, 5.2 mmol), same was dissolved in dioxane (40 mL), 5I (3.01 g, 6.24 mmol) and N,N-diisopropyl ethylamine (2.88 g, 22.29 mmol) were added, stirred at 90°C for 12 h, concentrated, and purified by column chromatography (petroleum ether : ethyl acetate = 10 : 1-5 : 1) to obtain the title compound **31E** (0.9 g, 33%).
LC-MS (ESI): m/z = 525.1 [M+H]$^+$.

Step 4: (R)-2,2-difluoro-3-((1-(6-fluoro-1H-indol-3-yl)propan-2-yl)amino) propan-1-ol **(31F)**

To a 50 mL three-neck bottle was added **31E** (0.9 g, 1.72 mmol), same was dissolved in tetrahydrofuran (20 mL), tetrabutylammonium fluoride (5.16 mL, 1 M) was added, stirred at room temperature for 3 h, washed with water (50 mL), extracted with ethyl acetate (2 × 20 mL), the organic phases were combined, dried over anhydrous sodium

sulfate, concentrated, and purified by column chromatography (petroleum ether : ethyl acetate = 2 : 1) to obtain the title compound **31F** (0.4 g, 81%).

LC-MS (ESI): m/z = 287.2 $[M+H]^+$.

Step 5: 3-((1R,3R)-1-(2,6-difluoro-4-(((S)-1-(3-fluoropropyl)pyrrolidin-3-yl)amino)phenyl)-7-fluoro-3-methyl-1,3,4,9-tetrahydro-2H-pyrido[3,4-b]indol-2-yl)-2,2-difluoropropan-1-ol**(compound 31)**

To a 50 mL single-neck bottle were added **31F** (0.1 g, 0.35 mmol) and **intermediate 1** (100 mg, 0.35 mmol), same was dissolved in toluene (8 mL), acetic acid (2 mL) was added, stirred under nitrogen protection at 100°C for 3 h, concentrated, and subjected to preparative HPLC to obtain the title compound **31** (30 mg, 15.5%).

Preparation method: instrument: waters 2767 (preparative liquid phase chromatographic instrument); chromatographic column: SunFire@ Prep C18 (19 mm × 250 mm); the sample was dissolved in methanol and filtered with a 0.45 μm filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 30%-65%; c. flow rate: 15 mL/min; d. elution time: 15 min; retention time: 8.40 min.

$^1H$ NMR (400 MHz, DMSO-d6) δ 10.63 (s, 1H), 7.33-7.37 (m, 1H), 6.95 (dd, 1H), 6.76-6.81 (m, 1H), 6.39 (d, 1H), 6.14 (d, 2H), 5.22 (t, 1H), 5.03 (s, 1H), 4.54 (t, 1H), 4.42 (t, 1H), 3.84 (s, 1H),3.60-3.70 (m, 1H), 3.36 - 3.47 (m, 2H), 3.01- 3.12 (m, 1H), 2.73 -2.82 (m, 2H), 2.54 - 2.67 (m, 3H), 2.31 - 2.47(m, 3H), 2.15 - 2.24 (m, 1H), 1.89 (s, 2H), 1.74-1.87 (m, 2H), 1.06 (d, 3H).

LC-MS (ESI): m/z = 555.1 $[M+H]^+$.

**Example 32: (S)-N-(3,5-difluoro-4-((1R,3R)-7-fluoro-3-methyl-2-(2,2,2-trifluoroethyl)-2,3,4,9-tetrahydro-1H-pyrido[3,4-b]indol-1-yl)phenyl)-1-(3-fluoropropyl)pyrrolidin-3-amine(compound 32)**

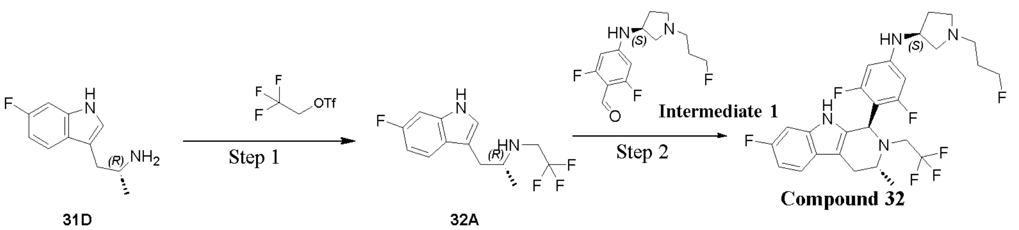


31D 32A Compound 32

Step 1: (R)-1-(6-fluoro-1H-indol-3-yl)-N-(2,2,2-trifluoroethyl)propan-2-amine **(32A)**

To a 100 mL three-neck bottle was added **31D** (0.6 g, 3.1 mmol), same was dissolved in dioxane (20 mL), 2,2,2-trifluoroethyl trifluoromethanesulfonate (1.45 g, 6.2 mmol) and N,N-diisopropyl ethylamine (1.21 g, 9.3 mmol) were added, stirred at 90°C for 12 h, concentrated, and purified by column chromatography (petroleum ether : ethyl acetate = 10 : 1-5 : 1) to obtain the title compound **32A** (0.35 g, 41%).

LC-MS (ESI): m/z = 275.1 $[M+H]^+$.

Step 2: (S)-N-(3,5-difluoro-4-((1R,3R)-7-fluoro-3-methyl-2-(2,2,2-trifluoroethyl)-2,3,4,9-tetrahydro-1H-pyrido[3,4-b]indol-1-yl)phenyl)-1-(3-fluoropropyl)pyrrolidin-3-amine **(compound 32)**

To a 50 mL single-neck bottle were added **32A** (0.15 g, 0.55 mmol) and **intermediate 1** (160 mg, 0.55 mmol), same was dissolved in toluene (8 mL), acetic acid (2 mL) was added, stirred under nitrogen protection at 100°C for 3 h, concentrated, and subjected to preparative HPLC to obtain the title compound **32** (25 mg, 8.4%).

Preparation method: instrument: waters 2767 (preparative liquid phase chromatographic instrument); chromatographic column: SunFire@ Prep C18 (19 mm × 250 mm); the sample was dissolved in methanol and filtered with a 0.45 μm filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 30%-65%; c. flow rate: 15 mL/min; d. elution time: 15 min; retention time: 8.40 min.

$^1H$ NMR (400 MHz, DMSO-d6) δ 10.68 (s, 1H), 7.34-7.38 (m, 1H), 6.96 (dd, 1H), 6.77-6.82 (m, 1H), 6.42 (d, 1H), 6.15 (d, 2H), 5.09 (s, 1H), 4.54 (t, 1H), 4.42 (t, 1H), 3.83 (s, 1H),3.46-3.72 (m, 2H), 2.90 - 3.00 (m, 1H), 2.74 -2.82 (m, 2H), 2.51 -2.68 (m, 2H), 2.38 - 2.48 (m, 3H), 2.33 - 2.36(m, 1H), 2.14 - 2.24(m, 1H), 1.74-1.86 (m, 2H), 1.10 (d, 3H).

LC-MS (ESI): m/z = 543.1 $[M+H]^+$.

**Example 33: 6-((1S,3R)-7-fluoro-3-methyl-2-(2,2,2-trifluoroethyl)-2,3,4,9-tetrahydro-1H-pyrido [3,4-b] indol-1-yl)-N-((S)-1-(3-fluoropropyl)pyrrolidin-3-yl)pyridin-3-amine(compound 33)**

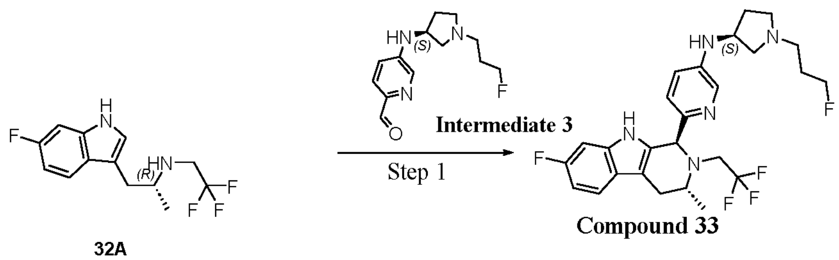


32A

Compound 33

Step 1: 6-((1S,3R)-7-fluoro-3-methyl-2-(2,2,2-trifluoroethyl)-2,3,4,9-tetrahydro-1H-pyrido[3,4-b]indol-1-yl)-N-((S)-1-(3-fluoropropyl)pyrrolidin-3-yl)pyridin-3-amine **(compound 33)**

To a 50 mL single-neck bottle were added **32A** (0.05 g, 0.18 mmol) and **intermediate 3** (45 mg, 0.18 mmol), same was dissolved in toluene (8 mL), acetic acid (2 mL) was added, stirred under nitrogen protection at 100°C for 3 h, concentrated, and subjected to preparative HPLC to obtain the title compound **33** (20 mg, 21.9%).

Preparation method: instrument: waters 2767 (preparative liquid phase chromatographic instrument); chromatographic column: SunFire@ Prep C18 (19 mm × 250 mm); the sample was dissolved in methanol and filtered with a 0.45 μm filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 30%-65%; c. flow rate: 15 mL/min; d. elution time: 15 min; retention time: 8.40 min.

$^1$H NMR (400 MHz, DMSO-d6) δ 10.69 (s, 1H), 7.85 (d, 1H), 7.36-7.39 (m, 1H), 7.08 (d, 1H), 7.02 (dd, 1H), 6.92 (dd, 1H), 6.77-6.82 (m, 1H), 5.96 (d, 1H), 4.89 (s, 1H), 4.54 (t, 1H), 4.42 (t, 1H), 3.85 (s, 1H),3.50-3.58 (m, 1H), 3.25 - 3.29 (m, 1H), 2.96 - 3.00 (m, 1H), 2.78 -2.82 (m, 2H), 2.54 - 2.65 (m, 3H), 2.40 - 2.47(m, 3H), 2.31 - 2.35(m, 1H), 1.73- 1.85 (m, 2H), 1.51 -1.58(m, 1H), 1.13 (d, 3H).

LC-MS (ESI): m/z = 508.2 $[M+H]^+$.

**Example 34: 3-((1R,3R)-1-(2,6-difluoro-4-((1-(3-fluoropropyl)azetidin-3-yl)amino)phenyl)-3-methyl-7-vinyl-1,3,4,9-tetrahydro-2H-pyrido[3,4-b]indol-2-yl)-2,2-difluoropropan-1-ol (compound 34)**

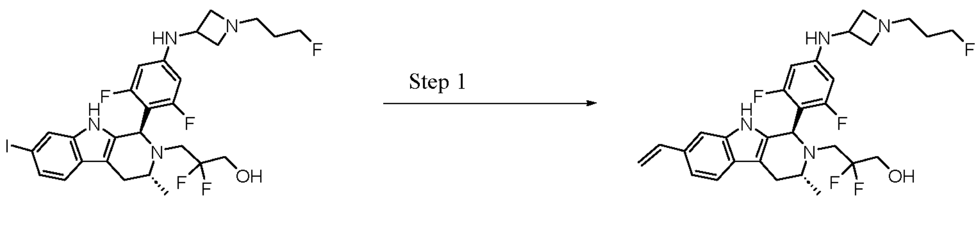


14F

Compound 34

Step 1: 3-((1R,3R)-1-(2,6-difluoro-4-((1-(3-fluoropropyl)azetidin-3-yl)amino)phenyl)-3-methyl-7-vinyl-1,3,4,9-tetrahydro-2H-pyrido[3,4-b]indol-2-yl)-2,2-difluoropropan-1-ol **(compound 34)**

Raw materials **14F** (300 mg, 0.46 mmol), tributyl(vinyl)tin (221 mg, 1.05 mmol), tetraethylammonium fluoride (130 mg, 1.58 mmol), and potassium carbonate (128mg, 1.86 mmol) were added into N,N-dimethylformamide solution (5ml), the reaction system was subjected to nitrogen replacement three times, a catalyst bis(triphenylphosphine)palladium(II) dichloride (30 mg, 0.05 mmol) was then added, and then the mixture was reacted at 100°C for 16 hours. After the reaction was completed, the mixture was poured into water (25 ml), extracted with EA, washed twice with saturated brine, dried over anhydrous sodium sulfate, and filtered, the filtrate was concentrated, and purified and separated by silica gel column chromatography (dichloromethane : methanol (v/v) = 1 : 0-10 : 1) to obtain the title compound **34** (25 mg, yield: 10%).

LCMS m/z = 412.3 [M-136].

$^1$H NMR (400 MHz, DMSO) δ 10.57 (s, 1H), 7.34 (d, *J* = 8.1 Hz, 1H), 7.21 (s, 1H), 7.13 (d, *J* = 8.2 Hz, 1H), 6.81 - 6.64 (m, 2H), 6.10 (d, *J* = 12.0 Hz, 2H), 5.66 (d, *J* = 17.0 Hz, 1H), 5.22 (s, 1H), 5.11 - 5.02 (m, 2H), 4.51 (t, *J* = 6.1 Hz, 1H), 4.39 (t, *J* = 6.1 Hz, 1H), 3.94 (d, *J* = 6.5 Hz, 1H), 3.63 (s, 3H), 3.49 - 3.36 (m, 2H), 3.08 (d, *J* = 14.5 Hz, 1H), 2.84 - 2.72 (m, 3H), 2.63 (d, *J* = 14.5 Hz, 1H), 2.55 (d, *J* = 5.6 Hz, 1H), 2.46 (d, *J* = 7.0 Hz, 2H), 1.65 (dt, *J* = 25.2, 6.4 Hz, 2H), 1.07 (d, *J* = 6.6 Hz, 3H).

**Example 35: 3-((1R,3R)-1-(2,6-difluoro-4-(((S)-1-(3-fluoropropyl) pyrrolidin-3-yl)amino)phenyl)-6,7-difluoro-3-methyl-1,3,4,9-tetrahydro-2H-pyrido[3,4-b]indol-2-yl)-2,2-difluoropropan-1-ol (compound 35)**

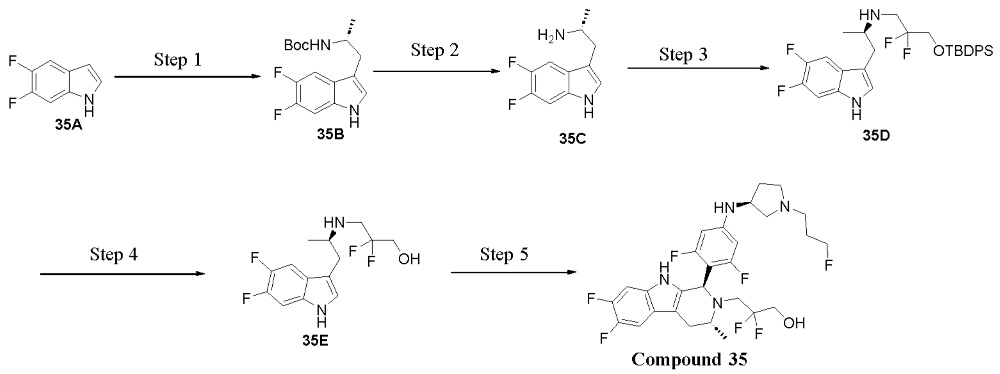


Step 1: tert-butyl (R)-(1-(5,6-difluoro-1H-indol-3-yl)propan-2-yl)carbamate (compound **35B**)

Raw materials 5,6-difluoroindole (2.5 g, 16.34 mmol) and cuprous chloride (2.6 g,10.95 mmol) were added into 50 mL of dichloromethane, placed at 0°C and under nitrogen protection, a solution of methylmagnesium chloride (4.36 mL, 3 mol/l) in THF was added dropwise at this temperature, and then reacted for 1 hour. Tert-butyl (R)-4-methyl-1,2,3-oxathiazolidine-3-carboxylate 2,2-dioxide (1.29 g, 13.07 mmol) was then added at -20°C and reacted for 20 hours. A saturated citric acid solution was added to quench the reaction, then a saturated ammonium chloride solution was added, extracted with DCM, dried over anhydrous sodium sulfate, and filtered, the filtrate was concentrated and then same was purified and separated by silica gel column chromatography (petroleum ether: ethyl acetate (v/v) = 1 : 0-10 : 1) to obtain the title compound **35B** (1.6 g, yield: 31%).

LCMS m/z = 311.1 [M+1].

Step 2: (R)-1-(5,6-difluoro-1H-indol-3-yl)propan-2-amine (compound **35C**)

Raw material **35B** (1.6 g, 5.16 mmol) was added into dichloromethane (5 mL), trifluoroacetic acid (3 mL) was then added, directly concentrated to dryness, a saturated aqueous sodium bicarbonate solution was then added, extracted with EA, dried over anhydrous sodium sulfate, filtered, and the filtrate was concentrated to obtain the title compound **35C** (1 g, yield: 92%).

LCMS m/z = 211.1[M+1].

Step 3: (R)-3-((tert-butyldiphenylsilyl)oxy)-N-(1-(5,6-difluoro-1H-indol-3-yl)propan-2-yl)-2,2-difluoropropan-1-amine (compound **35D**)

Reagent 3-((tert-butyldiphenylsilyl)oxy)-2,2-difluoropropyl trifluoromethanesulfonate (2.76 g, 5.71 mmol), raw materials **35C** (1 g, 4.76 mmol) and DIPEA (1.85 g, 14.28 mmol) were added into 1,4-dioxane solvent (20 mL), heated to 100°C and stirred for 6 hours, cooled, and concentrated, and the crude product was directly mixed with silica gel, and purified and separated by column chromatography (petroleum ether : ethyl acetate (v/v) = 1 : 0-4 : 1) to obtain the title compound **35D** (1.7 g, yield: 66%).

LCMS m/z = 543.2 [M+1].

Step 4: (R)-3-((1-(5,6-difluoro-1H-indol-3-yl)propan-2-yl)amino)-2,2-difluoropropan-1-ol (compound 3**5E**)

Raw material **35D** (1.7 g, 3.13 mmol) was dissolved in THF (20 mL) solvent, TBAF solution (6.26 mL, 6.26

mmol, 1M) was then added, stirred at room temperature for 2 hours, diluted with water, extracted with EA, dried over anhydrous sodium sulfate, and filtered, the filtrate was concentrated and then purified and separated by silica gel column chromatography (petroleum ether : ethyl acetate (v/v) = 1 : 0-1 : 4) to obtain the title compound **35E** (0.85 g, yield: 89%).

LCMS m/z = 305.1 [M+1].

Step 5: 3-((1R,3R)-1-(2,6-difluoro-4-(((S)-1-(3-fluoropropyl)pyrrolidin-3-yl)amino)phenyl)-6,7-difluoro-3-methyl-1,3,4,9-tetrahydro-2H-pyrido[3,4-b]indol-2-yl)-2,2-difluoropropan-1-ol (compound **35**)

Raw materials **35E** (100 mg, 0.33 mmol) and **intermediate 1** (94 mg, 0.33 mmol) were added into a mixed solvent of toluene (6 mL) and acetic acid (1.5 mL), then heated to 95°C and reacted for 4 hour, directly concentrated to dryness, and then purified by TLC (DCM : MeOH = 10 : 1) to obtain the title compound **35** (80 mg, yield: 42%).

LCMS m/z = 573.2 [M+1].

$^1$H NMR (400 MHz, $CD_3OD$) δ7.16-7.20 (m, 1H),7.00-7.04 (m, 1H), 6.14-6.19 (m, 2H), 5.13 (s, 1H), 4.56 (t, 1H), 4.44 (t, 1H), 4.00-4.06 (m, 1H), 3.73-3.83 (m, 1H), 3.58-3.62 (m, 1H), 3.42-3.51 (m, 1H), 3.08-3.18 (m, 2H), 2.99-3.05 (m, 1H), 2.90-2.95 (m, 1H), 2.81-2.89 (m, 3H), 2.70-2.78 (m, 2H), 2.52-2.57 (m, 1H), 2.33-2.42 (m, 1H), 1.98-2.04 (m, 1H),1.90-1.96(m, 1H), 1.76-1.84 (m, 1H), 1.13 (d, 3H).

**Example 36: 6-((1S,3R)-6,7-difluoro-3-methyl-2-(2,2,2-trifluoroethyl)-2,3,4,9-tetrahydro-1H-pyrido[3,4-b]indol-1-yl)-N-((S)-1-(3-fluoropropyl)pyrrolidin-3-yl)pyridin-3-amine (compound 36)**

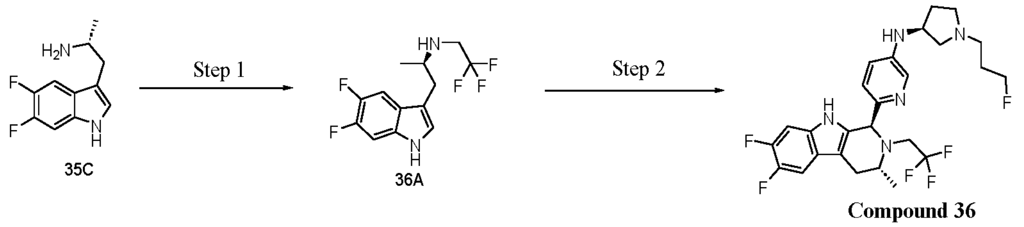


Step 1: (R)-1-(5,6-difluoro-1H-indol-3-yl)-N-(2,2,2-trifluoroethyl)propan-2-amine (compound **36A**)

2,2,2-trifluoroethyl trifluoromethanesulfonate (1.32 g, 5.68 mmol), raw materials **35C** (1 g, 4.72 mmol) and DIPEA (1.8 g, 14.2 mmol) were dissolved in 1,4-dioxane, and stirred and heated at 90°C for 3h. After the reaction was diluted with water, the mixture was extracted with EA, dried over anhydrous sodium sulfate, filtered, the filtrate was concentrated and purified and separated by silica gel column chromatography (petroleum ether : ethyl acetate (v/v) = 1 : 0-1 : 5) to obtain the title compound **36A** (0.83 g, yield: 60%).

LCMS m/z = 293.1 [M+1].

Step 2: 6-((1S,3R)-6,7-difluoro-3-methyl-2-(2,2,2-trifluoroethyl)-2,3,4,9-tetrahydro-1H-pyrido[3,4-b]indol-1-yl)-N-((S)-1-(3-fluoropropyl)pyrrolidin-3-yl)pyridin-3-amine (compound **36**)

Raw materials **36A** (400 mg, 1.36 mmol) and **intermediate 3** benzaldehyde (400 mg, 1.59 mmol) were added into a mixed solvent of toluene (6 mL) and acetic acid (1.5 mL), then heated to 95°C and reacted for 4 hour, directly concentrated to dryness, and then purified by TLC (DCM : MeOH = 10 : 1) to obtain the title compound **36** (420 mg, yield: 42%).

LCMS m/z = 526.2 [M+1].

$^1$H NMR(400 MHz, DMSO)δ10.79-10.77(s,1H), 7.84-7.82(s, 1H), 7.39-7.35(dd,1H), 7.25-7.21(m,1H), 7.10-7.08(d,1H), 6.94-6.91(m,1H),5.97-5.95(d,1H),4.89 (t, 1H),4.56 (t, 1H), 4.44 (t, 1H), 4.00-4.06 (m, 1H), 3.59-3.48 (m, 1H),3.23-3.25(m,1H) 3.04-2.98 (m, 1H), 2.82-2.78 (m, 1H), 2.64-2.56 (m, 2H), 2.52-2.41 (m, 4H), 2.37-2.31 (m, 1H), 2.23-2.16 (m, 1H), 1.86-1.73 (m, 2H), 1.58-1.50 (m, 1H), 1.14-1.12 (d, 3H).

**Example 37: 2,2-difluoro-3-((1S,3R)-6-fluoro-1-(5-(((S)-1-(3-fluoropropyl) pyrrolidin-3-yl)amino)pyridin-2-yl)-3-methyl-1,3,4,9-tetrahydro-2H-pyrido[3,4-b]indol-2-yl)propan-1-ol (compound 37)**

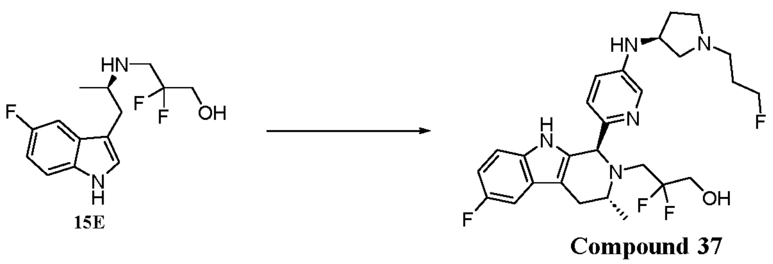

Raw materials **15E** (200 mg, 0.70 mmol) and **intermediate 3** (180 mg, 0.70 mmol) were added into a mixed solvent of toluene (7 mL) and acetic acid (2 mL), then heated to 95°C and reacted for 1 hour, directly concentrated to dryness, then washed with 1M sodium hydroxide solution, concentrated and then purified by TLC (DCM : MeOH = 10 : 1) to obtain the title compound 37 (65 mg, yield: 18%).

LCMS m/z = 520.2 [M+1].

$^1$H NMR (400 MHz, $CD_3OD$) δ7.89 (d, 1H),7.19-7.22 (m, 1H), 7.10-7.13 (m, 1H), 7.06-7.08 (m, 1H), 6.96-6.99 (m, 1H), 6.79-6.85 (m, 1H), 5.00 (s, 1H), 4.56 (t, 1H), 4.44 (t, 1H), 3.98-4.04 (m, 1H), 3.79 (t, 2H), 3.38-3.45 (m, 1H), 3.18-3.29 (m, 1H), 2.94-2.98 (m, 1H), 2.75-2.89 (m, 3H), 2.58-2.70 (m, 4H), 2.51-2.56 (m, 1H), 2.30-2.39 (m, 1H), 1.85-1.98(m, 2H), 1.68-1.76 (m, 1H), 1.19 (d, 3H).

**Example 38: (S)-N-(3,5-difluoro-4-((1R,3R)-6-fluoro-3-methyl-2-(2,2,3-trifluoropropyl)-2,3,4,9-tetrahydro-1H-pyrido[3,4-b]indol-1-yl)phenyl)-1-(3-fluoropropyl)pyrrolidin-3-amine(compound 38)**

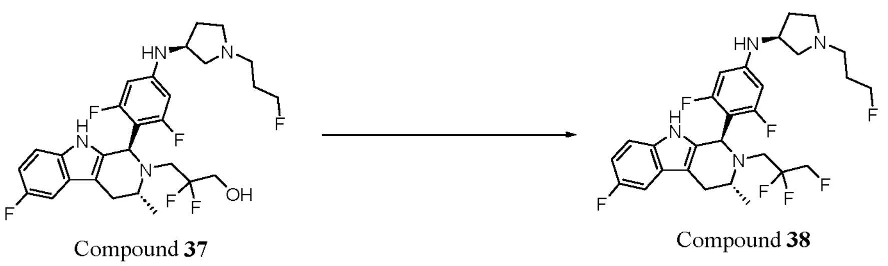

Raw material compound **37** (100 mg, 0.18 mmol) was dissolved in acetonitrile (8 mL) solution, triethylamine (0.15 mL, 1.08 mmol), triethylamine trihydrofluorate (0.059 mL, 0.36 mmol) and nonafluorobutyl sulfonyl fluoride (0.063 mL, 0.36 mmol) were then added, then heated to 50°C and reacted for 10 hours, cooled, then washed with a saturated sodium bicarbonate solution, extracted with EA, dried over anhydrous sodium sulfate, and filtered, the filtrate was concentrated, and then same was purified and separated by TLC (DCM : MeOH = 10 : 1) to obtain the title compound **38** (60 mg, yield: 60%).

LCMS m/z = 557.3 [M+1].

$^1$H NMR (400 MHz, DMSO-$d_6$) δ10.66 (s, 1H), 7.11-7.19 (m, 2H), 6.79-6.84 (m, 1H), 6.42 (d, 1H), 6.16 (d, 2H), 5.06 (s, 1H), 4.45-4.73 (m, 3H), 4.42 (t, 1H), 3.82-3.86 (m, 1H), 3.36-3.41 (m, 1H), 3.11-3.22 (m, 1H), 2.68-2.82 (m, 3H), 2.58-2.64 (m, 1H), 2.52-2.55 (m, 1H), 2.38-2.47 (m, 3H), 2.33-2.36 (m, 1H), 2.15-2.23 (m, 1H), 1.73-1.86 (m, 2H), 1.49-1.55 (m, 1H), 1.08 (d, 3H).

**Example 39: 6-((1S,3R)-6-ethynyl-3-methyl-2-(2,2,2-trifluoroethyl)-2,3,4,9-tetrahydro-1H-pyrido[3,4-b]indol-1-yl)-N-(1-(3-fluoropropyl)azetidin-3-yl)pyridin-3-amine (compound 39)**

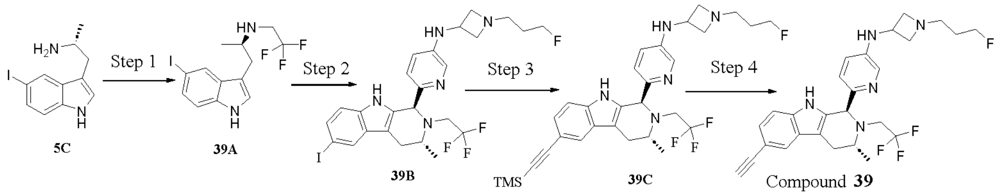


Step 1: (R)-1-(5-iodo-1H-indol-3-yl)-N-(2,2,2-trifluoroethyl)propan-2-amine (compound 3**9A**)

Raw materials **5C** (1.7 g, 5.66 mmol), 2,2,2-trifluoroethyl trifluoromethanesulfonate (1.45 g, 6.25 mmol) and DIPEA (2.19 g, 16.98 mmol) were added into 1,4-dioxane solvent (30 mL), heated to 100°C, stirred for 3 hours, cooled, and directly purified and separated by silica gel column chromatography (petroleum ether : ethyl acetate (v/v) = 1 : 0-5 : 1) to obtain the title compound **39A** (2 g, yield: 92%).

LCMS m/z = 383.1 [M+1].

Step 2: N-(1-(3-fluoropropyl)azetidin-3-yl)-6-((1S,3R)-6-iodo-3-methyl-2-(2,2,2-trifluoroethyl)-2,3,4,9-tetrahydro-1H-pyrido[3,4-b]indol-1-yl)pyridin-3-amine (compound **39B**)

Raw materials **39A** (400 mg, 1.05 mmol) and **intermediate 2** (270 mg, 1.16 mmol) were added into a mixed solvent of toluene (7 mL) and acetic acid (2 mL), then heated to 95°C and reacted for 1 hour, directly concentrated to dryness, then washed with 1M sodium hydroxide solution, concentrated and then purified by TLC (DCM : MeOH = 10 : 1) to obtain the title compound **39B** (500 mg, yield: 79%).

LCMS m/z = 602.1 [M+1].

Step 3: N-(1-(3-fluoropropyl)azetidin-3-yl)-6-((1S,3R)-3-methyl-2-(2,2,2-trifluoroethyl)-6-((trimethylsilyl)ethynyl)-2,3,4,9-tetrahydro-1H-pyrido[3,4-b]indol-1-yl)pyridin-3-amine (compound **39C**)

Raw materials **39B** (530 mg, 0.88 mmol), trimethylethynylsilane (430 mg, 4.4 mmol), triethylamine (180 mg, 1.76 mmol), tetrakis(triphenylphosphine)palladium (100 mg, 0.088 mmol) and cuprous iodide (84 mg, 0.44 mmol) were added into anhydrous DMF (20 mL) solvent, heated under nitrogen protection to 65°C and stirred for 10 hours, cooled, and filtered, the filtrate was concentrated under reduced pressure and then subjected to silica gel column chromatography (dichloromethane : anhydrous methanol (v/v) = 1 : 0-10 : 1) to obtain the title compound **39C** (450 mg, yield: 89%).

LCMS m/z = 572.3 [M+1].

Step 4: 6-((1S,3R)-6-ethynyl-3-methyl-2-(2,2,2-trifluoroethyl)-2,3,4,9-tetrahydro-1H-pyrido[3,4-b]indol-1-yl)-N-(1-(3-fluoropropyl)azetidin-3-yl)pyridin-3-amine (compound **39**)

Raw material **39C** (650 mg, 1.14 mmol) was dissolved in anhydrous methanol (10 mL) solvent, potassium carbonate (470 mg, 3.12 mmol) was added at room temperature, reacted for 3 hours, and filtered, the filtrate was concentrated and then purified and separated by silica gel column chromatography (dichloromethane : anhydrous methanol (v/v) = 1 : 0-10 : 1) to obtain the title compound **39** (100 mg, yield: 17%).

LCMS m/z = 500.2 [M+1].

$^1H$ NMR (400 MHz, DMSO-$d_6$) δ10.84 (s, 1H), 7.80 (d, 1H), 7.55 (s, 1H), 7.25 (d, 1H), 7.08-7.13 (m, 2H), 6.88-6.91 (m, 1H), 6.26 (d, 1H), 4.90 (s, 1H), 4.50 (t, 1H), 4.39 (t, 1H), 3.92-3.98 (m, 1H), 3.85 (s, 1H), 3.61-3.65 (m, 2H), 3.51-3.57 (m, 1H), 3.25-3.29 (m, 1H), 2.98-3.06 (m, 1H), 2.72-2.75 (m, 2H), 2.63-2.68 (m, 1H), 2.53-2.57 (m, 1H), 2.46 (t, 2H), 1.60-1.71 (m, 2H), 1.13 (d, 3H).

**Example 40: 6-((1S,3R)-6-ethynyl-3-methyl-2-(2,2,2-trifluoroethyl)-2,3,4,9-tetrahydro-1H-pyrido [3,4-b] indol-1-yl)-N-((S)-1-(3-fluoropropyl)pyrrolidin-3-yl)pyridin-3-amine (compound 40)**

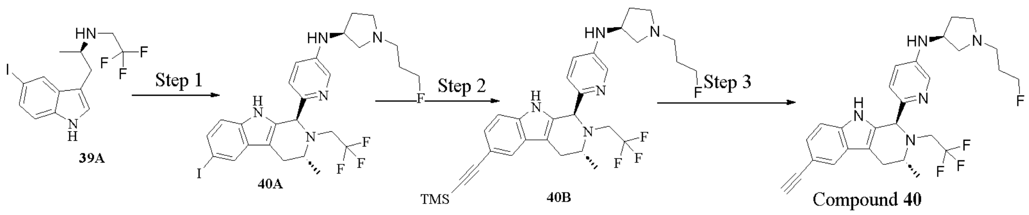


Step 1: N-((S)-1-(3-fluoropropyl)pyrrolidin-3-yl)-6-((1S,3R)-6-iodo-3-methyl-2-(2,2,2-trifluoroethyl)-2,3,4,9-tetrahydro-1H-pyrido[3,4-b]indol-1-yl)pyridin-3-amine (compound **40A**)

Raw materials **39A** (400 mg, 1.05 mmol) and **intermediate 3** (290 mg, 1.16 mmol) were added into a mixed solvent of toluene (7 mL) and acetic acid (2 mL), then heated to 95°C and reacted for 1 hour, directly concentrated to dryness, then washed with 1M sodium hydroxide solution, concentrated and then purified by TLC (DCM : MeOH = 10 : 1) to obtain the title compound **40A** (540 mg, yield: 83%).

LCMS m/z = 616.1 [M+1].

Step 2: N-((S)-1-(3-fluoropropyl)pyrrolidin-3-yl)-6-((1S,3R)-3-methyl-2-(2,2,2-trifluoroethyl)-6-((trimethylsilyl)ethynyl)-2,3,4,9-tetrahydro-1H-pyrido[3,4-b]indol-1-yl)pyridin-3-amine (compound **40B**)

Raw materials **40A** (540 mg, 0.88 mmol), trimethylethynylsilane (430 mg, 4.43 mmol), triethylamine (180 mg, 1.76 mmol), tetrakis(triphenylphosphine)palladium (100 mg, 0.088 mmol) and cuprous iodide (84 mg, 0.44 mmol) were added into anhydrous DMF (20 mL) solvent, heated under nitrogen protection to 65°C and stirred for 10 hours, cooled, and filtered, the filtrate was concentrated under reduced pressure and then subjected to silica gel column chromatography (dichloromethane : anhydrous methanol (v/v) = 1 : 0-10 : 1) to obtain the title compound **40B** (450 mg, yield: 87%).

LCMS m/z = 586.3 [M+1].

Step 3: 6-((1S,3R)-6-ethynyl-3-methyl-2-(2,2,2-trifluoroethyl)-2,3,4,9-tetrahydro-1H-pyrido[3,4-b]indol-1-yl)-N-((S)-1-(3-fluoropropyl)pyrrolidin-3-yl)pyridin-3-amine (compound **40**)

Raw material **40B** (420 mg, 0.72 mmol) was dissolved in anhydrous methanol (10 mL) solvent, potassium carbonate (300 mg, 2.16 mmol) was added at room temperature, reacted for 3 hours, and filtered, the filtrate was concentrated and then purified and separated by silica gel column chromatography (dichloromethane : anhydrous methanol (v/v) = 1 : 0-10 : 1) to obtain the title compound **40** (100 mg, yield: 27%).

LCMS m/z = 514.2 [M+1].

$^1H$ NMR (400 MHz, $CD_3OD$) δ7.86 (d, 1H), 7.61 (s, 1H), 7.16-7.23 (m, 3H), 7.00-7.03 (m, 1H), 4.95 (s, 1H), 4.56 (t, 1H), 4.44 (t, 1H), 3.99-4.05 (m, 1H), 3.35-3.50 (m, 2H), 3.24 (s, 1H), 2.89-3.07 (m,3H), 2.76-2.82 (m, 1H), 2.58-2.68 (m, 4H), 2.51-2.55 (m, 1H), 2.30-2.39 (m, 1H), 1.85-1.98 (m, 2H), 1.68-1.76 (m, 1H), 1.20 (d, 3H).

**Example 41: (S)-N-(4-((1R,3R)-6-ethynyl-3-methyl-2-(2,2,2-trifluoroethyl) -2,3,4,9-tetrahydro-1H-pyrido[3,4-b]indol-1-yl)-3,5-difluorophenyl)-1-(3-fluoropropyl)pyrrolidin-3-amine (compound 41)**

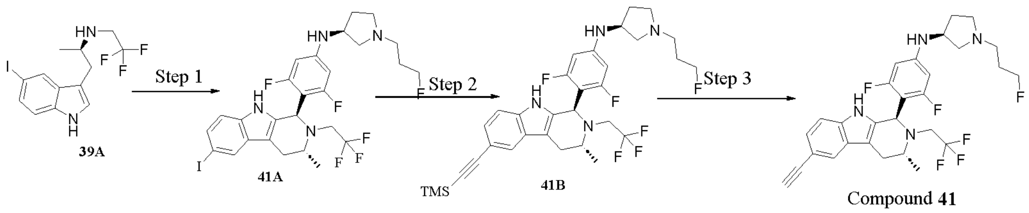


Step 1: (S)-N-(3,5-difluoro-4-((1R,3R)-6-iodo-3-methyl-2-(2,2,2-trifluoroethyl)-2,3,4,9-tetrahydro-1H-pyrido[3,4-b]indol-1-yl)phenyl)-1-(3-fluoropropyl)pyrrolidin-3-amine (compound **41A**)

Raw materials **39A** (400 mg, 1.05 mmol) and **intermediate 1** (330 mg, 1.16 mmol) were added into a mixed solvent of toluene (7 mL) and acetic acid (2 mL), then heated to 95°C and reacted for 1 hour, directly concentrated to dryness, then washed with 1M sodium hydroxide solution, concentrated and then purified by TLC (DCM : MeOH = 10:

1) to obtain the title compound **41A** (550 mg, yield: 81%).

LCMS m/z = 651.1 [M+1].

Step 2: (S)-N-(3,5-difluoro-4-((1R,3R)-3-methyl-2-(2,2,2-trifluoroethyl)-6-((trimethylsilyl)ethynyl)-2,3,4,9-tetrahydro-1H-pyrido[3,4-b]indol-1-yl)phenyl)-1-(3-fluoropropyl)pyrrolidin-3-amine (compound **41B**)

Raw materials **41A** (550 mg, 0.85 mmol), trimethylethynylsilane (420 mg, 4.25 mmol), triethylamine (180 mg, 1.76 mmol), tetrakis(triphenylphosphine)palladium (100 mg, 0.088 mmol) and cuprous iodide (84 mg, 0.44 mmol) were added into anhydrous DMF (20 mL) solvent, heated under nitrogen protection to 65°C and stirred for 10 hours, cooled, and filtered, the filtrate was concentrated under reduced pressure and then subjected to silica gel column chromatography (dichloromethane : anhydrous methanol (v/v) = 1 : 0-10 : 1) to obtain the title compound **41B** (400 mg, yield: 76%).

LCMS m/z = 621.3 [M+1].

Step 3: (S)-N-(4-((1R,3R)-6-ethynyl-3-methyl-2-(2,2,2-trifluoroethyl)-2,3,4,9-tetrahydro-1H-pyrido[3,4-b]indol-1-yl)-3,5-difluorophenyl)-1-(3-fluoropropyl)pyrrolidin-3-amine (compound **41**)

Raw material **41B** (450 mg, 0.72 mmol) was dissolved in anhydrous methanol (10 mL) solvent, potassium carbonate (300 mg, 2.16 mmol) was added at room temperature, reacted for 3 hours, and filtered, the filtrate was concentrated and then purified and separated by silica gel column chromatography (dichloromethane : anhydrous methanol (v/v) = 1 : 0-10 : 1) to obtain the title compound **41** (100 mg, yield: 25%).

LCMS m/z = 549.2 [M+1].

$^1$H NMR (400 MHz, DMSO-$d_6$) δ10.83 (s, 1H), 7.54 (s, 1H), 7.18-7.20 (m, 1H), 7.09-7.11 (m, 1H), 6.42 (d, 1H), 6.16 (d, 1H), 5.11 (s, 1H), 4.54 (t, 1H), 4.42 (t, 1H), 3.77-3.93 (m, 2H), 3.38-3.46 (m, 2H), 2.90-3.01 (m, 1H), 2.74-2.84 (m, 2H), 2.55-2.64 (m, 3H), 2.38-2.47 (m, 2H), 2.33-2.36 (m, 1H), 2.15-2.24 (m, 1H), 1.75-1.86 (m, 2H), 1.49-1.57 (m, 1H), 1.11 (d, 3H).

**Example 42: 6-((1S,3R)-7-ethynyl-3-methyl-2-(2,2,2-trifluoroethyl)-2,3,4,9-tetrahydro-1H-pyrido [3,4-b] indol-1-yl)-N-(1-(3-fluoropropyl)azetidin-3-yl)pyridin-3-amine (compound 42)**

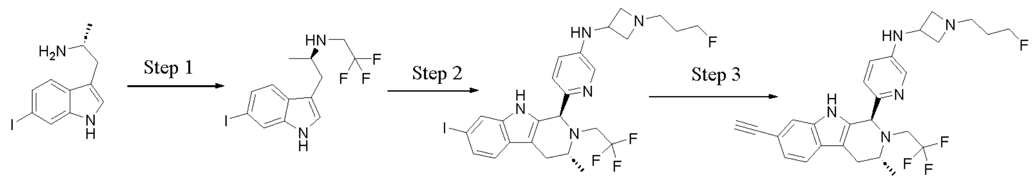


14C 42A 42B Compound 42

Step 1: (R)-1-(6-iodo-1H-indol-3-yl)-N-(2,2,2-trifluoroethyl)propan-2-amine (compound **42**)

Reagent **14C** (1.7 g, 6.67 mmol), raw material 2,2,2-trifluoroethyl trifluoromethanesulfonate (1.55 g, 6.67 mmol) and DIPEA (1.73 g, 13.34 mmol) were added into 1,4-dioxane solvent (35 mL), heated to 100°C, stirred for 2 hours, cooled, and directly purified and separated by silica gel column chromatography (petroleum ether : ethyl acetate (v/v) = 1 : 0-5 : 1) to obtain the title compound **42A** (2 g, yield: 79%).

LCMS m/z = 383.1 [M+1].

Step 2: N-((S)-1-(3-fluoropropyl)pyrrolidin-3-yl)-6-((1S,3R)-7-iodo-3-methyl-2-(2,2,2-trifluoroethyl)-2,3,4,9-tetrahydro-1H-pyrido[3,4-b]indol-1-yl)pyridin-3-amine (compound **42B**)

Raw materials **42A** (400 mg, 1.05 mmol) and **intermediate 2** (270 mg, 1.16 mmol) were added into a mixed solvent of toluene (8 mL) and acetic acid (2 mL), then heated to 95°C and reacted for 3 hour, directly concentrated to dryness, then washed with 1M sodium hydroxide solution, concentrated and then purified by TLC (DCM : MeOH = 10 : 1) to obtain the title compound **42B** (530 mg, yield: 79%).

LCMS m/z = 602.1 [M+1].

Step 3: 6-((1S,3R)-7-ethynyl-3-methyl-2-(2,2,2-trifluoroethyl)-2,3,4,9-tetrahydro-1H-pyrido[3,4-b]indol-1-yl)-N-(1-(3-fluoropropyl)azetidin-3-yl)pyridin-3-amine (**compound 42**)

Raw materials **42B** (530 mg, 0.88 mmol), trimethylethynylsilane (430 mg, 4.4 mmol), triethylamine (180 mg, 1.76 mmol), tetrakis(triphenylphosphine)palladium (100 mg, 0.088 mmol) and cuprous iodide (84 mg, 0.44 mmol) were added into anhydrous DMF (10 mL) solvent, heated under nitrogen protection to 75°C and stirred for 16 hours, cooled, and filtered, the filtrate was concentrated under reduced pressure and then subjected to silica gel column chromatography (dichloromethane : anhydrous methanol (v/v) = 1 : 0-10 : 1) to obtainthe product, an intermediate. The intermediate was dissolved in methanol (10Ml), potassium carbonate (1 g) was added and stirred for 3 hours, filtered, spun to dryness, and purified and separated by silica gel column chromatography (dichloromethane : anhydrous methanol (v/v) = 1 : 0-10 : 1) to obtain compound **42** (100 mg, yield: 23%).

LCMS m/z = 500.2 [M+1].

$^1$H NMR (400 MHz, DMSO) δ 10.79 (s, 1H), 7.80 (d, *J* = 2.7 Hz, 1H), 7.39 (d, *J* = 7.6 Hz, 2H), 7.07 (d, *J* = 15.0 Hz, 2H), 6.90 (dd, *J* = 8.5, 2.8 Hz, 1H), 6.27 (d, *J* = 6.9 Hz, 1H), 4.92 (s, 1H), 4.51 (s, 1H), 4.39 (s, 1H), 3.92 (s, 2H), 3.65 (d, *J* = 6.4 Hz, 2H), 3.54 (dd, *J* = 15.8, 10.1 Hz, 1H), 3.02 (dd, *J* = 15.8, 9.7 Hz, 1H), 2.76 (s, 2H), 2.65 (dd, *J* = 15.8, 4.1 Hz, 1H), 2.55 (dd, *J* = 13.6, 7.4 Hz, 1H), 2.48 (s, 2H), 1.91 (s, 2H), 1.72 - 1.56 (m, 2H), 1.13 (d, *J* = 6.7 Hz, 3H).

**Example 43: (S)-N-(4-((1R,3R)-7-ethynyl-3-methyl-2-(2,2,2-trifluoroethyl) -2,3,4,9-tetrahydro-1H-pyrido[3,4-b]indol-1-yl)-3,5-difluorophenyl)-1-(3-fluoropropyl)pyrrolidin-3-amine (compound 43)**

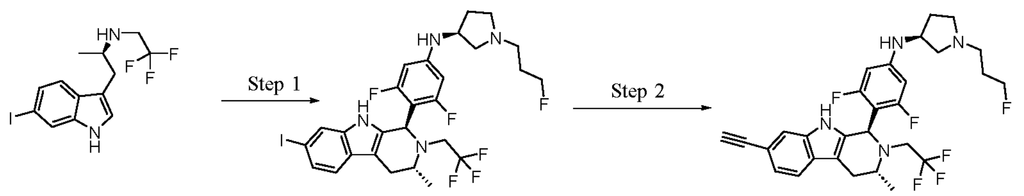


42A 43A Compound 43

Step 1: (S)-N-(3,5-difluoro-4-((1R,3R)-7-iodo-3-methyl-2-(2,2,2-trifluoroethyl)-2,3,4,9-tetrahydro-1H-pyrido[3,4-b]indol-1-yl)phenyl)-1-(3-fluoropropyl)pyrrolidin-3-amine (compound **43A**)

Raw materials **42A** (400 mg, 1.05 mmol) and **intermediate 1** (330 mg, 1.16 mmol) were added into a mixed solvent of toluene (8 mL) and acetic acid (2 mL), then heated to 95°C and reacted for 3 hour, directly concentrated to dryness, then washed with 1M sodium hydroxide solution, concentrated and then purified by TLC (DCM : MeOH = 10 : 1) to obtain the title compound **43A** (550 mg, yield: 81%).

LCMS m/z = 651.1 [M+1].

Step 2: (S)-N-(4-((1R,3R)-7-ethynyl-3-methyl-2-(2,2,2-trifluoroethyl)-2,3,4,9-tetrahydro-1H-pyrido[3,4-b]indol-1-yl)-3,5-difluorophenyl)-1-(3-fluoropropyl) pyrrolidin-3-amine (compound **43**)

**Raw materials 43A** (550 mg, 0.85 mmol), trimethylethynylsilane (420 mg, 4.25 mmol), triethylamine (180 mg, 1.76 mmol), tetrakis(triphenylphosphine)palladium (100 mg, 0.088 mmol) and cuprous iodide (84 mg, 0.44 mmol) were added into anhydrous DMF (10 mL) solvent, heated under nitrogen protection to 75°C and stirred for 16 hours, cooled, and filtered, the filtrate was concentrated under reduced pressure and then subjected to silica gel column chromatography (dichloromethane : anhydrous methanol (v/v) = 1 : 0-10 : 1) to obtain the product, an intermediate. The intermediate was dissolved in methanol (10 mL), potassium carbonate (1 g) was added and stirred for 3 hours, filtered, spun to dryness, and purified and separated by silica gel column chromatography (dichloromethane : anhydrous methanol (v/v) = 1 : 0-10 : 1) to obtain the title compound **43** (100 mg, yield: 21%).

LCMS m/z = 549.2 [M+1].

$^1$H NMR (400 MHz, DMSO) δ 10.78 (s, 1H), 7.38 (d, *J* = 8.1 Hz, 1H), 7.32 (s, 1H), 7.04 (dd, *J* = 8.1, 1.3 Hz, 1H), 6.43 (d, *J* = 6.7 Hz, 1H), 6.16 (d, *J* = 12.3 Hz, 2H), 5.12 (s, 1H), 4.54 (t, *J* = 6.0 Hz, 1H), 4.42 (t, *J* = 6.0 Hz, 1H), 3.91 (s, 1H), 3.85 (s, 1H), 3.52 - 3.35 (m, 2H), 3.04 - 2.88 (m, 1H), 2.86 - 2.72 (m, 2H), 2.60 (t, *J* = 6.4 Hz, 2H), 2.48 - 2.38 (m, 3H), 2.34 (dd, *J*= 9.4, 4.2 Hz, 1H), 2.19 (m, 1H), 1.80 (m, 2H), 1.59 - 1.45 (m, 1H), 1.10 (d, *J* = 6.6 Hz, 3H).

**Example 44: 6-((1S,3R)-7-ethynyl-3-methyl-2-(2,2,2-trifluoroethyl)-2,3,4,9-tetrahydro-1H-pyrido [3,4-b] indol-1-yl)-N-((S)-1-(3-fluoropropyl)pyrrolidin-3-yl)pyridin-3-amine (compound 44)**

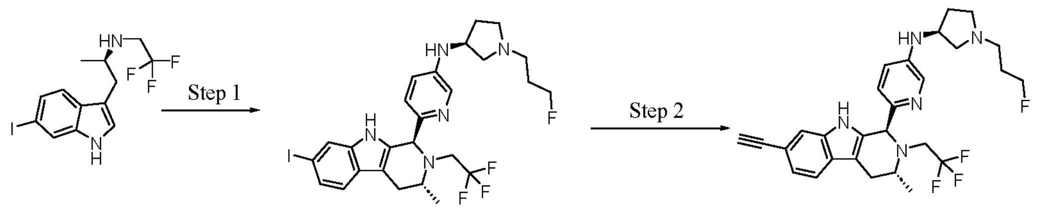


**42A** **44A** Compound 44

Step 1: N-((S)-1-(3-fluoropropyl)pyrrolidin-3-yl)-6-((1S,3R)-7-iodo-3-methyl-2-(2,2,2-trifluoroethyl)-2,3,4,9-tetrahydro-1H-pyrido[3,4-b]indol-1-yl)pyridin-3-amine (compound **44A**)

Raw materials **42A** (400 mg, 1.05 mmol) and **intermediate 3** (290 mg, 1.16 mmol) were added into a mixed solvent of toluene (8 mL) and acetic acid (2 mL), then heated to 95°C and reacted for 3 hour, directly concentrated to dryness, then washed with 1M sodium hydroxide solution, concentrated and then purified by TLC (DCM : MeOH = 10 : 1) to obtain the title compound **44A** (600 mg, yield: 93%).
LCMS m/z = 616.1 [M+1].

Step 2: 6-((1S,3R)-7-ethynyl-3-methyl-2-(2,2,2-trifluoroethyl)-2,3,4,9-tetrahydro-1H-pyrido[3,4-b]indol-1-yl)-N-((S)-1-(3-fluoropropyl)pyrrolidin-3-yl)pyridin-3-amine (compound **44**)

Raw materials **44A** (540 mg, 0.88 mmol), trimethylethynylsilane (430 mg, 4.43 mmol), triethylamine (180 mg, 1.76 mmol), tetrakis(triphenylphosphine)palladium (100 mg, 0.088 mmol) and cuprous iodide (84 mg, 0.44 mmol) were added into anhydrous DMF (10 mL) solvent, heated under nitrogen protection to 75°C and stirred for 16 hours, cooled, and filtered, the filtrate was concentrated under reduced pressure and then subjected to silica gel column chromatography (dichloromethane: anhydrous methanol (v/v) = 1 : 0-10 : 1) to obtain the product, an intermediate. The intermediate was dissolved in methanol (10 mL), potassium carbonate (1 g) was added and stirred for 3 hours, filtered, spun to dryness, and purified and separated by silica gel column chromatography (dichloromethane : anhydrous methanol (v/v) = 1 : 0-10 : 1) to obtain the title compound **44** (150 mg, yield: 32%).
LCMS m/z = 514.2 [M+1].
$^1$H NMR (400 MHz, DMSO) $\delta$ 10.79 (s, 1H), 7.84 (d, *J* = 2.7 Hz, 1H), 7.39 (d, *J* = 6.9 Hz, 2H), 7.10 - 7.02 (m, 2H), 6.93 (m, 1H), 5.96 (d, *J* = 6.8 Hz, 1H), 4.92 (s, 1H), 4.53 (t, *J* = 6.0 Hz, 1H), 4.42 (t, *J* = 6.0 Hz, 1H), 3.92 (s, 1H), 3.86 (s, 1H), 3.53 (m, 1H), 3.30 (s, 1H), 3.09 - 2.95 (m, 1H), 2.80 (dd, *J* = 9.2, 7.0 Hz, 1H), 2.70 - 2.53 (m, 3H), 2.49 - 2.40 (m, 3H), 2.33 (dd, *J* = 9.2, 4.6 Hz, 1H), 2.19 (m, 1H), 1.87 - 1.71 (m, 2H), 1.54 (m, 1H), 1.14 (d, *J* = 6.7 Hz, 3H).

**Example 45: (S)-N-(3,5-difluoro-4-((1R,3R)-7-fluoro-3-methyl-2-(2,2,3-trifluoropropyl)-2,3,4,9-tetrahydro-1H-pyrido[3,4-b]indol-1-yl)phenyl)-1-(3-fluoropropyl)pyrrolidin-3-amine (compound 45)**

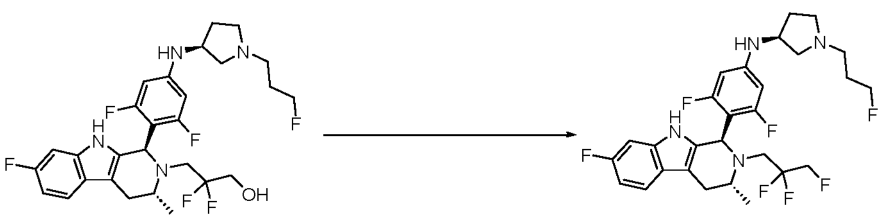

Compound **29** Compound **45**

Raw material compound **29** (300 mg, 0.54 mmol) was dissolved in acetonitrile (10 mL) solution, triethylamine (0.45 mL, 3.24 mmol), triethylamine trihydrofluorate (0.18 mL, 1.08 mmol) and nonafluorobutyl sulfonyl fluoride (0.18 mL, 1.08 mmol) were then added, then heated to 50°C and reacted for 10 hours, cooled, then washed with a saturated

sodium bicarbonate solution, extracted with EA, dried over anhydrous sodium sulfate, and filtered, the filtrate was concentrated, and then same was subjected to preparative HPLC to obtain the title **compound** 45 (80 mg, yield: 26.7%).

Preparation method: instrument: waters 2767 (preparative liquid phase chromatographic instrument); chromatographic column: SunFire@ Prep C18 (19 mm × 250 mm); the sample was dissolved in methanol and filtered with a 0.45 μm filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 30%-65%; c. flow rate: 15 mL/min; d. elution time: 15 min; retention time: 8.40 min.

LCMS m/z = 557.3 [M+1].

1H NMR (400 MHz, DMSO-d6) δ10.67 (s, 1H), 7.34-7.38 (m, 1H), 6.95-6.98 (m, 1H), 6.77-6.82 (m, 1H), 6.42 (d, 1H), 6.16 (d, 2H), 5.04 (s, 1H), 4.15-4.77 (m, 3H), 4.42 (t, 1H), 3.83 (s, 1H), 3.35-3.41 (m, 1H), 3.11-3.22 (m, 1H), 2.72-2.84 (m, 3H), 2.58-2.64 (m, 1H), 2.52-2.55 (m, 1H), 2.32-2.48 (m, 3H), 2.15-2.24 (m, 1H), 1.74-1.86 (m, 2H), 1.49-1.57 (m, 1H), 1.07 (d, 3H).

**Example 46: N-(1-(3-fluoropropyl)azetidin-3-yl)-6-((1S,3R)-3-methyl-2-(2,2,2-trifluoroethyl)-2,3,4,6,7,9-hexahydro-1H-cyclobuta[f]pyrido[3,4-b]indol-1-yl)pyridin-3-amine and N-(1-(3-fluoropropyl)azetidin-3-yl)-6-((1R,3S)-3-methyl-2-(2,2,2-trifluoroethyl)-2,3,4,6,7,9-hexahydro-1H-cyclobuta[f]pyrido[3,4-b]indol-1-yl)pyridin-3-amine (compound 46-1 and compound 46-2)**

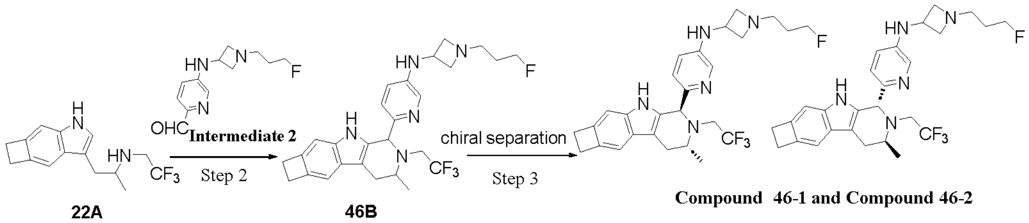


22A 46B Compound 46-1 and Compound 46-2

Step 1: N-(1-(3-fluoropropyl)azetidin-3-yl)-6-(3-methyl-2-(2,2,2-trifluoroethyl)-2,3,4,6,7,9-hexahydro-1H-cyclobuta[f]pyrido[3,4-b]indol-1-yl)pyridin-3-amine **(46B)**

To a 100 mL round bottom flask was added **22A** (0.21 g, 0.74 mmol), and same was dissolved in dioxane (10 mL), **intermediate 2** (0.35 g, 1.48 mmol) and trifluoroacetic acid (84 mg, 0.74 mmol) were added, stirred at 70°C for 2 h, concentrated, and separated by preparative HPLC to obtain the title compound **46B** (0.16 g, 43%).

Preparation method: instrument: waters 2767 (preparative liquid phase chromatographic instrument); chromatographic column: SunFire@ Prep C18 (19 mm × 250 mm); The sample was dissolved in DMF and filtered with a 0.45 μm filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 40%-70%; c. flow rate: 15 mL/min; d. elution time: 20 min; retention time: 10.5 min.

$^1$H NMR (400 MHz, DMSO-d6) δ7.79 (d, 1H), 7.16 (d, 1H), 7.06 (d, 1H), 6.94 - 6.89 (m, 2H), 4.89 (s, 1H), 4.51 (t, 1H), 4.39 (t, 1H), 4.10-4.07 (m, 1H), 3.82 - 3.77 (m, 2H), 3.47 - 3.33 (m, 2H), 3.14-3.12 (m, 5H), 3.02-2.96 (m, 3H), 2.86 (dd, 1H), 2.64 (t, 2H), 2.62 - 2.52 (m, 2H), 1.82 - 1.66 (m, 2H), 1.16 (d, 3H).

LC-MS (ESI): m/z = 502.3 $[M+H]^+$.

Step 2: N-(1-(3-fluoropropyl)azetidin-3-yl)-6-((1S,3R)-3-methyl-2-(2,2,2-trifluoroethyl)-2,3,4,6,7,9-hexahydro-1H-cyclobuta[f]pyrido[3,4-b]indol-1-yl)pyridin-3 -amine and N-(1-(3 -fluoropropyl)azetidin-3-yl)-6-((1R,3S)-3-methyl-2-(2,2,2-trifluoroethyl)-2,3,4,6,7,9-hexahydro-1H-cyclobuta[f]pyrido[3,4-b]indol-1-yl)pyridin-3-amine **(compound 46-1** and **compound 46-2)**

Compound **46B** (0.16 g, 0.32 mmol) was subjected to chiral preparation to obtain the title compound **46-1** (90 mg, retention time 1.53 min) and compound **46-2** (60 mg, retention time 2.04 min), the absolute configurations of the two compounds have not been determined, and NMR and MS are consistent with the racemate.

instrument: Waters 150 MGM; chromatographic column: Chiralpak Column; mobile phase: A: carbon dioxide, and B: ethanol (0.1% aqueous ammonia); isocratic elution: 40% mobile phase B; flow rate: 100 mL/min; back pressure: 100 bar; column temperature: 35°C; wavelength: 220 nm; elution time: 6.0 min.

**Example 47: N-((S)-1-(3-fluoropropyl)pyrrolidin-3-yl)-6-((1S,3R)-3-methyl-2-(2,2,2-trifluoroethyl)-2,3,4,6,7,9-hexahydro-1H-cyclobuta[f] pyrido [3,4-b] indol-1-yl)pyridin-3-amine and N-((S)-1-(3-fluoropropyl)pyrrolidin-3-yl)-6-((1R,3S)-3-methyl-2-(2,2,2-trifluoroethyl)-2,3,4,6,7,9-hexahydro-1H-cyclobuta[f]pyrido[3,4-b]indol-1-yl)pyridin-3-amine(compound 47-1 and compound 47-2)**

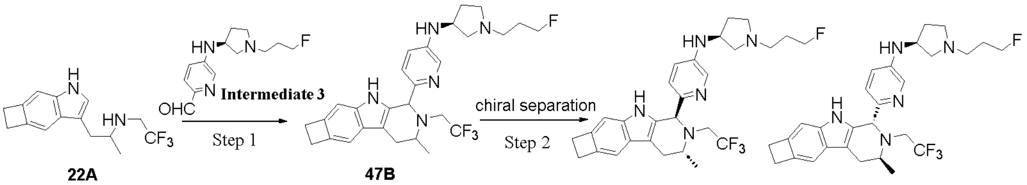


22A 47B

Compound 47-1 and Compound 47-2

Step 1: N-((S)-1-(3-fluoropropyl)pyrrolidin-3-yl)-6-(3-methyl-2-(2,2,2-trifluoroethyl)-2,3,4,6,7,9-hexahydro-1H-cyclobuta[f]pyrido[3,4-b]indol-1-yl)pyridin-3-amine **(47B)**

To a 100 mL round bottom flask was added **22A** (0.20 g, 0.71 mmol), and same was dissolved in dioxane (10 mL), **intermediate 3** (0.18 g, 0.71 mmol) and trifluoroacetic acid (81 mg, 0.71 mmol) were added, stirred at 70°C for 3 h, concentrated, and separated by preparative HPLC to obtain the title compound **47B** (0.13 g, 36%).

Preparation method: instrument: waters 2767 (preparative liquid phase chromatographic instrument); chromatographic column: SunFire@ Prep C18 (19 mm × 250 mm); the sample was dissolved in DMF and filtered with a 0.45 μm filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 40%-70%; c. flow rate: 15 mL/min; d. elution time: 20 min; retention time: 11.2 min.

$^1$H NMR (400 MHz, DMSO-d6) $\delta$ 10.39 (s, 1H), 7.85 (d, 1H), 7.07 (d, 1H), 7.01 (d, 1H), 6.96 (d, 1H), 6.90 (dd, 1H), 5.94 (d, 1H), 4.86 (s, 1H), 4.53 (t, 1H), 4.42 (t, 1H), 3.86 (s, 1H), 3.56-3.49 (m, 1H), 3.11 (s, 4H), 3.02-2.96 (m, 1H), 2.82-2.78 (m, 1H), 2.70 - 2.53 (m, 3H), 2.49-2.46 (m, 4H), 2.35-2.32 (m, 1H), 2.21-2.16 (m, 1H), 1.84-1.75 (m, 2H), 1.57-1.52 (m, 1H), 1.12 (d, 3H).

LC-MS (ESI): m/z = 516.3 $[M+H]^+$.

Step 2: N-((S)-1-(3-fluoropropyl)pyrrolidin-3-yl)-6-((1S,3R)-3-methyl-2-(2,2,2-trifluoroethyl)-2,3,4,6,7,9-hexahydro-1H-cyclobuta[f]pyrido[3,4-b]indol-1-yl)pyridin-3-amine and N-((S)-1-(3-fluoropropyl)pyrrolidin-3-yl)-6-((1R,3S)-3-methyl-2-(2,2,2-trifluoroethyl)-2,3,4,6,7,9-hexahydro-1H-cyclobuta[f]pyrido[3,4-b]indol-1-yl)pyridin-3-amine(**compound 47-1** and **compound 47-2**)

Compound **47B** (0.13 g, 0.25 mmol) was subjected to chiral preparation to obtain the title compound **47-1** (56 mg) and compound **47-2** (48 mg).
instrument: Waters 150 MGM; chromatographic column: Chiralpak Column; mobile phase: A: carbon dioxide, and B: ethanol (0.1% aqueous ammonia); isocratic elution: 35% mobile phase B; flow rate: 100 mL/min; back pressure: 100 bar; column temperature: 35°C; wavelength: 220 nm; elution time: 3.0 min.

Compound **47-1,** , retention time: 1.55 min, $^1$H NMR (400 MHz, DMSO-d6) $\delta$ 10.39 (s, 1H), 7.85 (d, 1H), 7.07 (d, 1H), 7.01 (d, 1H), 6.96 (d, 1H), 6.90 (dd, 1H), 5.94 (d, 1H), 4.86 (s, 1H), 4.53 (t, 1H), 4.42 (t, 1H), 3.86 (s, 1H), 3.56-3.50 (m, 1H), 3.11 (s, 4H), 3.04 - 2.92 (m, 1H), 2.80 (t, 1H), 2.70 - 2.53 (m, 3H), 2.50-2.46 (m, 4H), 2.35-2.32 (m, 1H), 2.23-2.14 (m, 1H), 1.86-1.73 (m, 2H), 1.57-1.52 (m, 1H), 1.12 (d, 3H).

Compound **47-2,** retention time: 2.16 min, $^1$H NMR (400 MHz, DMSO-d6) $\delta$ 10.39 (s, 1H), 7.85 (d, 1H), 7.07 (d, 1H), 7.01 (d, 1H), 6.97 (d, 1H), 6.90 (dd, 1H), 5.94 (d, 1H), 4.86 (s, 1H), 4.54 (t, 1H), 4.42 (t, 1H), 3.88-3.83 (m, 1H), 3.62 - 3.43 (m, 1H), 3.11 (s, 4H), 3.02-2.93 (m, 1H), 2.80 (dd, 1H), 2.68 - 2.51 (m, 3H), 2.52 - 2.38 (m, 4H), 2.35-2.31 (m, 1H), 2.23-2.14 (m, 1H), 1.89 - 1.70 (m, 2H), 1.60-1.48 (m, 1H), 1.12 (d, 3H).

LC-MS (ESI): m/z = 516.3 $[M+H]^+$.

**Example 48: (S)-N-(3,5-difluoro-4-((1R,3R)-3-methyl-2-(2,2,2-trifluoroethyl)-2,3,4,6,7,9-hexahydro-1H-cyclobuta[f]pyrido[3,4-b]indol-1-yl)phenyl)-1-(3-fluoropropyl)pyrrolidin-3-amine and (S)-N-(3,5-difluoro-4-((1S,3S)-3-methyl-2-(2,2,2-trifluoroethyl)-2,3,4,6,7,9-hexahydro-1H-cyclobuta[f]pyrido[3,4-b]indol-1-yl)phenyl)-1-(3-fluoropropyl)pyrrolidin-3-amine (compound 48-1 and compound 48-2)**

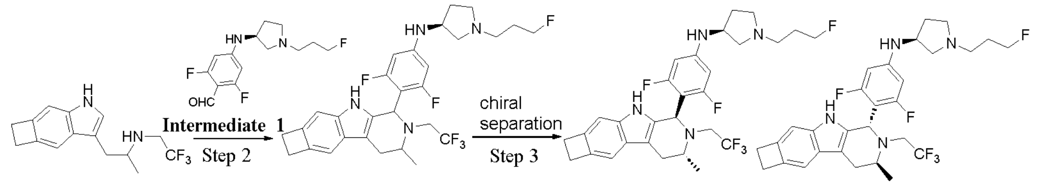


22A 48B Compound 48-1 and Compound 48-2

Step 1: (3S)-N-(3,5-difluoro-4-(3-methyl-2-(2,2,2-trifluoroethyl)-2,3,4,6,7,9-hexahydro-1H-cyclobuta[f]pyrido[3,4-b]indol-1-yl)phenyl)-1-(3-fluoropropyl)pyrrolidin-3-amine **(48B)**

To a 100 mL round bottom flask was added **22A** (0.20 g, 0.71 mmol), and same was dissolved in dioxane (10 mL), **intermediate 1** (0.20 g, 0.71 mmol) and trifluoroacetic acid (81 mg, 0.71 mmol) were added, stirred at 70°C for 3 h, concentrated, and separated by preparative HPLC to obtain the title compound **48B** (0.12 g, 31%).

Preparation method: instrument: waters 2767 (preparative liquid phase chromatographic instrument); chromatographic column: SunFire@ Prep C18 (19 mm × 250 mm); the sample was dissolved in DMF and filtered with a 0.45 μm filter to prepare a sample solution. Preparative chromatography conditions: a. composition of mobile phases A and B: mobile phase A: acetonitrile, and mobile phase B: water (containing 5 mM ammonium acetate); b. gradient elution: mobile phase A: 40%-70%; c. flow rate: 15 mL/min; d. elution time: 20 min; retention time: 10.8 min.

$^1$H NMR (400 MHz, DMSO-d6) $\delta$ 10.37 (s, 1H), 7.05 (d, 1H), 6.91 (d, 1H), 6.38 (d, 1H), 6.13 (d, 2H), 5.08 (s, 1H), 4.54 (t, 1H), 4.42 (t, 1H), 3.85-3.83 (m, 2H), 3.10 (s, 4H), 3.00 - 2.87 (m, 1H), 2.84 - 2.71 (m, 2H), 2.67 - 2.54 (m, 3H), 2.47 - 2.30 (m, 4H), 2.23 - 2.15 (m, 1H), 1.91 - 1.73 (m, 2H), 1.55-1.51 (m, 1H), 1.09 (d, 3H).

LC-MS (ESI): m/z = 551.4 $[M+H]^+$.

Step 2: (S)-N-(3,5-difluoro-4-((1R,3R)-3-methyl-2-(2,2,2-trifluoroethyl)-2,3,4,6,7,9-hexahydro-1H-cyclobuta[f]pyrido[3,4-b]indol-1-yl)phenyl)-1-(3-fluoropropyl)pyrrolidin-3-amine and (S)-N-(3,5-difluoro-4-((1S,3 S)-3-methyl-2-(2,2,2-trifluoroethyl)-2,3,4,6,7,9-hexahydro-1H-cyclobuta[f]pyrido[3,4-b]indol-1-yl)phenyl)-1-(3-fluoropropyl)pyrrolidin-3-amine **(compound 48-1** and **compound 48-2)**

Compound **48B** (0.12 g, 0.22 mmol) was subjected to chiral preparation to obtain the title compound **48-1** (55 mg) and compound **48-2** (51 mg).
instrument: Waters 150 MGM; chromatographic column: Chiralpak Column; mobile phase: A: carbon dioxide, and B: isopropanol (0.1% aqueous ammonia); isocratic elution: 45% mobile phase B; flow rate: 70 mL/min; back pressure: 100 bar; column temperature: 35°C; wavelength: 220 nm; elution time: 7.5 min.

Compound **48-1,** retention time: 2.06 min, $^1$H NMR (400 MHz, DMSO-d6) $\delta$ 10.38 (s, 1H), 7.05 (s, 1H), 6.91 (d, 1H), 6.38 (d, 1H), 6.13 (d, 2H), 5.08 (s, 1H), 4.54 (t, 1H), 4.42 (t, 1H), 3.84 (s, 1H), 3.51 - 3.32 (m, 2H), 3.10 (s, 4H), 2.96-2.90 (m, 1H), 2.80 - 2.71 (m, 2H), 2.69 - 2.53 (m, 2H), 2.46 - 2.27 (m, 4H), 2.22-2.17 (m, 1H), 1.85-1.75 (m, 2H), 1.55-1.51 (m, 1H), 1.09 (d, 3H).

Compound **48-2,** retention time: 2.29 min, $^1$H NMR (400 MHz, DMSO-d6) $\delta$ 10.37 (s, 1H), 7.05 (d, 1H), 6.91 (d, 1H), 6.38 (d, 1H), 6.13 (d, 2H), 5.08 (s, 1H), 4.54 (t, 1H), 4.42 (t, 1H), 3.84 (s, 1H), 3.51 - 3.32 (m, 2H), 3.10 (s, 4H), 2.96-2.90 (m, 1H), 2.82 - 2.72 (m, 2H), 2.69 - 2.54 (m, 2H), 2.48 - 2.31 (m, 4H), 2.21-2.16 (m, 1H), 1.91 - 1.72 (m, 2H), 1.55-1.51 (m, 1H), 1.09 (d, 3H).

LC-MS (ESI): m/z = 551.4 $[M+H]^+$.

**Example 49: 3-((1R,3R)-1-(2,6-difluoro-4-(((S)-1-(3-fluoropropyl) pyrrolidin-3-yl)amino)phenyl)-3,6-dimethyl-1,3,4,9-tetrahydro-2H-pyrido[3,4-b]indol-2-yl)-2,2-difluoropropan-1-ol (compound 49)**

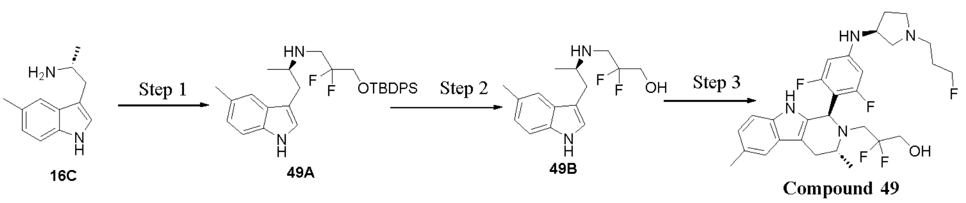


16C 49A 49B Compound 49

Step 1: (R)-3-((tert-butyldiphenylsilyl)oxy)-2,2-difluoro-N-(1-(5-methyl-1H-indol-3-yl)propan-2-yl)propan-1-amine **(compound 49A)**

Reagent 3-((tert-butyldiphenylsilyl)oxy)-2,2-difluoropropyl trifluoromethanesulfonate (2.26 g, 4.68 mmol), raw materials **16C** (0.68 g, 3.12 mmol) and DIPEA (1.21 g, 9.36 mmol) were added into 1,4-dioxane solvent (50 mL), heated to 100°C and stirred for 6 hours, cooled, and directly purified and separated by silica gel column chromatography (petroleum ether: ethyl acetate (v/v) = 1:0-5: 1) to obtain the title **compound 49A** (0.59 g, yield: 36%).

LCMS m/z = 521.3 [M+1].

Step 2: (R)-2,2-difluoro-3-((1-(5-methyl-1H-indol-3-yl)propan-2-yl)amino) propan-1-ol **(compound 49B)**

Raw material **49A** (0.59 g, 1.14 mmol) was dissolved in THF (20 mL) solvent, TBAF solution (2.28 mL, 2.28 mmol, 1M) was then added, stirred at room temperature for 2 hours, diluted with water, extracted with EA, dried over anhydrous sodium sulfate, and filtered, the filtrate was concentrated and then purified and separated by silica gel column chromatography (petroleum ether: ethyl acetate (v/v) = 1:0-1: 4) to obtain the title compound **49B** (0.3 g, yield: 93%).

LCMS m/z = 283.2 [M+1].

Step 3: 3-((1R,3R)-1-(2,6-difluoro-4-(((S)-1-(3-fluoropropyl)pyrrolidin-3-yl)amino)phenyl)-3,6-dimethyl-1,3,4,9-tetrahydro-2H-pyrido [3,4-b] indol-2-yl)-2,2-difluoropropan-1-ol **(compound 49)**

Raw materials **49B** (19 mg, 0.28 mmol) and **intermediate 1** (80 mg, 0.28 mmol) were added into a mixed solvent of toluene (4 mL) and acetic acid (1 mL), then heated to 95°C and reacted for 1 hour, directly concentrated to dryness, then washed with a saturated sodium bicarbonate solution, the filtrate was concentrated and then purified by TLC (DCM : MeOH = 10 : 1) to obtain the title compound **49** (30 mg, yield: 19%).

LCMS m/z = 551.3 [M+1].

1H NMR (400 MHz, $CD_3OD$) δ7.18 (s, 1H),7.07 (d, 1H),6.82-6.84(m, 1H), 6.12-6.15 (m, 2H), 5.13 (s, 1H), 4.54 (t, 1H), 4.42 (t, 1H), 3.91-3.97 (m, 1H), 3.73-3.84 (m, 1H), 3.57-3.62 (m, 1H), 3.45-3.50 (m, 1H), 3.09-3.16 (m, 1H), 2.90-2.96 (m, 2H), 2.72-2.79 (m, 2H), 2.50-2.67 (m, 5H), 2.39 (s, 3H), 2.27-2.35 (m, 1H), 1.83-1.97(m, 2H), 1.68-1.74 (m, 1H), 1.14 (d, 3H).

**Example 50: (S)-N-(4-((1R,3R)-3,6-dimethyl-2-(2,2,3-trifluoropropyl)-2,3,4,9-tetrahydro-1H-pyrido[3,4-b]indol-1-yl)-3,5-difluorophenyl)-1-(3-fluoropropyl)pyrrolidin-3-amine (compound 50)**

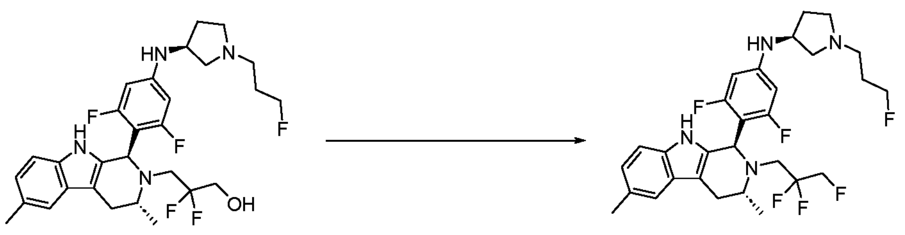

Compound **49** Compound **50**

Raw material **compound 49** (100 mg, 0.18 mmol) was dissolved in acetonitrile (8 mL) solution, triethylamine (0.15 mL, 1.08 mmol), triethylamine trihydrofluorate (0.059 mL, 0.36 mmol) and nonafluorobutyl sulfonyl fluoride (0.063 mL, 0.36 mmol) were then added, then heated to 50°C and reacted for 10 hours, cooled, then washed with a saturated sodium bicarbonate solution, extracted with EA, dried over anhydrous sodium sulfate, and filtered, the filtrate was concentrated, and then same was purified and separated by TLC (DCM : MeOH = 10 : 1) to obtain the title **compound 50** (50 mg, yield: 50%).

LCMS m/z = 553.2 [M+1].

1H NMR (400 MHz, DMSO-d6) δ10.39 (s, 1H), 7.15 (s, 1H), 7.07 (d, 1H), 6.81 (d, 1H), 6.40 (d, 1H), 6.15 (d, 2H), 5.04 (s, 1H), 4.47-4.73 (m, 3H), 4.42 (t, 1H), 3.82-3.86 (m, 1H), 3.34-3.42 (m, 1H), 3.10-3.21 (m, 1H), 2.67-2.82 (m, 3H), 2.58-2.64 (m, 1H), 2.51-2.54 (m, 1H), 2.38-2.49 (m, 3H), 2.35 (s, 3H), 2.33-2.34 (m, 1H), 2.15-2.23 (m, 1H), 1.73-1.86 (m, 2H), 1.49-1.57 (m, 1H), 1.07 (d, 3H).

**Example 51: N-(4-((1R,3R)-7-ethynyl-3-methyl-2-(2,2,2-trifluoroethyl)-2,3,4,9-tetrahydro-1H-pyrido[3,4-b]indol-1-yl)-3,5-difluorophenyl)-1-(3-fluoropropyl)azetidin-3-amine (compound 51)**

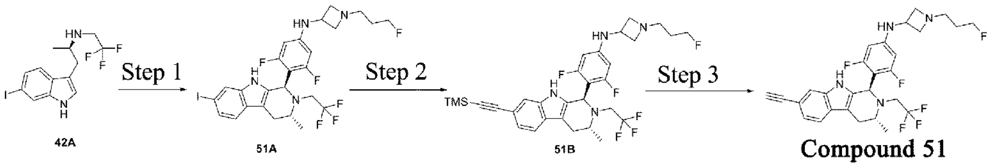


Step 1: N-(3,5-difluoro-4-((1R,3R)-7-iodo-3-methyl-2-(2,2,2-trifluoroethyl)-2,3,4,9-tetrahydro-1H-pyrido[3,4-b]indol-1-yl)phenyl)-1-(3-fluoropropyl)azetidin-3-amine (compound **51A**)

Raw materials **42A** (2 g, 5.23 mmol) and 2,6-difluoro-4-(1-(3-fluoropropyl)azetidin-3-yl)amino)benzaldehyde (intermediate **5L**) (1.42 g, 5.23 mmol) were added into a mixed solvent of toluene (20 mL) and acetic acid (6 mL), then heated to 95°C and reacted for 5 hour, directly concentrated to dryness, then washed with 1M sodium hydroxide solution, concentrated and then purified by silica gel column chromatography (DCM : MeOH = 10 : 1) to obtain the title compound (2.9 g, yield: 87%).
LCMS m/z = 637.1 [M+1].

Step 2: N-(3,5-difluoro-4-((1R,3R)-3-methyl-2-(2,2,2-trifluoroethyl)-7-((trimethylsilyl)ethynyl)-2,3,4,9-tetrahydro-1H-pyrido[3,4-b]indol-1-yl)phenyl)-1-(3-fluoropropyl)azetidin-3-amine (compound **51B**)

Raw materials **51A** (1.5 g, 2.36 mmol), trimethylethynylsilane (1.16 g, 11.80 mmol), triethylamine (0.48 g, 4.72 mmol), tetrakis(triphenylphosphine)palladium (270 mg, 0.24 mmol) and cuprous iodide (220 mg, 1.18 mmol) were added into anhydrous DMF (20 mL) solvent, then added into a 100 mL sealed tube, into which nitrogen was sparged for one minute, same was heated to 70°C, stirred for 8 hours, cooled, and filtered, the filtrate was concentrated under reduced pressure, diluted with water, extracted with EA, dried, and filtered, the filtrate was concentrated and then subjected to silica gel column chromatography (dichloromethane : anhydrous methanol (v/v) = 1 : 0-10 : 1) to obtain the title compound (1.2 g, yield: 84%).
LCMS m/z = 607.3 [M+1].

Step 3: N-(4-((1R,3R)-7-ethynyl-3-methyl-2-(2,2,2-trifluoroethyl)-2,3,4,9-tetrahydro-1H-pyrido[3,4-b]indol-1-yl)-3,5-difluorophenyl)-1-(3-fluoropropyl)azetidin-3-amine (compound **51**)

Raw material **51B** (1.2 g, 1.98 mmol) was dissolved in anhydrous methanol (20 mL) solvent, potassium carbonate (550 mg, 3.96 mmol) was added at room temperature, reacted for 3 hours, and filtered, the filtrate was concentrated and then purified and separated by silica gel column chromatography (dichloromethane : anhydrous methanol (v/v) = 1 : 0-10 : 1) to obtain the target compound (350 mg, yield: 33%).
LCMS m/z = 535.2 [M+1].
$^1H$ NMR (400 MHz, DMSO-$d_6$) δ10.78 (s, 1H), 7.38 (d, 1H), 7.31 (s, 1H), 7.02-7.05 (m, 1H), 6.72 (d, 1H), 6.12 (d, 2H), 5.12 (s, 1H), 4.51 (t, 1H), 4.39 (t, 1H), 3.93-3.96 (m, 1H), 3.92 (s, 1H), 3.60-3.63 (m, 2H), 3.38-3.44 (m, 2H), 2.89-3.01 (m, 1H), 2.79-2.84 (m, 1H), 2.73 (t, 2H), 2.55-2.60 (m, 1H), 2.46 (t, 2H), 1.59-1.70 (m, 2H), 1.10 (d, 3H).

**Biological test**

**1. Inhibitory effect of compounds on the proliferation of MCF-7 cells**

Breast cancer cells MCF-7 were purchased from ATCC, the cell media were all EMEM+10%FBS, and the cells were cultured in an incubator at 37°C under 5% $CO_2$. On the first day, cells in the exponential growth phase were collected and cultured in a 10% css-FBS phenol red-free medium without cytokines for 3 days. After the completion of culture, a 10% css-FBS phenol red-free medium was used to adjust the cell suspension to the corresponding concentration and same was plated to 1000 cells/well. β-estradiol and compounds of different concentrations were added. A plate without adding any reagent was taken and used as Day0 with a reading value $RLU_{day0}$, determined with CellTiter-Glo kit, placed in an incubator and incubated for 7 days. After the incubation was completed, according to operation instructions for a CellTiter-Glo kit (Promega, G7573), 50 μL of CellTiter-Glo solutions, which were pre-melted and equilibrated to

room temperature, were added to each well, and the mixtures were uniformly mixed for 2 min using a microplate shaker. The plate was placed at room temperature for 10 min, and then fluorescence signal values were measured using a microplate reader (PHERAstar FSX). Chemiluminescence readouts were processed using origin9.2 software and the DoseResp function was used to calculate the $GI_{50}$ value for the inhibitory activity of compounds on the proliferation of cells (Table 1). The results were processed according to equation (1), the inhibition rate of the compound at different concentrations was calculated, and the $GI_{50}$ value of the compound with a proliferation rate of 50% was calculated using origin9.2 software, wherein $RLU_{compound}$ was the readout of the treated group, and $RLU_{control}$ was the average value of the solvent control group.

$$\text{Growth }\% = (RLU_{compound} - RLU_{day0}) / (RLU_{control} - RLU_{day0}) \times 100 \quad \text{equation (1)}$$

Results: The compound of the present invention had a significant inhibitory effect on the proliferation of MCF-7 cells. The example compounds had an inhibitory activity with GI50 value of less than 100 nM on the proliferation of MCF-7 cells, some excellent compounds had a $GI_{50}$value of less than 1 nM on MCF-7 cells, and more excellent compounds had a $GI_{50}$ value of less than 0.03 nM on MCF-7 cells. The test results of some examples were shown in Table 1.

Table 1 GI50 value for the inhibitory activity of the compound of the present invention on the proliferation of MCF-7 cells

| Compound | MCF-7 $GI_{50}$ (nM) |
|---|---|
| Compound 35 | <0.026 |
| Compound 38 | <0.026 |
| Compound 43 | 0.219 |
| Compound 49 | <0.026 |
| Conclusion: the compounds of the present invention had a significant inhibitory effect on the proliferation of MCF-7 cells. | |

**2. Degradation effect of compounds on ERα in MCF-7 and CAMA-1 cells**

Breast cancer cells MCF-7 and CAMA-1 were purchased from ATCC, the cell media were all EMEM+10%FBS, and the cells were cultured in an incubator at 37°C under 5% $CO_2$. On the first day, the cells in an exponential phase were collected. With a 10% css-FBS phenol red-free medium, the cell suspension was adjusted to a corresponding concentration, plated at 300000 cells/well and incubated for 3 days. After 3 days, the liquid in the well was aspirated, and a 10% css-FBS phenol red-free medium containing the compound to be tested was added and cultured in an incubator at 37°C under 5% $CO_2$. After 24 hours, 60 μl of RIPA lysis solution (containing 1X protease inhibitor cocktail) was added to each well, lysed on ice for 15 minutes, and then centrifuged at 4°C for 10 minutes at 12000 g. The supernatant protein samples were collected, and protein quantification was performed by a BCA method. ERα was detected using fully automatic protein expression quantitative analysis, which involved diluting the protein to be tested to 1.5 mg/mL. 4 μL of the diluted protein samples were added to 1 μL of 5 × Master Mix (provided in the kit), and the prepared samples were denatured at 95°C for 5 min and placed on ice for use. Antibody Diluent II (provided in the kit) was used to dilute the primary antibody, in which the dilution ratios of ERα (CST, 13258S) and β-actin (CST, 3700) antibodies were 1 : 40 and 1 : 500, respectively, the secondary antibody was a 1 : 1 mixture of goat anti-mouse and goat anti-rabbit secondary antibodies, and the chromogenic solution was a 1 : 1 mixture of Lumino-S and Peroxide. The prepared reagents were successively added to an assay plate according to instructions of the kit and detected on an instrument. Western blot band processing was performed using "Compass for SW", a fully automatic protein expression quantitative analysis software, in which western blot bands were automatically simulated based on signal values. The proportion of ERα relative to the solvent control at different drug concentrations according to equation (1), wherein $A_{compound}$ was the relative peak area of ERα in the treated group, and $A_{solvent}$ was the relative peak area of ERα in the solvent control group.

$$ER\alpha\ \% = A_{compound}/A_{solvent} \times 100 \quad \text{equation (1)}$$

$DC_{50}$ calculation: Calculation was performed according to equation (1), and using Graphpad Prism 8.0 software, the compound concentration $DC_{50}$ values at which the degradation rate of ERα was 50% were calculated using a non-linear fourparameter function (Non linear regression (cruve fit)) (Table 1).

Results: The compound of the present invention had a significant inhibitory effect on ERα in MCF-7 and CAMA-1 cells, and the DC50 value of the example compound on ERα was less than 1 μM. The test results of some examples were shown in Table 2.

Table 2 Degradation activity on ERα in MCF-7 and CAMA-1 cells

| Compound | MCF-7 | |
|---|---|---|
| | $DC_{50}$ (nM) | **Dmax,%** |
| Compound 8 | 0.594 | 70%@100nM |
| Compound 15 | 0.447 | 72 @100 nM |
| Compound 18 | 0.47 | 70%@100nM |
| Compound 22-1 | 1.13 | 69%@100nM |
| Compound 31 | 0.244 | 77%@100nM |
| Compound 32 | 1.23 | 88%@100nM |
| Compound 35 | 0.269 | 68%@100nM |
| Compound 38 | 1.7 | 72%@100nM |
| Compound 49 | 0.726 | 70%@100nM |
| Compound 51 | 0.712 | 68%@100nM |
| Conclusion: the compounds of the present invention had a significant inhibitory effect on the activity of ERα. | | |

**3. Pharmacokinetic test in rats**

3.1 Experimental animals: male SD rats, about 220 g, 6-8 weeks old, 6 rats/compound, purchased from Chengdu Ddossy Experimental Animals Co., Ltd.

3.2 Experimental design: on the day of the test, 24 SD rats were randomly grouped according to their body weight; the animals were fasted with water available for 12 to 14 h one day before the administration, and were fed 4 h after the administration;

Table 3 Administration information

| Group | Quantity | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of Administration |
| G1 | 3 | WO 2021228210 | 2.5 | 0.5 | 5 | Plasma | Intravenously |
| G2 | 3 | **Compound 4** | 10 | 1 | 10 | Plasma | Intragastrically |
| G3 | 3 | **Compound 32** | 2.5 | 0.5 | 5 | Plasma | Intravenously |
| G4 | 3 | | 10 | 1 | 10 | Plasma | Intragastrically |
| G5 | 3 | **Compound 18** | 2.5 | 0.5 | 5 | Plasma | Intravenously |
| G6 | 3 | | 10 | 1 | 10 | Plasma | Intragastrically |
| G7 | 3 | **Compound 43** | 2.5 | 0.5 | 5 | Plasma | Intravenously |
| G8 | 3 | | 10 | 1 | 10 | Plasma | Intragastrically |

Notes: Solvent for intravenous administration: 5%DMA+5%Solutol+90%Saline; Solvent for intragastric administration: 0.5% MC (DMA: dimethylacetamide; Solutol: Polyethylene glycol-15-hydroxystearate; Saline: Normal saline; MC: methylcellulose)

Before and after the administration, 0.15 ml of blood was taken from the orbit of the animals under isoflurane anesthesia and placed in an EDTAK2 centrifuge tube. The blood was centrifuged at 5000 rpm and 4°C for 10 min to collect plasma. The blood collection time points for the intravenous administration group and intragastric administration group were: 0, 5 min, 15 min, 30 min, 1 h, 2 h, 4 h, 6 h, 8 h, and 24 h. Before analysis and detection, all samples were stored at -80°C, and a quantitative analysis of samples was performed using LC-MS/MS.

The test results were as shown in Table 4.

Table 4 Pharmacokinetic parameters of compounds in plasma of rats

| Test compounds | Mode of administration | CL (mL/min/kg) | $Vd_{ss}$ (L/kg) | $AUC_{0-t}$ (hr*ng/mL) | F (%) |
|---|---|---|---|---|---|
| WO 2021228210 **Compound 4** | i.v. (2.5 mg/kg) | 42.2±14 | 21.9±4.3 | 942±240 | - |
| | i.g. (10 mg/kg) | - | - | 1493±48 | 39.6±1.3 |
| **Compound 32** | i.v. (2.5 mg/kg) | 39.2±2.8 | 31.4±5.9 | 853±48 | - |
| | i.g. (10 mg/kg) | - | - | 1966±469 | 57.6±14 |
| **Compound 18** | i.v. (2.5 mg/kg) | 16.9±4.0 | 12.2±1.5 | 2074±294 | - |
| | i.g. (10 mg/kg) | - | - | 4563±1042 | 55.0±13 |
| **Compound 43** | i.v. (2.5 mg/kg) | 20.8±2.0 | 18.1±2.1 | 1568±143 | - |
| | i.g. (10 mg/kg) | - | - | 3200±129 | 51.0±2.1 |
| -: not applicable.<br>Conclusion: Compared with **compound 4** reported in WO 2021228210, the compound in this patent exhibits excellent pharmacokinetic properties in rat PK testing, and has certain advantages in terms of exposure and oral bioavailability. | | | | | |

**4. Pharmacokinetic test in mice**

4.1 Experimental animals: male ICR mice, 20-25 g, 18 mice/compound. purchased from Chengdu Ddossy Experimental Animals Co., Ltd.

4.2 Experimental design: on the day of the test, 72 ICR mice were randomly grouped according to their body weights. the animals were fasted with water available for 12 to 14 h one day before the administration, and were fed 4 h after the administration;

Table 5 Administration information

| Group | Quantity | Administration information | | | | | |
|---|---|---|---|---|---|---|---|
| | Male | Test compound | Administration dosage (mg/kg) | Administration concentration (mg/mL) | Administration volume (mL/kg) | Collected samples | Mode of Administration |
| G1 | 9 | WO 2021228210 | 5 | 1 | 5 | Plasma | Intravenously |
| G2 | 9 | **Compound 4** | 10 | 1 | 10 | Plasma | Intragastrically |
| G3 | 9 | **Compound 32** | 5 | 1 | 5 | Plasma | Intravenously |
| G4 | 9 | | 10 | 1 | 10 | Plasma | Intragastrically |
| G5 | 9 | **Compound 18** | 5 | 1 | 5 | Plasma | Intravenously |
| G6 | 9 | | 10 | 1 | 10 | Plasma | Intragastrically |
| G7 | 9 | **Compound 43** | 5 | 1 | 5 | Plasma | Intravenously |
| G8 | 9 | | 10 | 1 | 10 | Plasma | Intragastrically |

Notes: Solvent for intravenous administration: 5%DMA+5%Solutol+90%Saline; Solvent for intragastric administration: 0.5% MC (DMA: dimethylacetamide; Solutol: Polyethylene glycol-15-hydroxystearate; Saline: Normal saline; MC: methylcellulose)

Before and after the administration, 0.06 mL of blood was taken from the orbit of the animals under isoflurane anesthesia and placed in an EDTAK2 centrifuge tube. The blood was centrifuged at 5000 rpm and 4°C for 10 min to collect plasma. The blood collection time points for the intravenous group and intragastric administration group were: 0 min, 5 min, 15 min, 30 min, 1 h, 2 h, 4 h, 6 h, 8 h, and 24 h. Before analysis and detection, all samples were stored at -80°C, and a quantitative analysis of samples was performed using LC-MS/MS. The test results were as shown in Table 6.

Table 6 Pharmacokinetic parameters of compounds in plasma of mice

| Test compounds | Mode of administration | CL (mL/min/kg) | $Vd_{ss}$ (L/kg) | $AUC_{0-t}$ (hr*ng/mL) | F (%) |
|---|---|---|---|---|---|
| WO 2021228210 | i.v. (5 mg/kg) | 15.3 | 28.2 | 5450 | - |
| **Compound 4** | i.g. (10 mg/kg) | - | - | 5695 | 52.3 |
| **Compound 32** | i.v. (5 mg/kg) | 16.3 | 21.6 | 3356 | - |
| | i.g. (10 mg/kg) | - | - | 4406 | 65.6 |
| **Compound 18** | i.v. (5 mg/kg) | 15.0 | 18.2 | 3806 | - |
| | i.g. (10 mg/kg) | - | - | 5654 | 74.3 |
| **Compound 43** | i.v. (5 mg/kg) | 8.21 | 16.6 | 10153 | - |
| | i.g. (10 mg/kg) | - | - | 10187 | 50.2 |
| -: not applicable.<br>Conclusion: Compared with **compound 4** reported in WO 2021228210, the compound of the present invention exhibits excellent pharmacokinetic properties in mouse PK testing. | | | | | |

**5. Test for hERG potassium ion channel**

Test platform: electrophysiological manual patch-clamp system
Cell line: Chinese hamster ovary (CHO) cell lines stably expressing hERG potassium ion channel
Test method: In CHO (Chinese Hamster Ovary) cells stably expressing hERG potassium channel, whole cell patch-clamp technique was used to record hERG potassium channel current at room temperature. The glass microelectrode was made of a glass electrode blank (BF150-86-10, Sutter) by a puller. The tip resistance after filling the liquid in the electrode was about 2-5 MΩ. The glass microelectrode can be connected to the patch-clamp amplifier by inserting the glass microelectrode into an amplifier probe. The clamping voltage and data recording were controlled and recorded by the pClamp 10 software through a computer. The sampling frequency was 10 kHz, and the filtering frequency was 2 kHz. After the whole cell records were obtained, the cells were clamped at -80 mV, and the step voltage that induced the hERG potassium current ($I_{hERG}$) was depolarized from -80 mV to +20 mV for 2 s, then repolarized to -50 mV, and returned to -80 mV after 1 s. This voltage stimulation was given every 10 s, and the administration process was started after the hERG potassium current was confirmed to be stable (at least 1 minute). The compound was administered for at least 1 minute at each test concentration, and at least 2 cells (n ≥ 2) were tested at each concentration.

Data processing: Data analysis processing was carried out by using pClamp 10, GraphPad Prism 5 and Excel software. The inhibition degree of hERG potassium current (peak value of hERG tail current induced at -50 mV) at different compound concentrations was calculated by the following formula:

$$\text{Inhibition\%} = [1 - (I / I\text{o})] \times 100\%$$

wherein, Inhibition % represents the percentage of inhibition of hERG potassium current by the compound, and $I$ and $I_O$ represent the amplitude of hERG potassium current after and before dosing, respectively.

$IC_{50}$ for the compound was calculated using GraphPad Prism 5 software by fitting according to the following equation:

$$\text{Y = Bottom + (Top-Bottom)/(1+10\^{}((LogIC50-X)*HillSlope))}$$

wherein, X represents the Log value of the tested concentration of the test sample, Y represents the inhibition percentage at the corresponding concentration, and Bottom and Top represent the minimum and maximum inhibition percentage, respectively.

Test results: The $IC_{50}$ value for the inhibitory effect of the test compound on hERG potassium channel current is shown in Table 7 below.

Table 7 Inhibitory activity of test compounds on hERG potassium channel current

| Compound | Inhibition rate at the highest concentration tested | $IC_{50}$ (μM) |
|---|---|---|
| WO 2021228210 **Compound 4** | 97.65±1.26%@10 μM | 0.7021 |
| **Compound 32** | 100.20±0.74%@40 μM | 4.14 |
| **Compound 18** | 99.98±1.79%@40 μM | 2.82 |
| **Compound 43** | 98.28±0.93%@40 μM | 12.3 |
| Conclusion: The in this patent has more advantages in hERG testing than **compound 4** reported in WO 2021228210. | | |

**6. CYP450 enzyme inhibition test**

The purpose of this study was to evaluate the effect of the test compound on the activity of five isoenzymes (CYP1A2, CYP2C9, CYP2C19, CYP2D6 and CYP3A4) of human liver microsomal cytochrome P450 (CYP) by using an *in vitro* testing system. The specific probe substrates of CYP450 isoenzymes were incubated with human liver microsomes and test compounds of different concentrations, and reduced nicotinamide adenine dinucleotide phosphate (NADPH) was added to initiate the reaction. After the completion of the reaction, the sample was treated and liquid chromatography-tandem mass spectrometry (LC-MS/MS) was used to quantitatively detect metabolites produced by specific substrates, changes in CYP enzyme activity were determined, and $IC_{50}$value was calculated to evaluate the inhibitory potential of the test compound on each CYP enzyme subtype.

Test results: Under the test conditions, when the incubation concentration was 0-50/100 μM, the $IC_{50}$ values for the inhibitory effect of each compound on CYP enzyme were shown in Table 8 below.

Table 8 Inhibitory activity of test compounds on CYP enzyme

| CYP enzyme subtypes/compounds | $IC_{50}$ (μM) | | | |
|---|---|---|---|---|
| | WO 2021228210 **Compound 4** | **Compound 32** | **Compound 18** | **Compound 43** |
| CYP1A2 | 15.6 | >100 | >100 | 41.6 |
| CYP2C9 | 15.6 | 19.5 | 20.0 | >42.8 |
| CYP2C19 | 19.1 | NA | NA | >50 |
| CYP2D6 | 13.8 | 23.4 | 34.1 | 21.5 |
| CYP3A-M | 12.8 | 18.9 | 15.7 | >12.7 |
| Conclusion: The of the present invention has more advantages in CYP testing than **compound 4** reported in WO 2021228210. | | | | |

7. **Growth inhibition experiment in mouse MCF-7 brain orthotopic tumor model**

Cell culture: MCF-7-Luc cells (derived from WuXi Apptec Co., Ltd.) were cultured *in vitro.* The culture conditions included a medium supplemented with 10% fetal calf serum and 10 μg/mL recombinant insulin at 37 °C under 5% $CO_2$. Passaging was performed twice a week. When the cells were maintained in the exponential growth phase, cells were harvested, counted, and inoculated.

Animal: BALB/c nude mice, female, 6-8 weeks old, weighing 19-23 grams, provided by Beijing Vital River Laboratory Animal Technology Co., Ltd.

Tumor inoculation: the mice were anesthetized and placed on a brain stereotaxic apparatus, and the brain skin was disinfected. Sagittal incisions of about 2 mm on the parietal bone and occipital bone were made to expose the skull,

and a 25G sharp needle was used to perforate the skull 2 mm to the right of the bregma and 1 mm anterior to the coronal suture. A syringe was used to draw the cell suspension, the needle was inserted vertically into the previous puncture to a depth of 3 mm, and the cell suspension (3 $\mu$L, containing $3 \times 10^5$ MCF-7-Luc cells and 20% Matrigel, injected for 3 minutes) was slowly injected. After the completion of the injection, the syringe was left in for 1 minute, and then slowly withdrew to reduce the reflux of cell fluid. The perforation was sealed with glue, the scalp was reset, and the incision was sutured. After the operation, the animal was monitored until it fully woke up. 17-beta-estradiol tablets (0.36 mg, 60 day release, innovrsrch, Cat#: SE-121-0.36 mg) were implanted subcutaneously at least one day before cell implantation. On day 8 after cell inoculation, the mice were randomized into groups for administration according to the body weight thereof, and the day of grouping was Day0.

Administration: Compound 18 and Compound 32 were administered at a dose of 30 mg/kg orally (PO), once daily (QD), with 8 mice in each group. All groups were continuously administered for 56 days.

Experiment observation and endpoint:

The body weight of the mice was measured twice a week, and the intracranial MCF-luc fluorescence value of the mice was measured using the live animal in vivo imaging system IVIS. The experiment was terminated after 49-day administration, and all mice were euthanized.

Test results:

see Figures 1 and 2. In the mouse brain orthotopic MCF-7 model, the body weight of the solvent control group continued to decrease after 35 days of administration. This was due to the progression of intracranial tumors and the heavier tumor burden in the mice. The mice in the group with the compound in the example of the present invention had a stable body weight and no abnormalities until the end of the experiment, and no mice died. Compared with the solvent control group, the compound in the example had a significant inhibitory effect on mice in MCF-7 brain orthotopic tumor model.

## Claims

1. A compound as represented by formula (I), or a stereoisomer, solvate, deuterated compound, or pharmaceutically acceptable salt thereof,

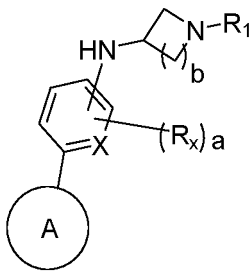

(I)

**characterized in that**, ring A is selected from I-a or I-b:

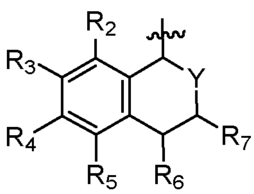

I-a

,

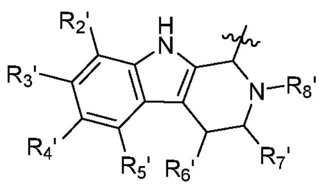

I-b

;

X is N or $CR_x$;
a is 0, 1, 2 or 3;

b is 1 or 2;

Y is $NR_8$ or $CHR_8$;

$R_1$ is $C_{1-6}$alkyl, $C_{2-6}$alkenyl or $C_{2-6}$alkynyl, the $R_1$ is optionally substituted with 1-3 groups selected from halogen, OH, $NH_2$, -$OC_{1-4}$alkyl, -$NHC_{1-4}$alkyl, $C_{3-6}$cycloalkyl, and 4- to 7-membered heterocycloalkyl containing 1-3 heteroatoms selected from N, S or O, and the cycloalkyl and heterocycloalkyl are optionally further substituted with 1-3 groups selected from halogen, OH, $C_{1-4}$alkyl and -$OC_{1-4}$alkyl;

each $R_x$ is independently H, $NH_2$, OH, halogen, $C_{1-4}$alkyl, halo $C_{1-4}$alkyl, -$N(C_{1-4}alkyl)_2$, -$NHC_{1-4}$alkyl or -$OC_{1-4}$alkyl;

$R_2$, $R_5$, $R_6$, $R_2$', $R_5$' and $R_6$' are independently H, CN, OH, $NH_2$, SH or halogen;

$R_3$ and $R_4$ are independently H, halogen, $SF_5$, CN, -$NR_aSO_2R_b$, -$SO_2NHR_a$, - $P(=O)(R_b)_2$, or -$NR_aP(=O)(R_b)_2$;

$R_a$ is H or $R_b$;

$R_b$ is $C_{1-4}$alkyl, $C_{3-6}$cycloalkyl, phenyl, 5- to 6-membered heteroaryl containing 1-3 heteroatoms selected from N, S, or O, or 4- to 7-membered heterocycloalkyl containing 1-3 heteroatoms selected from N, S, or O, and the alkyl, cycloalkyl, phenyl, heteroaryl and heterocycloalkyl are optionally substituted with 1-3 groups selected from halogen, CN, $NH_2$, OH, $C_{1-4}$alkyl, $C_{3-6}$cycloalkyl, 5- to 6-membered heteroaryl containing 1-3 heteroatoms selected from N, S, or O, and 4- to 7-membered heterocycloalkyl containing 1-3 heteroatoms selected from N, S, or O;

$R_3$' and $R_4$' are independently H, -$NR_cR_c$', $OR_d$, $C_{2-6}$alkenyl, $C_{2-6}$alkynyl, halogen, $SF_5$, CN, $C_{1-4}$alkyl, $C_{3-6}$cycloalkyl, phenyl, 5- to 6-membered heteroaryl containing 1-3 heteroatoms selected from N, S, or O, or 4- to 7-membered heterocycloalkyl containing 1-3 heteroatoms selected from N, S, or O, and the alkyl, alkenyl, alkynyl, cycloalkyl, phenyl, heteroaryl and heterocycloalkyl are optionally substituted with 1-3 groups selected from halogen, CN, $NH_2$, OH, $C_{1-4}$alkyl, $C_{3-6}$cycloalkyl, 5- to 6-membered heteroaryl containing 1-3 heteroatoms selected from N, S, or O, and 4- to 7-membered heterocycloalkyl containing 1-3 heteroatoms selected from N, S, or O;

optionally, one $R_3$' and one $R_4$' together with the atoms to which they are attached form $C_{3-8}$cycloalkyl, or 4- to 12-membered heterocycloalkyl containing 1-3 heteroatoms selected from N, S, or O, and the cycloalkyl or heterocycloalkyl is optionally substituted with 1-3 groups selected from halogen, OH, $NH_2$, $C_{1-4}$alkyl and halo $C_{1-4}$alkyl;

provided that, when b is selected from 2, $R_3$' and $R_4$' are not both selected from H;

$R_c$ is H, $C_{1-4}$alkyl or $C_{3-6}$cycloalkyl;

$R_c$' is $C_{2-6}$alkyl, $C_{2-6}$alkenyl, $C_{2-6}$alkynyl, -$COR_e$, -OH, -$OC_{1-4}$alkyl or -$SO_2R_e$;

$R_e$ is $C_{2-6}$alkyl, $C_{2-6}$alkenyl, $C_{2-6}$alkynyl, $C_{4-6}$cycloalkyl, -$C_{1-4}$alkyl$C_{3-6}$cycloalkyl, 5- to 6-membered heteroaryl containing 1-3 heteroatoms selected from N, S, or O, or 4- to 7-membered heterocycloalkyl containing 1-3 heteroatoms selected from N, S, or O, and the alkyl, alkenyl, alkynyl, cycloalkyl, heteroaryl and heterocycloalkyl are optionally substituted with 1-3 groups selected from halogen, OH, CN, $NH_2$, $C_{1-4}$alkyl and halo $C_{1-4}$alkyl;

$R_d$ is $C_{1-4}$alkyl, $C_{1-4}$alkyl, halo $C_{1-4}$alkyl, deuterated $C_{1-4}$alkyl, or halo $C_{1-4}$alkyl substituted with $C_{3-6}$cycloalkyl, 5- to 6-membered heteroaryl containing 1-3 heteroatoms selected from N, S, or O, or 4- to 7-membered heterocycloalkyl containing 1-3 heteroatoms selected from N, S, or O, and the cycloalkyl, heteroaryl and heterocycloalkyl are optionally substituted with 1-3 groups selected from halogen, OH, CN, $NH_2$, $C_{1-4}$alkyl and halo $C_{1-4}$alkyl;

$R_7$ and $R_7$' are H, $C_{1-4}$alkyl or $C_{3-6}$cycloalkyl, and the alkyl and cycloalkyl are optionally substituted with 1-3 groups selected from halogen, OH, SH, $C_{1-4}$alkyl and $C_{3-6}$cycloalkyl;

$R_8$ and $R_8$' are independently $C_{1-6}$alkyl, -$OR_f$, -$SR_f$, -$NHR_f$, $C_{3-6}$cycloalkyl, or 4- to 7-membered heterocycloalkyl containing 1-3 heteroatoms selected from N, S, or O, and the alkyl, cycloalkyl and heterocycloalkyl are optionally substituted with 1-3 groups selected from halogen, OH, CN, $NH_2$, $C_{1-4}$alkyl, -$C_{1-4}$alkyl-OH, $C_{3-6}$cycloalkyl, and 4- to 7-membered heterocycloalkyl containing 1-3 heteroatoms selected from N, S, or O;

$R_f$ is H, $C_{1-4}$alkyl, $C_{3-6}$cycloalkyl, or 4- to 7-membered heterocycloalkyl containing 1-3 heteroatoms selected from N, S, or O, and the alkyl, cycloalkyl and heterocycloalkyl are optionally substituted with 1-3 groups selected from halogen, OH, CN, $NH_2$, $C_{1-4}$alkyl, $C_{3-6}$cycloalkyl, and 4- to 7-membered heterocycloalkyl containing 1-3 heteroatoms selected from N, S, or O.

**2.** The compound, or the stereoisomer, solvate, deuterated compound, or pharmaceutically acceptable salt thereof according to claim 1, **characterized in that**,

(i) $R_3$ is -$NR_aSO_2R_b$, -$SO_2NR_a$, CN or halogen; or

(ii) $R_4$ is $SF_5$, -$NR_aSO_2R_B$, -$SO_2NR_a$, -$P(=O)(R_b)_2$ or -$NR_aP(=O)(R_b)_2$, wherein,

$R_B$ is $C_{1-4}$alkyl, $C_{3-6}$cycloalkyl, phenyl, 5- to 6-membered heteroaryl containing 1-3 heteroatoms selected from N, S, or O, or 4- to 7-membered heterocycloalkyl containing 1-3 heteroatoms selected from N, S, or O; when

$R_B$ is the alkyl, $R_B$ is further substituted with 1-3 groups selected from halogen, CN, $C_{3-6}$cycloalkyl, 5- to 6-membered heteroaryl containing 1-3 heteroatoms selected from N, S, or O, and 4-to 7-membered heterocycloalkyl containing 1-3 heteroatoms selected from N, S, or O; when $R_B$ is the cycloalkyl, phenyl, heteroaryl or heterocycloalkyl, $R_B$ is optionally substituted with 1-3 groups selected from halogen, CN, $NH_2$, OH and $C_{1-4}$alkyl; or

(iii) $R_5$ is CN or halogen; or

(iv) $R_8$ is $-OR_f$, $-SR_f$, $-NHR_f$, $C_{1-6}$alkyl, $C_{3-6}$cycloalkyl, or 4- to 7-membered heterocycloalkyl containing 1-3 heteroatoms selected from N, S, or O, when Rs is the alkyl, Rs is further substituted with $C_{3-6}$cycloalkyl, 5- to 6-membered heteroaryl containing 1-3 heteroatoms selected from N, S, or O, or 4- to 7-membered heterocycloalkyl containing 1-3 heteroatoms selected from N, S, or O; when Rs is the cycloalkyl or heterocycloalkyl, Rs is optionally substituted with 1-3 groups selected from halogen, OH, CN, $NH_2$, $C_{1-4}$alkyl, $C_{3-6}$cycloalkyl, and 4- to 7-membered heterocycloalkyl containing 1-3 heteroatoms selected from N, S, or O; or

(v) at least one of $R_3$' and $R_4$' is $-NR_cR_c$', $OR_d$, $C_{2-6}$alkenyl, $C_{2-6}$alkynyl, 5- to 6-membered heteroaryl containing 1-3 heteroatoms selected from N, S, or O, or 4-to 7-membered heterocycloalkyl containing 1-3 heteroatoms selected from N, S, or O, the alkenyl, alkynyl, heteroaryl and heterocycloalkyl are optionally substituted with 1-3 groups selected from halogen, CN, $NH_2$, OH, $C_{1-4}$alkyl, $C_{3-6}$cycloalkyl, 5-to 6-membered heteroaryl containing 1-3 heteroatoms selected from N, S, or O, and 4- to 7-membered heterocycloalkyl containing 1-3 heteroatoms selected from N, S, or O, and $R_c$' is $C_{2-6}$alkyl, $C_{2-6}$alkenyl, $C_{2-6}$alkynyl, $-OC_{1-4}$alkyl or OH;

optionally, one $R_3$' and one $R_4$' together with the atoms to which they are attached form $C_{3-8}$cycloalkyl, or 4- to 12-membered heterocycloalkyl containing 1-3 heteroatoms selected from N, S, or O, the cycloalkyl or heterocycloalkyl is optionally substituted with 1-3 groups selected from halogen, OH, $NH_2$, $C_{1-4}$alkyl and halo $C_{1-4}$alkyl; or

(vi) $R_8$' is $-OR_f$ or $C_{3-6}$cycloalkyl, and the cycloalkyl is optionally substituted with 1-3 groups selected from halogen, OH, CN, $NH_2$, $C_{1-4}$alkyl, $-C_{1-4}$alkyl-OH, $C_{3-6}$cycloalkyl, and 4- to 7-membered heterocycloalkyl containing 1-3 heteroatoms selected from N, S, or O;

$R_f$ is $C_{1-4}$alkyl, $C_{3-6}$cycloalkyl, or 4- to 7-membered heterocycloalkyl containing 1-3 heteroatoms selected from N, S, or O, the alkyl, cycloalkyl and heterocycloalkyl are optionally substituted with 1-3 groups selected from halogen, OH, CN, $NH_2$, $C_{1-4}$alkyl, $C_{3-6}$cycloalkyl, and 4- to 7-membered heterocycloalkyl containing 1-3 heteroatoms selected from N, S, or O.

3. The compound, or the stereoisomer, solvate, deuterated compound, or pharmaceutically acceptable salt thereof according to claim 2, **characterized in that** the compound has a structure of formula (II'),

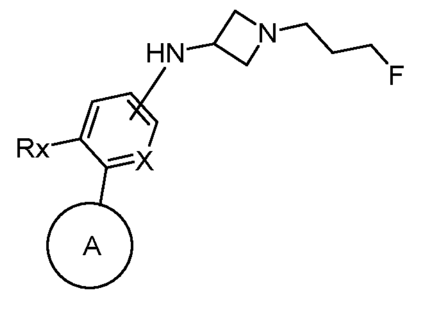

(II')

wherein, ring A is selected from II-a or II-b:

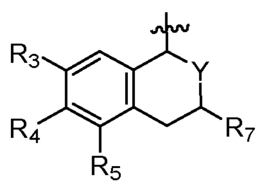

II-a

,

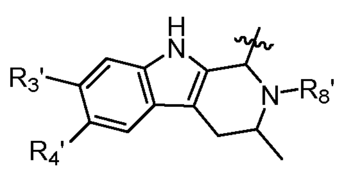

II-b

X is N or $CR_x$;

Y is $NR_8$ or $CHR_8$;
each $R_x$ is independently H or halogen.

**4.** The compound, or the stereoisomer, solvate, deuterated compound, or pharmaceutically acceptable salt thereof according to claim 3,

**characterized in that**, $R_3$' and $R_4$' are independently H, $-NR_cR_c$', $OR_d$, $C_{2-6}$alkenyl, $C_{2-6}$alkynyl, halogen, $SF_5$, CN, $C_{1-4}$alkyl, $C_{3-6}$cycloalkyl, 5-membered heteroaryl containing 1-3 heteroatoms selected from N, S, or O, or 5- to 6-membered heterocycloalkyl containing 1-3 heteroatoms selected from N, S, or O, the alkyl, alkenyl, alkynyl, cycloalkyl, heteroaryl and heterocycloalkyl are optionally substituted with 1-3 groups selected from halogen, CN, $NH_2$, OH, $C_{1-4}$alkyl, $C_{3-6}$cycloalkyl, 5- to 6-membered heteroaryl containing 1-3 heteroatoms selected from N, S, or O, and 5- to 6-membered heterocycloalkyl containing 1-3 heteroatoms selected from N, S, or O;
optionally, one $R_3$' and one $R_4$' together with the atoms to which they are attached form $C_{4-6}$cycloalkyl, the cycloalkyl is optionally substituted with 1-3 groups selected from halogen, OH, and $C_{1-4}$alkyl;
$R_c$ is H, $C_{1-4}$alkyl or $C_{3-6}$cycloalkyl;
$R_c$' is $C_{2-6}$alkyl, $C_{2-6}$alkenyl, $C_{2-6}$alkynyl, -OH or $-OC_{1-4}$alkyl;
$R_d$ is $C_{1-4}$alkyl, $C_{1-4}$alkyl, halo $C_{1-4}$alkyl, deuterated $C_{1-4}$alkyl, or halo $C_{1-4}$alkyl substituted with $C_{3-6}$cycloalkyl, 5- to 6-membered heteroaryl containing 1-3 heteroatoms selected from N, S, or O, or 5- to 6-membered heterocycloalkyl containing 1-3 heteroatoms selected from N, S, or O, the cycloalkyl, heteroaryl and heterocycloalkyl are optionally substituted with 1-3 groups selected from halogen, OH, $C_{1-4}$alkyl and halo $C_{1-4}$alkyl;
Rs is independently H, CN, OH or halogen;
$R_7$ is H or $C_{1-4}$alkyl;
$R_8$ and $R_8$' are independently $C_{1-4}$alkyl, $-OR_f$, $-NHR_f$, $C_{3-6}$cycloalkyl, or 5- to 6-membered heterocycloalkyl containing 1-3 heteroatoms selected from N, S, or O, the alkyl, cycloalkyl and heterocycloalkyl are optionally substituted with 1-3 groups selected from halogen, OH, CN, $NH_2$, $C_{1-4}$alkyl, $-C_{1-4}$alkyl-OH, $C_{3-6}$cycloalkyl, and 5- to 6-membered heterocycloalkyl containing 1-3 heteroatoms selected from N, S, or O;
$R_f$ is $C_{1-4}$alkyl, $C_{3-6}$cycloalkyl, or 5-to 6-membered heterocycloalkyl containing 1-3 heteroatoms selected from N, S, or O, the alkyl, cycloalkyl and heterocycloalkyl are optionally substituted with 1-3 groups selected from halogen, OH, CN, $NH_2$, $C_{1-4}$alkyl, $C_{3-6}$cycloalkyl, and 5- to 6-membered heterocycloalkyl containing 1-3 heteroatoms selected from N, S, or O;
provided that, the compound satisfies that:

(i) $R_3$ is $-NR_aSO_2R_b$, $-SO_2NR_a$, CN or halogen; or
(ii) $R_4$ is $SF_5$, $-NR_aSO_2R_B$, $-SO_2NR_a$, $-P(=O)(R_b)_2$ or $-NR_aP(=O)(R_b)_2$, wherein, $R_B$ is $C_{1-4}$alkyl, $C_{3-6}$cycloalkyl, phenyl, 5- to 6-membered heteroaryl containing 1-3 heteroatoms selected from N, S, or O, or 5- to 6-membered heterocycloalkyl containing 1-3 heteroatoms selected from N, S, or O; when $R_B$ is the alkyl, $R_B$ is further substituted with 1-3 groups selected from halogen, CN, $C_{3-6}$cycloalkyl, 5- to 6-membered heteroaryl containing 1-3 heteroatoms selected from N, S, or O, and 5-to 6-membered heterocycloalkyl containing 1-3 heteroatoms selected from N, S, or O; when $R_B$ is the cycloalkyl, phenyl, heteroaryl or heterocycloalkyl, $R_B$ is optionally substituted with 1-3 groups selected from halogen, CN, $NH_2$, OH and $C_{1-4}$alkyl; or
(iii) $R_5$ is CN or halogen; or
(iv) $R_8$ is $-OR_f$, $-NHR_f$, $C_{1-4}$alkyl, $C_{3-6}$cycloalkyl, or 5- to 6-membered heterocycloalkyl containing 1-3 heteroatoms selected from N, S, or O, when Rs is the alkyl, Rs is further substituted with $C_{3-6}$cycloalkyl, 5- to 6-membered heteroaryl containing 1-3 heteroatoms selected from N, S, or O, or 5- to 6-membered heterocycloalkyl containing 1-3 heteroatoms selected from N, S, or O; when Rs is the cycloalkyl or heterocycloalkyl, Rs is optionally substituted with 1-3 groups selected from halogen, OH, CN, $NH_2$, $C_{1-4}$alkyl, $C_{3-6}$cycloalkyl, and 5- to 6-membered heterocycloalkyl containing 1-3 heteroatoms selected from N, S, or O; or
(v) at least one of $R_3$' and $R_4$' is $-NHR_c$', $OR_d$, $C_{2-6}$alkenyl, $C_{2-6}$alkynyl, 5-membered heteroaryl containing 1-3 heteroatoms selected from N, S, or O, or 5- to 6-membered heterocycloalkyl containing 1-3 heteroatoms selected from N, S, or O, the alkenyl, alkynyl, heteroaryl and heterocycloalkyl are optionally substituted with 1-3 groups selected from halogen, CN, $NH_2$, OH, $C_{1-4}$alkyl, $C_{3-6}$cycloalkyl, 5- to 6-membered heteroaryl containing 1-3 heteroatoms selected from N, S, or O, and 5- to 6-membered heterocycloalkyl containing 1-3 heteroatoms selected from N, S, or O, $R_c$' is $C_{2-6}$alkyl, $C_{2-6}$alkenyl, $C_{2-6}$alkynyl, $-OC_{1-4}$alkyl or OH;
optionally, one $R_3$' and one $R_4$' together with the atoms to which they are attached form $C_{3-8}$cycloalkyl, or 4- to 12-membered heterocycloalkyl containing 1-3 heteroatoms selected from N, S, or O, the cycloalkyl

or heterocycloalkyl is optionally substituted with 1-3 groups selected from halogen, OH, $NH_2$, $C_{1-4}$alkyl and halo $C_{1-4}$alkyl; or
(vi) $R_8$' is -$OR_f$ or $C_{3-6}$cycloalkyl, the cycloalkyl is optionally substituted with 1-3 groups selected from halogen, OH, CN, $NH_2$, $C_{1-4}$alkyl, -$C_{1-4}$alkyl-OH, $C_{3-6}$cycloalkyl, and 4- to 7-membered heterocycloalkyl containing 1-3 heteroatoms selected from N, S, or O;

$R_f$ is $C_{1-4}$alkyl, $C_{3-6}$cycloalkyl, or 5-to 6-membered heterocycloalkyl containing 1-3 heteroatoms selected from N, S, or O, the alkyl, cycloalkyl and heterocycloalkyl are optionally substituted with 1-3 groups selected from halogen, OH, CN, $NH_2$, $C_{1-4}$alkyl, $C_{3-6}$cycloalkyl, and 5- to 6-membered heterocycloalkyl containing 1-3 heteroatoms selected from N, S, or O.

5. The compound, or the stereoisomer, solvate, deuterated compound, or pharmaceutically acceptable salt thereof according to claim 1, **characterized in that** the compound has a structure of formula (II'-1),

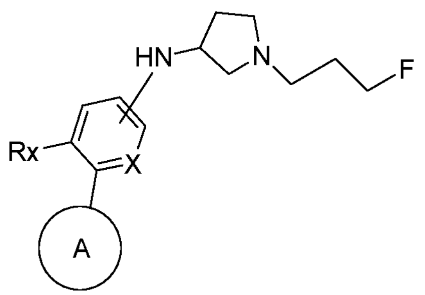

(II'-1)

wherein, ring A is selected from II-a or II-b:

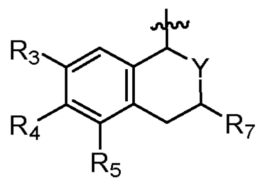

II-a

,

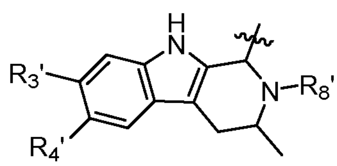

II-b

X is N or $CR_x$;
Y is $NR_8$ or $CHR_8$;
each $R_x$ is independently H or halogen;
provided that, $R_3$' and $R_4$' are not both selected from H.

6. The compound, or the stereoisomer, solvate, deuterated compound, or pharmaceutically acceptable salt thereof according to claim 1, **characterized in that** the compound has a structure of formula (II'-a) or formula (II'-a-1),

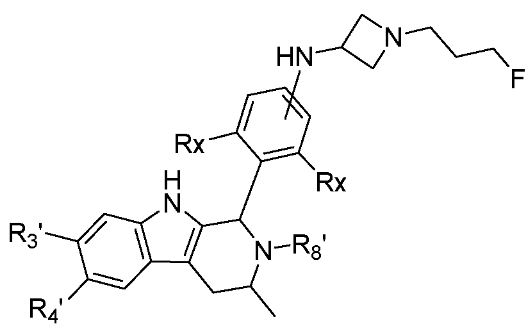

(II'-a)

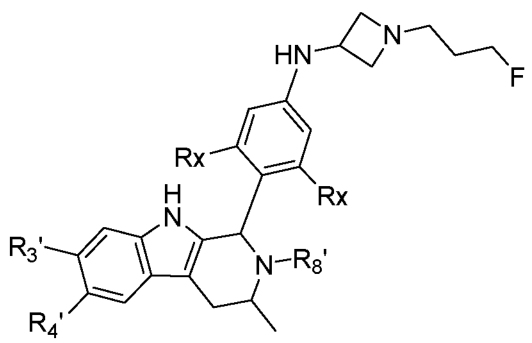

(II'-a-1)

each Rx is independently H or halogen;
$R_3$' and $R_4$' are independently H, $C_{1-6}$alkyl, $C_{1-6}$alkoxy, -O-$CH_2$-$C_{3-6}$cycloalkyl, 5- to 6-membered heteroaryl containing 1-3 heteroatoms selected from N, S, or O, $C_{2-6}$alkenyl or $C_{2-6}$alkynyl, or $R_3$' and $R_4$' form 4-membered cycloalkyl or 5-membered cycloalkyl;
provided that, $R_3$' and $R_4$' are not both selected from H;
$R_8$' is independently $C_{1-6}$alkyl, $C_{3-6}$cycloalkyl, or 4- to 7-membered heterocycloalkyl containing 1-3 heteroatoms selected from N, S, or O, the alkyl, cycloalkyl and heterocycloalkyl are optionally substituted with 1-3 groups selected from halogen, OH, CN, $NH_2$, $C_{1-4}$alkyl, -$C_{1-4}$alkyl-OH, $C_{3-6}$cycloalkyl, and 4- to 7-membered heterocycloalkyl containing 1-3 heteroatoms selected from N, S, or O.

**7.** The compound, or the stereoisomer, solvate, deuterated compound, or pharmaceutically acceptable salt thereof according to claim 6, **characterized in that**,

each Rx is independently H or F;
$R_3$' and $R_4$' are independently H, $C_{1-4}$alkyl, $C_{1-4}$alkoxy, -O-$CH_2$-$C_{3-6}$cycloalkyl, 5- to 6-membered heteroaryl containing 1-3 heteroatoms selected from N, S, or O, $C_{2-4}$alkenyl, or $C_{2-4}$alkynyl, provided that $R_3$' and $R_4$' are not both selected from H;
$R_8$' is independently $C_{1-4}$alkyl, and the alkyl is substituted with 1, 2, or 3 groups selected from F, Cl, OH, CN, $NH_2$, methyl, hydroxymethyl, cyclopropyl, azetidinyl, or oxetanyl.

**8.** The compound, or the stereoisomer, solvate, deuterated compound, or pharmaceutically acceptable salt thereof according to claim 1, **characterized in that** the compound has a structure of formula (II'-1a) or formula (II'-1a-1),

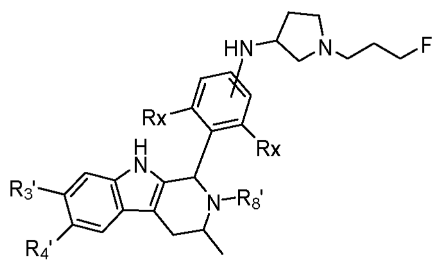

(II'-1a)

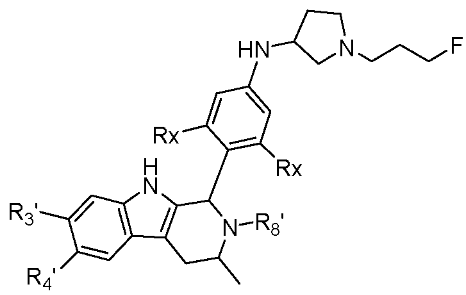

(II'-1a-1)

each Rx is independently H or halogen;

$R_3$' and $R_4$' are independently H, halo $C_{1-4}$alkyl, $C_{1-6}$alkyl, $C_{2-6}$alkenyl, $C_{2-6}$alkynyl or halogen; or
$R_3$' and $R_4$' together form cyclobutyl;
$R_8$' is independently $C_{1-6}$alkyl, $C_{3-6}$cycloalkyl, or 4- to 7-membered heterocycloalkyl containing 1-3 heteroatoms selected from N, S, or O, the alkyl, cycloalkyl and heterocycloalkyl are optionally substituted with 1-3 groups selected from halogen, OH, CN, $NH_2$, $C_{1-4}$alkyl, -$C_{1-4}$alkyl-OH, $C_{3-6}$cycloalkyl, and 4- to 7-membered heterocycloalkyl containing 1-3 heteroatoms selected from N, S, or O;
provided that, $R_3$' and $R_4$' are not both selected from H.

**9.** The compound, or the stereoisomer, solvate, deuterated compound, or pharmaceutically acceptable salt thereof according to claim 1, **characterized in that**

each Rx is independently F;
$R_3$' and $R_4$' are independently H, difluoromethyl, trifluoromethyl, ethenyl, propenyl, ethynyl, propynyl, F or Cl;
$R_8$' is independently methyl, ethyl or propyl, and the methyl, ethyl or propyl is substituted with 1, 2, or 3 groups selected from F, Cl, OH, CN, $NH_2$, methyl, hydroxymethyl, cyclopropyl, azetidinyl or oxetanyl;
provided that, $R_3$' and $R_4$' are not both selected from H.

**10.** The compound, or the stereoisomer, solvate, deuterated compound, or pharmaceutically acceptable salt thereof according to claim 1, **characterized in that**

each Rx is independently H or F;
$R_3$' and $R_4$' are independently H, $C_{2-4}$alkenyl, $C_{2-4}$alkynyl, F or Cl;
$R_8$' is independently $C_{1-4}$alkyl, the alkyl is substituted with 1, 2, or 3 groups selected from F, Cl, OH, CN, $NH_2$, methyl, hydroxymethyl, cyclopropyl, azetidinyl or oxetanyl;
provided that, $R_3$' and $R_4$' are not both selected from H.

**11.** The compound, or the stereoisomer, solvate, deuterated compound, or pharmaceutically acceptable salt thereof according to claim 1, **characterized in that** the compound is selected from one of the following structures:

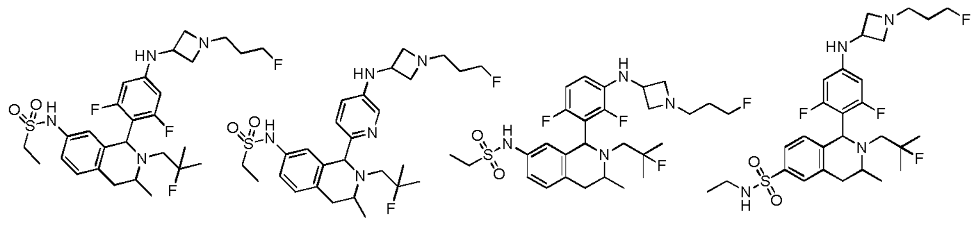

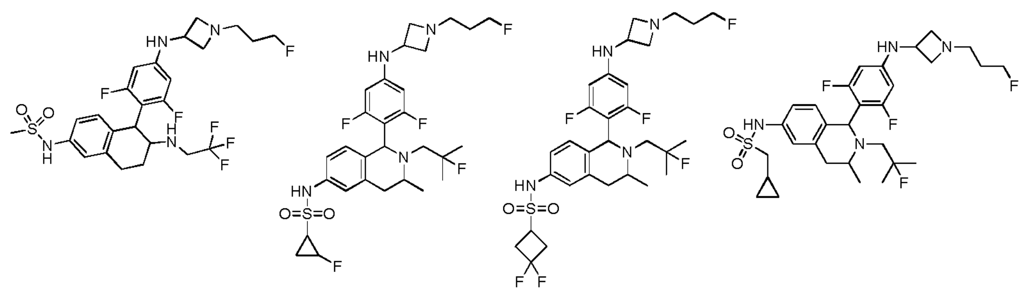

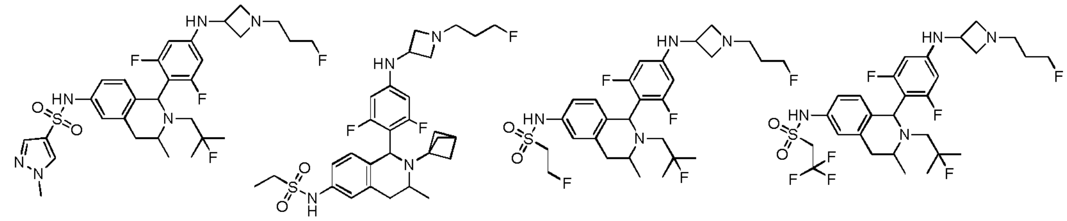

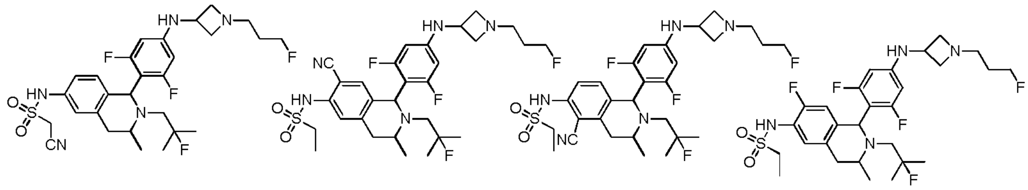

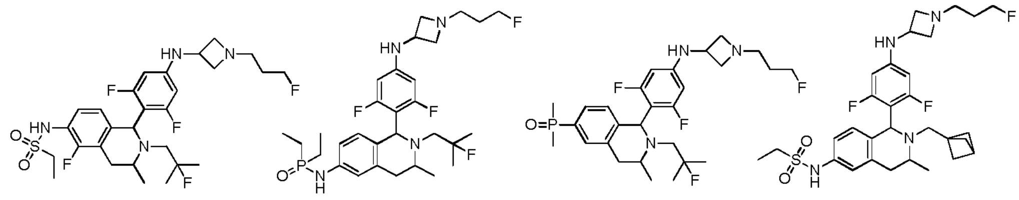

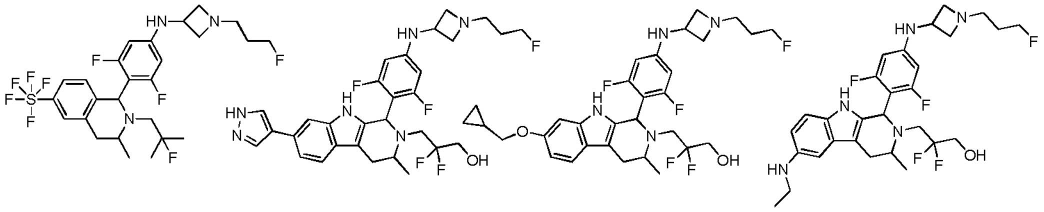

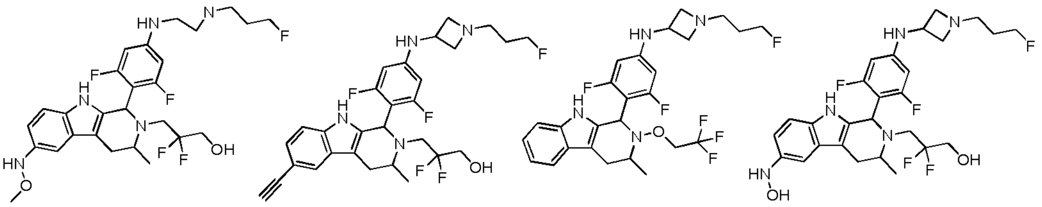

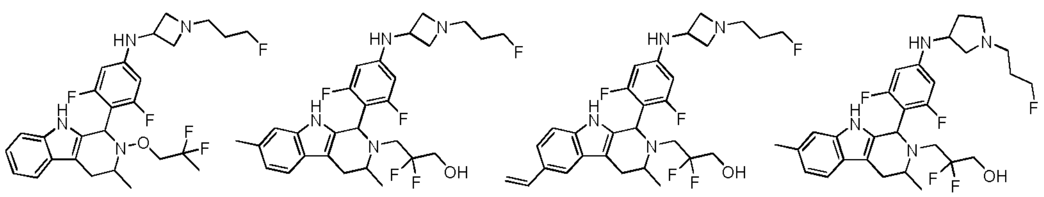

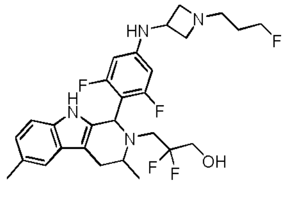

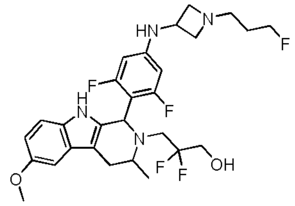

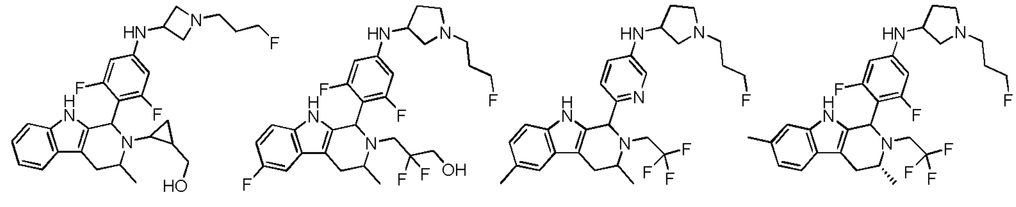

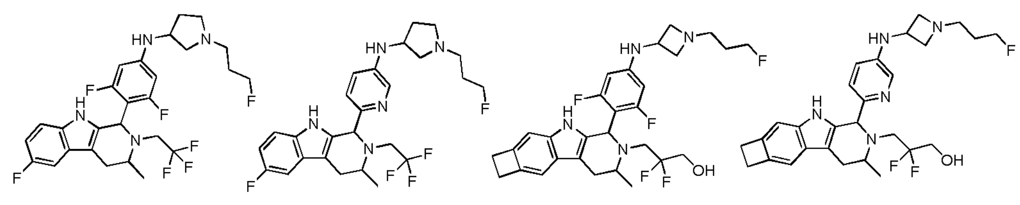

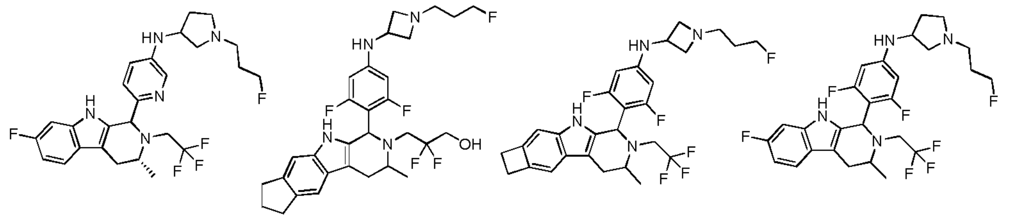

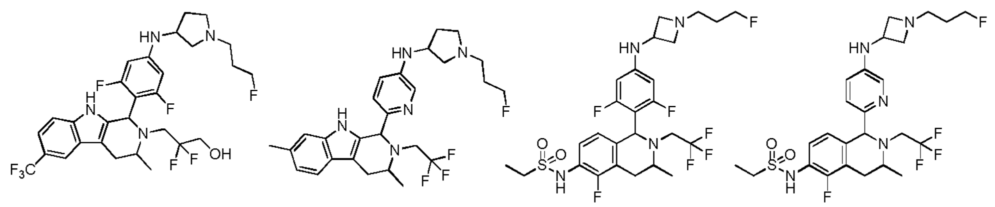

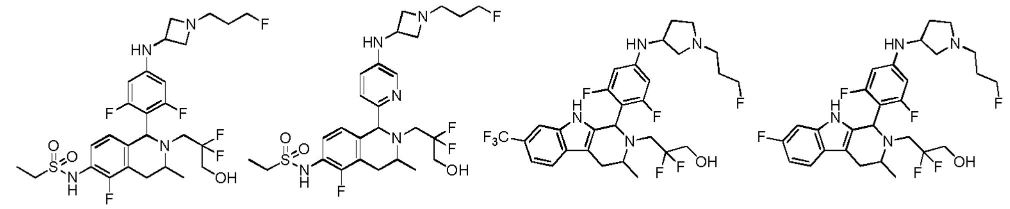

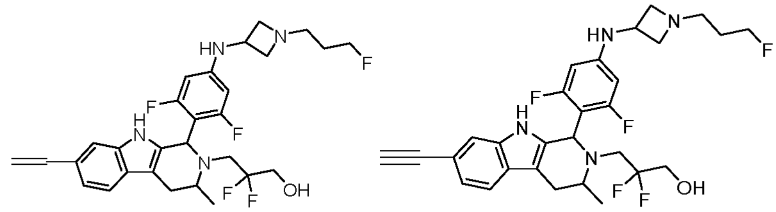

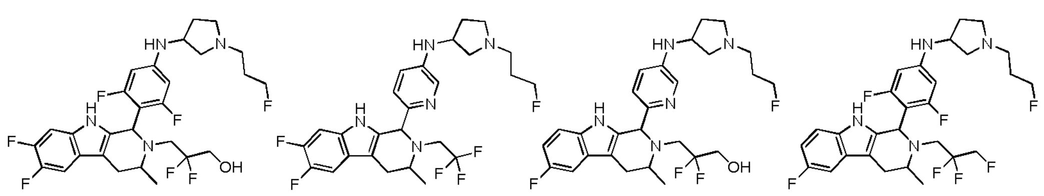

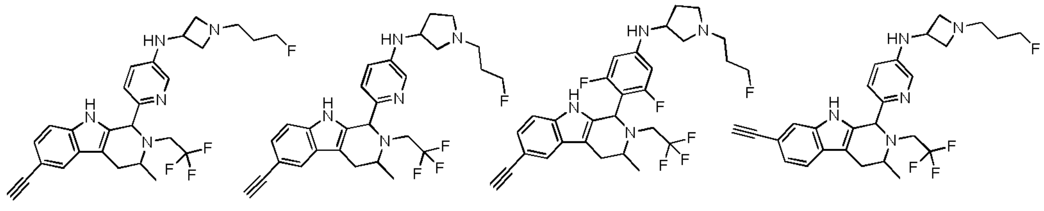

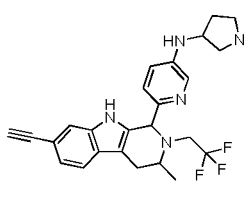
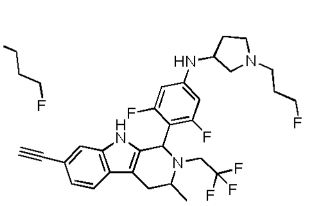

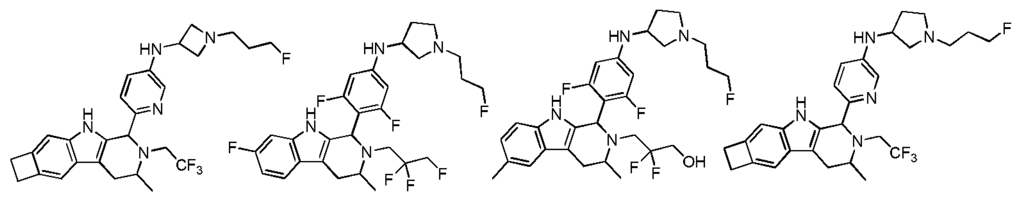

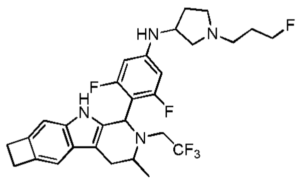
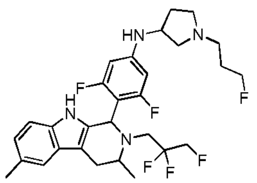

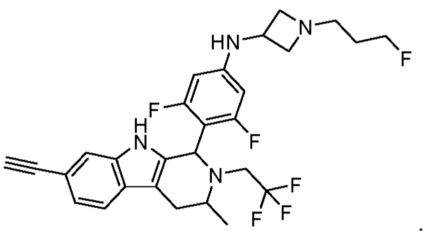

.

12. The compound, or the stereoisomer, solvate, deuterated compound, or pharmaceutically acceptable salt thereof according to claim 1, **characterized in that** the compound is selected from one of the following structures:

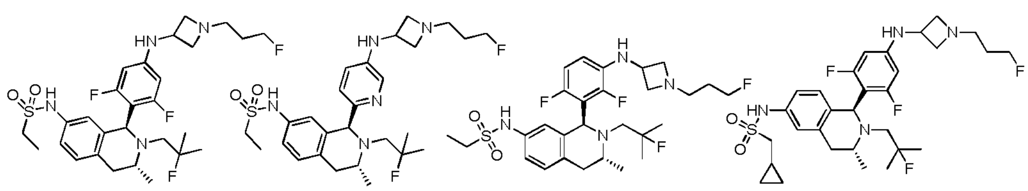

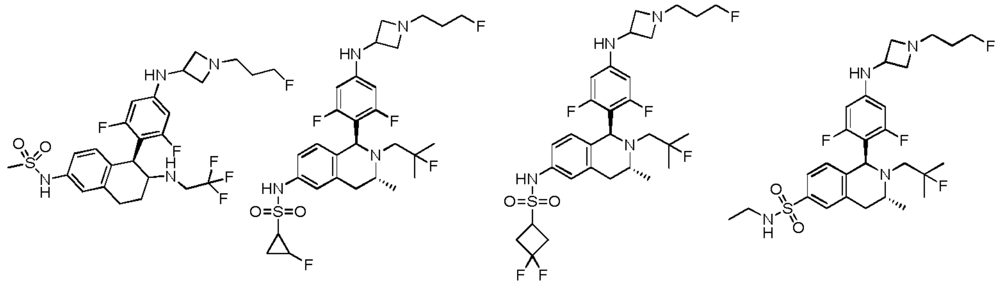

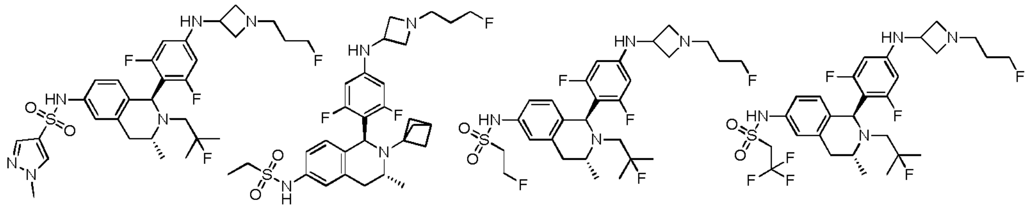

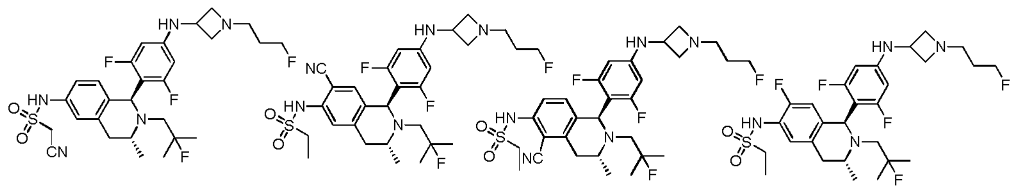

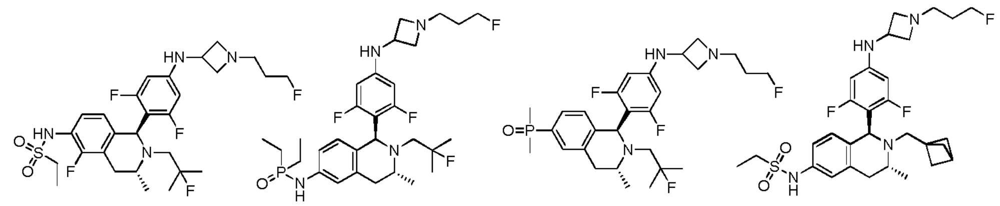

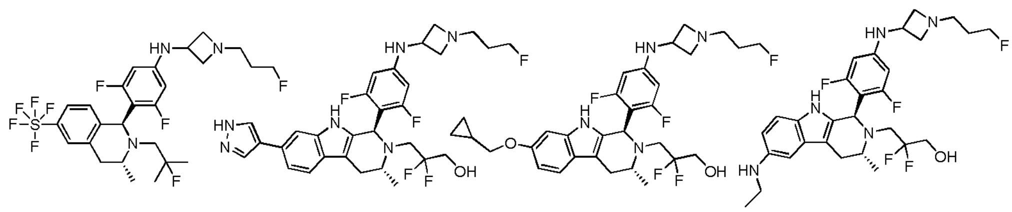

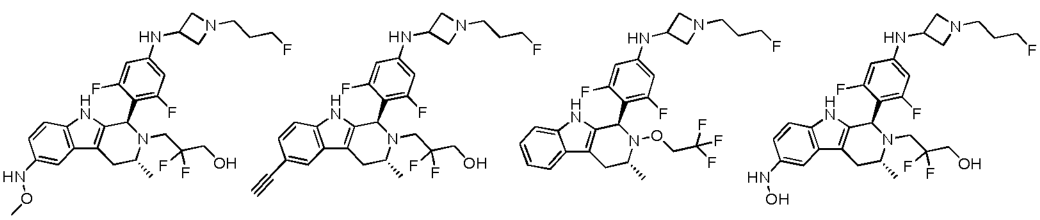

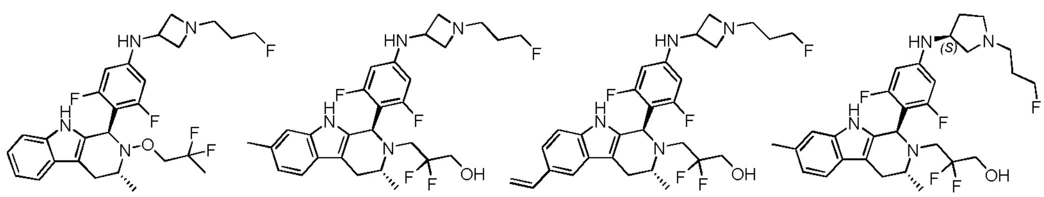

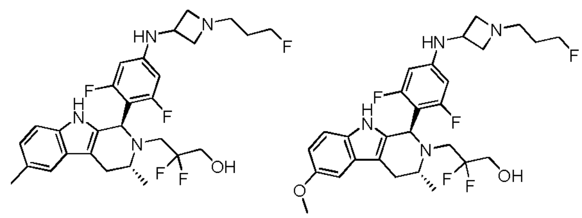

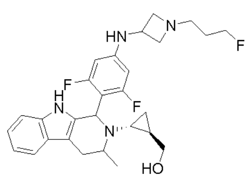
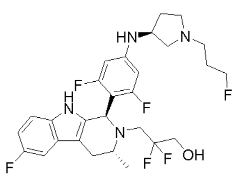
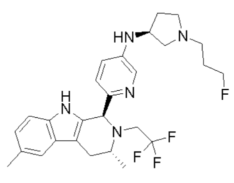
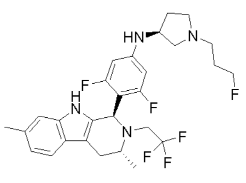
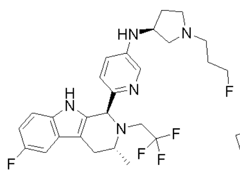
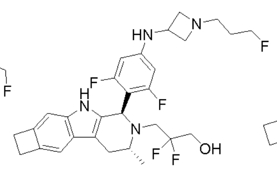
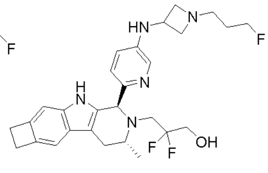
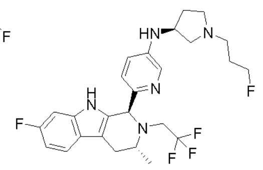
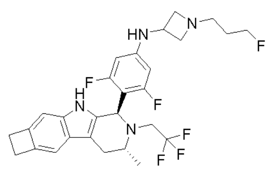
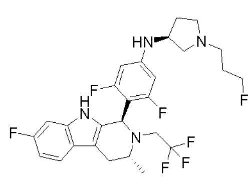
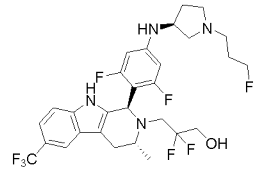
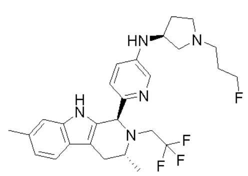
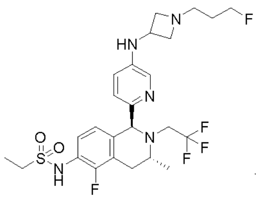
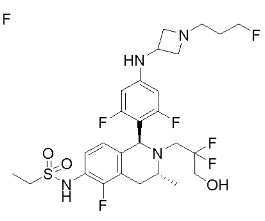
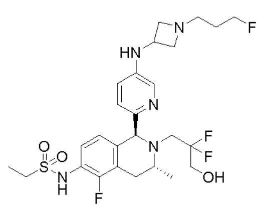
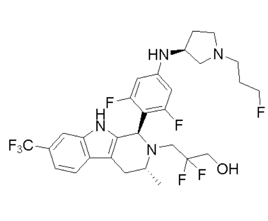
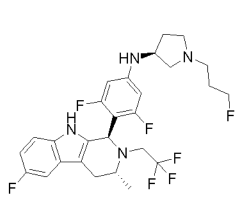
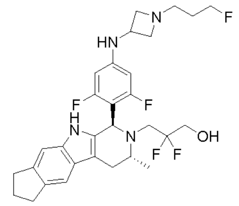
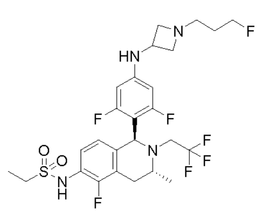
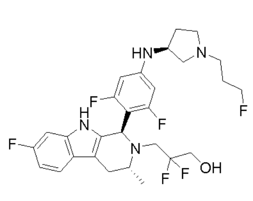
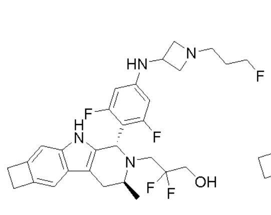
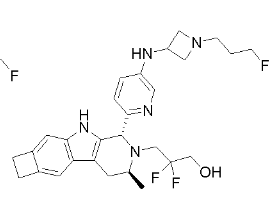
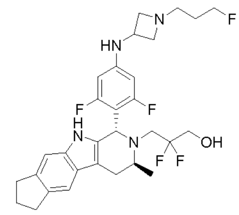
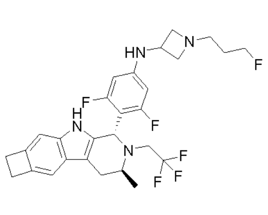
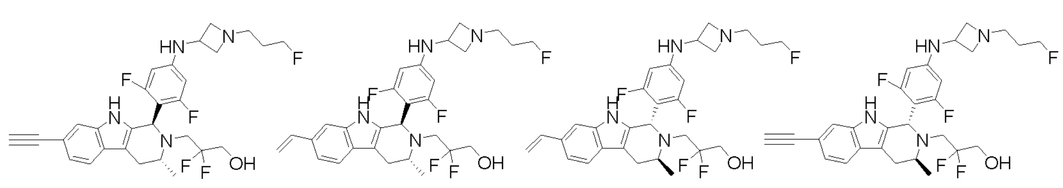

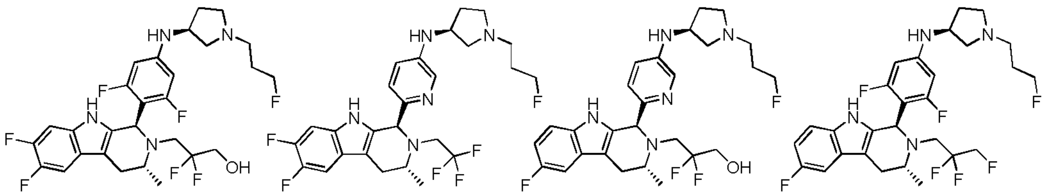

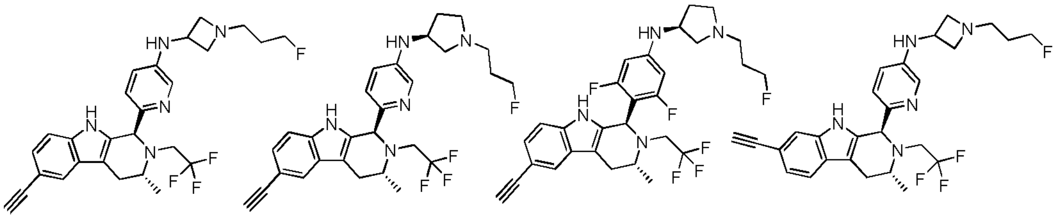

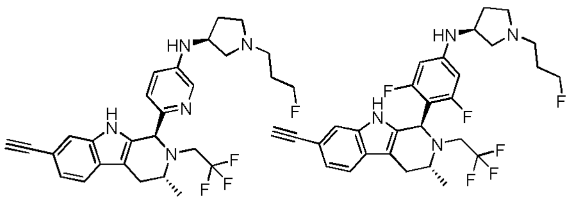

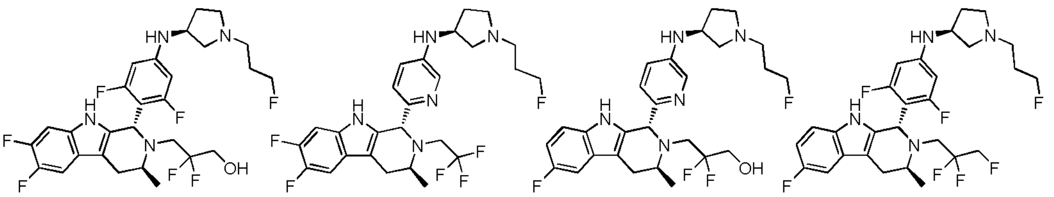

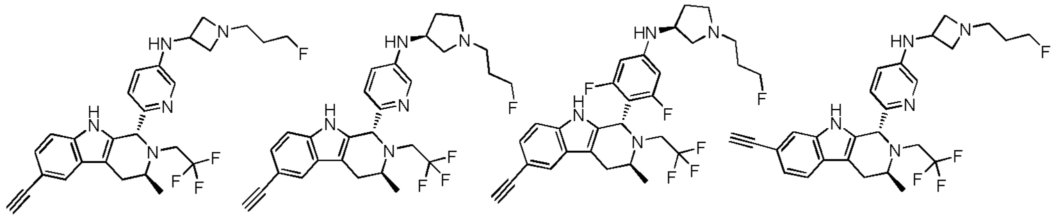

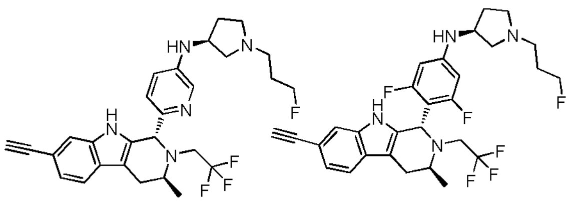

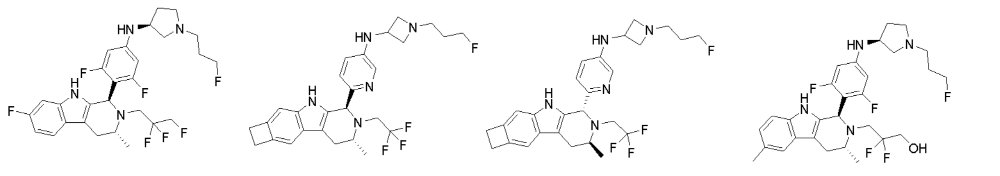

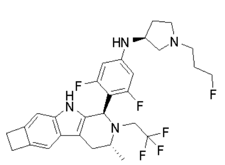
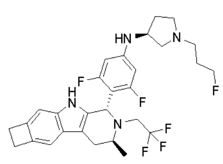
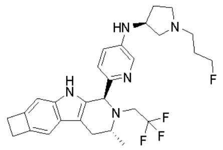
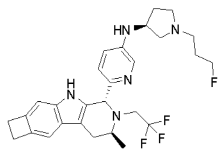
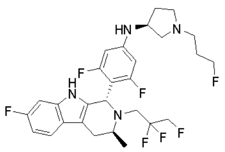
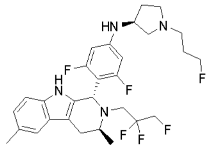
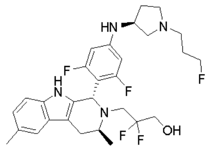
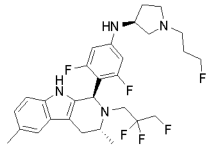
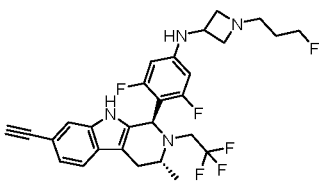
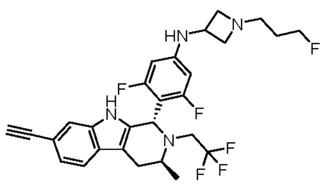
.

**13.** A pharmaceutical composition, **characterized in that** the pharmaceutical composition comprises the compound, or the stereoisomer, solvate, deuterated compound, or pharmaceutically acceptable salt thereof according to any one of claims 1-12, and a pharmaceutically acceptable adjuvant and/or carrier.

**14.** Use of the compound, or the stereoisomer, solvate, deuterated compound, or pharmaceutically acceptable salt thereof according to any one of claims 1-12, or the pharmaceutical composition according to claim 13 in the preparation of a drug for treating/preventing an ER-mediated disease.

**15.** The use according to claim 14, **characterized in that** the ER-mediated disease is selected from breast cancer, ovarian cancer, uterine cancer or cervical cancer.

**16.** A pharmaceutical composition or pharmaceutical preparation, **characterized in that** the pharmaceutical composition or pharmaceutical preparation comprises 1-600 mg of the compound, or the stereoisomer, solvate, deuterated compound, or pharmaceutically acceptable salt thereof according to any one of claims 1-12, and a pharmaceutically acceptable adjuvant and/or carrier.

**17.** A method for treating a disease in mammals, **characterized in that** the method comprises administering to a subject a therapeutically effective amount of the compound, or the stereoisomer, solvate, deuterated compound, or pharmaceutically acceptable salt thereof according to any one of claims 1-12, wherein the therapeutically effective amount is preferably 1-600 mg, and the disease is preferably breast cancer, ovarian cancer, uterine cancer or cervical cancer.

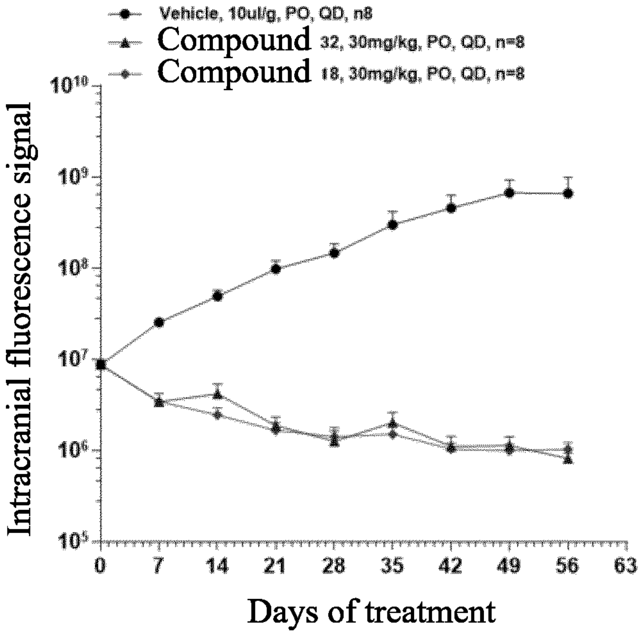
Vehicle, 10ul/g, PO, QD, n8
Compound 32, 30mg/kg, PO, QD, n=8
Compound 18, 30mg/kg, PO, QD, n=8
Intracranial fluorescence signal
Days of treatment

*FIG. 1*

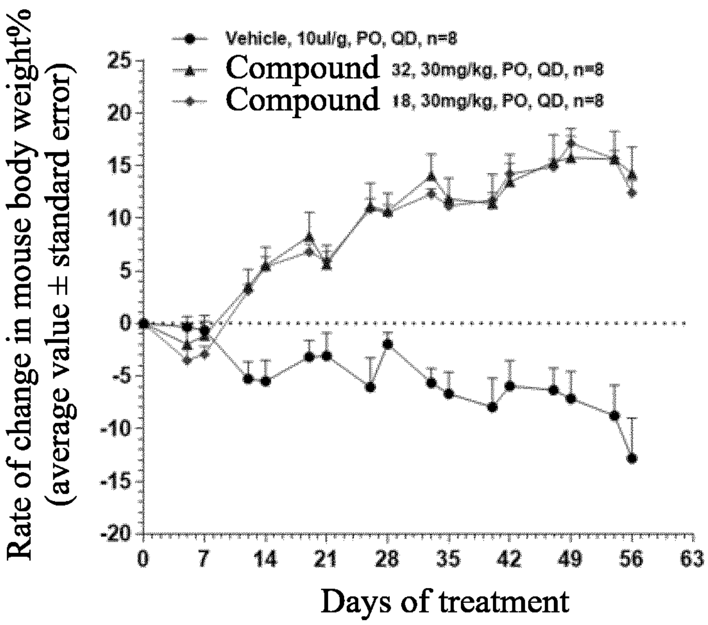
Vehicle, 10ul/g, PO, QD, n=8
Compound 32, 30mg/kg, PO, QD, n=8
Compound 18, 30mg/kg, PO, QD, n=8
Rate of change in mouse body weight%
(average value ± standard error)
Days of treatment

*FIG. 2*

**INTERNATIONAL SEARCH REPORT**

International application No.
**PCT/CN2022/105788**

**A. CLASSIFICATION OF SUBJECT MATTER**

C07D 217/14(2006.01)i; C07D 401/12(2006.01)i; C07D 401/14(2006.01)i; C07D 471/04(2006.01)i; A61K 31/435(2006.01)i; A61K 31/397(2006.01)i; A61K 31/437(2006.01)i; A61K 31/4545(2006.01)i; A61P 35/00(2006.01)i

According to International Patent Classification (IPC) or to both national classification and IPC

**B. FIELDS SEARCHED**

Minimum documentation searched (classification system followed by classification symbols)

C07D; A61K; A61P

Documentation searched other than minimum documentation to the extent that such documents are included in the fields searched

Electronic data base consulted during the international search (name of data base and, where practicable, search terms used)

CNABS, WPI, SIPOABS, CNKI, Caplus(STN), Registry(STN): 雌激素, 芳氨基, 吡咯, 吡啶, 吲哚, 氮杂环丁基, 海思科, 李瑶, 王文晶, 石宗军, 结构检索, structural search, estrogen, arylamino, pyrrole, pyridine, indole, azacyclobutyl

**C. DOCUMENTS CONSIDERED TO BE RELEVANT**

| Category* | Citation of document, with indication, where appropriate, of the relevant passages | Relevant to claim No. |
|---|---|---|
| X | CN 111825613 A (SHANGHAI XUNHE PHARMACEUTICAL TECHNOLOGY CO., LTD.) 27 October 2020 (2020-10-27)<br>claims 1, 5, and 8-10, and description, paragraph 56 | 1-5, 9-17 |
| X | CN 107108611 A (F. HOFFMANN-LA ROCHE AG) 29 August 2017 (2017-08-29)<br>description, pp. 32, 63-64, 66, 74, 81, 85, 87-89, and 91-97, and claim 1 | 1-17 |
| PX | WO 2021228210 A1 (JIANGSU SIMCERE PHARMACEUTICAL CO., LTD.) 18 November 2021 (2021-11-18)<br>claims 1 and 10-13, and description, pp. 16-32 | 1-17 |
| X | WO 2019245974 A1 (GENENTECH INC. et al.) 26 December 2019 (2019-12-26)<br>claims 34-38 and 102 | 1-17 |
| X | WO 2020037203 A2 (GENENTECH INC. et al.) 20 February 2020 (2020-02-20)<br>abstract, and claims 1, 77, and 78 | 1-17 |
| X | CN 109219604 A (F. HOFFMANN-LA ROCHE AG) 15 January 2019 (2019-01-15)<br>abstract, and claims 1-34 | 1-5, 9-17 |
| PX | US 11339162 B1 (RECURIUM IP HOLDINGS LLC.) 24 May 2022 (2022-05-24)<br>abstract, table 1, and claims 1 and 27-28 | 1-8, 13-17 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **20 September 2022** | **09 October 2022** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/105788**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **17**
because they relate to subject matter not required to be searched by this Authority, namely:

   [1] Claim 17 relates to a method for treating diseases (PCT Rule 39.1(iv)), and the following search was conducted on the basis of a pharmaceutical use of the compound.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2022/105788**

| Patent document cited in search report | Publication date (day/month/year) | Patent family member(s) | Publication date (day/month/year) |
|---|---|---|---|
| CN 111825613 A | 27 October 2020 | None | |
| CN 107108611 A | 29 August 2017 | ES 2819448 T3 | 16 April 2021 |
| | | SI 3233852 T1 | 30 October 2020 |
| | | RS 60824 B1 | 30 October 2020 |
| | | CL 2017000814 A1 | 29 December 2017 |
| | | IL 287326 A | 01 December 2021 |
| | | JP 2020073493 A | 14 May 2020 |
| | | JP 2022088416 A | 14 June 2022 |
| | | AU 2020200407 A1 | 13 February 2020 |
| | | US 2016175289 A1 | 23 June 2016 |
| | | HU E050466 T2 | 28 December 2020 |
| | | TW 202028204 A | 01 August 2020 |
| | | CA 2963736 A1 | 23 June 2016 |
| | | CL 2019000431 A1 | 12 July 2019 |
| | | DK 3233852 T3 | 14 September 2020 |
| | | AU 2015367509 A1 | 13 April 2017 |
| | | PL 3233852 T3 | 14 December 2020 |
| | | JP 2017538725 A | 28 December 2017 |
| | | US 2021236473 A1 | 05 August 2021 |
| | | LT 3233852 T | 12 October 2020 |
| | | SG 10201913913V A | 30 March 2020 |
| | | CN 112375077 A | 19 February 2021 |
| | | CR 20170250 A | 19 July 2017 |
| | | CO 2017003070 A2 | 11 July 2017 |
| | | PT 3233852 T | 10 September 2020 |
| | | US 2018235945 A1 | 23 August 2018 |
| | | IL 251370 D0 | 29 May 2017 |
| | | NZ 730448 A | 26 February 2021 |
| | | TW 201636348 A | 16 October 2016 |
| | | KR 20170097651 A | 28 August 2017 |
| | | CN 112375078 A | 19 February 2021 |
| | | HR P20201396 T1 | 27 November 2020 |
| | | PH 12017501140 A1 | 05 March 2018 |
| | | CN 112457310 A | 09 March 2021 |
| | | MA 53837 A | 10 November 2021 |
| | | EA 201791239 A1 | 31 October 2017 |
| | | EP 3760623 A1 | 06 January 2021 |
| | | SG 10202100799 P A | 30 March 2021 |
| | | WO 2016097072 A1 | 23 June 2016 |
| | | SG 11201704401 T A | 28 July 2017 |
| | | MX 2017007489 A | 18 October 2017 |
| | | HK 1243074 A1 | 06 July 2018 |
| | | PE 20171330 A1 | 13 September 2017 |
| | | BR 112017007662 A2 | 19 December 2017 |
| | | AU 2021200352 A1 | 18 March 2021 |
| | | IL 270653 B | 01 December 2021 |
| | | EP 3233852 A1 | 25 October 2017 |
| WO 2021228210 A1 | 18 November 2021 | None | |
| WO 2019245974 A1 | 26 December 2019 | US 2022041587 A1 | 10 February 2022 |
| | | US 2020002331 A1 | 02 January 2020 |

Form PCT/ISA/210 (patent family annex) (January 2015)

**INTERNATIONAL SEARCH REPORT**
**Information on patent family members**

International application No.
**PCT/CN2022/105788**

| Patent document cited in search report | Publication date (day/month/year) | Patent family member(s) | Publication date (day/month/year) |
|---|---|---|---|
| | | SG 11202012171 S A | 28 January 2021 |
| | | TW 202016098 A | 01 May 2020 |
| | | PH 12020500676 A1 | 14 June 2021 |
| | | EP 3810283 A1 | 28 April 2021 |
| | | MA 52973 A | 28 April 2021 |
| | | JP 2021191750 A | 16 December 2021 |
| | | AU 2022202967 A1 | 26 May 2022 |
| | | CO 2020014604 A2 | 10 December 2020 |
| | | AU 2019290545 A1 | 19 November 2020 |
| | | JP 6916969 B1 | 11 August 2021 |
| | | CN 112437685 A | 02 March 2021 |
| | | IL 278063 A | 30 November 2020 |
| | | CL 2020003022 A1 | 04 June 2021 |
| | | CR 20200621 A | 03 February 2021 |
| | | BR 112020024073 A2 | 17 February 2021 |
| | | KR 20210022701 A | 03 March 2021 |
| | | CA 3098203 A1 | 26 December 2019 |
| | | JP 2021191751 A | 16 December 2021 |
| | | PE 20211207 A1 | 05 July 2021 |
| WO 2020037203 A2 | 20 February 2020 | JP 2021533787 A | 09 December 2021 |
| | | US 2022044819 A1 | 10 February 2022 |
| | | IL 280707 A | 25 March 2021 |
| | | CN 113490850 A | 08 October 2021 |
| | | US 2020082944 A1 | 12 March 2020 |
| | | CA 3109090 A1 | 20 February 2020 |
| | | EP 3837548 A2 | 23 June 2021 |
| | | TW 202022124 A | 16 June 2020 |
| | | AU 2019321588 A1 | 04 March 2021 |
| | | KR 20210046031 A | 27 April 2021 |
| CN 109219604 A | 15 January 2019 | WO 2017174757 A1 | 12 October 2017 |
| | | US 2017320871 A1 | 09 November 2017 |
| | | JP 2019510799 A | 18 April 2019 |
| | | EP 3440067 A1 | 13 February 2019 |
| US 11339162 B1 | 24 May 2022 | US 2022235050 A1 | 28 July 2022 |
| | | WO 2022140744 A1 | 30 June 2022 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2021139756 A
- WO 2019245974 A
- WO 2021228210 A


### Non-patent literature cited in the description


- Synthetic Organic Chemistry. John Wiley & Sons, Inc
- **S. R. SANDLER et al.** Organic Functional Group Preparations. Academic Press, 1983
- **H. O. HOUSE.** Modern Synthetic Reactions. W. A. Benjamin, Inc, 1972
- **T. L. GILCHRIST.** Heterocyclic Chemistry. John Wiley & Sons, 1992
- **J. MARCH.** Advanced Organic Chemistry: Reactions, Mechanisms and Structure. Wiley-Interscience, 1992
- **FUHRHOP, J. ; PENZLIN G.** Organic Synthesis: Concepts, Methods, Starting Materials. John Wiley & Sons, 1994
- **HOFFMAN, R.V.** Organic Chemistry, An Intermediate Text. Oxford University Press, 1996
- **LAROCK, R. C.** Comprehensive Organic Transformations: A Guide to Functional Group Preparations. Wiley-VCH, 1999
- **MARCH, J.** Advanced Organic Chemistry: Reactions, Mechanisms, and Structure. John Wiley & Sons, 1992
- Modern Carbonyl Chemistry. Wiley-VCH, 2000
- **PATAI, S.** Patai's 1992 Guide to the Chemistry of Functional Groups. 1992
- **SOLOMONS, T. W. G.** Organic Chemistry. John Wiley & Sons, 2000
- **; STOWELL, J.C.** Intermediate Organic Chemistry. Wiley-Interscience, 1993
- Industrial Organic Chemicals: Starting Materials and Intermediates: An Ullmann's Encyclopedia. John Wiley & Sons, 1999
- Organic Reactions. John Wiley & Sons, 1942, vol. 8
- Chemistry of Functional Groups. John Wiley & Sons, vol. 73
- **P. H. STAHL ; C. G. WERMUTH.** Handbook of Pharmaceutical Salts. Verlag Helvetica Chimica Acta, 2002